(19) Europäisches Patentamt
European Patent Office
Office européen des brevets

(11) **EP 2 813 227 A1**

(12) **EUROPEAN PATENT APPLICATION**

(43) Date of publication:
**17.12.2014 Bulletin 2014/51**

(21) Application number: **14184159.3**

(22) Date of filing: **22.10.2010**

(51) Int Cl.:
***A61K 31/404*** *(2006.01)* ***A61K 31/443*** *(2006.01)*
***A61K 31/4965*** *(2006.01)* ***A61K 31/501*** *(2006.01)*
***A61K 45/06*** *(2006.01)* ***A61P 11/00*** *(2006.01)*
***A61P 19/10*** *(2006.01)* ***A61P 43/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2009 US 254180 P**

(62) Document number(s) of the earlier application(s) in accordance with Art. 76 EPC:
**10773489.9 / 2 490 687**

(71) Applicant: **Vertex Pharmaceuticals Incorporated**
**Boston, MA 02210 (US)**

(72) Inventors:
- **Van Goor, Fredrick F.**
  **San Diego, CA California 92109 (US)**
- **Burton, William Lawrence**
  **San Diego, CA California 92122 (US)**

(74) Representative: **Oates, Edward Christopher et al**
**Carpmaels & Ransford LLP**
**One Southampton Row**
**London WC1B 5HA (GB)**

Remarks:
This application was filed on 09-09-2014 as a divisional application to the application mentioned under INID code 62.

(54) **Compositions for treatment of cystic fibrosis and other chronic diseases**

(57) The present invention relates to pharmaceutical compositions comprising an inhibitor of epithelial sodium channel activity in combination with at least one compound of Formula I, Formula II, or Formula III. The invention also relates to solid forms and to pharmaceutical formulations thereof, and to methods of using such compositions in the treatment of CFTR mediated diseases, particularly cystic fibrosis using the pharmaceutical combination compositions.

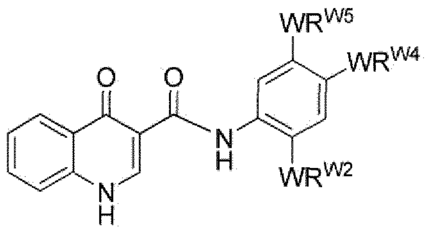

Formula I



Printed by Jouve, 75001 PARIS (FR)

**(Cont. next page)**

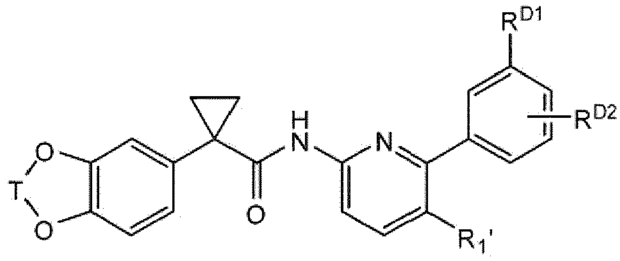

Formula II

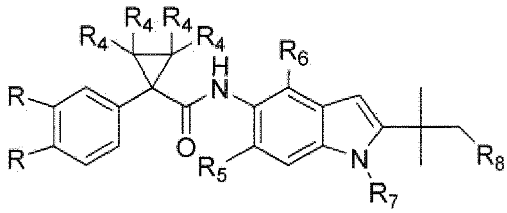

Formula III

## Description

CROSS REFERENCE TO RELATED APPLICATIONS

This application claims the benefit of U.S. Provisional Patent Application No. 61/254,180 filed on October 22, 2009. The disclosure of the above referenced application is incorporated herein by reference in its entirety.

TECHNICAL FIELD

The present invention relates to compositions for the treatment of cystic fibrosis (CF) and other chronic diseases, methods for preparing the compositions and methods for using the compositions for the treatment of CF and other chronic diseases, including chronic diseases involving regulation of fluid volumes across epithelial membranes.

BACKGROUND

Cystic fibrosis (CF) is a recessive genetic disease that affects approximately 30,000 children and adults in the United States and approximately 30,000 children and adults in Europe. Despite progress in the treatment of CF, there is no cure.

CF is caused by mutations in the cystic fibrosis transmembrane conductance regulator (CFTR) gene that encodes an epithelial chloride ion channel responsible for aiding in the regulation of salt and water absorption and secretion in various tissues. Small molecule drugs, known as potentiators that increase the probability of CFTR channel opening, represent one potential therapeutic strategy to treat CF. Potentiators of this type are disclosed in WO 2006/002421, which is herein incorporated by reference in its entirety. Another potential therapeutic strategy involves small molecule drugs known as CF correctors that increase the number and function of CFTR channels. Correctors of this type are disclosed in WO 2005/075435, which are herein incorporated by reference in their entirety.

Specifically, CFTR is a cAMP/ATP-mediated anion channel that is expressed in a variety of cells types, including absorptive and secretory epithelia cells, where it regulates anion flux across the membrane, as well as the activity of other ion channels and proteins. In epithelia cells, normal functioning of CFTR is critical for the maintenance of electrolyte transport throughout the body, including respiratory and digestive tissue. CFTR is composed of approximately 1480 amino acids that encode a protein made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking.

The gene encoding CFTR has been identified and sequenced (See Gregory, R. J. et al. (1990) Nature 347:382-386; Rich, D. P. et al. (1990) Nature 347:358-362), (Riordan, J. R. et al. (1989) Science 245:1066-1073). A defect in this gene causes mutations in CFTR resulting in cystic fibrosis ("CF"), the most common fatal genetic disease in humans. Cystic fibrosis affects approximately one in every 2,500 infants in the United States. Within the general United States population, up to 10 million people carry a single copy of the defective gene without apparent ill effects. In contrast, individuals with two copies of the CF associated gene suffer from the debilitating and fatal effects of CF, including chronic lung disease.

In patients with CF, mutations in CFTR endogenously expressed in respiratory epithelia leads to reduced apical anion secretion causing an imbalance in ion and fluid transport. The resulting decrease in anion transport contributes to enhanced mucus accumulation in the lung and the accompanying microbial infections that ultimately cause death in CF patients. In addition to respiratory disease, CF patients typically suffer from gastrointestinal problems and pancreatic insufficiency that, if left untreated, results in death. In addition, the majority of males with cystic fibrosis are infertile and fertility is decreased among females with cystic fibrosis. In contrast to the severe effects of two copies of the CF associated gene, individuals with a single copy of the CF associated gene exhibit increased resistance to cholera and to dehydration resulting from diarrhea - perhaps explaining the relatively high frequency of the CF gene within the population.

Sequence analysis of the CFTR gene of CF chromosomes has revealed a variety of disease causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S et al. (1990) Proc. Natl. Acad. Sci. USA 87:8447-8451). To date, greater than 1000 disease causing mutations in the CF gene have been identified (http://www.genet.sickkids.on.ca/cftr/app). The most prevalent mutation is a deletion of phenylalanine at position 508 of the CFTR amino acid sequence, and is commonly referred to as ΔF508-CFTR. This mutation occurs in approximately 70% of the cases of cystic fibrosis and is associated with a severe disease.

The deletion of residue 508 in ΔF508-CFTR prevents the nascent protein from folding correctly. This results in the inability of the mutant protein to exit the ER, and traffic to the plasma membrane. As a result, the number of channels present in the membrane is far less than observed in cells expressing wild-type CFTR. In addition to impaired trafficking, the mutation results in defective channel gating. Together, the reduced number of channels in the membrane and the

defective gating lead to reduced anion transport across epithelia leading to defective ion and fluid transport. (Quinton, P. M. (1990), FASEB J. 4: 2709-2727). Studies have shown, however, that the reduced numbers of ΔF508-CFTR in the membrane are functional, albeit less than wild-type CFTR. (Dalemans et al. (1991), Nature Lond. 354: 526-528; Denning et al., supra; Pasyk and Foskett (1995), J. Cell. Biochem. 270: 12347-50). In addition to ΔF508-CFTR, other disease causing mutations in CFTR that result in defective trafficking, synthesis, and/or channel gating could be up- or down-regulated to alter anion secretion and modify disease progression and/or severity.

Although CFTR transports a variety of molecules in addition to anions, it is clear that this role (the transport of anions) represents one element in an important mechanism of transporting ions and water across the epithelium. The other elements include the epithelial Na+ channel ("ENaC"), Na+/2Cl-/K+ co-transporter, Na+-K+-ATPase pump and the basolateral membrane K+ channels, that are responsible for the uptake of chloride into the cell.

These elements work together to achieve directional transport across the epithelium via their selective expression and localization within the cell. Chloride absorption takes place by the coordinated activity of ENaC and CFTR present on the apical membrane and the Na+-K+-ATPase pump and Cl- ion channels expressed on the basolateral surface of the cell. Secondary active transport of chloride from the luminal side leads to the accumulation of intracellular chloride, which can then passively leave the cell via Cl- channels, resulting in a vectorial transport. Arrangement of Na+/2Cl-/K+ co-transporter, Na+-K+-ATPase pump and the basolateral membrane K+ channels on the basolateral surface and CFTR on the luminal side coordinate the secretion of chloride via CFTR on the luminal side. Because water is probably never actively transported itself, its flow across epithelia depends on tiny transepithelial osmotic gradients generated by the bulk flow of sodium and chloride.

As discussed above, it is believed that the deletion of residue 508 in ΔF508-CFTR prevents the nascent protein from folding correctly, resulting in the inability of this mutant protein to exit the ER, and traffic to the plasma membrane. As a result, insufficient amounts of the mature protein are present at the plasma membrane and chloride transport within epithelial tissues is significantly reduced. In fact, this cellular phenomenon of defective ER processing of CF Modulators by the ER machinery has been shown to be the underlying basis not only for CF disease, but for a wide range of other isolated and inherited diseases.

There is a need for methods of treating CF Modulator/ENaC mediated diseases using such combination compositions comprising at least one modulator of CF Modulator activity and at least one inhibitor of ENaC activity.

There is a need for methods for modulating an CF Modulator activity and/or ENaC activity in an ex vivo cell membrane of a mammal.

There is a need for modulators of CFTR activity that can be used to modulate the activity of CFTR in the cell membrane of a mammal.

There is a need for methods for treating CFTR-mediated diseases using such modulators of CFTR activity.

There is a need for methods for treating ENaC-mediated diseases using such modulators, in particular, inhibitors of ENaC activity.

There is a need for methods of modulating CFTR activity in an ex vivo cell membrane of a mammal.

SUMMARY

These and other needs are met by the present invention which is directed to a pharmaceutical composition comprising a Compound of formula I , II, or III and an ENaC inhibitor, wherein:

A the Compound of Formula I is

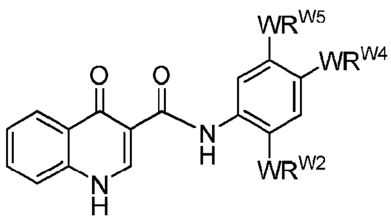

Formula I

or pharmaceutically acceptable salts thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is

independently and optionally substituted with up to three substituents selected from -OR', $-CF_3$, $-OCF_3$, SR', S(O)R', $SO_2R'$, $-SCF_3$, halo, CN, -COOR', -COR', $-O(CH_2)_2N(R')_2$, - $O(CH_2)N(R')_2$, $-CON(R')_2$, $-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$; $WR^{W5}$ is selected from hydrogen, $-OCF_3$ $-CF_3$, -OH, $-OCH_3$, $-NH_2$, -CN, $-CHF_2$, -NHR', $-N(R')_2$, -NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and

Each R' is independently selected from an optionally substituted group selected from a $C_{1-8}$ aliphatic group, a 3-8-mcmbcrcd saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;

provided that:

i) $WR^{W2}$ and $WR^{W4}$ are not both -Cl; and

$WR^{W2}$, $WR^{W4}$ and $WR^{W5}$ are not $-OCH_2CH_2Ph$, $-OCH_2CH_2$(2-trifluoromethyl-phenyl), $-OCH_2CH_2$-(6,7-dimethoxy-1,2,3,4-tetrahydroisoquinolin-2-yl), or substituted 1*H*-pyrazol-3-yl;

B the Compound of Formula II is

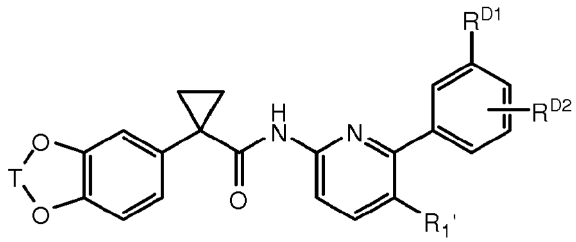

Formula II

or pharmaceutically acceptable salts thereof, wherein:

T is $-CH_2-$, $-CH_2CH_2-$, $-CF_2-$, $-C(CH_3)_2-$, or -C(O)-;

$R_1$' is H, $C_{1-6}$ aliphatic, halo, $CF_3$, $CHF_2$, $O(C_{1-6}$ aliphatic); and

$R^{D1}$ or $R^{D2}$ is $Z^DR_9$

wherein:

$Z^D$ is a bond, CONH, $SO_2NH$, $SO_2N(C_{1-6}$ alkyl), $CH_2NHSO_2$, $CH_2N(CH_3)SO_2$, $CH_2NHCO$, COO, $SO_2$, or CO; and $R_9$ is H, $C_{1-6}$ aliphatic, or aryl;

C. the Compound of Formula III is

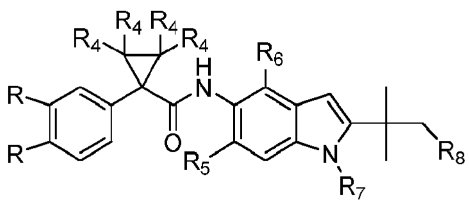

Formula III

or pharmaceutically acceptable salts thereof, wherein:

Each R is independently H, OH, $OCH_3$ or two R taken together form $-OCH_2O-$ or $-OCF_2O-$;

Each $R_4$ is independently H or alkyl;

$R_5$ is H or F;

$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

In one aspect, the pharmaceutical composition comprises an ENaC inhibitor and at least one Compound of Formula I, Formula II and Formula III.

In another aspect, pharmaceutical composition comprises at least one Compound of Formula I, Formula II and Formula III and an ENaC inhibitor selected from amiloride, benzamil, dimethyl-amiloride camostat (a trypsin-like protease inhibitor), QAU145, 552-02, GS-9411, INO-4995, and aerolytic.

In another aspect, the ENaC inhibitor is a compound of Formula IV

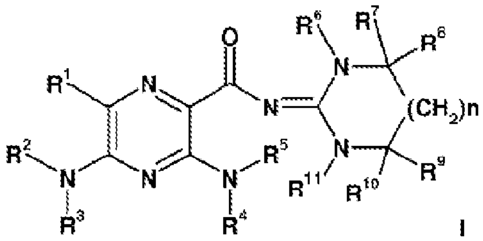

I

Formula IV

or pharmaceutically acceptable salts thereof.

In another aspect, the pharmaceutical composition comprises an inhibitor of ENaC activity and Compound 1.

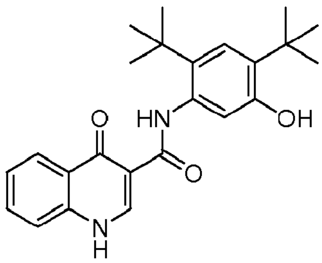

Compound 1

In some embodiments of this aspect, the ENaC inhibitor is selected from amiloride, benzamil, and dimethyl-amiloride.

In another aspect, the pharmaceutical composition comprises an inhibitor of ENaC activity and Compound 2.

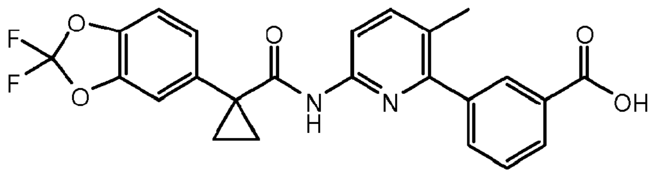

Compound 2

In some embodiments of this aspect, the ENaC inhibitor is selected from amiloride, benzamil, and dimethyl-amiloride.

In another aspect, the pharmaceutical composition comprises an inhibitor of ENaC activity and Compound 3.

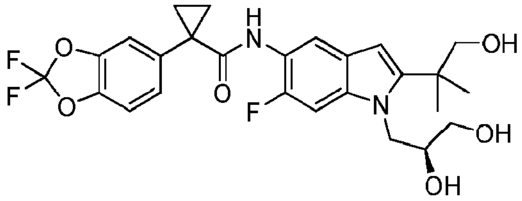

Compound 3

In some embodiments of this aspect, the ENaC inhibitor is selected from amiloride, benzamil, and dimethylamiloride.

In another aspect, the invention is directed to a composition, preferably a pharmaceutical composition comprising at least one component from: Column A of Table I, or Column B of Table I, or Column C of Table I, in combination with at least one component from Column D of Table I. These components are described in the corresponding sections of the following pages as embodiments of the invention. For convenience, Table I recites the section number and corresponding heading title of the embodiments of the compounds.

Table I.

| Column A Embodiments | | Column B Embodiments | | Column C Embodiments | | Column D Embodiments | |
|---|---|---|---|---|---|---|---|
| Section | | Section | Heading | Section | Heading | Section | Heading |
| II.A.1. | Compound of Formula I | H.B.1. | Compound of Formula II | II.C.1. | Compound of Formula III | II.D.1. | ENaC Compounds |
| | | | | | | | |
| II.A.2. | Compound 1 | II.B.2. | Compound 2 | II.C.2. | Compound 3 | II.D.2 | ENaC Compounds of Formula IV |

For example, the embodiments of the compounds of Formula I are disclosed in section II.A.1. of this specification.

For another example, the embodiments of the compounds of Formula II are disclosed in section II.B.1. of this specification.

For another example, the embodiments of the compounds of Formula III are disclosed in section II.C.1. of this specification.

For another example, the embodiments of the ENaC compounds are illustratively described in section II.D.1. of this specification.

In any embodiment of this aspect, any embodiment or group of embodiments included in Column A can comprise the first component, and any embodiment or group of embodiments of Column D can comprise the second component.

In one embodiment based on Table I, the Column A component is Compound 1, the Column B Component is Compound 2, and the Column C Component is Compound 3.

In another aspect, the invention is directed to method of treating a CFTR mediated disease in a human comprising administering to the human, an effective amount of a pharmaceutical composition comprising an ENaC inhibitor component of Column D and an CF modulator component selected from at least one of Columns A, B, or C according to Table I.

It has now been found that pharmaceutically acceptable compositions of the present invention, include the combination of a modulator of CF Modulator activity or cAMP/ATP-mediated anion channel, Cystic Fibrosis Transmembrane Conductance Regulator ("CFTR") and a modulator of ENaC activity.

In another aspect, the combination compounds are provided to treat a variety of diseases and disorders mediated by CF Modulators and/or ENaC. The combination composition can include a modulator of an CF Modulator corresponding to one or more of Formulas I, II and III and an inhibitor of ENaC, for example, compounds of Formula IV. While the methods for treating said variety of diseases and disorders mediated by CF Modulators and/or ENaC comprises a combination of a an ENaC inhibitor component of Column D and an CF modulator component selected from at least one of Columns A, B, or C according to Table I, the individual active agents can be administered in a single dose unit, as separate dosage units, administered simultaneously, or may be administered sequentially, optionally within a specified time period of the other's administration.

In another aspect, the invention is directed to method of treating a CFTR mediated disease in a human comprising administering to the human an effective amount of a ENaC inhibitor component of Column D and at least one of Compounds 1, 2, or 3 according to Table I.

In another aspect, the invention is directed to method of treating a CFTR mediated disease in a human comprising administering to the human an effective amount of a ENaC inhibitor component of Column D and at least one solid form component of Columns A, B, or C according to Table I.

Methods are provided to treat CF and other chronic diseases mediated by dysregulation or dysfunctional CF Modulator activity or cAMP/ATP-mediated anion channel and epithelial sodium channel (ENaC) activity using the pharmaceutical compositions described herein.

In another aspect, the invention is directed to a kit for the treatment of a CFTR mediated disease in a human, the kit comprising an ENaC inhibitor component of Column D and an CF modulator component selected from at least one of Columns A, B, or C according to Table I, and optionally, instructions for preparing and administering a pharmaceutical composition for the treatment of said disease.

Various components listed in Table I have been disclosed and can be found in US Pat. No. 7,741,321, US Pat. No. 7,645,789, US Pat. No. 7,495,103, US Pat. No. 7,776,905, US Pat. No. 7,659,268, U.S. Patent Application publications US 2007/0244159A1, US 2008/0113985A1, US 2008/0019915A1, US 2008/0306062A1, US 2006/0074075A1 and US 2009/0131492A1 the contents of all of the above published patent applications and patents are incorporated herein by reference in their entireties.

DETAILED DESCRIPTION

The invention relates to a combination, particularly a pharmaceutical combination, such as a combined preparation or pharmaceutical composition, respectively, which comprises 1) a modulator of ATP-Binding Cassette ("ABC") transporters or fragments thereof, including Cystic Fibrosis Transmembrane Conductance Regulator ("CFTR")and 2) an epithelial sodium channel inhibitor ("ENaC"), for simultaneous, separate or sequential use, especially in the prevention, delay of progression or treatment of conditions mediated by CFTR and ENaC, conditions directly caused by CF Modulator and/or CFTR activities and alleviation of symptoms of diseases not directly caused by CF Modulator and/or CFTR anion channel activities. Examples of diseases whose symptoms may be affected by CF Modulator e.g. CFTR and/or ENaC activity include, but are not limited to, Cystic fibrosis, Hereditary emphysema, Hereditary hemochromatosis, Coagulation-Fibrinolysis deficiencies, such as Protein C deficiency, Type 1 hereditary angioedema, Lipid processing deficiencies, such as Familial hypercholesterolemia, Type 1 chylomicronemia, Abetalipoproteinemia, Lysosomal storage diseases, such as I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Hereditary emphysema, Congenital hyperthyroidism, Osteogenesis imperfecta, Hereditary hypofibrinogenemia, ACT deficiency, Diabetes insipidus (DI), Neurophysiol DI, Nephrogenic DI, Charcot-Marie Tooth syndrome, Perlizaeus-Merzbacher disease, neurodegenerative diseases such as Alzheimer’s disease, Parkinson’s disease, Amyotrophic lateral sclerosis, Progressive supranuclear palsy, Pick’s disease, several polyglutamine neurological disorders such as Huntington, Spinocerebullar ataxia type I, Spinal and bulbar muscular atrophy, Dentatorubal pallidoluysian, and Myotonic dystrophy, as well as Spongiform encephalopathies, such as Hereditary Creutzfeldt-Jakob disease, Fabry disease, Straussler-Scheinker syndrome, COPD, dry-eye disease, and Sjogren’s disease. In some embodiments, the present invention also provides for the use of such combination of active agents, for the preparation of a pharmaceutical composition, for the prevention, delay, of progression or treatment of such conditions, diseases and disorders; and for providing kits comprising such combinations for the treatment of a mammal.

DEFINITIONS

As used herein, the following definitions shall apply unless otherwise indicated.

The term "ABC-transporter" as used herein means an ABC-transporter protein or a fragment thereof comprising at least one binding domain, wherein said protein or fragment thereof is present *in vivo* or *in vitro.* The term "binding domain" as used herein means a domain on the ABC-transporter that can bind to a modulator. See, e.g., Hwang, T. C. et al., J. Gen. Physiol. (1998): 111(3), 477-90.

The term "CFTR" as used herein means cystic fibrosis transmembrane conductance regulator or a mutation thereof capable of regulator activity, including, but not limited to, ΔF508 CFTR, R117H CFTR, and G551D CFTR (see, e.g., http://www.genet.sickkids.on.ca/cftr/, for CFTR mutations).

As used herein, the term "active pharmaceutical ingredient" or "API" refers to a biologically active compound. Exemplary APIs include the CF potentiators N-[2,4-bis(1,1-dimethylethyl)-5-hydroxyphenyl]-1,4-dihydro-4-oxoquinoline-3-carboxamide (Compound 1) and N-(4-(7-azabicyclo[2.2.1]heptan-7-yl)-2-(trifluoromethyl)phenyl)-4-oxo-5-(trifluoromethyl)-1,4-dihydroquinoline-3-carboxamide (Compound 2). Exemplary APIs also include the CF correctors 3-(6-(1-(2,2-Difluorobenzo[d][1,3]dioxol-5-yl)cyclopropanecarboxamido)-3-methylpyridin-2-yl)benzoic acid (Compound 3) and (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (Compound 3).

The term "modulating" as used herein means increasing or decreasing by a measurable amount.

The term "normal CFTR" or "normal CFTR function" as used herein means wild-type like CFTR without any impairment due to environmental factors such as smoking, pollution, or anything that produces inflammation in the lungs.

The term "reduced CFTR" or "reduced CFTR function" as used herein means less than normal CFTR or less than normal CFTR function.

As used herein, the term "amorphous" refers to a solid material having no long range order in the position of its molecules. Amorphous solids are generally supercooled liquids in which the molecules are arranged in a random manner so that there is no well-defined arrangement, e.g., molecular packing, and no long range order. Amorphous solids are generally isotropic, i.e. exhibit similar properties in all directions and do not have definite melting points. For example, an amorphous material is a solid material having no sharp characteristic crystalline peak(s) in its X-ray power diffraction (XRPD) pattern (i.e., is not crystalline as determined by XRPD). Instead, one or several broad peaks (e.g., halos) appear in its XRPD pattern. Broad peaks are characteristic of an amorphous solid. See, US 2004/0006237 for a comparison of XRPDs of an amorphous material and crystalline material.

As used herein, the term "substantially amorphous" refers to a solid material having little or no long range order in the position of its molecules. For example, substantially amorphous materials have less than about 15% crystallinity (e.g., less than about 10% crystallinity or less than about 5% crystallinity). It is also noted that the term 'substantially amorphous' includes the descriptor, 'amorphous', which refers to materials having no (0%) crystallinity.

As used herein, the term "dispersion" refers to a disperse system in which one substance, the dispersed phase, is distributed, in discrete units, throughout a second substance (the continuous phase or vehicle). The size of the dispersed phase can vary considerably (e.g. single molecules, colloidal particles of nanometer dimension, to multiple microns in size). In general, the dispersed phases can be solids, liquids, or gases. In the case of a solid dispersion, the dispersed and continuous phases are both solids. In pharmaceutical applications, a solid dispersion can include: an amorphous drug in an amorphous polymer; an amorphous drug in crystalline polymer; a crystalline drug in an amorphous polymer; or a crystalline drug in crystalline polymer. In this invention, a solid dispersion can include an amorphous drug in an amorphous polymer or an amorphous drug in crystalline polymer. In some embodiments, a solid dispersion includes the polymer constituting the dispersed phase, and the drug constitutes the continuous phase. Or, a solid dispersion includes the drug constituting the dispersed phase, and the polymer constitutes the continuous phase.

As used herein, the term "solid dispersion" generally refers to a solid dispersion of two or more components, usually one or more drugs (e.g., one drug (e.g., Compound 1)) and polymer, but possibly containing other components such as surfactants or other pharmaceutical excipients, where the drug(s) (e.g., Compound 1) is substantially amorphous (e.g., having about 15% or less (e.g., about 10% or less, or about 5% or less)) of crystalline drug (e.g., N-[2,4-bis(1,1-dimethylethyl)-5-hydroxyphenyl]-1,4-dihydro-4-oxoquinoline-3-carboxamide) or amorphous (i.e., having no crystalline drug), and the physical stability and/or dissolution and/or solubility of the substantially amorphous or amorphous drug is enhanced by the other components. Solid dispersions typically include a compound dispersed in an appropriate carrier medium, such as a solid state carrier. For example, a carrier comprises a polymer (e.g., a water-soluble polymer or a partially water-soluble polymer) and can include optional excipients such as functional excipients (e.g., one or more surfactants) or nonfunctional excipients (e.g., one or more fillers). Another exemplary solid dispersion is a co-precipitate or a co-melt of N-[2,4-bis(1,1-dimethylethyl)-5-hydroxyphenyl]-1,4-dihydro-4-oxoquinoline-3-carboxamide with at least one polymer.

A "Co-precipitate" is a product after dissolving a drug and a polymer in a solvent or solvent mixture followed by the removal of the solvent or solvent mixture. Sometimes the polymer can be suspended in the solvent or solvent mixture. The solvent or solvent mixture includes organic solvents and supercritical fluids. A "co-melt" is a product after heating a drug and a polymer to melt, optionally in the presence of a solvent or solvent mixture, followed by mixing, removal of at least a portion of the solvent if applicable, and cooling to room temperature at a selected rate.

As used herein "crystalline" refers to compounds or compositions where the structural units are arranged in fixed geometric patterns or lattices, so that crystalline solids have rigid long range order. The structural units that constitute the crystal structure can be atoms, molecules, or ions. Crystalline solids show definite melting points.

As used herein the phrase "substantially crystalline", means a solid material that is arranged in fixed geometric patterns or lattices that have rigid long range order. For example, substantially crystalline materials have more than about 85% crystallinity (e.g., more than about 90% crystallinity or more than about 95% crystallinity). It is also noted that the term 'substantially crystalline' includes the descriptor 'crystalline', which is defined in the previous paragraph.

As used herein, "crystallinity" refers to the degree of structural order in a solid. For example, Compound 1, which is substantially amorphous, has less than about 15% crystallinity, or its solid state structure is less than about 15% crystalline. In another example, Compound 1, which is amorphous, has zero (0%) crystallinity.

As used herein, an "excipient" is an inactive ingredient in a pharmaceutical composition. Examples of excipients include fillers or diluents, surfactants, binders, glidants, lubricants, disintegrants, and the like.

As used herein, a "disintegrant" is an excipient that hydrates a pharmaceutical composition and aids in tablet

dispersion. Examples of disintegrants include sodium croscarmellose and/or sodium starch glycolate.

As used herein, a "diluent" or "filler" is an excipient that adds bulkiness to a pharmaceutical composition. Examples of fillers include lactose, sorbitol, celluloses, calcium phosphates, starches, sugars (e.g., mannitol, sucrose, or the like) or any combination thereof.

As used herein, a "surfactant" is an excipient that imparts pharmaceutical compositions with enhanced solubility and/or wetability. Examples of surfactants include sodium lauryl sulfate (SLS), sodium stearyl fumarate (SSF), polyoxyethylene 20 sorbitan mono-oleate (e.g., Tween™), or any combination thereof.

As used herein, a "binder" is an excipient that imparts a pharmaceutical composition with enhanced cohesion or tensile strength (e.g., hardness). Examples of binders include dibasic calcium phosphate, sucrose, corn (maize) starch, microcrystalline cellulose, and modified cellulose (e.g., hydroxymethyl cellulose).

As used herein, a "glidant" is an excipient that imparts a pharmaceutical compositions with enhanced flow properties. Examples of glidants include colloidal silica and/or talc.

As used herein, a "colorant" is an excipient that imparts a pharmaceutical composition with a desired color. Examples of colorants include commercially available pigments such as FD&C Blue # 1 Aluminum Lake, FD&C Blue #2, other FD&C Blue colors, titanium dioxide, iron oxide, and/or combinations thereof.

As used herein, a "lubricant" is an excipient that is added to pharmaceutical compositions that are pressed into tablets. The lubricant aids in compaction of granules into tablets and ejection of a tablet of a pharmaceutical composition from a die press. Examples of lubricants include magnesium stearate, stearic acid (stearin), hydrogenated oil, sodium stearyl fumarate, or any combination thereof.

As used herein, "friability" refers to the property of a tablet to remain intact and withhold its form despite an external force of pressure. Friability can be quantified using the mathematical expression presented in equation 1:

$$\% friabiliy = 100 \times \frac{(W_0 - W_f)}{W_0} \qquad (1)$$

wherein $W_0$ is the original weight of the tablet and $W_f$ is the final weight of the tablet after it is put through the friabilator.

Friability is measured using a standard USP testing apparatus that tumbles experimental tablets for 100 revolutions. Some tablets of the present invention have a friability of less than about 1 % (e.g., less than about 0.75%, less than about 0.50%, or less than about 0.30%)

As used herein, "mean particle diameter" is the average particle diameter as measured using techniques such as laser light scattering, image analysis, or sieve analysis.

As used herein, "bulk density" is the mass of particles of material divided by the total volume the particles occupy. The total volume includes particle volume, inter-particle void volume and internal pore volume. Bulk density is not an intrinsic property of a material; it can change depending on how the material is processed.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle" "cycloaliphatic" or "cycloalkyl"), that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. In some embodiments, "cycloaliphatic" (or "carbocycle" or "cycloalkyl") refers to a monocyclic $C_3$-$C_8$ hydrocarbon or bicyclic or tricyclic $C_8$-$C_{14}$ hydrocarbon that is completely saturated or that contains one or more units of unsaturation, but which is not aromatic, that has a single point of attachment to the rest of the molecule wherein any individual ring in said bicyclic ring system has 3-7 members. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl. Suitable cycloaliphatic groups include cycloalkyl, bicyclic cycloalkyl (e.g., decalin), bridged bicycloalkyl such as norbornyl or [2.2.2]bicyclo-octyl, or bridged tricyclic such as adamantyl.

The term "heteroaliphatic", as used herein, means aliphatic groups wherein one or two carbon atoms are independently replaced by one or more of oxygen, sulfur, nitrogen, phosphorus, or silicon. Heteroaliphatic groups may be substituted or unsubstituted, branched or unbranched, cyclic or acyclic, and include "heterocycle", "heterocyclyl", "heterocycloaliphatic", or "heterocyclic" groups.

The term "heterocycle", "heterocyclyl", "heterocycloaliphatic", or "heterocyclic" as used herein means non-aromatic, monocyclic, bicyclic, or tricyclic ring systems in which one or more ring members is an independently selected

heteroatom. In some embodiments, the "heterocycle", "heterocyclyl", "heterocycloaliphatic", or "heterocyclic" group has three to fourteen ring members in which one or more ring members is a heteroatom independently selected from oxygen, sulfur, nitrogen, or phosphorus, and each ring in the system contains 3 to 7 ring members.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or $NR^+$ (as in N-substituted pyrrolidinyl)).

The term "unsaturated", as used herein, means that a moiety has one or more units of unsaturation.

The term "aryl" used alone or as part of a larger moiety as in "aralkyl", "aralkoxy", or "aryloxyalkyl", refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring". The term "aryl" also refers to heteroaryl ring systems as defined hereinbelow.

An aliphatic or heteroaliphatic group, or a non-aromatic heterocyclic ring may contain one or more substituents. Suitable substituents on the saturated carbon of an aliphatic or heteroaliphatic group, or of a non-aromatic heterocyclic ring are selected from those listed above for the unsaturated carbon of an aryl or heteroaryl group and additionally include the following: =O, =S, =NNHR*, $=NN(R^*)_2$, =NNHC(O)R*, $=NNHCO_2$(alkyl), $=NNHSO_2$(alkyl), or =NR*, where each R* is independently selected from hydrogen or an optionally substituted $C_{1-6}$ aliphatic. Optional substituents on the aliphatic group of R* are selected from $NH_2$, NH($C_{1-4}$ aliphatic), N($C_{1-4}$ aliphatic)$_2$, halo, $C_{1-4}$ aliphatic, OH, O($C_{1-4}$ aliphatic), $NO_2$, CN, $CO_2H$, $CO_2$($C_{1-4}$ aliphatic), O(halo $C_{1-4}$ aliphatic), or halo($C_{1-4}$ aliphatic), wherein each of the foregoing $C_{1-4}$aliphatic groups of R* is unsubstituted.

Optional substituents on the nitrogen of a non-aromatic heterocyclic ring are selected from $-R^+$, $-N(R^+)_2$, $-C(O)R^+$, $-CO_2R^+$, $-C(O)C(O)R^+$, $-C(O)CH_2C(O)R^+$, $-SO_2R^+$, $-SO_2N(R^+)_2$, $-C(=S)N(R^+)_2$, $-C(=NH)-N(R^+)_2$, or $-NR^+SO_2R^+$; wherein $R^+$ is hydrogen, an optionally substituted $C_{1-6}$ aliphatic, optionally substituted phenyl, optionally substituted -O(Ph), optionally substituted $-CH_2$(Ph), optionally substituted $-(CH_2)_{1-2}$(Ph); optionally substituted -CH=CH(Ph); or an unsubstituted 5-6 membered heteroaryl or heterocyclic ring having one to four heteroatoms independently selected from oxygen, nitrogen, or sulfur, or, notwithstanding the definition above, two independent occurrences of $R^+$, on the same substituent or different substituents, taken together with the atom(s) to which each $R^+$ group is bound, form a 3-8-membered cycloalkyl, heterocyclyl, aryl, or heteroaryl ring having 0-3 heteroatoms independently selected from nitrogen, oxygen, or sulfur. Optional substituents on the aliphatic group or the phenyl ring of $R^+$ are selected from $NH_2$, NH($C_{1-4}$ aliphatic), N($C_{1-4}$ aliphatic)$_2$, halo, $C_{1-4}$ aliphatic, OH, O($C_{1-4}$ aliphatic), $NO_2$, CN, $CO_2H$, $CO_2$($C_{1-4}$ aliphatic), O(halo $C_{1-4}$ aliphatic), or halo($C_{1-4}$ aliphatic), wherein each of the foregoing $C_{1-4}$aliphatic groups of $R^+$ is unsubstituted.

As detailed above, in some embodiments, two independent occurrences of R' (or any other variable similarly defined herein), are taken together with the atom(s) to which each variable is bound to form a 3-8-membered cycloalkyl, heterocyclyl, aryl, or heteroaryl ring having 0-3 heteroatoms independently selected from nitrogen, oxygen, or sulfur. Exemplary rings that are formed when two independent occurrences of R (or any other variable similarly defined herein) are taken together with the atom(s) to which each variable is bound include, but are not limited to the following: a) two independent occurrences of R (or any other variable similarly defined herein) that are bound to the same atom and are taken together with that atom to form a ring, for example, N(R')$_2$, where both occurrences of R' are taken together with the nitrogen atom to form a piperidin-1-yl, piperazin-1-yl, or morpholin-4-yl group; and b) two independent occurrences of R (or any other variable similarly defined herein) that are bound to different atoms and are taken together with both of those atoms to form a ring, for example where a phenyl group is substituted with two occurrences of OR'

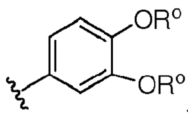
,

these two occurrences of R° are taken together with the oxygen atoms to which they are bound to form a fused 6-membered oxygen containing ring:

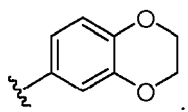
.

It will be appreciated that a variety of other rings can be formed when two independent occurrences of R (or any other variable similarly defined herein) are taken together with the atom(s) to which each variable is bound and that the

examples detailed above are not intended to be limiting.

A substituent bond in, e.g., a bicyclic ring system, as shown below, means that the substituent can be attached to any substitutable ring atom on either ring of the bicyclic ring system:

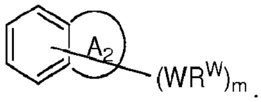

The term "protecting group" (PG) as used herein, represents those groups intended to protect a functional group, such as, for example, an alcohol, amine, carboxyl, carbonyl, etc., against undesirable reactions during synthetic procedures. Commonly used protecting groups are disclosed in Greene and Wuts, Protective Groups in Organic Synthesis, 3rd Edition (John Wiley & Sons, New York, 1999), which is incorporated herein by reference. Examples of nitrogen protecting groups include acyl, aroyl, or carbamyl groups such as formyl, acetyl, propionyl, pivaloyl, *t*-butylacetyl, 2-chloroacetyl, 2-bromoacetyl, trifluoroacetyl, trichloroacetyl, phthalyl, o-nitrophenoxyacetyl, $\alpha$-chlorobutyryl, benzoyl, 4-chlorobenzoyl, 4-bromobenzoyl, 4-nitrobenzoyl and chiral auxiliaries such as protected or unprotected D, L or D, L-amino acids such as alanine, leucine, phenylalanine and the like; sulfonyl groups such as benzenesulfonyl, p-toluenesulfonyl and the like; carbamate groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, $\alpha,\alpha$-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl and the like, arylalkyl groups such as benzyl, triphenylmethyl, benzyloxymethyl and the like and silyl groups such as trimethylsilyl and the like. Preferred *N*-protecting groups are *tert*-butyloxycarbonyl (Boc).

Examples of useful protecting groups for acids are substituted alkyl esters such as 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, pivaloyloxymethyl, phenylacetoxymethyl, triisopropropylsysilylmethyl, cyanomethyl, acetol, phenacyl, substituted phenacyl esters, 2,2,2-trichloroethyl, 2-haloethyl, $\omega$-chloroalkyl, 2-(trimethylsilyl)ethyl, 2-methylthioethyl, t-butyl, 3-methyl-3-pentyl, dicyclopropylmethyl, cyclopentyl, cyclohexyl, allyl, methallyl, cynnamyl, phenyl, silyl esters, benzyl and substituted benzyl esters, 2,6-dialkylphenyl esters such as pentafluorophenyl, 2,6-dialkylpyhenyl. Preferred protecting groups for acids are methyl or ethyl esters.

Methods of adding (a process generally referred to as "protection") and removing (process generally referred to as "deprotection") such amine and acid protecting groups are well-known in the art and available, for example in P.J.Kocienski, Protecting Groups, Thieme, 1994, which is hereby incorporated by reference in its entirety and in Greene and Wuts, Protective Groups in Organic Synthesis, 3rd Edition (John Wiley & Sons, New York, 1999).

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention.

Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a $^{13}$C- or $^{14}$C-enriched carbon are within the scope of this invention. Such compounds are useful, for example, as analytical tools or probes in biological assays.

Examples of suitable solvents are, but not limited to, water, methanol, dichloromethane (DCM), acetonitrile, dimethylformamide (DMF), ethyl acetate (EtOAc), isopropyl alcohol (IPA), isopropyl acetate (IPAc), tetrahydrofuran (THF), methyl ethyl ketone (MEK), *t*-butanol and N-methyl pyrrolidone (NMP).

PHARMACEUTICAL COMPOSITIONS

In one aspect, the invention is directed to a pharmaceutical composition comprising an ENaC inhibitor component of Column D and at least one component from Columns A, B, or C. In some embodiments, the pharmaceutical composition includes a combination of an ENaC inhibitor component from Column D and a compound of Formula I. In

another embodiment, the pharmaceutical composition includes a combination of an ENaC inhibitor component from Column D and a compound of Formula II. In another embodiment, the pharmaceutical composition includes a combination of an ENaC inhibitor component from Column D and a compound of Formula III.

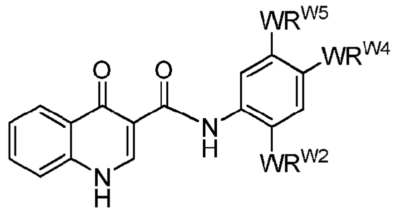

Formula I

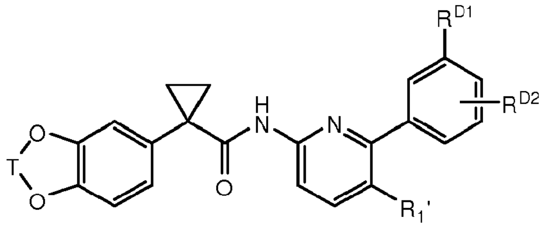

Formula II

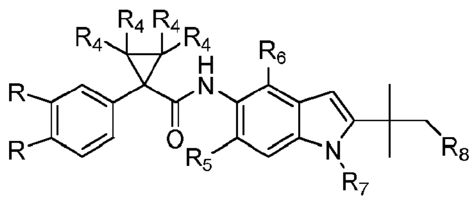

Formula III

In another embodiment, the pharmaceutical composition includes a combination of an ENaC inhibitor component from Column D and Compound I. In another embodiment, the pharmaceutical composition includes a combination of an ENaC inhibitor component from Column D and Compound 11. In another embodiment, the pharmaceutical composition includes a combination of an ENaC inhibitor component from Column D and Compound III. In another embodiment, the pharmaceutical composition includes a combination of an ENaC inhibitor component from Column D and a Compound 1 solid form. In another embodiment, the pharmaceutical composition includes a combination of an ENaC inhibitor component from Column D and a Compound 2 solid form. In another embodiment, the pharmaceutical composition includes a combination of an ENaC inhibitor component from Column D and a Compound 3 solid form. In another embodiment, the pharmaceutical composition includes a combination of an ENaC inhibitor component from Column D and a Compound 1 formulation. In another embodiment, the pharmaceutical composition includes a combination of an ENaC inhibitor component from Column D and a Compound 2 formulation. In another embodiment, the pharmaceutical composition includes a combination of an ENaC inhibitor component from Column D and a Compound 3 formulation. In another embodiment, the pharmaceutical composition includes a combination of an ENaC inhibitor of Column D, at least one component of Columns A, B, or C and at least one additional therapeutic agent.

In another embodiment the at least one additional therapeutic agent includes a CFTR modulator other than the components from Columns A, B and/or C, i.e., an agent that has the effect of modulating CFTR activity.

II.A.1. COMPOUNDS OF FORMULA I

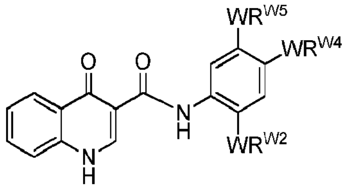

Formula I

The modulators of ABC transporter activity in Column A are fully described and exemplified in U.S. Patent 7,495,103 and US Application Publication US 2010/0184739 which are commonly assigned to the Assignee of the present invention. All of the compounds recited in the above patents are useful in the present invention and are hereby incorporated into the present disclosure in their entirety.

In one embodiment, in the compound of Formula I of the composition or pharmaceutically acceptable salts thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is independently and optionally substituted with up to three substituents selected from -OR', $-CF_3$, $-OCF_3$, SR', S(O)R', $SO_2R'$, $-SCF_3$, halo, CN, -COOR', -COR', $-O(CH_2)_2N(R')_2$, - $O(CH_2)N(R')_2$, $-CON(R')_2$, $-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$; $WR^{W5}$ is selected from hydrogen,
$-OCF_3$, $-CF_3$, -OH, $-OCH_3$, $-NH_2$, -CN, $-CHF_2$, -NHR', $-N(R')_2$, -NHC(O)R', -NHC(O)OR',$-NHSO_2R'$, $-CH_2OH$, $-CH_2N(R')_2$, -C(O)OR', $-SO_2NHR'$, $-SO_2N(R')_2$, or $-CH_2NHC(O)OR'$; and
Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that:

i) $WR^{W2}$ and $WR^{W4}$ are not both -Cl; and
$WR^{W2}$, $WR^{W4}$ and $WR^{W5}$ are not $-OCH_2CH_2Ph$, $-OCH_2CH_2$(2-trifluoromethyl-phenyl), $-OCH_2CH_2$-(6,7-dimethoxy-1,2,3,4-tetrahydroisoquinolin-2-yl), or substituted 1*H*-pyrazol-3-yl.

COMPOUND OF FORMULA I EMBODIMENTS

In one embodiment of the compound of Formula I, or a pharmaceutical salt thereof, wherein each of $WR^{W2}$ and $WR^{W4}$ is independently selected from CN, $CF_3$, halo, $C_{2-6}$ straight or branched alkyl, $C_{3-12}$ membered cycloaliphatic, or phenyl, wherein said $WR^{W2}$ and $WR^{W4}$ is independently and optionally substituted with up to three substituents selected from -OR', $-CF_3$, $-OCF_3$, $-SCF_3$, halo,-COOR', -COR', $-O(CH_2)_2N(R')_2$, $-O(CH_2)N(R')_2$, $-CON(R')_2$, $-(CH_2)_2OR'$, $-(CH_2)OR'$, optionally substituted phenyl, $-N(R')_2$, -NC(O)OR', -NC(O)R', $-(CH_2)_2N(R')_2$, or $-(CH_2)N(R')_2$; and $WR^{W5}$ is selected from hydrogen, $-OCF_3$, $-CF_3$, -OH, $-OCH_3$, $-NH_2$, -CN, -NHR', $-N(R')_2$, -NHC(O)R',-NHC(O)OR', $-NHSO_2R'$, $-CH_2OH$, -C(O)OR', $-SO_2NHR'$, or $-CH_2NHC(O)O-R'$).

Alternatively, each of $WR^{W2}$ and $WR^{W4}$ is independently selected from -CN, $-CF_3$, $C_{2-6}$ straight or branched alkyl, $C_{3-12}$ membered cycloaliphatic, or phenyl, wherein each of said $WR^{W2}$ and $WR^{W4}$ is independently and optionally substituted with up to three substituents selected from -OR', $-CF_3$, $-OCF_3$,$-SCF_3$, halo, -COOR', -COR', $-O(CH_2)_2N(R')_2$, $-O(CH_2)N(R')_2$, $-CON(R')_2$, $-(CH_2)_2OR'$, $-(CH_2)OR'$, optionally substituted phenyl, $-N(R')_2$, -NC(O)OR', -NC(O)R', $-(CH_2)_2N(R')_2$, or $-(CH_2)N(R')_2$; and $WR^{W5}$ is selected from -OH, -CN, -NHR', $-N(R')_2$, -NHC(O)R', -NHC(O)OR', $-NHSO_2R'$, $-CH_2OH$, - C(O)OR', $-SO_2NHR'$, or $-CH_2NHC(O)O-(R')$.

In a further embodiment, $WR^{W2}$ is a phenyl ring optionally substituted with up to three substituents selected from -OR', $-CF_3$, $-OCF_3$, -SR', -S(O)R', $-SO_2R'$, $-SCF_3$, halo, -CN, -COOR', - COR', $-O(CH_2)_2N(R')_2$, $-O(CH_2)N(R')_2$, $-CON(R')_2$, $-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$; $WR^{W4}$ is $C_{2-6}$ straight or branched alkyl; and $WR^{W5}$ is -OH. In another embodiment, each of $WR^{W2}$ and $WR^{W4}$ is independently $-CF_3$, -CN, or a $C_{2-6}$ straight or branched alkyl.

In another embodiment, each of $WR^{W2}$ and $WR^{W4}$ is $C_{2-6}$ straight or branched alkyl optionally substituted with up to three substituents independently selected from -OR', $-CF_3$, $-OCF_3$, -SR', -S(O)R',$-SO_2R'$, $-SCF_3$, halo, -CN, -COOR', -COR', $-O(CH_2)_2N(R')_2$, $-O(CH_2)N(R')_2$, $-CON(R')_2$, $-(CH_2)_2OR'$,$-(CH_2)OR'$, $-CH_2CN$, optionally substituted phenyl or phenoxy, $-N(R')_2$, -NR'C(O)OR', -NR'C(O)R',$-(CH_2)_2N(R')_2$, or $-(CH_2)N(R')_2$.

In another embodiment, each of $WR^{W2}$ and $WR^{W4}$ is independently selected from optionally substituted n-propyl, isopropyl, n-butyl, sec-butyl, t-butyl, 1,1-dimethyl-2-hydroxyethyl, 1,1-dimethyl-2-(ethoxycarbonyl)-ethyl, 1,1-dimethyl-3-(t-butoxycarbonyl-amino) propyl, or n-pentyl.

In another embodiment, $WR^{W5}$ is selected from -CN, -NHR', $-N(R')_2$, $-CH_2N(R')_2$,-NHC(O)R', -NHC(O)OR', -OH, C(O)OR', or $-SO_2NHR'$.

In another embodiment, $WR^{W5}$ is selected from -CN, -NH($C_{1-6}$ alkyl), -N($C_{1-6}$ alkyl)$_2$,-NHC(O)($C_{1-6}$ alkyl), $-CH_2NHC(O)O$($C_{1-6}$ alkyl), -NHC(O)O($C_{1-6}$ alkyl), -OH, -O($C_{1-6}$ alkyl), -C(O)O($C_{1-6}$ alkyl), $-CH_2O$($C_{1-6}$ alkyl), or $-SO_2NH_2$.

In another embodiment, $WR^{W5}$ is selected from -OH, $-CH_2OH$, -NIIC(O)OMe, -NIIC(O)OEt, -CN, $-CII_2NIIC(O)O$(t-butyl), -C(O)OMe, or $-SO_2NII_2$.

In another embodiment:

$WR^{W2}$ is $C_{2-6}$ straight or branched alkyl;
$WR^{W4}$ is $C_{2-6}$ straight or branched alkyl or monocyclic or bicyclic aliphatic; and
$WR^{W5}$ is selected from -CN, -NH($C_{1-6}$ alkyl), -N($C_{1-6}$ alkyl)$_2$, -NHC(O)($C_{1-6}$ alkyl), - NHC(O)O($C_{1-6}$ alkyl), $-CH_2C(O)O$($C_{1-6}$ alkyl), -OH, -O($C_{1-6}$ alkyl), -C(O)O($C_{1-6}$ alkyl), or $-SO_2NH_2$.

In another embodiment:

$WR^{W2}$ is $C_{2-6}$ alkyl, $-CF_3$, -CN, or phenyl optionally substituted with up to 3 substituents selected from $C_{1-4}$ alkyl, -O($C_{1-4}$ alkyl), or halo;
$WR^{W4}$ is $-CF_3$, $C_{2-6}$ alkyl, or $C_{6-10}$ cycloaliphatic; and
$WR^{W5}$ is -OH, -NH($C_{1-6}$ alkyl), or -N($C_{1-6}$ alkyl)$_2$.

In another embodiment, $WR^{W2}$ is *tert*-butyl.

In another embodiment, $WR^{W4}$ is *tert*-butyl.

In another embodiment, $WR^{W5}$ is -OH.

II.A.2. COMPOUND 1

In another embodiment, the compound of Formula I is Compound 1.

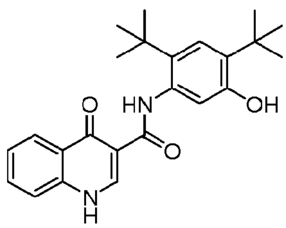

Compound 1

Compound 1 is known by the name N-[2,4-bis(1,1-dimethylethyl)-5-hydroxyphenyl]-1,4-dihydro-4-oxoquinoline-3-carboxamide and by the name N-(5-hydroxy-2,4-di-*tert*-butyl-phenyl)-4-oxo-1H-quinoline-3-carboxamide.

SYNTHESIS OF THE COMPOUNDS OF FORMULA I

Compounds of Formula I

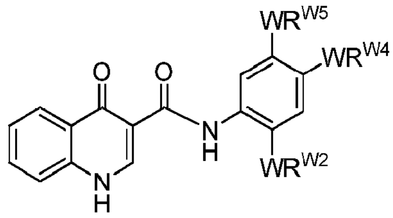

are readily prepared by combining an acid moiety

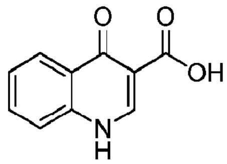

with an amine moiety

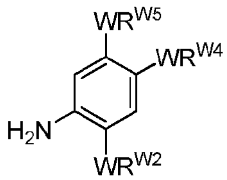

as described herein, wherein $WR^{W2}$, $WR^{W4}$, and $WR^{W5}$ are as defined previously.

a. SYNTHESIS OF THE ACID MOIETY OF COMPOUNDS OF FORMULA I

The acid precursor of compounds of Formula I, dihydroquinoline carboxylic acid, can be synthesized according to Scheme 1-1, by conjugate addition of $EtOCII=C(COOEt)_2$ to aniline, followed by thermal rearrangement and hydrolysis.

Scheme 1-1: General Synthesis of Compound of Formula I Acid Moiety.

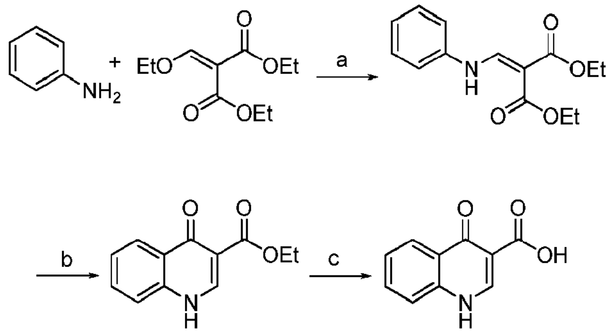


a) 140-150 °C; b) PPA, $POCl_3$, 70 °C or diphenyl ether, 220 °C; c) i) 2N NaOH ii) 2N HCl

b. SYNTHESIS OF THE AMINE MOIETY OF COMPOUNDS OF FORMULA I

Amine precursors of compounds of Formula I are prepared as depicted in Scheme 1-2, wherein $WR^{W2}$, $WR^{W4}$, and $WR^{W5}$ are as defined previously. Thus, ortho alkylation of the para-substituted benzene in step (a) provides a tri-substituted intermediate. Optional protection when $WR^{W5}$ is OH (step (b) and nitration (step c) provides the trisubstituted nitrated intermediate. Optional deprotection (step d) and hydrogenation (step e) provides the desired amine moiety.

Scheme 1-2: General Synthesis of the Amine Moiety.

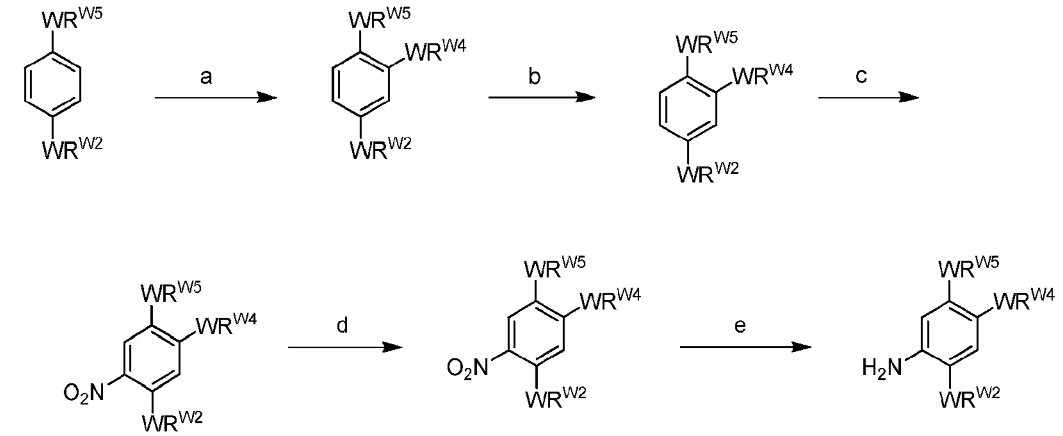


a) $WR^{W4}OH$, $WR^{W4}$ = alkyl; b) $ClCO_2R$, TEA; c) $HNO_3$, $H_2SO_4$; d) base; e) hydrogenation.

c. COUPLING OF ACID MOIETY TO AMINE MOIETY TO FORM COMPOUNDS OF FORMULA I

Compounds of Formula I are prepared by coupling an acid moiety with an amine moiety as depicted in Scheme 1-3. In general, the coupling reaction requires a coupling reagent, a base, as well as a solvent. Examples of conditions used include HATU, DIEA; BOP, DIEA, DMF; HBTU, $Et_3N$, $CH_2Cl_2$; PFPTFA, pyridine.

Scheme 1-3: Preparation of Compounds of Formula I.

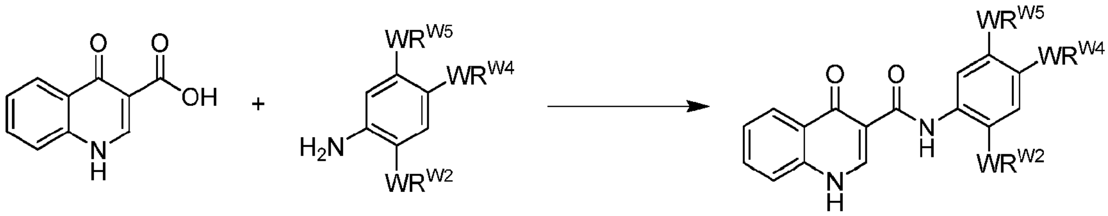

**Formula I**

2. COMPOUND 1 SYNTHESIS

Compound 1 can be prepared generally as provided in Schemes 1-3 through 1-6, wherein an acid moiety

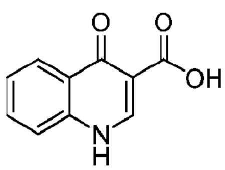

is coupled with an amine moiety

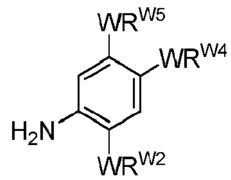

wherein $WR^{W2}$ and $WR^{W4}$ are t-butyl, and $WR^{W5}$ is OH. More detailed schemes and examples are provided below.

a. SYNTHESIS OF COMPOUND 1 ACID MOIETY

The synthesis of the acid moiety 4-Oxo-1,4-dihydroquinoline-3-carboxylic acid 26, is summarized in Scheme 1-4.

Scheme 1-4: Synthesis of 4-Oxo-1,4-Dihydroquinoline-3-Carboxylic Acid.

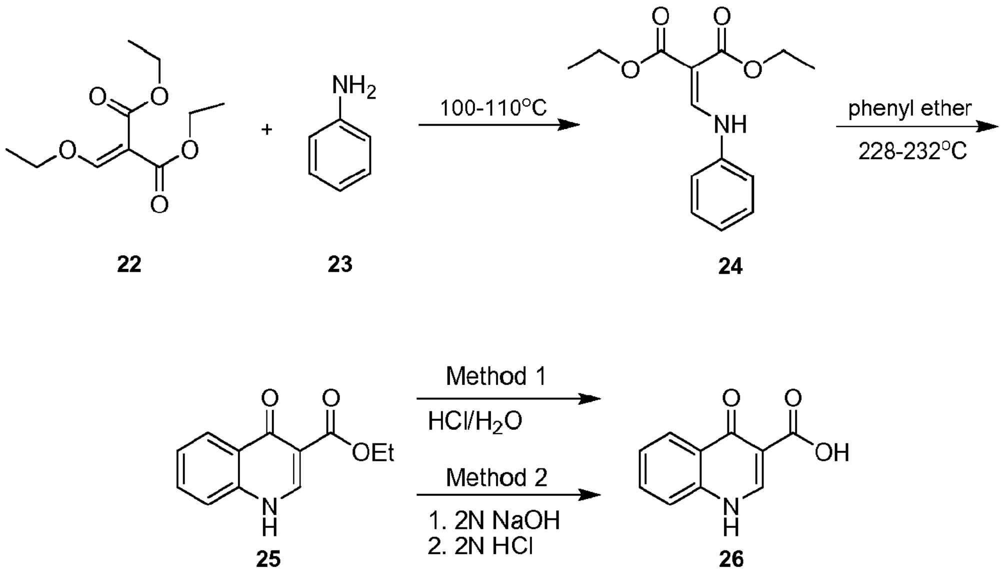


22 23 24

25 26

Ethyl 4-oxo-1,4-dihydroquinoline-3-carboxylate (25).

Compound 23 (4.77 g, 47.7 mmol) was added dropwise to Compound 22 (10 g, 46.3 mmol) with subsurface $N_2$ flow to drive out ethanol below 30 °C for 0.5 hours. The solution was then heated to 100-110 °C and stirred for 2.5 hours. After cooling the mixture to below 60 °C, diphenyl ether was added. The resulting solution was added dropwise to diphenyl ether that had been heated to 228-232 °C for 1.5 hours with subsurface $N_2$ flow to drive out ethanol. The mixture was stirred at 228-232 °C for another 2 hours, cooled to below 100 °C and then heptane was added to precipitate the product. The resulting slurry was stirred at 30 °C for 0.5 hours. The solids were then filtrated, and the cake was washed with heptane and dried *in vacuo* to give Compound 25 as a brown solid. $^1$H NMR (DMSO-$d_6$; 400 MHz) δ 12.25 (s), δ 8.49 (d), δ 8.10 (m), δ 7.64 (m), δ 7.55 (m), δ 7.34 (m), δ 4.16 (q), δ 1.23 (t).

4-Oxo-1,4-dihydroquinoline-3-carboxylic acid (26).

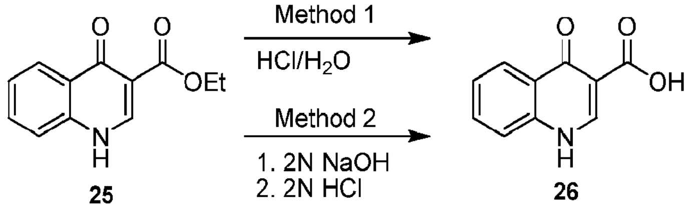


25 26

Method 1

Compound 25 (1.0 eq) was suspended in a solution of HCl (10.0 eq) and $H_2O$ (11.6 vol). The slurry was heated to 85 - 90 °C, although alternative temperatures are also suitable for this hydrolysis step. For example, the hydrolysis can alternatively be performed at a temperature of from about 75 to about 100 °C. In some instances, the hydrolysis is performed at a temperature of from about 80 to about 95 °C. In others, the hydrolysis step is performed at a temperature of from about 82 to about 93 °C (e.g., from about 82.5 to about 92.5 °C or from about 86 to about 89 °C). After stirring at 85 - 90 °C for approximately 6.5 hours, the reaction was sampled for reaction completion. Stirring may be performed under any of the temperatures suited for the hydrolysis. The solution was then cooled to 20 - 25 °C and filtered. The reactor/cake was rinsed with $H_2O$ (2 vol x 2). The cake was then washed with 2 vol $H_2O$ until the pH ≥ 3.0. The cake was then dried under vacuum at 60 °C to give Compound 26.

Method 2

Compound 25 (11.3 g, 52 mmol) was added to a mixture of 10% NaOH (aq) (10 mL) and ethanol (100 mL). The solution was heated to reflux for 16 hours, cooled to 20-25 °C and then the pH was adjusted to 2-3 with 8% HCl. The mixture was then stirred for 0.5 hours and filtered. The cake was washed with water (50 mL) and then dried *in vacuo* to give Compound 26 as a brown solid. $^1$H NMR (DMSO-$d_6$; 400 MHz) δ 15.33 (s), δ 13.39 (s), δ 8.87 (s), δ 8.26 (m), δ 7.87 (m), δ 7.80 (m), δ 7.56 (m).

b. SYNTHESIS OF COMPOUND 1 AMINE MOIETY

The synthesis of the amine moiety 32, is summarized in Scheme 1-5. Scheme 1-5: Synthesis of 5-Amino-2,4-Di-Tert-Butylphenyl Methyl Carbonate (32).

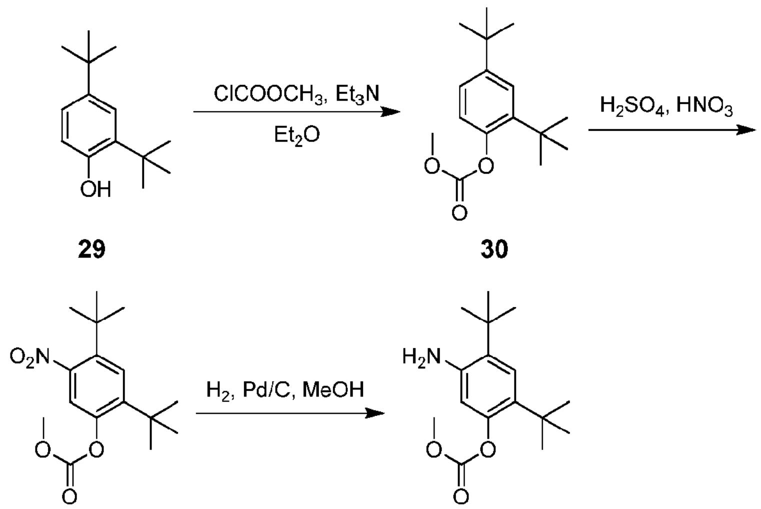


29 30

31 32

2,4-Di-tert-butylphenyl methyl carbonate (30).

Method 1

To a solution of 2,4-di-*tert*-butyl phenol, 29, (10 g, 48.5mmol) in diethyl ether (100 mL) and triethylamine (10.1 mL, 72.8 mmol), was added methyl chloroformate (7.46 mL, 97 mmol) dropwise at 0 °C. The mixture was then allowed to warm to room temperature and stir for an additional 2 hours. An additional 5 mL triethylamine and 3.7 mL methyl chloroformate was then added and the reaction stirred overnight. The reaction was then filtered, the filtrate was cooled to 0 °C, and an additional 5 mL triethylamine and 3.7 mL methyl chloroformate was then added and the reaction was allowed to warm to room temperature and then stir for an addition 1 hours. At this stage, the reaction was almost complete and was worked up by filtering, then washing with water (2x), followed by brine. The solution was then concentrated to produce a yellow oil and purified using column chromatography to give Compound 30. $^1$H NMR (400 MHz, DMSO-$d_6$) δ 7.35 (d, *J*= 2.4 Hz, 1H), 7.29 (dd, *J*= 8.4, 2.4 Hz, 1H), 7.06 (d, *J*= 8.4 Hz, 1H), 3.85 (s, 3H), 1.30 (s, 9H), 1.29 (s, 9H).

Method 2

To a reactor vessel charged with 4-dimethylaminopyridine (DMAP, 3.16 g, 25.7 mmol) and 2,4-di*tert*-butyl phenol (Compound 29, 103.5 g, 501.6 mmol) was added methylene chloride (415 g, 313 mL) and the solution was agitated until all solids dissolved. Triethylamine (76 g, 751 mmol) was then added and the solution was cooled to 0 - 5 °C. Methyl chloroformate (52 g, 550.3 mmol) was then added dropwise over 2.5 - 4 hours, while keeping the solution temperature between 0 - 5 °C. The reaction mixture was then slowly heated to 23 - 28 °C and stirred for 20 hours. The reaction was then cooled to 10 - 15 °C and charged with 150 mL water. The mixture was stirred at 15 - 20 °C for 35 - 45 minutes and the aqueous layer was then separated and extracted with 150 mL methylene chloride. The organic layers were combined and neutralized with 2.5% IICl (aq) at a temperature of 5 - 20 °C to give a final pH of 5 - 6. The organic layer was then washed with water and concentrated *in vacuo* at a temperature below 20 °C to 150 mL to give Compound 30 in methylene chloride.

5-Nitro-2,4-di-tert-butylphenyl methyl carbonate (31).

Method 1

To a stirred solution of Compound 30 (6.77g, 25.6 mmol) was added 6 mL of a 1:1 mixture of sulfuric acid and nitric acid at 0 °C dropwise. The mixture was allowed to warm to room temperature and stirred for 1 hour. The product was purified using liquid chromatography (ISCO, 120 g, 0-7% EtOAc/Hexanes, 38 min) producing about an 8:1 - 10:1 mixture of regioisomers of Compound 31 as a white solid. $^1H$ NMR (400 MHz, DMSO-$d_6$) $\delta$ 7.63 (s, 1H), 7.56 (s, 1H), 3.87 (s, 3H), 1.36 (s, 9H), 1.32 (s, 9H). HPLC ret. time 3.92 min 10-99% $CH_3CN$, 5 min run; ESI-MS 310 m/z $(MH)^+$.

Method 2

To Compound 30 (100g, 378 mmol) was added DCM (540 g, 408 mL). The mixture was stirred until all solids dissolved, and then cooled to -5 - 0 °C. Concentrated sulfuric acid (163 g) was then added dropwise, while maintaining the initial temperature of the reaction, and the mixture was stirred for 4.5 hours. Nitric acid (62 g) was then added dropwise over 2-4 hours while maintaining the initial temperature of the reaction, and was then stirred at this temperature for an additional 4.5 hours. The reaction mixture was then slowly added to cold water, maintaining a temperature below 5 °C. The quenched reaction was then heated to 25 °C and the aqueous layer was removed and extracted with methylene chloride. The combined organic layers were washed with water, dried using $Na_2SO_4$, and concentrated to 124 - 155 mL. Hexane (48 g) was added and the resulting mixture was again concentrated to 124 - 155 mL. More hexane (160 g) was subsequently added to the mixture. The mixture was then stirred at 23 - 27 °C for 15.5 hours, and was then filtered. To the filter cake was added hexane (115 g), the resulting mixture was heated to reflux and stirred for 2 - 2.5 hours. The mixture was then cooled to 3 - 7 °C, stirred for an additional 1 - 1.5 hours, and filtered to give Compound 31 as a pale yellow solid.

5-Amino-2,4-di-tert-butylphenyl methyl carbonate (32).

2,4-Di-tert-butyl-5-nitrophenyl methyl carbonate (1.00 eq) was charged to a suitable hydrogenation reactor, followed by 5% Pd/C (2.50 wt% dry basis, Johnson-Matthey Type 37). MeOH (15.0 vol) was charged to the reactor, and the system was closed. The system was purged with $N_2$ (g), and was then pressurized to 2.0 Bar with $H_2$ (g). The reaction was performed at a reaction temperature of 25 °C +/- 5 °C. When complete, the reaction was filtered, and the reactor/cake was washed with MeOH (4.00 vol). The resulting filtrate was distilled under vacuum at no more than 50 °C to 8.00 vol. Water (2.00 vol) was added at 45 °C +/- 5 °C. The resultant slurry was cooled to 0 °C +/- 5. The slurry was held at 0 °C +/- 5 °C for no less than 1 hour, and filtered. The cake was washed once with 0 °C +/- 5 °C MeOH/$H_2O$ (8:2) (2.00 vol). The cake was dried under vacuum (-0.90 bar and -0.86 bar) at 35 °C - 40 °C to give Compound 32. $^1H$ NMR (400 MHz, DMSO-$d_6$) $\delta$ 7.05 (s, 1H), 6.39 (s, 1H), 4.80 (s, 2H), 3.82 (s, 3H), 1.33 (s, 9H), 1.23 (s, 9H).

Once the reaction was complete, the resulting mixture was diluted with from about 5 to 10 volumes of MeOH (e.g., from about 6 to about 9 volumes of MeOH, from about 7 to about 8.5 volumes of MeOH, from about 7.5 to about 8 volumes of MeOH, or about 7.7 volumes of MeOH), heated to a temperature of about 35 $\pm$ 5 °C, filtered, washed, and dried, as described above.

c. COUPLING OF ACID AND AMINE MOIETY TO FORM COMPOUND 1

The coupling of the acid moiety to the amine moiety is summarized in Scheme 1-6.

Scheme 1-6: Synthesis of Compound 1

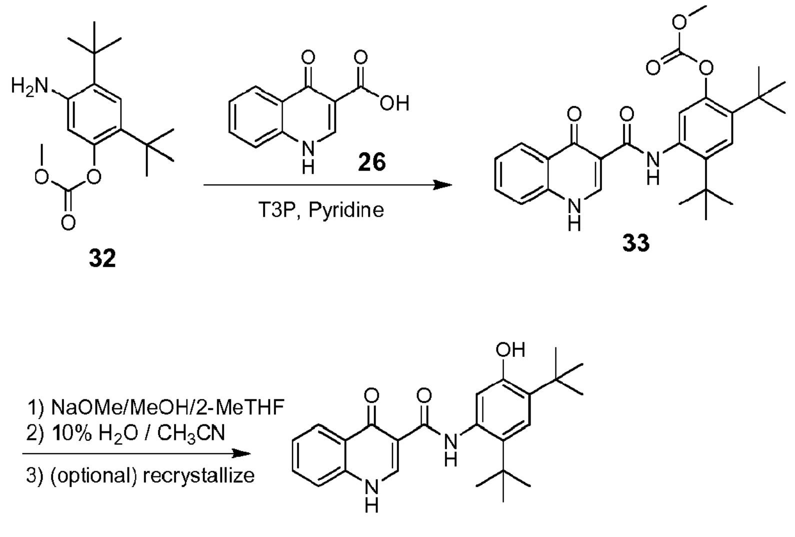


32

33

1

N-(2,4-di-tert-butyl-5-hydroxyphenyl)-4-oxo-1,4-dihydroquinoline-3-carboxamide (1).

4-Oxo-1,4-dihydroquinoline-3-carboxylic acid 26 (1.0 eq) and 5-amino-2,4-di-tert-butylphenyl methyl carbonate 32 (1.1 eq) were charged to a reactor. 2-MeTHF (4.0 vol, relative to the acid) was added followed by T3P® 50% solution in 2-MeTHF (1.7 eq). The T3P charged vessel was washed with 2-MeTHF (0.6 vol). Pyridine (2.0 eq) was then added, and the resulting suspension was heated to 47.5 +/- 5.0 °C and held at this temperature for 8 hours. A sample was taken and checked for completion by HPLC. Once complete, the resulting mixture was cooled to 25.0 °C +/- 2.5 °C. 2-MeTHF was added (12.5 vol) to dilute the mixture. The reaction mixture was washed with water (10.0 vol) 2 times. 2-MeTHF was added to bring the total volume of reaction to 40.0 vol (~16.5 vol charged). To this solution was added NaOMc/McOH (1.7 cquiv) to perform the mcthanolysis. The reaction was stirred for no less than 1.0 hour, and checked for completion by HPLC. Once complete, the reaction was quenched with 1 N HCl (10.0 vol), and washed with 0.1 N HCl (10.0 vol). The organic solution was polish filtered to remove any particulates and placed in a second reactor. The filtered solution was concentrated at no more than 35 °C (jacket temperature) and no less than 8.0 °C (internal reaction temperature) under reduced pressure to 20 vol. $CH_3CN$ was added to 40 vol and the solution concentrated at no more than 35 °C (jacket temperature) and no less than 8.0 °C (internal reaction temperature) to 20 vol. The addition of $CH_3CN$ and concentration cycle was repeated 2 more times for a total of 3 additions of $CH_3CN$ and 4 concentrations to 20 vol. After the final concentration to 20 vol, 16.0 vol of $CH_3CN$ was added followed by 4.0 vol of $H_2O$ to make a final concentration of 40 vol of 10% $H_2O/CH_3CN$ relative to the starting acid. This slurry was heated to 78.0 °C +/- 5.0 °C (reflux). The slurry was then stirred for no less than 5 hours. The slurry was cooled to 0.0 °C +/- 5 °C over 5 hours, and filtered. The cake was washed with 0.0 °C +/- 5.0 °C $CH_3CN$ (5 vol) 4 times. The resulting solid (Compound 1) was dried in a vacuum oven at 50.0 °C +/- 5.0 °C. $^1H$ NMR (400 MHz, DMSO-$d_6$) δ 12.8 (s, 1H), 11.8 (s, 1H), 9.2 (s, 1H), 8.9 (s, 1H), 8.3 (s, 1H), 7.2 (s, 1H), 7.9 (t, 1H), 7.8 (d, 1H), 7.5 (t, 1H), 7.1 (s, 1H), 1.4 (s, 9H), 1.4 (s, 9H).

An alternative synthesis of Compound 1 is depicted in Scheme 1-7.

Scheme 1-7: Alternate Synthesis of Compound 1.

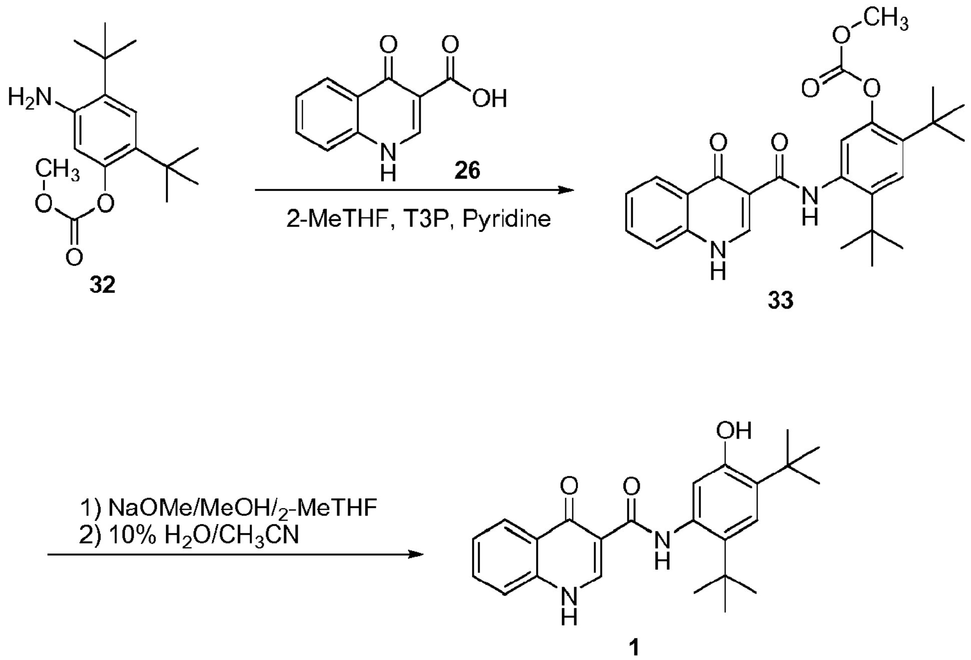


4-Oxo-1,4-dihydroquinoline-3-carboxylic acid 26 (1.0 eq) and 5-amino-2,4-di-tert-butylphenyl methyl carbonate 32 (1.1 eq) were charged to a reactor. 2-MeTHF (4.0 vol, relative to the acid) was added followed by T3P® 50% solution in 2-MeTHF (1.7 eq). The T3P charged vessel was washed with 2-MeTHF (0.6 vol). Pyridine (2.0 eq) was then added, and the resulting suspension was heated to 47.5 +/- 5.0 °C and held at this temperature for 8 hours. A sample was taken and checked for completion by HPLC. Once complete, the resulting mixture was cooled to 20 °C +/- 5 °C. 2-MeTHF was added (12.5 vol) to dilute the mixture. The reaction mixture was washed with water (10.0 vol) 2 times and 2-MeTHF (16.5 vol) was charged to the reactor. This solution was charged with 30% w/w NaOMe/MeOH (1.7 equiv) to perform the methanolysis. The reaction was stirred at 25.0 °C +/- 5.0 °C for no less than 1.0 hour, and checked for completion by HPLC. Once complete, the reaction was quenched with 1.2 N $HCl/H_2O$ (10.0 vol), and washed with 0.1 N $HCl/H_2O$ (10.0 vol). The organic solution was polish filtered to remove any particulates and placed in a second reactor.

The filtered solution was concentrated at no more than 35 °C (jacket temperature) and no less than 8.0 °C (internal reaction temperature) under reduced pressure to 20 vol. $CH_3CN$ was added to 40 vol and the solution concentrated at no more than 35 °C (jacket temperature) and no less than 8.0 °C (internal reaction temperature) to 20 vol. The addition of $CH_3CN$ and concentration cycle was repeated 2 more times for a total of 3 additions of $CH_3CN$ and 4 concentrations to 20 vol. After the final concentration to 20 vol, 16.0 vol of $CH_3CN$ was charged followed by 4.0 vol of $H_2O$ to make a final concentration of 40 vol of 10% $H_2O/CH_3CN$ relative to the starting acid. This slurry was heated to 78.0 °C +/- 5.0 °C (reflux). The slurry was then stirred for no less than 5 hours. The slurry was cooled to 20 to 25 °C over 5 hours, and filtered. The cake was washed with $CH_3CN$ (5 vol) heated to 20 to 25 °C 4 times. The resulting solid (Compound 1) was dried in a vacuum oven at 50.0 °C +/- 5.0 °C. $^1$H NMR (400 MHz, DMSO-$d_6$) δ 12.8 (s, 1H), 11.8 (s, 1H), 9.2 (s, 1H), 8.9 (s, 1H), 8.3 (s, 1H), 7.2 (s, 1H), 7.9 (t, 1II), 7.8 (d, 1II), 7.5 (t, 1II), 7.1 (s, 1II), 1.4 (s, 9II), 1.4 (s, 9II).

II.B.1. COMPOUND OF FORMULA II

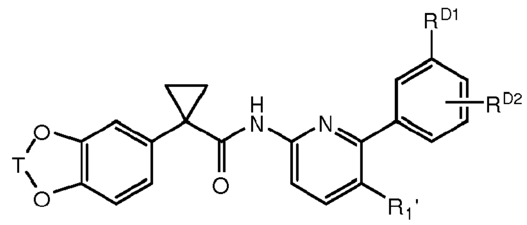

Formula II

1. EMBODIMENTS OF THE COMPOUNDS OF FORMULA II

The modulators of ABC transporter activity in Column B are fully described and exemplified in U.S. Patents 7,741,321 and 7,659,268, and also in U.S. Patent Application Serial Number 12/114,935, published as US 2008/0306062 A1. All of which are commonly assigned to the Assignee of the present invention. All of the compounds recited in the above publications are useful in the present invention and are hereby incorporated into the present disclosure in their entirety.

In one embodiment, in the compound of Formula II of the composition

T is $-CH_2-$, $-CH_2CH_2-$, $-CF_2-$, $-C(CH_3)_2-$, or -C(O)-;
$R_1$' is H, $C_{1-6}$ aliphatic, halo, $CF_3$, $CHF_2$, O($C_{1-6}$ aliphatic); and
$R^{D1}$ or $R^{D2}$ is $Z^DR_9$
wherein:

$Z^D$ is a bond, CONH, $SO_2NH$, $SO_2N(C_{1-6}$ alkyl), $CH_2NHSO_2$, $CH_2N(CH_3)SO_2$, $CH_2NHCO$, COO, $SO_2$, or CO; and $R_9$ is H, $C_{1-6}$ aliphatic, or aryl.

II.B.2. COMPOUND 2

In another embodiment, the compound of Formula II is Compound 2, depicted below, which is also known by its chemical name 3-(6-(1-(2,2-Difluorobenzo[d][1,3]dioxol-5-yl)cyclopropanecarboxamido)-3-methylpyridin-2-yl)benzoic acid.

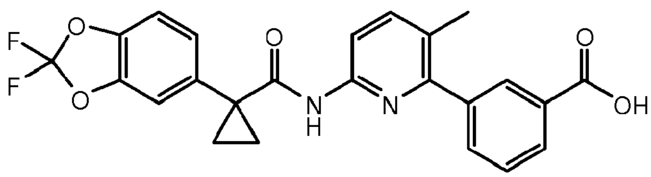

Compound 2

1. SYNTHESIS OF COMPOUNDS OF FORMULA II

Compound 2 can be prepared by coupling an acid chloride moiety with an amine moiety according to following Schemes 2-1 to 2-3.

Scheme 2-1: Synthesis of the Acid Chloride Moiety.

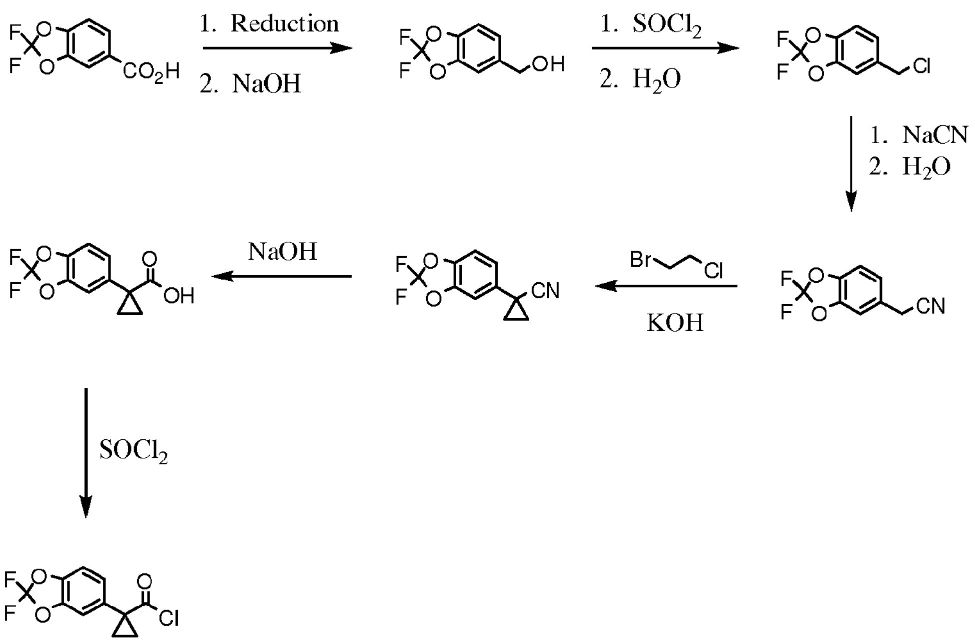


Scheme 2-1 depicts the preparation of 1-(2,2-difluorobenzo[d][1,3]dioxol-5-yl)cyclopropanecarbonyl chloride, which is used in Scheme 2-3 to make the amide linkage of Compound 2.

The starting material, 2,2-difluorobenzo[d][1,3]dioxole-5-carboxylic acid, is commercially available from Saltigo (an affiliate of the Lanxess Corporation). Reduction of the carboxylc acid moiety in 2,2-difluorobenzo[d][1,3]dioxole-5-carboxylic acid to the primary alcohol, followed by conversion to the corresponding chloride using thionyl chloride ($SOCl_2$), provides 5-(chloromethyl)-2,2-difluorobenzo[d][1,3]dioxole, which is subsequently converted to 2-(2,2-difluorobenzo[d][1,3]dioxol-5-yl)acetonitrile using sodium cyanide. Treatment of 2-(2,2-difluorobenzo[d][1,3]dioxol-5-yl)acetonitrile with base and 1-bromo-2-chloroethane provides 1-(2,2-difluorobenzo[d][1,3]dioxol-5-yl)cyclopropanecarbonitrile. The nitrile moiety in 1-(2,2-difluorobenzo[d] [1,3]dioxol-5-yl)cyclopropanecarbonitrile is converted to a carboxylic acid using base to give 1-(2,2-difluorobenzo[d][1,3]dioxol-5-yl)cyclopropanecarboxylic acid, which is converted to the desired acid chloride using thionyl chloride.

Scheme 2-2: Synthesis of the Amine Moiety.

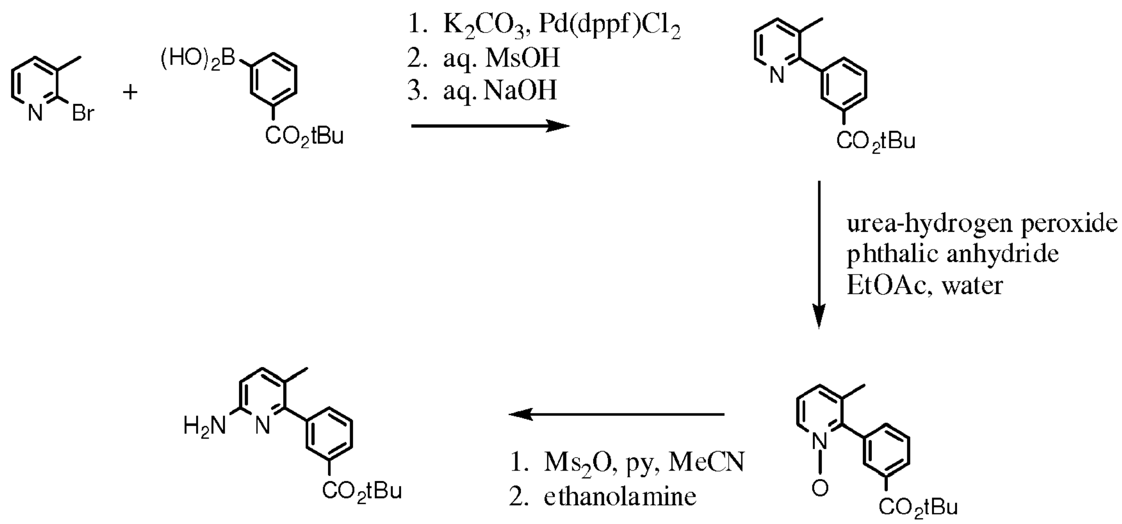


Scheme 2-2 depicts the preparation of the requisite tert-butyl 3-(6-amino-3-methylpyridin-2-yl)benzoate, which is coupled with 1-(2,2-difluorobenzo[d][1,3]dioxol-5-yl)cyclopropanecarbonyl chloride in Scheme 3-3 to give Compound

2. Palladium-catalyzed coupling of 2-bromo-3-methylpyridine with 3-(tert-butoxycarbonyl)phenylboronic acid gives tert-butyl 3-(3-methylpyridin-2-yl)benzoate, which is subsequently converted to the desired compound.

Scheme 2-3. Formation of an Acid Salt of 3-(6-(1-(2,2-difluorobenzo[d][1,3]dioxol-5-yl) cyclopropanecarboxamido)-3-methylpyridin-2-yl)benzoic Acid.

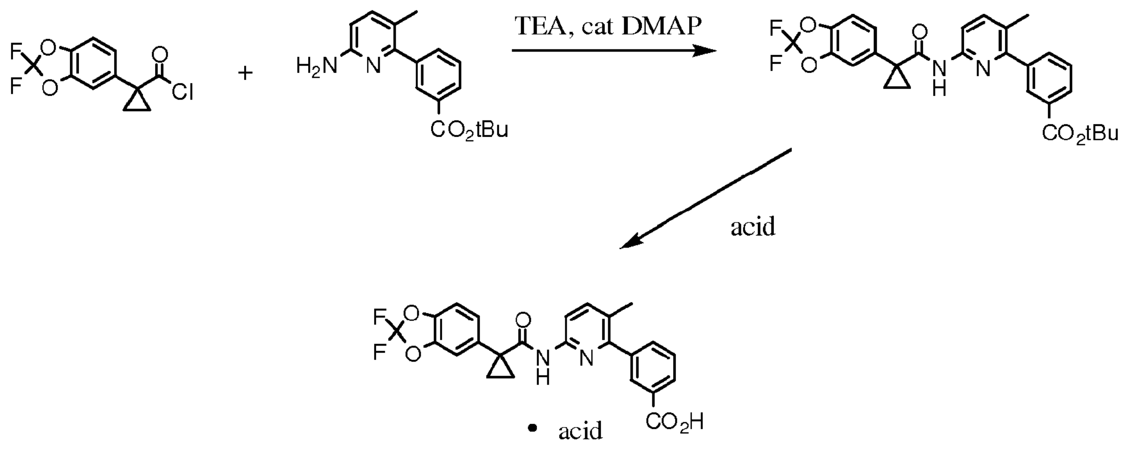


Scheme 2-3 depicts the coupling of 1-(2,2-difluorobenzo[d][1,3]dioxol-5-yl)cyclopropanecarbonyl chloride with tert-butyl 3-(6-amino-3-methylpyridin-2-yl)benzoate using triethyl amine and 4-dimethylaminopyridine to initially provide the tert-butyl ester of Compound 2. Treatment of the tert-butyl ester with an acid such as HCl, gives the HCl salt of Compound 2, which is typically a crystalline solid.

Experimentals

Vitride® (sodium bis(2-methoxyethoxy)aluminum hydride [or $NaAlH_2(OCH_2CH_2OCH_3)_2$], 65 wgt% solution in toluene) was purchased from Aldrich Chemicals.

2,2-Difluoro-1,3-benzodioxole-5-carboxylic acid was purchased from Saltigo (an affiliate of the Lanxess Corporation).

(2,2-Difluoro-1,3-benzodioxol-5-yl)-methanol.

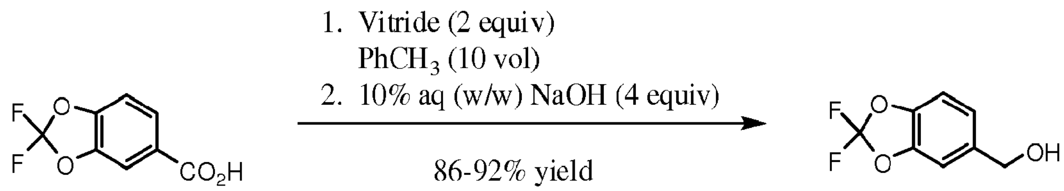


Commercially available 2,2-difluoro-1,3-benzodioxole-5-carboxylic acid (1.0 eq) was slurried in toluene (10 vol). Vitride® (2 eq) was added via addition funnel at a rate to maintain the temperature at 15-25 °C. At the end of the addition, the temperature was increased to 40 °C for 2 hours (h), then 10% (w/w) aqueous (aq) NaOH (4.0 eq) was carefully added via addition funnel, maintaining the temperature at 40-50 °C. After stirring for an additional 30 minutes (min), the layers were allowed to separate at 40 °C. The organic phase was cooled to 20 °C, then washed with water (2 x 1.5 vol), dried ($Na_2SO_4$), filtered, and concentrated to afford crude (2,2-difluoro-1,3-benzodioxol-5-yl)-methanol that was used directly in the next step.

5-Chloromethyl-2,2-difluoro-1,3-benzodioxole.

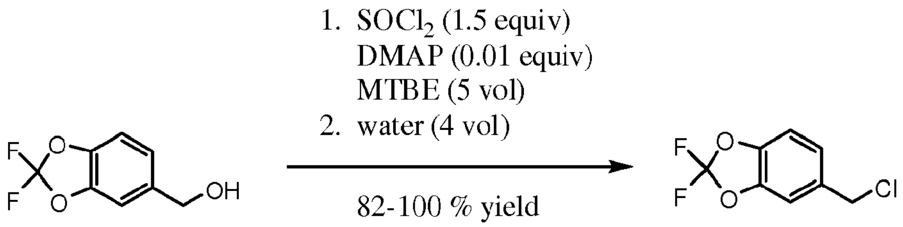


(2,2-difluoro-1,3-benzodioxol-5-yl)-methanol (1.0 eq) was dissolved in MTBE (5 vol). A catalytic amount of 4-(N,N-dimethyl)aminopyridine (DMAP) (1 mol %) was added and $SOCl_2$ (1.2 eq) was added via addition funnel. The $SOCl_2$ was added at a rate to maintain the temperature in the reactor at 15-25 °C. The temperature was increased to 30 °C for 1 h, and then was cooled to 20 °C. Water (4 vol) was added via addition funnel while maintaining the temperature at less than 30 °C. After stirring for an additional 30 min, the layers were allowed to separate. The organic layer was stirred and 10% (w/v) aq NaOH (4.4 vol) was added. After stirring for 15 to 20 min, the layers were allowed to separate. The organic phase was then dried ($Na_2SO_4$), filtered, and concentrated to afford crude 5-chloromethyl-2,2-difluoro-1,3-benzodioxole that was used directly in the next step.

(2,2-Difluoro-1,3-benzodioxol-5-yl)-acetonitrile.

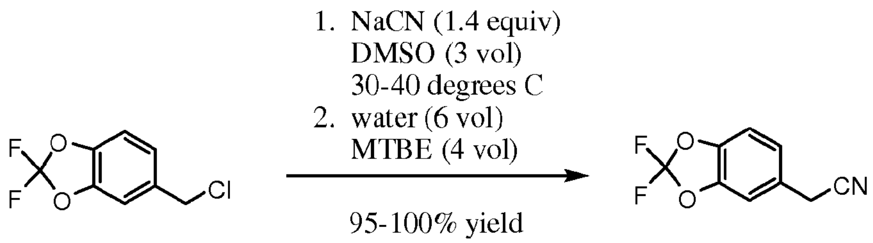


A solution of 5-chloromethyl-2,2-difluoro-1,3-benzodioxole (1 eq) in DMSO (1.25 vol) was added to a slurry of NaCN (1.4 eq) in DMSO (3 vol), while maintaining the temperature between 30-40 °C. The mixture was stirred for 1 h, and then water (6 vol) was added, followed by methyl *tert*-butyl ether (MTBE) (4 vol). After stirring for 30 min, the layers were separated. The aqueous layer was extracted with MTBE (1.8 vol). The combined organic layers were washed with water (1.8 vol), dried ($Na_2SO_4$), filtered, and concentrated to afford crude (2,2-difluoro-1,3-benzodioxol-5-yl)-acetonitrile (95%) that was used directly in the next step.

(2,2-Difluoro-1,3-benzodioxol-5-yl)-cyclopropanecarbonitrile.

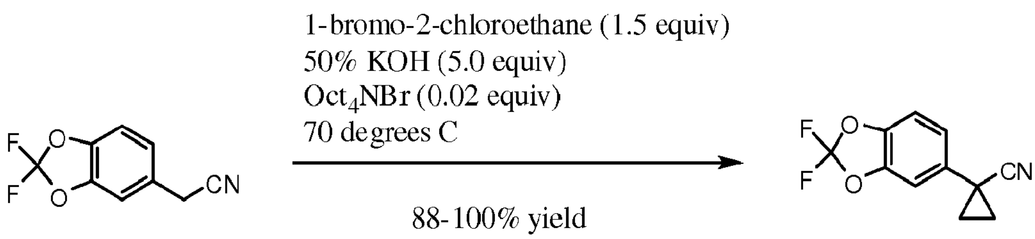


A mixture of (2,2-difluoro-1,3-benzodioxol-5-yl)-acetonitrile (1.0 eq), 50 wt % aqueous KOH (5.0 eq) 1-bromo-2-chloroethane (1.5 eq), and $Oct_4NBr$ (0.02 eq) was heated at 70 °C for 1 h. The reaction mixture was cooled, then worked up with MTBE and water. The organic phase was washed with water and brine. The solvent was removed to afford (2,2-difluoro-1,3-benzodioxol-5-yl)-cyclopropanecarbonitrile.

1-(2,2-Difluoro-1,3-benzodioxol-5-yl)-cyclopropanecarboxylic acid.

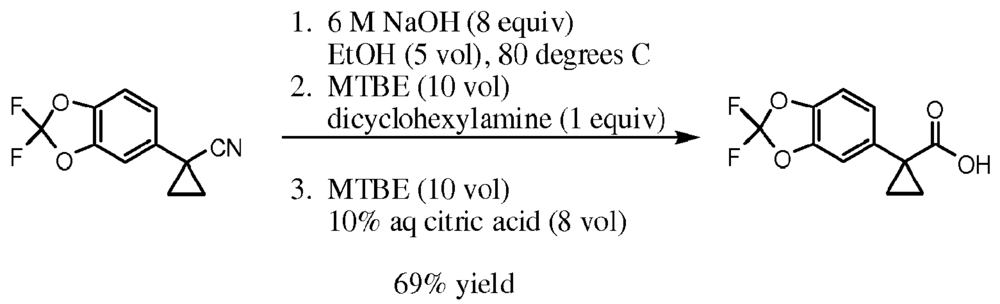


(2,2-difluoro-1,3-benzodioxol-5-yl)-cyclopropanecarbonitrile was hydrolyzed using 6 M NaOH (8 equiv) in ethanol (5 vol) at 80 °C overnight. The mixture was cooled to room temperature and the ethanol was evaporated under vacuum. The residue was taken up in water and MTBE, 1 M HCl was added, and the layers were separated. The MTBE layer was then treated with dicyclohexylamine (DCHA) (0.97 equiv). The slurry was cooled to 0 °C, filtered and washed with heptane to give the corresponding DCHA salt. The salt was taken into MTBE and 10% citric acid and stirred until all the solids had dissolved. The layers were separated and the MTBE layer was washed with water and brine. A solvent swap to heptane followed by filtration gave 1-(2,2-difluoro-1,3-benzodioxol-5-yl)-cyclopropanecarboxylic acid after drying in a vacuum oven at 50 °C overnight.

1-(2,2-Difluoro-1,3-benzodioxol-5-yl)-cyclopropanecarbonyl chloride.

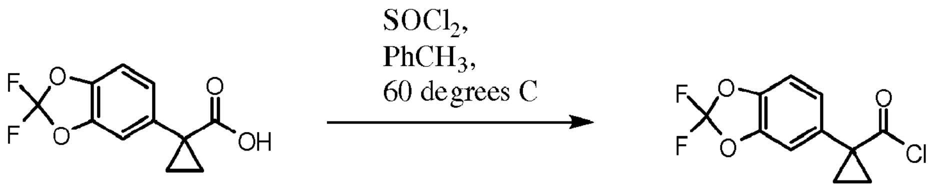


1-(2,2-difluoro-1,3-benzodioxol-5-yl)-cyclopropanecarboxylic acid (1.2 eq) is slurried in toluene (2.5 vol) and the mixture was heated to 60 °C. $SOCl_2$ (1.4 eq) was added via addition funnel. The toluene and $SOCl_2$ were distilled from the reaction mixture after 30 minutes. Additional toluene (2.5 vol) was added and the resulting mixture was distilled again, leaving the product acid chloride as an oil, which was used without further purification.

*tert*-Butyl-3-(3-methylpyridin-2-yl)benzoate.

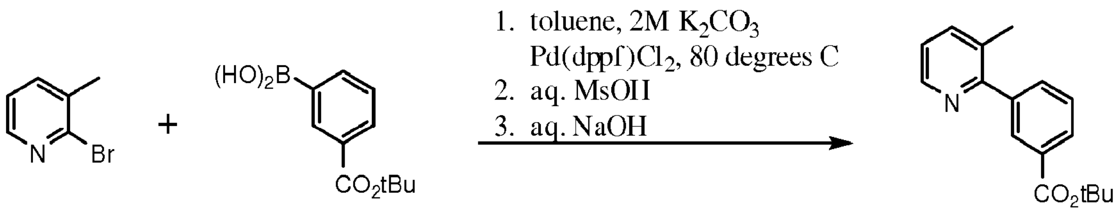


2-Bromo-3-methylpyridine (1.0 eq) was dissolved in toluene (12 vol). $K_2CO_3$ (4.8 eq) was added, followed by water (3.5 vol). The resulting mixture was heated to 65 °C under a stream of $N_2$ for 1 hour. 3-(*t*-Butoxycarbonyl)phenylboronic acid (1.05 eq) and $Pd(dppf)Cl_2 \cdot CH_2Cl_2$ (0.015 eq) were then added and the mixture was heated to 80 °C. After 2 hours, the heat was turned off, water was added (3.5 vol), and the layers were allowed to separate. The organic phase was then washed with water (3.5 vol) and extracted with 10% aqueous methanesulfonic acid (2 eq MsOH, 7.7 vol). The aqueous phase was made basic with 50% aqueous NaOH (2 eq) and extracted with EtOAc (8 vol). The organic layer

was concentrated to afford crude *tert*-butyl-3-(3-methylpyridin-2-yl)benzoate (82%) that was used directly in the next step.

2-(3-(*tert*-Butoxycarbonyl)phenyl)-3-methylpyridine-1-oxide.

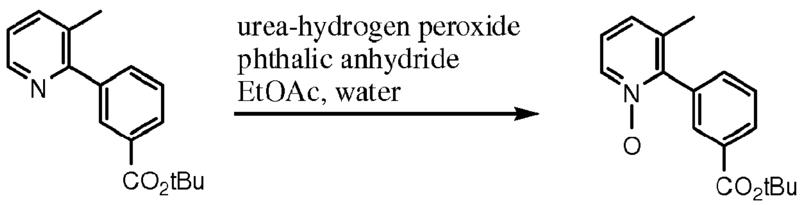


*Tert*-butyl-3-(3-methylpyridin-2-yl)benzoate (1.0 eq) was dissolved in EtOAc (6 vol). Water (0. 3 vol) was added, followed by urea-hydrogen peroxide (3 eq). Phthalic anhydride (3 eq) was then added portionwise to the mixture as a solid at a rate to maintain the temperature in the reactor below 45 °C. After completion of the phthalic anhydride addition, the mixture was heated to 45 °C. After stirring for an additional 4 hours, the heat was turned off. 10% w/w aqueous $Na_2SO_3$ (1.5 eq) was added via addition funnel. After completion of $Na_2SO_3$ addition, the mixture was stirred for an additional 30 min and the layers separated. The organic layer was stirred and 10% wt/wt aqueous. $Na_2CO_3$ (2 eq) was added. After stirring for 30 minutes, the layers were allowed to separate. The organic phase was washed 13% w/v aq NaCl. The organic phase was then filtered and concentrated to afford crude 2-(3-(*tert*-butoxycarbonyl)phenyl)-3-methylpyridine-1-oxide (95%) that was used directly in the next step.

*tert*-Butyl-3-(6-amino-3-methylpyridin-2-yl)benzoate.

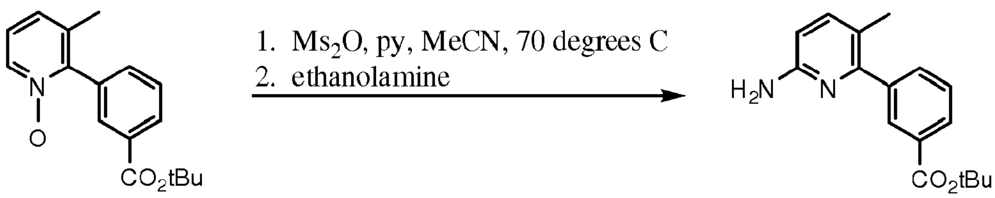


A solution of 2-(3-(*tert*-butoxycarhonyl)phenyl)-3-methylpyridine-1-oxide (1 eq) and pyridine (4 eq) in acetonitrile (8 vol) was heated to 70 °C. A solution of methanesulfonic anhydride (1.5 eq) in MeCN (2 vol) was added over 50 min via addition funnel while maintaining the temperature at less than 75 °C. The mixture was stirred for an additional 0.5 hours after complete addition. The mixture was then allowed to cool to ambient. Ethanolamine (10 eq) was added via addition funnel. After stirring for 2 hours, water (6 vol) was added and the mixture was cooled to 10 °C. After stirring for 3 hours, the solid was collected by filtration and washed with water (3 vol), 2:1 acetonitrile/water (3 vol), and acetonitrile (2 x 1.5 vol). The solid was dried to constant weight (<1 % difference) in a vacuum oven at 50 °C with a slight $N_2$ bleed to afford *tert*-butyl-3-(6-amino-3-methylpyridin-2-yl)benzoate as a red-yellow solid (53% yield).

3-(6-(1-(2,2-Difluorobenzo[d][1,3]dioxol-5-yl)- cyclopropanecarboxamido)-3-methylpyridin-2-yl)-t-butylbenzoate.

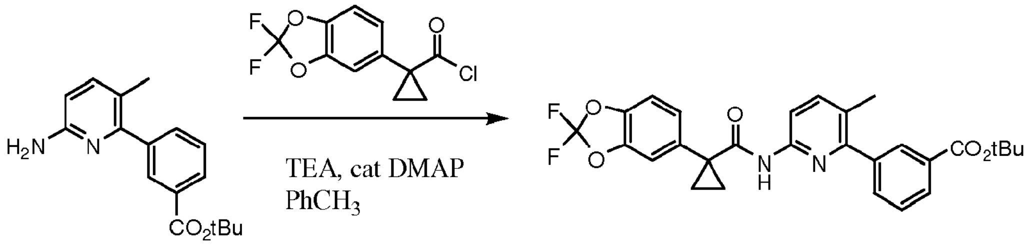

The crude acid chloride described above was dissolved in toluene (2.5 vol based on acid chloride) and added via addition funnel to a mixture of *tert*-butyl-3-(6-amino-3-methylpyridin-2-yl)benzoate (1 eq), DMAP, (0.02 eq), and triethylamine (3.0 eq) in toluene (4 vol based on *tert*-butyl-3-(6-amino-3-methylpyridin-2-yl)benzoate). After 2 hours, water (4 vol based on *tert*-butyl-3-(6-amino-3-methylpyridin-2-yl)benzoate) was added to the reaction mixture. After stirring for 30 minutes, the layers were separated. The organic phase was then filtered and concentrated to afford a thick oil of 3-(6-(1-(2,2-difluorobenzo[d][1,3]dioxol-5-yl) cyclopropanecarboxamido)-3-methylpyridin-2-yl)-t-butylbenzoate (quantitative crude yield). Acetonitrile (3 vol based on crude product) was added and distilled until crystallization occurs. Water (2 vol based on crude product) was added and the mixture stirred for 2 h. The solid was collected by filtration, washed with 1:1 (by volume) acetonitrile/water (2 x 1 volumes based on crude product), and partially dried on the filter under vacuum. The solid was dried to a constant weight (<1 % difference) in a vacuum oven at 60 °C with a slight $N_2$ bleed to afford 3-(6-(1-(2,2-difluorobenzo[d][1,3]dioxol-5-yl) cyclopropanecarboxamido)-3-methylpyridin-2-yl)-t-butylbenzoate as a brown solid.

3-(6-(1-(2,2-Difluorobenzo[d][1,3]dioxol-5-yl) cyclopropanecarboxamido)-3-methylpyridin-2-yl)benzoic acid • HCl salt.

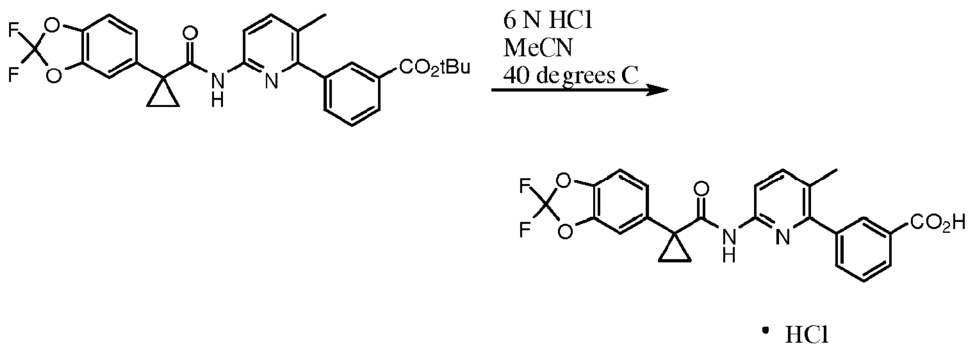


To a slurry of 3-(6-(1-(2,2-difluorobenzo[d][1,3]dioxol-5-yl) cyclopropanecarboxamido)-3-methylpyridin-2-yl)-t-butylbenzoate (1.0 eq) in MeCN (3.0 vol) was added water (0.83 vol) followed by concentrated aqueous HCl (0.83 vol). The mixture was heated to 45 ± 5 °C. After stirring for 24 to 48 h, the reaction was complete, and the mixture was allowed to cool to ambient. Water (1.33 vol) was added and the mixture stirred. The solid was collected by filtration, washed with water (2 x 0.3 vol), and partially dried on the filter under vacuum. The solid was dried to a constant weight (<1 % difference) in a vacuum oven at 60 °C with a slight $N_2$ bleed to afford 3-(6-(1-(2,2-difluorobenzo[d][1,3]dioxol-5-yl) cyclopropanecarboxamido)-3-methylpyridin-2-yl)benzoic acid • HCl as an off-white solid.

Table 2-1 below recites physical data for Compound 2.

Table 2-1.

| Compound | LC/MS M+1 | LC/RT minutes | NMR |
|---|---|---|---|
| Compound 2 | 453.3 | 1.93 | $^1$HNMR (400 MHz, DMSO-d6) 9.14 (s, 1H), 7.99-7.93 (m, 3H), 7.80-7.78 (m,1H), 7.74-7.72 (m,1H), 7.60-7.55 (m,2H), 7.41-7.33 (m,2H), 2.24 (s, 3H), 1.53-1.51 (m, 2H), 1.19-1.17 (m, 2H). |

II.C.1. COMPOUND OF FORMULA III

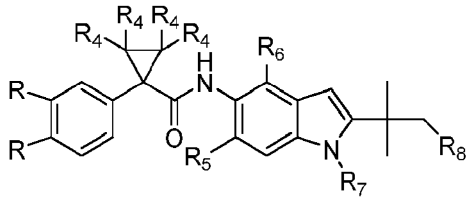

Formula III

The modulators of ABC transporter activity in Column C are fully described and exemplified in U.S. Patents 7,645,789 and 7,776,905, which are commonly assigned to the Assignee of the present invention. All of the compounds recited in the above patents are useful in the present invention and are hereby incorporated into the present disclosure in their entirety.

1. EMBODIMENTS OF COMPOUNDS OF FORMULA III

In one embodiment, in the compound of Formula III:

R is H, OH, $OCH_3$ or two R taken together form $-OCH_2O-$ or $-OCF_2O-$;
$R_4$ is H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

II.C.2. COMPOUND 3

In another embodiment, the compound of Formula III is Compound 3, which is known by its chemical name (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide.

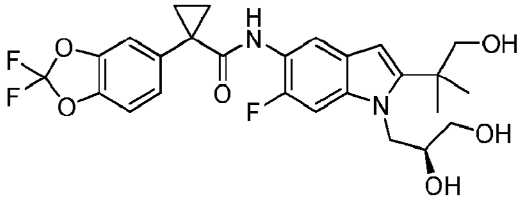

Compound 3

1. SYNTHESIS OF COMPOUNDS OF FORMULA III

a. GENERAL SCHEMES

Compound 3 can be prepared by coupling an acid chloride moiety with an amine moiety according to Schemes 3-1 through 3-3.

Scheme 3-1: Synthesis of the Acid Chloride Moiety.

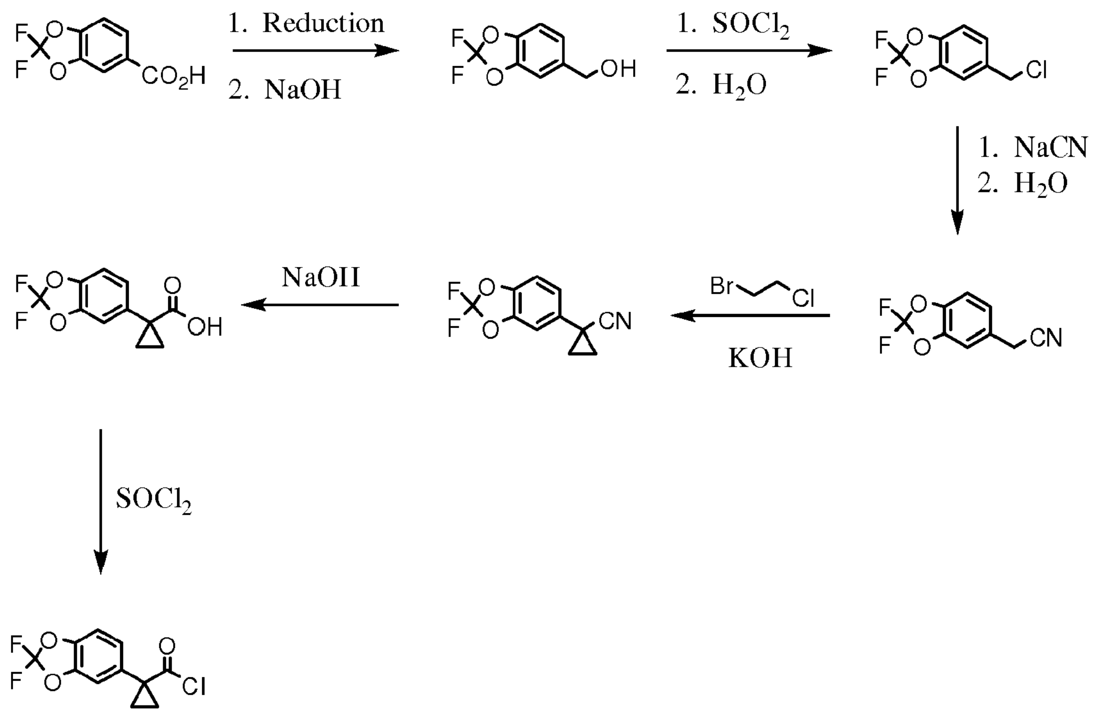


Scheme 3-2: Synthesis of the Amine Moiety.

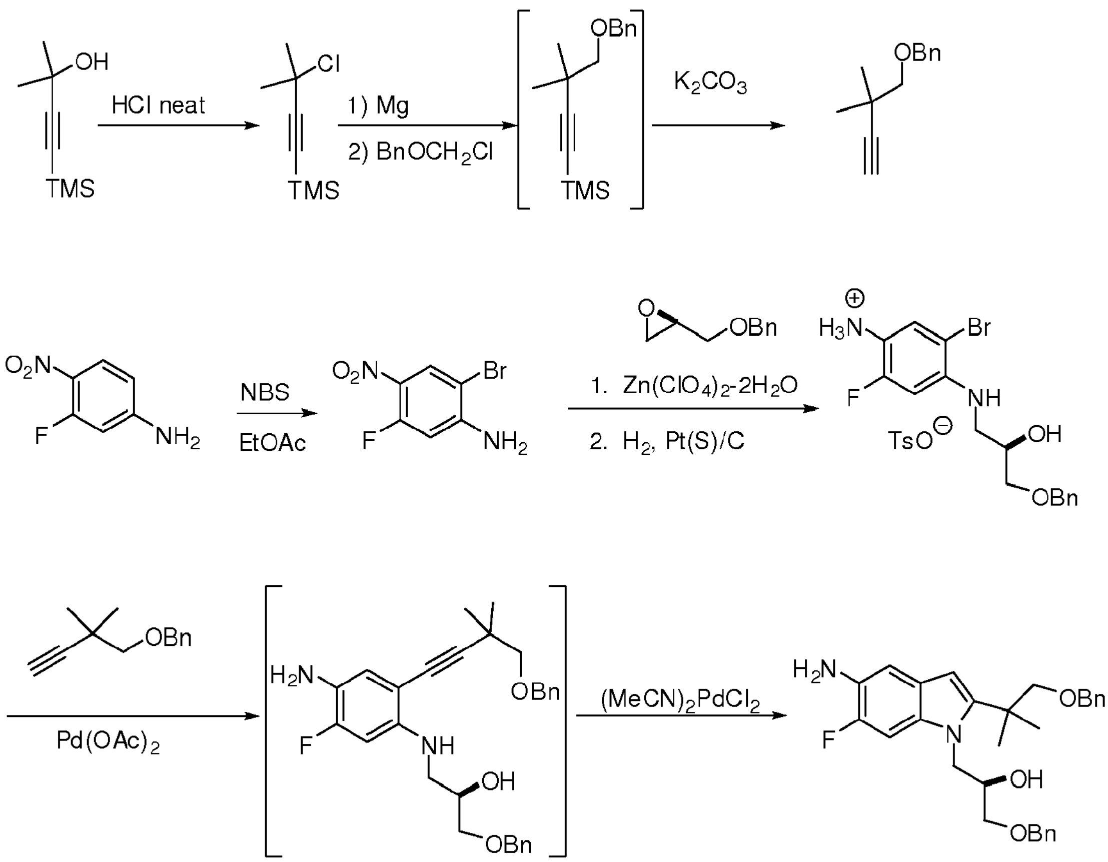

Scheme 3-3: Formation of Compound 3.

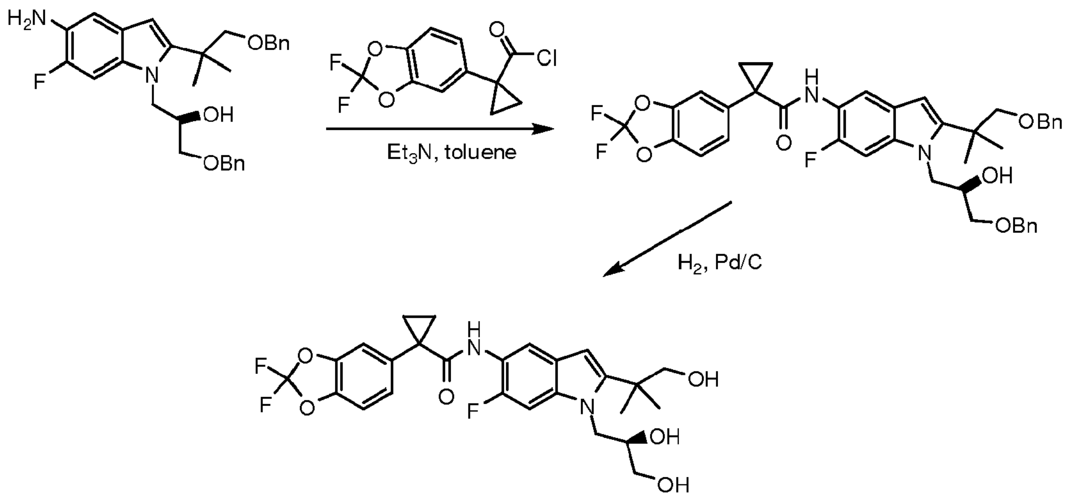


b. EXAMPLES

Vitride® (sodium bis(2-methoxyethoxy)aluminum hydride [or $NaAlH_2(OCH_2CH_2OCH_3)_2$], 65 wgt% solution in toluene) was purchased from Aldrich Chemicals.

2,2-Difluoro-1,3-benzodioxole-5-carboxylic acid was purchased from Saltigo (an affiliate of the Lanxess Corporation).

Compound 3 Acid Moiety Synthesis

(2,2-Difluoro-1,3-benzodioxol-5-yl)-methanol.

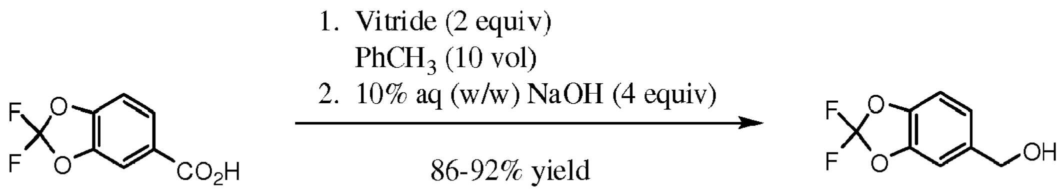


Commercially available 2,2-difluoro-1,3-benzodioxole-5-carboxylic acid (1.0 eq) was slurried in toluene (10 vol). Vitride® (2 eq) was added via addition funnel at a rate to maintain the temperature at 15-25 °C. At the end of addition the temperature was increased to 40 °C for 2 hours (h) then 10% (w/w) aq. NaOH (4.0 eq) was carefully added via addition funnel maintaining the temperature at 40-50 °C. After stirring for an additional 30 minutes (min), the layers were allowed to separate at 40 °C. The organic phase was cooled to 20 °C then washed with water (2 x 1.5 vol), dried ($Na_2SO_4$), filtered, and concentrated to afford crude (2,2-difluoro-1,3-benzodioxol-5-yl)-methanol that was used directly in the next step.

5-Chloromethyl-2,2-difluoro-1,3-benzodioxole.

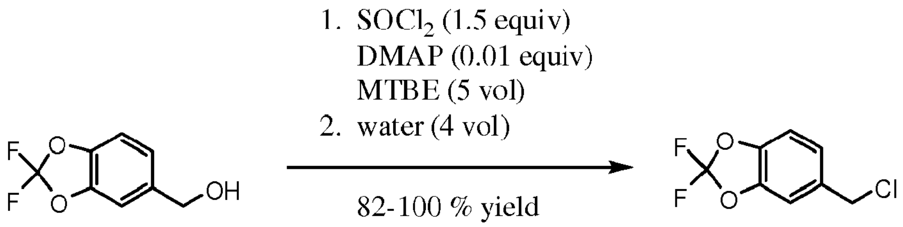


(2,2-difluoro-1,3-benzodioxol-5-yl)-methanol (1.0 eq) was dissolved in MTBE (5 vol). A catalytic amount of DMAP (1 mol %) was added and $SOCl_2$ (1.2 eq) was added via addition funnel. The $SOCl_2$ was added at a rate to maintain the temperature in the reactor at 15-25 °C. The temperature was increased to 30 °C for 1 hour then cooled to 20 °C then water (4 vol) was added via addition funnel maintaining the temperature at less than 30 °C. After stirring for an additional 30 minutes, the layers were allowed to separate. The organic layer was stirred and 10% (w/v) aq. NaOH (4.4 vol) was added. After stirring for 15 to 20 minutes, the layers were allowed to separate. The organic phase was then dried ($Na_2SO_4$), filtered, and concentrated to afford crude 5-chloromethyl-2,2-difluoro-1,3-benzodioxole that was used directly in the next step.

(2,2-Difluoro-1,3-benzodioxol-5-yl)-acetonitrile.

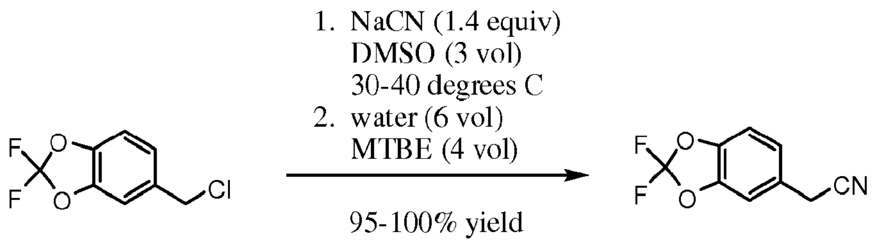


A solution of 5-chloromethyl-2,2-difluoro-1,3-benzodioxole (1 eq) in DMSO (1.25 vol) was added to a slurry of NaCN (1.4 eq) in DMSO (3 vol) maintaining the temperature between 30-40 °C. The mixture was stirred for 1 hour then water (6 vol) was added followed by MTBE (4 vol). After stirring for 30 min, the layers were separated. The aqueous layer was extracted with MTBE (1.8 vol). The combined organic layers were washed with water (1.8 vol), dried ($Na_2SO_4$), filtered, and concentrated to afford crude (2,2-difluoro-1,3-benzodioxol-5-yl)-acetonitrile (95%) that was used directly in the next step. $^1$H NMR (500 MHz, DMSO) δ 7.44 (br s, 1H), 7.43 (d, *J* = 8.4 Hz, 1H), 7.22 (dd, *J* = 8.2, 1.8 Hz, 1H), 4.07 (s, 2H).

(2,2-Difluoro-1,3-benzodioxol-5-yl)-cyclopropanecarbonitrile.

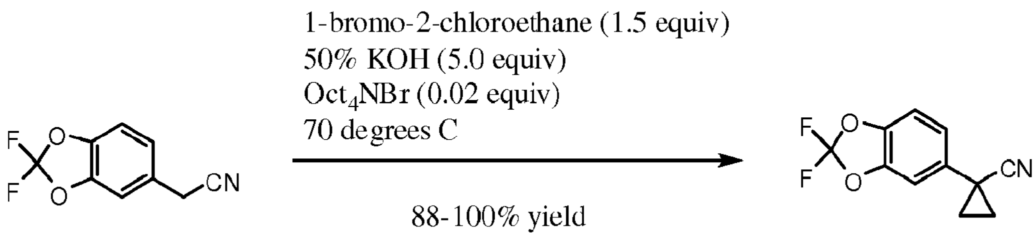


A mixture of (2,2-difluoro-1,3-benzodioxol-5-yl)-acetonitrile (1.0 eq), 50 wt % aqueous KOH (5.0 eq) 1-bromo-2-chloroethane (1.5 eq), and $Oct_4NBr$ (0.02 eq) was heated at 70 °C for 1 h. The reaction mixture was cooled then worked up with MTBE and water. The organic phase was washed with water and brine then the solvent was removed to afford (2,2-difluoro-1,3-benzodioxol-5-yl)-cyclopropanecarbonitrile. $^1$H NMR (500 MHz, DMSO) δ 7.43 (d, *J* = 8.4 Hz, 1H), 7.40 (d, *J* = 1.9 Hz, 1H), 7.30 (dd, J= 8.4, 1.9 Hz, 1H), 1.75 (m, 2H), 1.53 (m, 2H).

1-(2,2-Difluoro-1,3-benzodioxol-5-yl)-cyclopropanecarboxylic Acid.

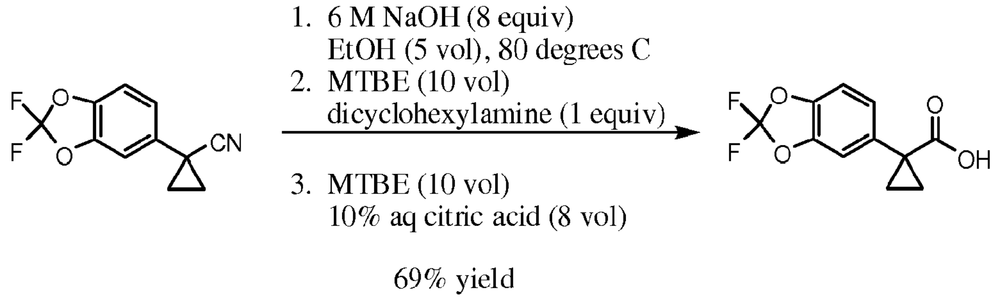


(2,2-difluoro-1,3-benzodioxol-5-yl)-cyclopropanecarbonitrile was hydrolyzed using 6 M NaOH (8 equiv) in ethanol (5 vol) at 80 °C overnight. The mixture was cooled to room temperature and ethanol was evaporated under vacuum. The residue was taken into water and MTBE, 1 M HCl was added and the layers were separated. The MTBE layer was then treated with dicyclohexylamine (0.97 equiv). The slurry was cooled to 0 °C, filtered and washed with heptane to give the corresponding DCHA salt. The salt was taken into MTBE and 10% citric acid and stirred until all solids dissolve. The layers were separated and the MTBE layer was washed with water and brine. Solvent swap to heptane followed by filtration gives 1-(2,2-difluoro-1,3-benzodioxol-5-yl)-cyclopropanecarboxylic acid after drying in a vacuum oven at 50 °C overnight. ESI-MS *m/z* calc. 242.04, found 241.58 $(M+1)^+$; $^1$II NMR (500 MHz, DMSO) δ 12.40 (s, 1H), 7.40 (d, *J* = 1.6 Hz, 1H), 7.30 (d, *J* = 8.3 Hz, 1H), 7.17 (dd, *J*= 8.3, 1.7 Hz, 1H), 1.46 (m, 2H), 1.17 (m, 2H).

Compound 3 Amine Moiety Synthesis

2-Bromo-5-fluoro-4-ntroaniline.

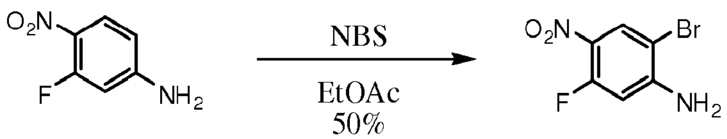


A flask was charged with 3-fluoro-4-nitroaniline (1.0 equiv) followed by ethyl acetate (10 vol) and stirred to dissolve all solids. N-Bromosuccinimide (1.0 equiv) was added portion-wise as to maintain an internal temperature of 22 °C. At the end of the reaction, the reaction mixture was concentrated *in vacuo* on a rotavap. The residue was slurried in distilled water (5 vol) to dissolve and remove succinimide. (The succinimide can also be removed by water workup procedure.) The water was decanted and the solid was slurried in 2-propanol (5 vol) overnight. The resulting slurry was filtered and the wetcake was washed with 2-propanol, dried in vacuum oven at 50 °C overnight with $N_2$ bleed until constant weight was achieved. A yellowish tan solid was isolated (50% yield, 97.5% AUC). Other impurities were a bromo-regioisomer (1.4% AUC) and a di-bromo adduct (1.1% AUC). $^1$H NMR (500 MHz, DMSO) δ 8.19 (1 H, d, *J* = 8.1 Hz), 7.06 (br. s, 2 H), 6.64 (d, 1 H, *J* = 14.3 Hz).

Benzylglycolated-4-ammonium-2-bromo-5-fluoroaniline tosylate salt.

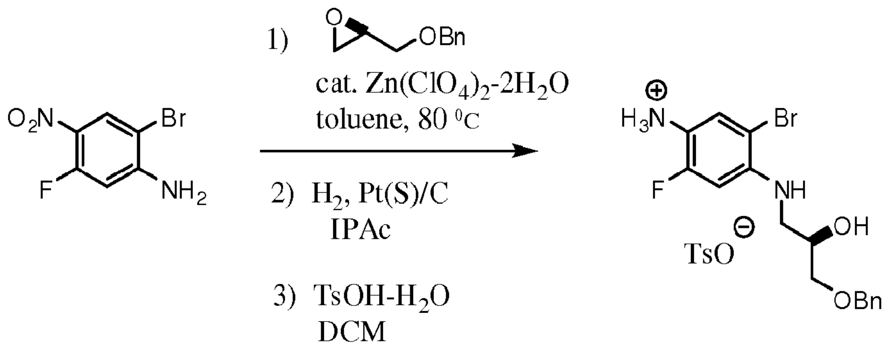


A thoroughly dried flask under $N_2$ was charged with the following: Activated powdered 4 Å molecular sieves (50 wt% based on 2-bromo-5-fluoro-4-nitroaniline), 2-Bromo-5-fluoro-4-nitroaniline (1.0 equiv), zinc perchlorate dihydrate (20 mol%), and toluene (8 vol). The mixture was stirred at room temperature for no more than 30 min. Lastly, (R)-benzyl glycidyl ether (2.0 equiv) in toluene (2 vol) was added in a steady stream. The reaction was heated to 80 °C (internal temperature) and stirred for approximately 7 hours or until 2-Bromo-5-fluoro-4-nitroaniline was <5%AUC.

The reaction was cooled to room temperature and Celite® (50 wt%) was added, followed by ethyl acetate (10 vol). The resulting mixture was filtered to remove Celite® and sieves and washed with ethyl acetate (2 vol). The filtrate was washed with ammonium chloride solution (4 vol, 20% w/v). The organic layer was washed with sodium bicarbonate solution (4 vol x 2.5% w/v). The organic layer was concentrated *in vacuo* on a rotovap. The resulting slurry was dissolved in isopropyl acetate (10 vol) and this solution was transferred to a Buchi hydrogenator.

The hydrogenator was charged with 5wt% Pt(S)/C (1.5 mol%) and the mixture was stirred under $N_2$ at 30 °C (internal temperature). The reaction was flushed with $N_2$ followed by hydrogen. The hydrogenator pressure was adjusted to 1 Bar of hydrogen and the mixture was stirred rapidly (>1200 rpm). At the end of the reaction, the catalyst was filtered through a pad of Celite® and washed with dichloromethane (10 vol). The filtrate was concentrated *in vacuo.* Any remaining isopropyl acetate was chased with dichloromethane (2 vol) and concentrated on a rotavap to dryness.

The resulting residue was dissolved in dichloromethane (10 vol). *p*-Toluenesulfonic acid monohydrate (1.2 equiv) was added and stirred overnight. The product was filtered and washed with dichloromethane (2 vol) and suction dried. The wetcake was transferred to drying trays and into a vacuum oven and dried at 45 °C with $N_2$ bleed until constant weight was achieved. Benzylglycolated-4-ammonium-2-bromo-5-fluoroanilinc tosylate salt was isolated as an off-white solid.

(3-Chloro-3-methylbut-1-ynyl)trimethylsilane.

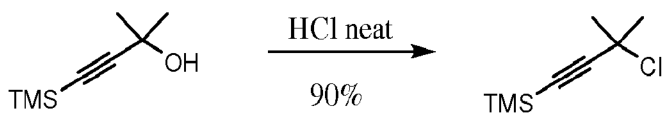


Propargyl alcohol (1.0 equiv) was charged to a vessel. Aqueous hydrochloric acid (37%, 3.75 vol) was added and stirring begun. During dissolution of the solid alcohol, a modest endotherm (5-6 °C) was observed. The resulting mixture was stirred overnight (16 h), slowly becoming dark red. A 30 L jacketed vessel was charged with water (5 vol) which was then cooled to 10 °C. The reaction mixture was transferred slowly into the water by vacuum, maintaining the internal temperature of the mixture below 25 °C. Hexanes (3 vol) was added and the resulting mixture was stirred for 0.5 h. The phases were settled and the aqueous phase (pH < 1) was drained off and discarded. The organic phase was concentrated *in vacuo* using a rotary evaporator, furnishing the product as red oil.

(4-(Benzyloxy)-3,3-dimethylbut-1-ynyl)trimethylsilane.

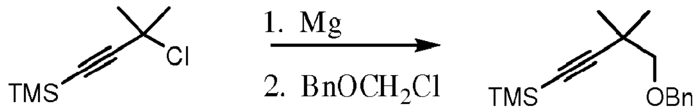


Method A

All equivalent and volume descriptors in this part are based on a 250g reaction. Magnesium turnings (69.5 g, 2.86 mol, 2.0 equiv) were charged to a 3 L 4-neck reactor and stirred with a magnetic stirrer under nitrogen for 0.5 h. The reactor was immersed in an ice-water bath. A solution of the propargyl chloride (250 g, 1.43 mol, 1.0 equiv) in THF (1.8 L, 7.2 vol) was added slowly to the reactor, with stirring, until an initial exotherm (about 10 °C) was observed. The Grignard reagent formation was confirmed by IPC using $^1$H-NMR spectroscopy. Once the exotherm subsided, the remainder of the solution was added slowly, maintaining the batch temperature <15 °C. The addition required about 3.5 h. The resulting dark green mixture was decanted into a 2 L capped bottle.

All equivalent and volume descriptors in this part are based on a 500g reaction. A 22 L reactor was charged with a solution of benzyl chloromethyl ether (95%, 375 g, 2.31 mol, 0.8 equiv) in THF (1.5 L, 3 vol). The reactor was cooled in an ice-water bath. Two of the four Grignard reagent batches prepared above were combined and then added slowly to the benzyl chloromethyl ether solution *via* an addition funnel, maintaining the batch temperature below 25 °C. The addition required 1.5 h. The reaction mixture was stirred overnight (16 h).

All equivalent and volume descriptors in this part are based on a 1 kg reaction. A solution of 15% ammonium chloride was prepared in a 30 L jacketed reactor (1.5 kg in 8.5 kg of water, 10 vol). The solution was cooled to 5 °C. The two Grignard reaction mixtures above were combined and then transferred into the ammonium chloride solution *via* a header vessel. An exotherm was observed in this quench, which was carried out at a rate such as to keep the internal temperature below 25 °C. Once the transfer was complete, the vessel jacket temperature was set to 25 °C. Hexanes (8 L, 8 vol) was added and the mixture was stirred for 0.5 h. After settling the phases, the aqueous phase (pH 9) was drained off and discarded. The remaining organic phase was washed with water (2 L, 2 vol). The organic phase was concentrated *in vacuo* using a 22 L rotary evaporator, providing the crude product as an orange oil.

Method B

Magnesium turnings (106 g, 4.35 mol, 1.0 eq) were charged to a 22 L reactor and then suspended in THF (760 mL, 1 vol). The vessel was cooled in an ice-water bath such that the batch temperature reached 2 °C. A solution of the propargyl chloride (760 g, 4.35 mol, 1.0 equiv) in THF (4.5 L, 6 vol) was added slowly to the reactor. After 100 mL was added, the addition was stopped and the mixture stirred until a 13 °C exotherm was observed, indicating the Grignard reagent initiation. Once the exotherm subsided, another 500 mL of the propargyl chloride solution was added slowly, maintaining the batch temperature <20 °C. The Grignard reagent formation was confirmed by IPC using $^1$H-NMR spectroscopy. The remainder of the propargyl chloride solution was added slowly, maintaining the batch temperature <20 °C. The addition required about 1.5 h. The resulting dark green solution was stirred for 0.5 h. The Grignard reagent formation was confirmed by IPC using $^1$H-NMR spectroscopy. Neat benzyl chloromethyl ether was charged to the reactor addition funnel and then added dropwise into the reactor, maintaining the batch temperature below 25 °C. The addition required 1.0 h. The reaction mixture was stirred overnight. The aqueous work-up and concentration was carried out using the same procedure and relative amounts of materials as in Method A to give the product as an orange oil.

Benzyloxy-3,3-dimethylbut-1-yne.

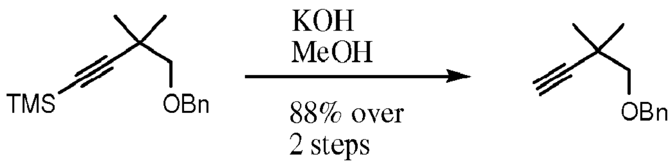


A 30 L jacketed reactor was charged with methanol (6 vol) which was then cooled to 5 °C. Potassium hydroxide (85%, 1.3 equiv) was added to the reactor. A 15-20 °C exotherm was observed as the potassium hydroxide dissolved. The jacket temperature was set to 25 °C. A solution of 4-benzyloxy-3,3-dimethyl-1-trimethylsilylbut-1-yne (1.0 equiv) in methanol (2 vol) was added and the resulting mixture was stirred until reaction completion, as monitored by HPLC.

Typical reaction time at 25 °C was 3-4 h. The reaction mixture was diluted with water (8 vol) and then stirred for 0.5 h. Hexanes (6 vol) was added and the resulting mixture was stirred for 0.5 h. The phases were allowed to settle and then the aqueous phase (pH 10-11) was drained off and discarded. The organic phase was washed with a solution of KOH (85%, 0.4 equiv) in water (8 vol) followed by water (8 vol). The organic phase was then concentrated down using a rotary evaporator, yielding the title material as a yellow-orange oil. Typical purity of this material was in the 80% range with primarily a single impurity present. $^1$H NMR (400 MHz, $C_6D_6$) δ 7.28 (d, 2 H, *J* = 7.4 Hz), 7.18 (t, 2 H, *J* = 7.2 Hz), 7.10 (d, 1H, *J* = 7.2 Hz), 4.35 (s, 2 H), 3.24 (s, 2 H), 1.91 (s, 1 H), 1.25 (s, 6 H).

Benzylglycolated 4-Amino-2-(4-benzyloxy-3,3-dimethylbut-1-ynyl)-5-fluoroaniline.

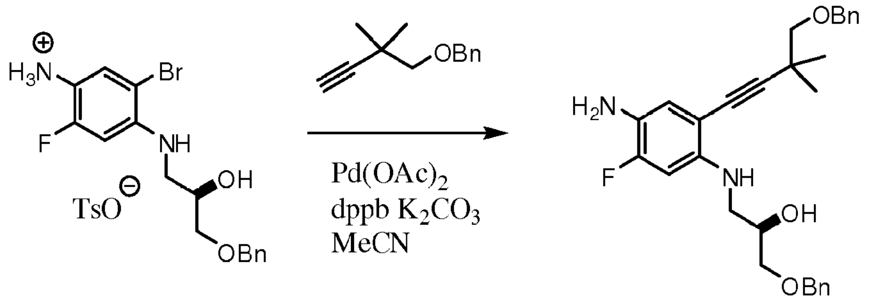


Benzylglocolated 4-ammonium-2-bromo-5-flouroaniline tosylate salt was freebased by stirring the solid in EtOAc (5 vol) and saturated $NaHCO_3$ solution (5 vol) until a clear organic layer was achieved. The resulting layers were separated and the organic layer was washed with saturated $NaHCO_3$ solution (5 vol) followed by brine and concentrated *in vacuo* to obtain benzylglocolated 4-ammonium-2-bromo-5-flouroaniline tosylate salt as an oil.

Then, a flask was charged with benzylglocolated 4-ammonium-2-bromo-5-flouroaniline tosylate salt (freebase, 1.0 equiv), Pd(OAc) (4.0 mol%), dppb (6.0 mol%) and powdered $K_2CO_3$ (3.0 equiv) and stirred with acetonitrile (6 vol) at room temperature. The resulting reaction mixture was degassed for approximately 30 min by bubbling in $N_2$ with vent. Then 4-benzyloxy-3,3-dimethylbut-1-yne (1.1 equiv) dissolved in acetonitrile (2 vol) was added in a fast stream and heated to 80 °C and stirred until complete consumption of 4-ammonium-2-bromo-5-flouroaniline tosylate salt was achieved. The reaction slurry was cooled to room temperature and filtered through a pad of Celite® and washed with acetonitrile (2 vol). Filtrate was concentrated *in vacuo* and the residue was redissolved in EtOAc (6 vol). The organic layer was washed twice with $NH_4Cl$ solution (20% w/v, 4 vol) and brine (6 vol). The resulting organic layer was concentrated to yield brown oil and used as is in the next reaction.

*N*-Benzylglycolated-5-amino-2-(2-benzyloxy-1,1-dimethylethyl)-6-fluoroindole.

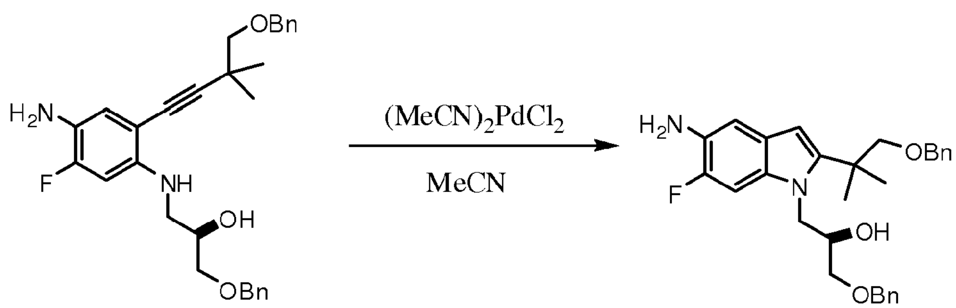


Crude oil of benzylglycolated 4-amino-2-(4-benzyloxy-3,3-dimethylbut-1-ynyl)-5-fluoroanilinc was dissolved in acctonitrilc (6 vol) and added $(MeCN)_2PdCl_2$ (15 mol%) at room temperature. The resulting mixture was degassed using $N_2$ with vent for approximately 30 min. Then the reaction mixture was stirred at 80 °C under $N_2$ blanket overnight. The reaction mixture was cooled to room temperature and filtered through a pad of Celite® and washed the cake with acetonitrile (1 vol). The resulting filtrate was concentrated *in vacuo* and redissolved in EtOAc (5 vol). Deloxan-II® THP (5 wt% based on the theoretical yield of *N*-benzylglycolated-5-amino-2-(2-benzyloxy-1,1-dimethylethyl)-6-fluoroindole)

was added and stirred at room temperature overnight. The mixture was then filtered through a pad of silica (2.5 inch depth, 6 inch diameter filter) and washed with EtOAc (4 vol). The filtrate was concentrated down to a dark brown residue, and used as is in the next reaction.

Repurification of crude *N*-benzylglycolated-5-amino-2-(2-benzyloxy-1,1-dimethylethyl)-6-fluoroindole:

The crude *N*-benzylglycolated-5-amino-2-(2-benzyloxy-1,1-dimethylethyl)-6-fluoroindole was dissolved in dichloromethane (about 1.5 vol) and filtered through a pad of silica initially using 30% EtOAc/heptane where impurities were discarded. Then the silica pad was washed with 50% EtOAc/heptane to isolate *N*-benzylglycolated-5-amino-2-(2-benzyloxy-1,1-dimethylethyl)-6-fluoroindole until faint color was observed in the filtrate. This filtrate was concentrated in vacuo to afford brown oil which crystallized on standing at room temperature. $^1H$ NMR (400 MHz, DMSO) $\delta$ 7.38-7.34 (m, 4 H), 7.32-7.23 (m, 6 H), 7.21 (d, 1 H, *J* = 12.8 Hz), 6.77 (d, 1H, *J* = 9.0 Hz), 6.06 (s, 1 H), 5.13 (d, 1H, *J* = 4.9 Hz), 4.54 (s, 2 H), 4.46 (br. s, 2 H), 4.45 (s, 2 H), 4.33 (d, 1 H, *J* = 12.4 Hz), 4.09-4.04 (m, 2 H), 3.63 (d, 1H, J = 9.2 Hz), 3.56 (d, 1H, J = 9.2 Hz), 3.49 (dd, 1H, *J* = 9.8, 4.4 Hz), 3.43 (dd, 1H, J = 9.8, 5.7 Hz), 1.40 (s, 6 H).

Synthesis of Compound 3.

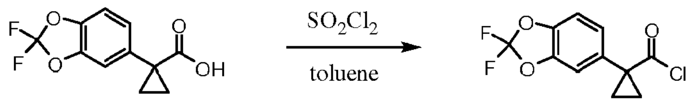


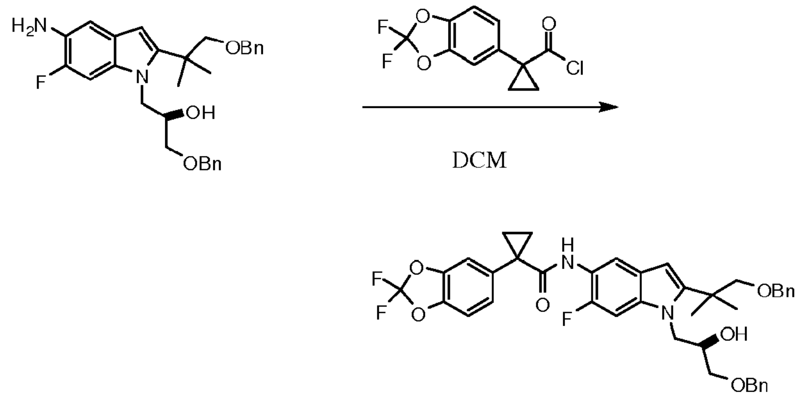


1-(2,2-Difluoro-1,3-benzodioxol-5-yl)-cyclopropanecarboxylic acid (1.3 cquiv) was slurried in toluene (2.5 vol, based on 1-(2,2-difluoro-1,3-benzodioxol-5-yl)-cyclopropanecarboxylic acid) and the mixture was heated to 60 °C. $SOCl_2$ (1.7 equiv) was added via addition funnel. The resulting mixture was stirred for 2 h. The toluene and the excess $SOCl_2$ were distilled off using rotavop. Additional toluene (2.5 vol, based on 1-(2,2-difluoro-1,3-benzodioxol-5-yl)-cyclopropanecarboxylic acid) was added and distilled again. The crude acid chloride was dissolved in dichloromethane (2 vol) and added via addition funnel to a mixture of *N*-benzylglycolated-5-amino-2-(2-benzyloxy-1,1-dimethylethyl)-6-fluoroindole (1.0 equiv), and triethylamine (2.0 equiv) in dichloromethane (7 vol) while maintaining 0-3 °C (internal temperature). The resulting mixture was stirred at 0 °C for 4 h and then warmed to room temperature overnight. Distilled water (5 vol) was added to the reaction mixture and stirred for no less than 30 min and the layers were separated. The organic phase was washed with 20 wt% $K_2CO_3$ (4 vol x 2) followed by a brine wash (4 vol) and concentrated to afford crude benzyl protected Compound 2 as a thick brown oil, which was purified further using silica pad filtration.

Silica gel pad filtration: Crude benzyl protected Compound 3 was dissolved in ethyl acetate (3 vol) in the presence of activated carbon Darco-G (10wt%, based on theoretical yield of benzyl protected Compound 3) and stirred at room temperature overnight. To this mixture was added heptane (3 vol) and filtered through a pad of silica gel (2x weight of crude benzyl protected Compound 3). The silica pad was washed with ethyl acetate/heptane (1:1, 6 vol) or until little color was detected in the filtrate. The filtrate was concentrated *in vacuo* to afford benzyl protected Compound 3 as viscous reddish brown oil, and used directly in the next step.

Repurification: Benzyl protected Compound 3 was redissolved in dichloromethane (1 vol, based on theoretical yield of benzyl protected Compound 3) and loaded onto a silica gel pad (2x weight of crude benzyl protected Compound 3). The silica pad was washed with dichloromethane (2 vol, based on theoretical yield of benzyl protected Compound

3) and the filtrate was discarded. The silica pad was washed with 30% ethyl acetate/heptane (5 vol) and the filtrate was concentrated *in vacuo* to afford benzyl protected Compound 3 as viscous reddish orange oil, and used directly in the next step.

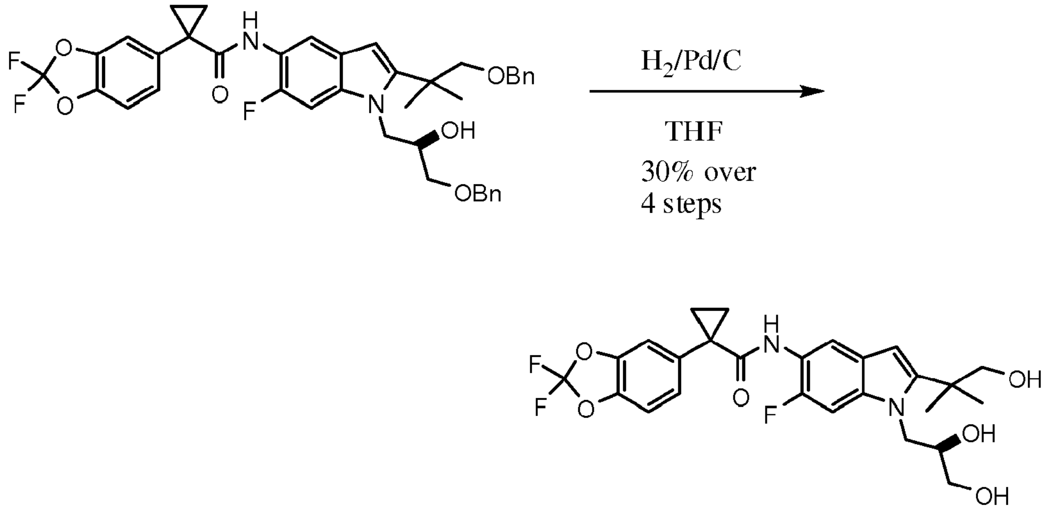


Method A

A 20 L autoclave was flushed three times with nitrogen gas and then charged with palladium on carbon (Evonik E 101 NN/W, 5% Pd, 60% wet, 200 g, 0.075 mol, 0.04 equiv). The autoclave was then flushed with nitrogen three times. A solution of crude benzyl protected Compound 3 (1.3 kg, about 1.9 mol) in THF (8 L, 6 vol) was added to the autoclave *via* suction. The vessel was capped and then flushed three times with nitrogen gas. With gentle stirring, the vessel was flushed three times with hydrogen gas, evacuating to atmosphere by diluting with nitrogen. The autoclave was pressurized to 3 Bar with hydrogen and the agitation rate was increased to 800 rpm. Rapid hydrogen uptake was observed (dissolution). Once uptake subsided, the vessel was heated to 50 °C.

For safety purposes, the thermostat was shut off at the end of every work-day. The vessel was pressurized to 4 Bar with hydrogen and then isolated from the hydrogen tank.

After 2 full days of reaction, more Pd / C (60 g, 0.023 mol, 0.01 equiv) was added to the mixture. This was done by flushing three times with nitrogen gas and then adding the catalyst through the solids addition port. Resuming the reaction was done as before. After 4 full days, the reaction was deemed complete by HPLC by the disappearance of not only the starting material, but also the peak corresponding to a mono-benzylated intermediate.

The reaction mixture was filtered through a Celite® pad. The vessel and filter cake were washed with THF (2 L, 1.5 vol). The Celite® pad was then wetted with water and the cake discarded appropriately. The combined filtrate and THF wash were concentrated using a rotary evaporator yielding the crude product as a black oil, 1 kg.

The equivalents and volumes in the following purification are based on 1 kg of crude material. The crude black oil was dissolved in 1:1 ethyl acetate-heptane. The mixture was charged to a pad of silica gel (1.5 kg, 1.5 wt. equiv) in a fritted funnel that had been saturated with 1:1 ethyl acetate-heptane. The silica pad was flushed first with 1:1 ethyl acetate-heptane (6 L, 6 vol) and then with pure ethyl acetate (14 L, 14 vol). The eluent was collected in 4 fractions that were analyzed by HPLC.

The equivalents and volumes in the following purification are based on 0.6 kg of crude material. Fraction 3 was concentrated by rotary evaporation to give a brown foam (600 g) and then redissolved in MTBE (1.8 L, 3 vol). The dark brown solution was stirred overnight at ambient temperature, during which time, crystallization occurred. Heptane (55 mL, 0.1 vol) was added and the mixture was stirred overnight. The mixture was filtered using a Buchner funnel and the filter cake was washed with 3:1 MTBE-heptane (900 mL, 1.5 vol). The filter cake was air-dried for 1 h and then vacuum dried at ambient temperature for 16 h, furnishing 253 g of Compound 3 as an off-white solid.

The equivalents and volumes for the following purification are based on 1.4 kg of crude material. Fractions 2 and 3 from the above silica gel filtration as well as material from a previous reaction were combined and concentrated to give 1.4 kg of a black oil. The mixture was resubmitted to the silica gel filtration (1.5 kg of silica gel, eluted with 3.5 L, 2.3 vol of 1:1 ethyl acetate-heptane then 9 L, 6 vol of pure ethyl acetate) described above, which upon concentration gave a tan foamy solid (390 g).

The equivalents and volumes for the following purification are based on 390 g of crude material. The tan solid was insoluble in MTBE, so was dissolved in methanol (1.2 L, 3 vol). Using a 4 L Morton reactor equipped with a long-path distillation head, the mixture was distilled down to 2 vol. MTBE (1.2 L, 3 vol) was added and the mixture was distilled back down to 2 vol. A second portion of MTBE (1.6 L, 4 vol) was added and the mixture was distilled back down to 2 vol. A third portion of MTBE (1.2 L, 3 vol) was added and the mixture was distilled back down to 3 vol. Analysis of the distillate by GC revealed it to consist of about 6% methanol. The thermostat was set to 48 °C (below the boiling temp of the MTBE-methanol azeotrope, which is 52 °C). The mixture was cooled to 20 °C over 2 h, during which time a relatively fast crystallization occurred. After stirring the mixture for 2 h, heptane (20 mL, 0.05 vol) was added and the mixture was stirred overnight (16 h). The mixture was filtered using a Buchner funnel and the filter cake was washed with 3:1 MTBE-heptane (800 mL, 2 vol). The filter cake was air-dried for 1 h and then vacuum dried at ambient temperature for 16 h, furnishing 130 g of Compound 3 as an off-white solid.

Method B

Benzyl protected Compound 3 was dissolved and flushed with THF (3 vol) to remove any remaining residual solvent. Benzyl protected Compound 3 was redissolved in THF (4 vol) and added to the hydrogenator containing 5 wt% Pd/C (2.5 mol%, 60% wet, Degussa E5 E101 NN/W). The internal temperature of the reaction was adjusted to 50 °C, and flushed with $N_2$ (x5) followed by hydrogen (x3). The hydrogenator pressure was adjusted to 3 Bar of hydrogen and the mixture was stirred rapidly (>1100 rpm). At the end of the reaction, the catalyst was filtered through a pad of Celite® and washed with THF (1 vol). The filtrate was concentrated *in vacuo* to obtain a brown foamy residue. The resulting residue was dissolved in MTBE (5 vol) and 0.5N HCl solution (2 vol) and distilled water (1 vol) were added. The mixture was stirred for no less than 30 min and the resulting layers were separated. The organic phase was washed with 10wt% $K_2CO_3$ solution (2 vol x2) followed by a brine wash. The organic layer was added to a flask containing silica gel (25 wt%), Deloxan-II® THP (5wt%, 75% wet), and $Na_2SO_4$ and stirred overnight. The resulting mixture was filtered through a pad of Celite® and washed with 10%THF/MTBE (3 vol). The filtrate was concentrated *in vacuo* to afford crude Compound 3 as a pale tan foam.

Recovery of Compound 3 Mother Liquor:

Option A.

Silica gel pad filtration: The mother liquor was concentrated *in* vacuo to obtain a brown foam, dissolved in dichloromethane (2 vol), and filtered through a pad of silica (3x weight of the crude Compound 3). The silica pad was washed with ethyl acetate/heptane (1:1, 13 vol) and the filtrate was discarded. The silica pad was washed with 10% THF/ethyl acetate (10 vol) and the filtrate was concentrated *in vacuo* to afford Compound 3 as pale tan foam. The above crystallization procedure was followed to isolate the remaining Compound 3.

Option B.

Silica gel column chromatography: After chromatography on silica gel (50% ethyl acetate/hexanes to 100% ethyl acetate), the desired compound was isolated as pale tan foam. The above crystallization procedure was followed to isolate the remaining Compound 3.
Compound 3 may also be prepared by one of several synthetic routes disclosed in US published patent application US 2009/0131492, incorporated herein by reference.

Table 3-1: Physical Data for Compound 3.

| Cmpd. No. | LC/MS M+1 | LC/RT min | NMR |
|---|---|---|---|
| 3 | 521.5 | 1.69 | 1H NMR (400.0 MHz, $CD_3CN$) d 7.69 (d, J = 7.7 Hz, 1H), 7.44 (d, J = 1.6 Hz, 1H), 7.39 (dd, J = 1.7, 8.3 Hz, 1H), 7.31 (s, 1H), 7.27 (d, J = 8.3 Hz, 1H), 7.20 (d, J = 12.0 Hz, 1H), 6.34 (s, 1H), 4.32 (d, J = 6.8 Hz, 2H), 4.15 - 4.09 (m, 1H), 3.89 (dd, J = 6.0, 11.5 Hz, 1H), 3.63 - 3.52 (m, 3H), 3.42 (d, J = 4.6 Hz, 1H), 3.21 (dd, J = 6.2, 7.2 Hz, 1H), 3.04 (t, J = 5.8 Hz, 1H), 1.59 (dd, J = 3.8, 6.8 Hz, 2H), 1.44 (s, 3H), 1.33 (s, 3H) and 1.18 (dd, J = 3.7, 6.8 Hz, 2H) ppm. |

II.D.1 ENaC INHIBITORS

The present invention is directed to pharmaceutical compositions comprising an CF modulator component as

provided by Columns A-C in Table I and at least one ENaC inhibitor as provided in Column D of Table I. The invention also provides methods for treating CF and other chronic diseases, methods for preparing the compositions and methods for using the compositions for the treatment of CF and other chronic diseases, including chronic diseases involving regulation of fluid volumes across epithelial membranes, using compositions containing an CF Modulator modulator compound and ENaC inhibitor compounds.

In other embodiments, the ENaC inhibitors of Column D compounds can include ENaC inhibitors of Formula IV which are fully described and exemplified in International Patent Application No. PCT/EP2008/067110 filed: 12/09/2008 and is Assigned to Novartis AG. All of the compounds recited in PCT/EP2008/067110, are useful in the present invention and the compounds and methods for making such compounds are hereby incorporated into the present disclosure in their entirety.

In some embodiments, in addition to compounds of formula IV, ENaC inhibitors can also include camostat (a trypsin-like protease inhibitor), QAU145, 552-02, GS-9411, INO-4995, aerolytic, amiloride, benzamil, dimethyl-amiloride, and ENaC inhibitor compounds disclosed in International Applications: PCT/EP2006/003387 filed October 19, 2006; PCT/EP2006/012314 filed June 28, 2007 and PCT/EP2006/012320 filed June 28, 2007. All of these International Patent Application disclosures are hereby incorporated by reference herein in their entireties. In some embodiments, the ENaC inhibitor is amiloride. Methods for determining whether a compound is an ENaC inhibitor are known in the art and can be used to identify an ENaC inhibitor that can be used in the combination with CF modulator component described herein.

II.D.2 COMPOUNDS OF FORMULA IV

In one aspect, the invention provides ENaC inhibitor compounds according to Formula IV:

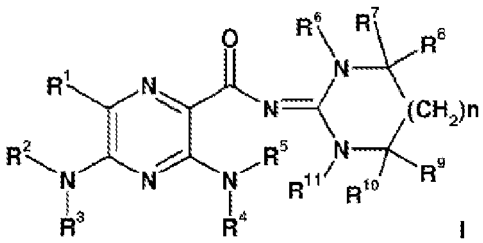

I

Formula IV.

or solvates, hydrates or pharmaceutically acceptable salts thereof, wherein $R^1$ is H, halogen, $C_1$-$C_8$-alkyl, $C_1$-$C_8$-haloalkyl, $C_1$-$C_8$-haloalkoxy, $C_3C_{15}$-carbocyclic group, nitro, cyano, a $C_6$-$C_{15}$-membered aromatic carbocyclic group, or a $C_1$-$C_8$-alkyl substituted by a $C_6$-$C_{15}$-membered aromatic carbocyclic group;

$R^2$, $R^3$, $R^4$ and $R^5$ are each independently selected from H and $C_1$-$C_6$ alkyl;

$R^6$, $R^7$, $R^8$, $R^9$, $R^{10}$ and $R^{11}$ are each independently selected from H; $SO_2R^{16}$; aryl optionally substituted by one or more Z groups; a $C_3$-$C_{10}$ carbocyclic group optionally substituted by one or more Z groups; $C_3$-$C_{14}$ heterocyclic group optionally substituted by one or more Z groups; $C_1$-$C_8$ alkyl optionally substituted by an aryl group which is optionally substituted by one or more Z groups, a $C_3$-$C_{10}$ carbocyclic group optionally substituted by one or more Z groups or a $C_3$-$C_{14}$ heterocyclic group optionally substituted by one or more Z groups; or is represented by the formula 2:

$-(C_0$-$C_6$ alkylene)-A-($C_0$-$C_6$ alkylene)-B-$(X$-$R^{12})_q$-$R^{22}$, wherein the alkylene groups are optionally substituted by one or more Z groups;

or $R^6$ and $R^7$ together with the atoms to which they are attached form a 3- to 10-membered heterocyclic group, the heterocyclic group including one or more further heteroatoms selected from N, O and S, and the heterocyclic group being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2;

or $R^7$ and $R^8$ together with the carbon atom to which they are attached form a 3- to 10-membered carbocyclic or a 3- to 10-membered heterocyclic group, the heterocyclic group including one or more heteroatoms selected from N, O and S, and the carbocyclic and heterocyclic groups being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2;

or $R^9$ and $R^{10}$ together with the carbon atom to which they are attached form a 3- to 10-membered carbocyclic or a 3- to 10-membered heterocyclic group, the heterocyclic group including one or more heteroatoms selected from

N, O and S, and the carbocyclic and heterocyclic groups being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2;

or $R^8$ and $R^9$ together with the carbon atoms to which they are attached form a 3- to 10-membered cycloalkyl or a 3- to 10-membered heterocyclic group, the heterocyclic group including one or more heteroatoms selected from N, O and S, and the carbocyclic and heterocyclic groups being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2;

or $R^{10}$ and $R^{11}$ together with the atoms to which they are attached form a 3- to 10-membered heterocyclic group, the heterocyclic group including one or more further heteroatoms selected from N, O and S, and the heterocyclic group being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2;

A is selected from a bond, $-NR^{13}(SO_2)-$, $-(SO_2)NR^{13}-$, $-(SO_2)-$, $-NR^{13}C(O)-$, $-C(O)NR^{13}-$, $-NR^{13}C(O)NR^{14}-$, $-NR^{13}C(O)O-$, $-NR^{13}-$, C(O)O, OC(O), C(O), O and S;

B is selected from a bond, -($C_2$-$C_4$ alkenyl group)-, -($C_2$-$C_4$ alkynyl group)-, -NH-, aryl, O-aryl, NH-aryl, a $C_3$-$C_{14}$ carbocyclic group and a 3- to 14-membered heterocyclic group, the heterocyclic group including one or more heteroatoms selected from N, O and S, wherein the aryl, carbocyclic and heterocyclic groups arc each optionally substituted by one or more Z groups;

X is selected from a bond, $-NR^{15}(SO_2)-$, $-(SO_2)NR^{15}-$, $-(SO_2)-$, $-NR^{15}C(O)-$, $-C(O)NR^{15}$, $-NR^{15}C(O)NR^{17}-$, $-NR^{15}C(O)O-$, $-NR^{15}-$, C(O)O, OC(O), C(O), O and S;

$R^{12}$ is selected from $C_1$-$C_8$ alkylene, $C_1$-$C_8$ alkenylene, -$C_3$-$C_8$ cycloalkyl-, -$C_1$-$C_8$ alkylene-$C_3$-$C_8$ cycloalkyl-, and -aryl-, wherein the alkylene, cycloalkyl and aryl groups are optionally substituted by one or more Z groups;

$R^{13}$, $R^{14}$, $R^{15}$ and $R^{17}$ are each independently selected from H and $C_1$-$C_6$ alkyl;

$R^{16}$ is selected from $C_1$-$C_8$ alkyl, aryl and a 3- to 14-membered heterocyclic group, the heterocyclic group including one or more heteroatoms selected from N, O and S;

Z is independently selected from OH, aryl, O-aryl, $C_7$-$C_{14}$ aralkyl, O-$C_7$-$C_{14}$ aralkyl, $C_1$-$C_6$ alkyl, $C_1$-$C_6$ alkoxy, $NR^{19}(SO_2)R^{21}$, $(SO_2)NR^{19}R^{21}$, $(SO_2)R^{20}$, $NR^{19}C(O)R^{20}$, $C(O)NR^{19}R^{20}$, $NR^{19}C(O)NR^{20}R^{18}$, $NR^{19}C(O)OR^{20}$, $NR^{19}R^{21}$, $C(O)OR^{19}$, $C(O)R^{19}$, $SR^{19}$, $OR^{19}$, oxo, CN, $NO_2$, and halogen, wherein the alkyl, alkoxy, aralkyl and aryl groups are each optionally substituted by one or more substituents selected from OH, halogen, $C_1$-$C_4$ haloalkyl and $C_1$-$C_4$ alkoxy;

$R^{18}$ and $R^{20}$ are each independently selected from II and $C_1$-$C_6$ alkyl;

$R^{19}$ and $R^{21}$ are each independently selected from H; $C_1$-$C_8$ alkyl; $C_3$-$C_8$ cycloalkyl; $C_1$-$C_4$ alkoxy-$C_1$-$C_4$ alkyl; ($C_0$-$C_4$ alkyl)-aryl optionally substituted by one or more groups selected from $C_1$-$C_6$ alkyl, $C_1$-$C_6$ alkoxy and halogen; ($C_0$-$C_4$ alkyl)- 3- to 14-membered heterocyclic group, the heterocyclic group including one or more heteroatoms selected from N, O and S, optionally substituted by one or more groups selected from halogen, oxo, $C_1$-$C_6$ alkyl and C(O)$C_1$-$C_6$ alkyl; ($C_0$-$C_4$ alkyl)-O-aryl optionally substituted by one or more groups selected from $C_1$-$C_6$ alkyl, $C_1$-$C_6$ alkoxy and halogen; and ($C_0$-$C_4$ alkyl)-O- 3- to 14-membered heterocyclic group, the heterocyclic group including one or more heteroatoms selected from N, O and S, optionally substituted by one or more groups selected from halogen, $C_1$-$C_6$ alkyl and C(O)$C_1$-$C_6$ alkyl; wherein the alkyl groups are optionally substituted by one or more halogen atoms, $C_1$-$C_4$ alkoxy, $C(O)NH_2$, C(O)NH$C_1$-$C_6$ alkyl or C(O)N($C_1$-$C_6$ alkyl)$_2$; or

$R^{19}$ and $R^{20}$ together with the nitrogen atom to which they attached form a 5- to 10-membered heterocyclic group, the heterocyclic group including one or more further heteroatoms selected from N, O and S, the heterocyclic group being optionally substituted by one or more substituents selected from OH; halogen; aryl; 5- to 10-membered heterocyclic group including one or more heteroatoms selected from N, O and S; $S(O)_2$-aryl; $S(O)_2$-$C_1$-$C_6$ alkyl; $C_1$-$C_6$ alkyl optionally substituted by one or more halogen atoms; $C_1$-$C_6$ alkoxy optionally substituted by one or more OH groups or $C_1$-$C_4$ alkoxy; and C(O)O$C_1$-$C_6$ alkyl, wherein the aryl and heterocyclic substituent groups are themselves optionally substituted by $C_1$-$C_6$ alkyl, $C_1$-$C_6$ haloalkyl or $C_1$-$C_6$ alkoxy;

$R^{22}$ is selected from H, halogen, $C_1$-$C_8$ alkyl, $C_1$-$C_8$ alkoxy, aryl, O-aryl, $S(O)_2$-aryl, $S(O)_2$-$C_1$-$C_6$ alkyl, $S(O)_2NR^{23}R^{24}$, $NHS(O)_2NR^{23}R^{24}$, a $C_3$-$C_{14}$ carbocyclic group, a 3-to 14-membered heterocyclic group, the heterocyclic group including one or more heteroatoms selected from N, O and S, and O-(3- to 14-membered heterocyclic group, the heterocyclic group including one or more heteroatoms selected from N, O and S), wherein the alkyl, aryl, carbocyclic and heterocyclic groups are each optionally substituted by one or more Z groups;

$R^{23}$ and $R^{24}$ are each independently selected from H, $C_1$-$C_8$ alkyl and $C_3$-$C_8$ cycloalkyl; or

$R^{23}$ and $R^{24}$ together with the nitrogen atom to which they are attached form a 5- to 10-membered heterocyclic group, optionally including one or more further heteroatoms selected from N, O and S, wherein the heterocyclic group is optionally substituted by one or more Z groups;

n is 0, 1 or 2;
and p are each independently an integer from 0 to 6; and
q is 0, 1, 2 or 3;
with the proviso that when n is 0, at least one of $R^6$, $R^7$, $R^8$, $R^9$, $R^{10}$ and $R^{11}$ is other than H.

In an embodiment of the invention, there is provided a compound according to the Formula IVa:

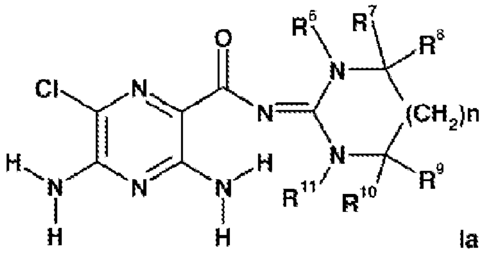

Ia

wherein

$R^6$, $R^7$, $R^8$, $R^9$, $R^{10}$ and $R^{11}$ are each independently selected from H; $SO_2R^{16}$; aryl optionally substituted by one or more Z groups; a $C_3$-$C_{10}$ carbocyclic group optionally substituted by one or more Z groups; $C_3$-$C_{14}$ heterocyclic group optionally substituted by one or more Z groups; $C_1$-$C_8$ alkyl optionally substituted by an aryl group, a $C_3$-$C_{10}$ carbocyclic group optionally substituted by one or more Z groups or a $C_3$-$C_{14}$ heterocyclic group optionally substituted by one or more Z groups; or is represented by the formula 2a:

$$-(CH_2)_o-A-(CH_2)_p-B-(X-R^{12})_q-R^{22};$$

or $R^7$ and $R^8$ together with the carbon atom to which they are attached form a 3- to 7-membered carbocyclic or a 3- to 7-membered heterocyclic group, the heterocyclic group including one or more heteroatoms selected from N, O and S, and the carbocyclic and heterocyclic groups being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2a;
or $R^9$ and $R^{10}$ together with the carbon atom to which they are attached form a 3- to 7-membered carbocyclic or a 3- to 7-membered heterocyclic group, the heterocyclic group including one or more heteroatoms selected from N, O and S, and the carbocyclic and heterocyclic groups being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2a;
or $R^8$ and $R^9$ together with the carbon atoms to which they are attached form a 3- to 7-membered cycloalkyl or a 3- to 7-membered heterocyclic group, the heterocyclic group including one or more heteroatoms selected from N, O and S, and the carbocyclic and heterocyclic groups being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula (IV)2a;
A is selected from a bond, $-NR^{13}(SO_2)-$, $-(SO_2)NR^{13}-$, $-(SO_2)-$, $-NR^{13}C(O)-$, $-(O)NR^{13}$, $-NR^{13}C(O)NR^{14}-$, $-NR^{13}C(O)O-$, $-NR^{13}-$, C(O)O, OC(O), C(O), O and S;
B is selected from a bond, aryl, a $C_3$-$C_{14}$ carbocyclic group and a $C_3$-$C_{14}$ heterocyclic group, wherein the ring systems are optionally substituted by one or more Z groups;
X is selected from a bond, $-NR^{15}(SO_2)-$, $-(SO_2)NR^{15}-$, $-(SO_2)-$, $-NR^{15}C(O)-$, $-C(O)NR^{15}-$, $-NR^{15}C(O)NR^{17}-$, $-NR^{15}C(O)O-$, $-NR^{15}-$, C(O)O, OC(O), C(O), O and S;
$R^{12}$ is selected from H, $C_1$-$C_8$ alkyl, $C_3$-$C_8$ cycloalkyl, $C_1$-$C_8$ alkyl- $C_3$-$C_8$ cycloalkyl, $C_1$-$C_8$ alkyl-aryl and aryl, wherein the alkyl, cycloalkyl and aryl groups are optionally substituted by one or more Z groups;
$R^{13}$, $R^{14}$, $R^{15}$ and $R^{17}$ are each independently selected from H and $C_1$-$C_6$ alkyl;
$R^{16}$ is selected from $C_1$-$C_8$ alkyl, aryl and a 3- to 14-membered heterocyclic group; Z is independently selected from OH, aryl, O-aryl, $C_7$-$C_{14}$ aralkyl, O-$C_7$-$C_{14}$ aralkyl, $C_1$-$C_6$ alkyl, $C_1$-$C_6$ alkoxy, $NR^{19}(SO_2)R^{21}$, $(SO_2)NR^{19}R^{21}$, $(SO_2)R^{20}$, $NR^{19}C(O)R^{20}$, $C(O)NR^{19}R^{20}$, $NR^{19}C(O)NR^{20}R^{18}$, $NR^{19}C(O)OR^{20}$, $NR^{19}R^{21,}$ $C(O)OR^{19}$, $C(O)R^{19}$, $SR^{19}$, $OR^{19}$, oxo, CN, $NO_2$, and halogen, wherein the alkyl, alkoxy, aralkyl and aryl groups are each optionally substituted by one or more substituents selected from OH, halogen, $C_1$-$C_4$ haloalkyl and $C_1$-$C_4$ alkoxy;
$R^{18}$, $R^{19}$ and $R^{20}$ are each independently selected from H and $C_1$-$C_6$ alkyl;

$R^{21}$ is selected from $C_1$-$C_8$ alkyl, aryl and a 3- to 14-membered heterocyclic group;
$R^{22}$ is selected from H and $C_1$-$C_8$ alkyl;
n is 0, 1 or 2;
and p are each independently an integer from 0 to 6; and
q is 0, 1, 2 or 3;
with the proviso that when n is 0, at least one of $R^6$, $R^7$, $R^8$, $R^9$, $R^{10}$ and $R^{11}$ is other than H.

In a further embodiment of the invention as defined anywhere above, $R^6$ is selected from H, $C_1$-$C_3$ alkyl and $(CH_2)_d$-phenyl, where the phenyl group is optionally substituted by $OR^{23}$;

$R^{23}$ is H or $C_1$-$C_6$ alkyl; and
d is an integer from 1 to 5 (optionally 2 to 4).

In a still further embodiment of the invention as defined anywhere above, $R^7$ is H or $C_1$-$C_6$; and

$R^8$ is selected from H, $C_1$-$C_6$ alkyl; $(CH_2)_e$phenyl, where the phenyl group is optionally substituted by one or more groups selected from halo and $OR^{24}$; $(CH_2)_fCOOR^{25}$; $(CH_2)_gOC_1$-$C_6$ alkyl, where the alkyl group is optionally substituted by 1 to 3 groups selected from OH, $C_1$-$C_3$ alkyl and phenyl; and $(CH_2)_hNHCO_2(CH_2)_i$phenyl;
$R^{24}$ is H or $C_1$-$C_6$ alkyl, where the alkyl group is optionally substituted by 1 to 3 groups selected from OH and $OC_1$-$C_3$ alkyl;
$R^{25}$ is H or $C_1$-$C_3$ alkyl;
e is 0, 1, 2, 3, 4 or 5 (optionally 0, 1, 2, 3 or 4);
f, g and h are each independently an integer from 1 to 4; and
i is 1 or 2;
or $R^7$ and $R^8$ together with the carbon atom to which they attached form a 5- or 6-membered non-aromatic carbocyclic ring system or a 5- or 6- membered non-aromatic heterocyclic ring system containing one or more heteroatoms selected from N, O and S, the ring systems being optionally substituted by one or more Z groups; $SO_2R^{16}$; $C_6$-$C_{15}$-aromatic carbocyclic group optionally substituted by one or more Z groups; a $C_3$-$C_{10}$ carbocyclic group; a $C_3$-$C_{14}$ heterocyclic group optionally substituted by one or more Z groups; or a group represented by the formula 2 or 2a. Suitably, the ring system defined by $R^7$, $R^8$ and the carbon to which they are attached is optionally substituted by $C_1$-$C_3$ alkyl, halo or benzyl.

Optionally, f is 2 or 3. Additionally or alternatively, g may be 2 or 3. Additionally or alternatively, h may be 2, 3 or 4. Additionally or alternatively, i may be 1. In the immediately preceding sub-definitions of f, g, h and i, each sub-definition may be combined with more other sub-definitions or they may be combined with the definitions for the relevant variables given above.

In a yet further embodiment of the invention as defined anywhere above, $R^9$ is H, $C_1$-$C_6$ alkyl or phenyl; or $R^8$ and $R^9$ together with the carbon atoms to which they attached form a 5-, 6- or 7-membered non-aromatic carbocyclic ring system or a 5-, 6- or 7- membered non-aromatic heterocyclic ring system containing one or more heteroatoms selected from N, O and S, the ring systems being optionally substituted by $C_1$-$C_3$ alkyl, halo or benzyl.

In a further embodiment of the invention as defined anywhere above, $R^{11}$ is H, $SO_2C_1$-$C_6$ alkyl or $SO_2$phenyl.

In a further embodiment of the invention as defined anywhere above, $R^6$ and R" are both H.

A further embodiment of the invention provides a compound according to the formula IV(1b):

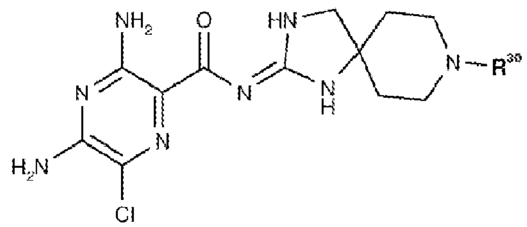

or the formula IV(1c):

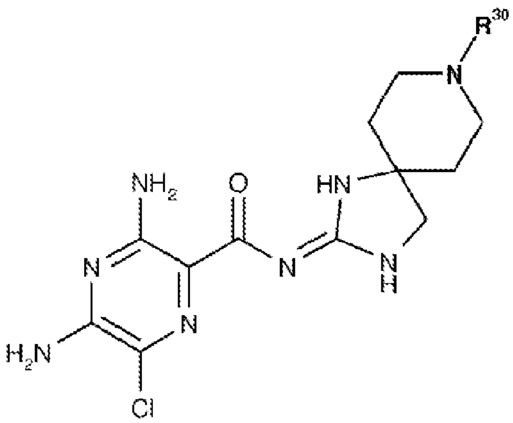

wherein $R^{30}$ is $-A-(C_0-C_6$ alkylene$)-B-(X-R^{12})_q-R^{22}$
and A, B, X, $R^{12}$, q and $R^{22}$ are as defined anywhere herein.
A further aspect of the invention provides a compound of Formula IV(2)

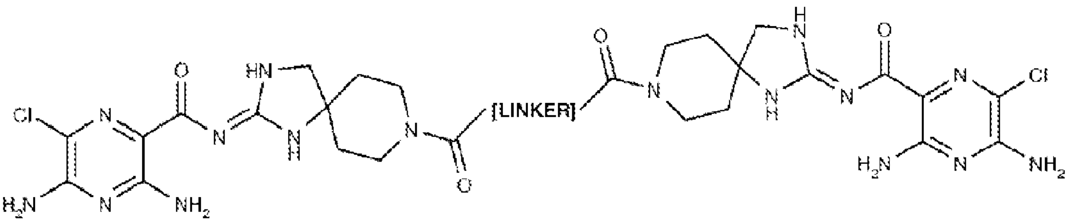

2

Formula IV(2)

Table II.D-1 Exemplary Compounds of Formula IV

| | |
|---|---|
| 1 | 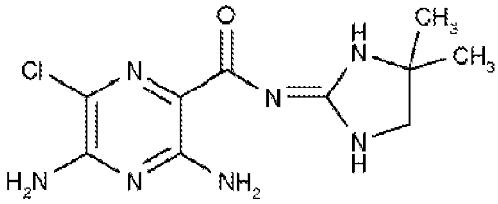 |
| 2 | 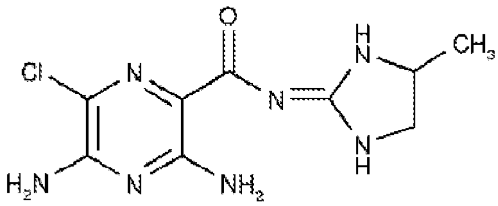 |
| 3 | 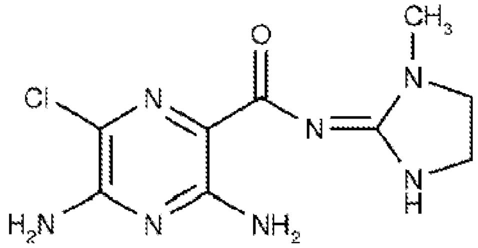 |

(continued)

4

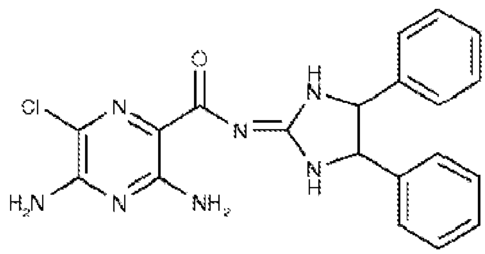

5

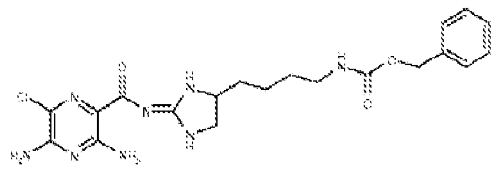

6

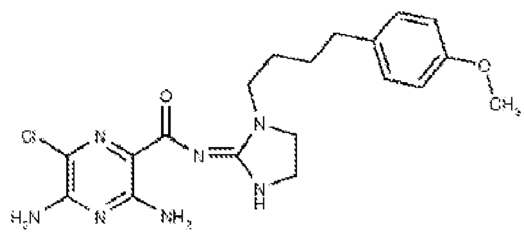

7

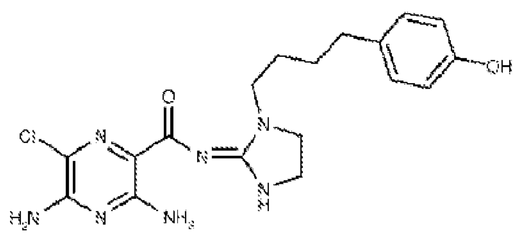

8

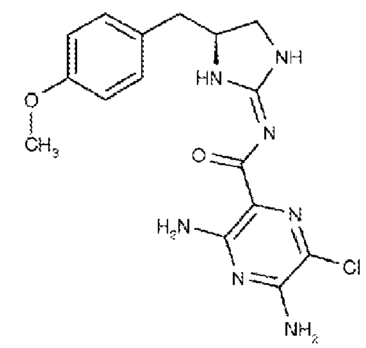

9

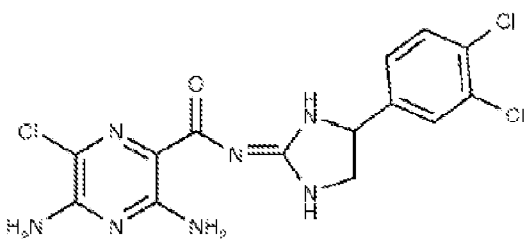

10

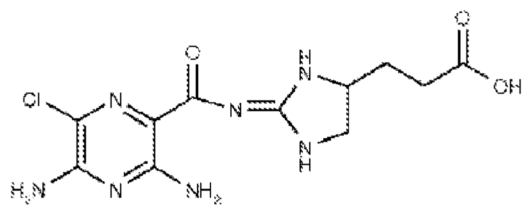

(continued)

11

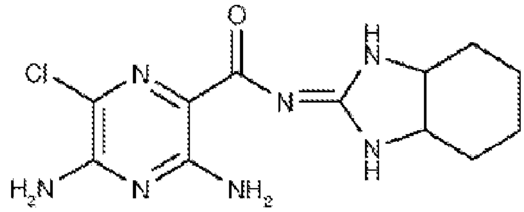

12

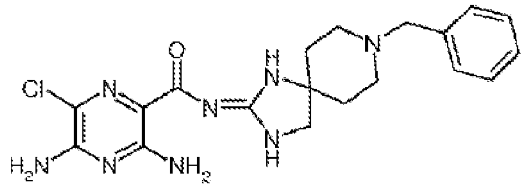

13

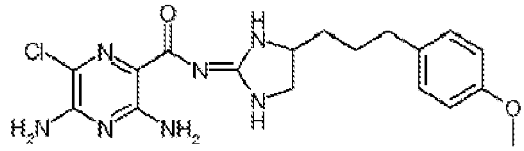

14

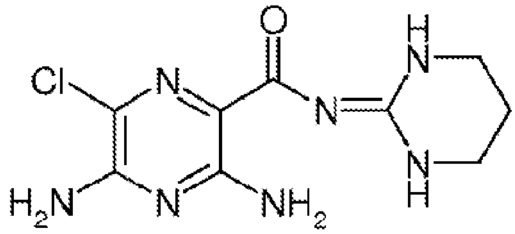

15

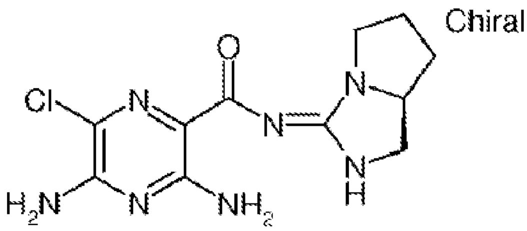


16

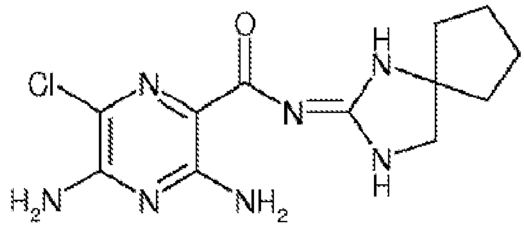

17

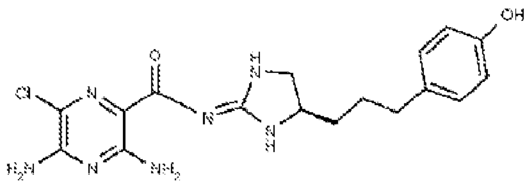

18

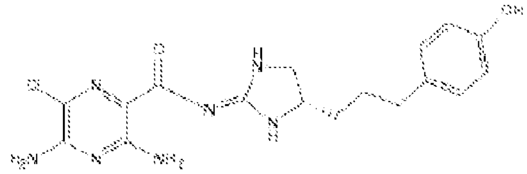

(continued)

19 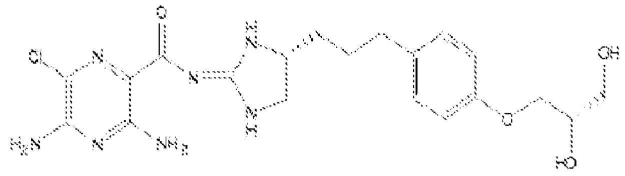

20 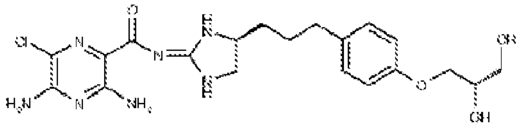

21 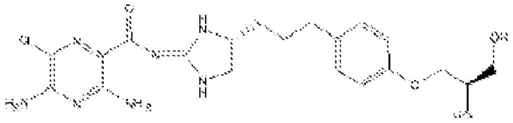

22 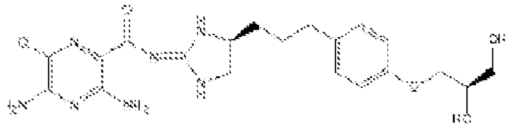

23 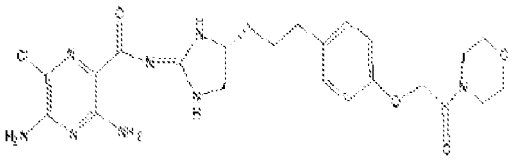

24 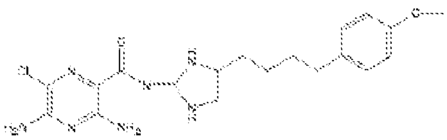

25 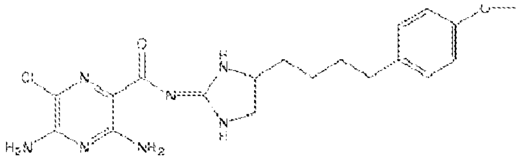

26 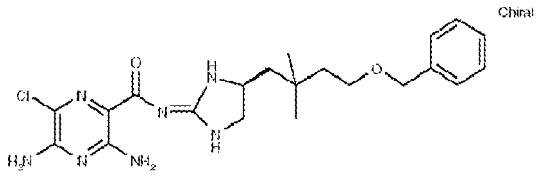

27 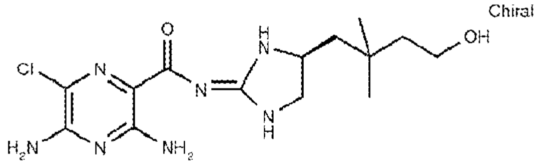

28 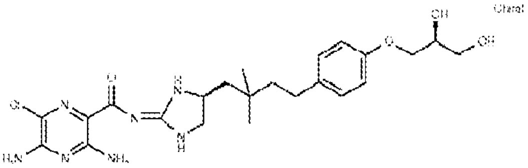

(continued)

29 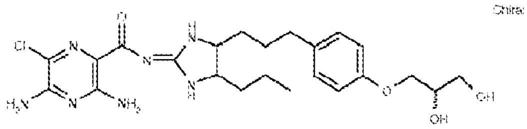

30 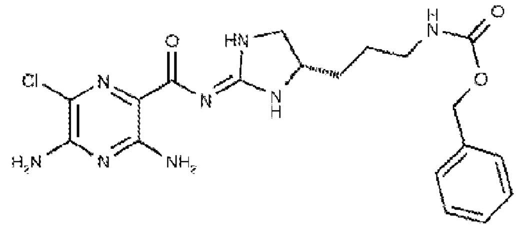

31 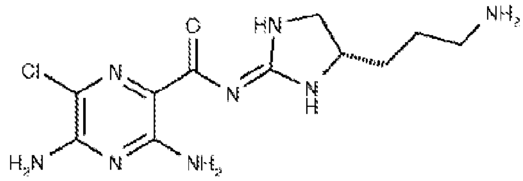

32 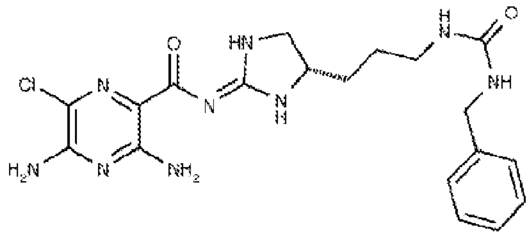

33 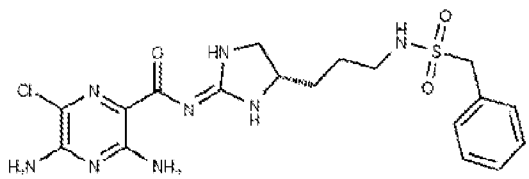

34 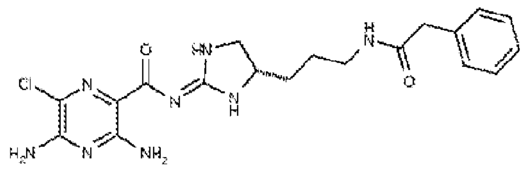

35 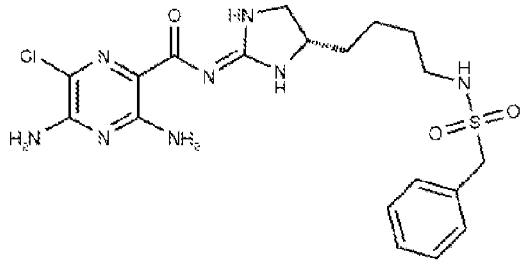

36 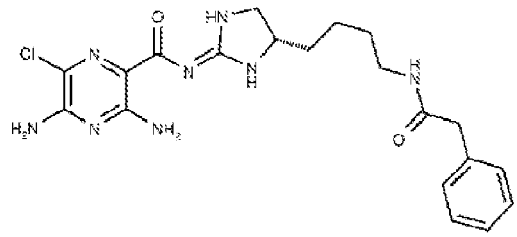

(continued)

37

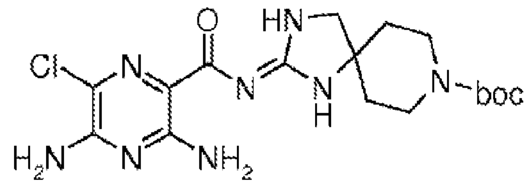

38

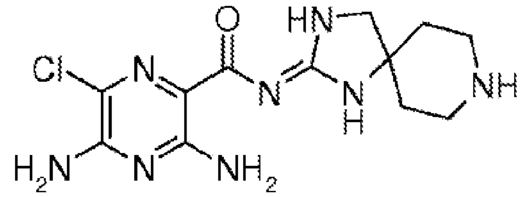

39

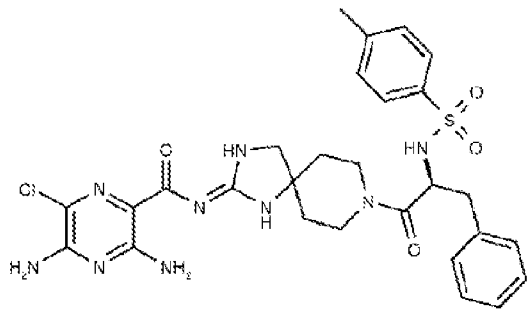

40

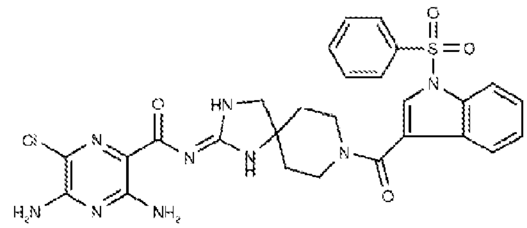

41

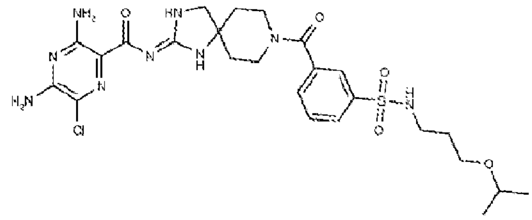

42

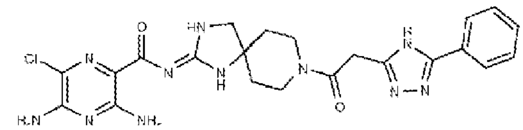

43

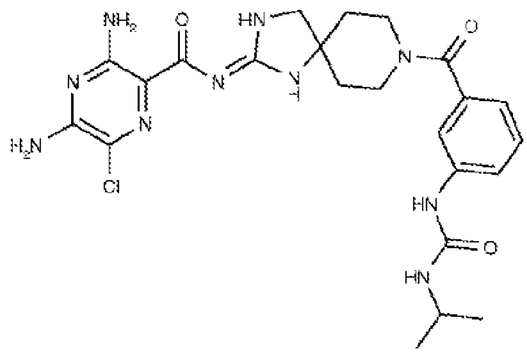

(continued)

44
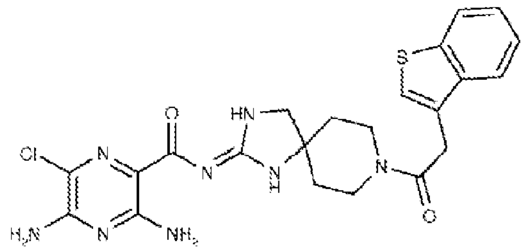

45
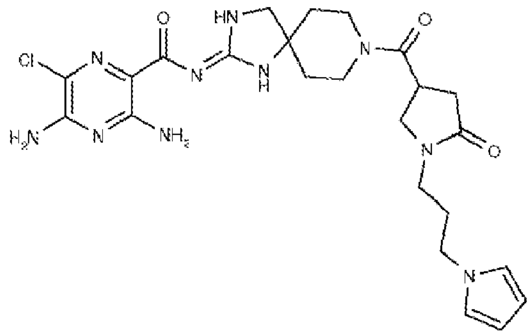

46
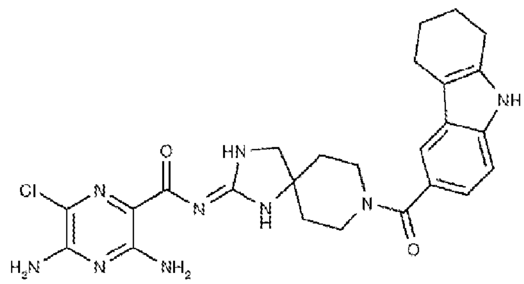

47
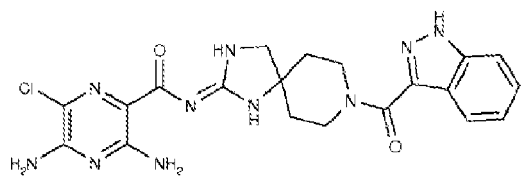

48
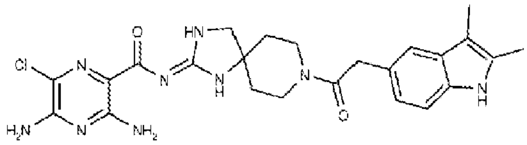

49
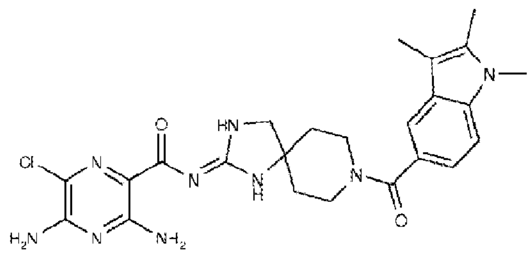

50
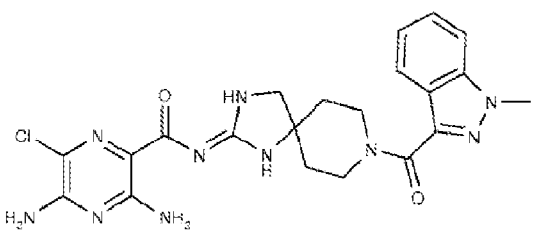

(continued)

51

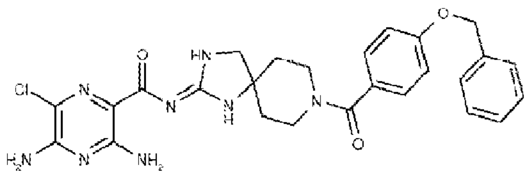

52

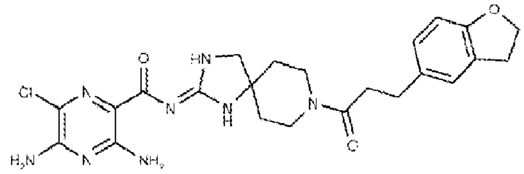

53

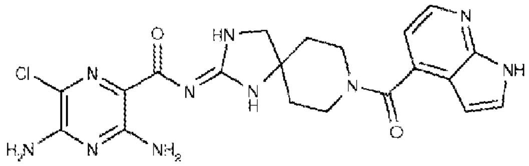

54

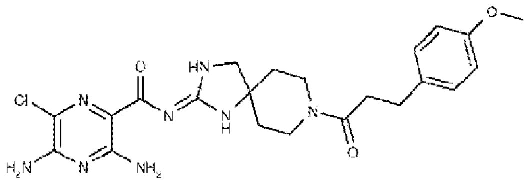

55

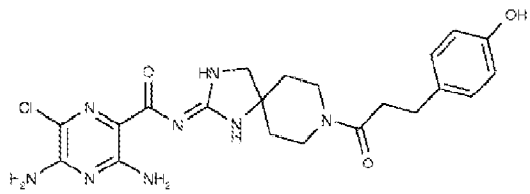

56

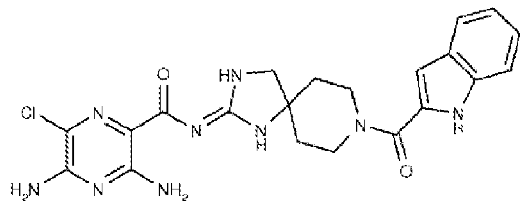

57

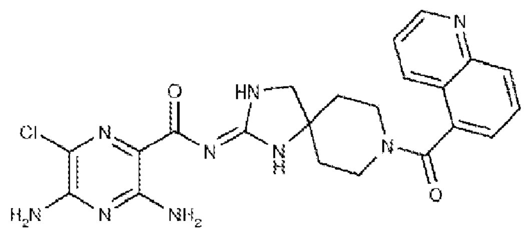

58

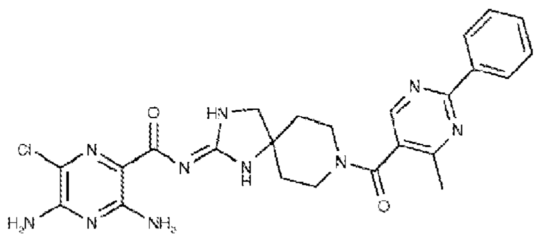

(continued)

59 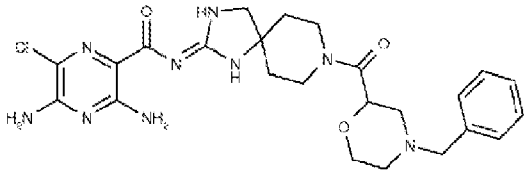

60 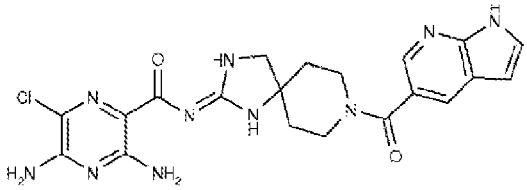

61 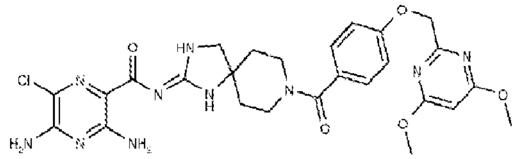

62 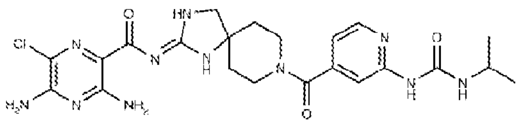

63 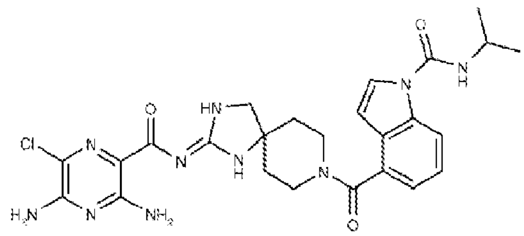

64 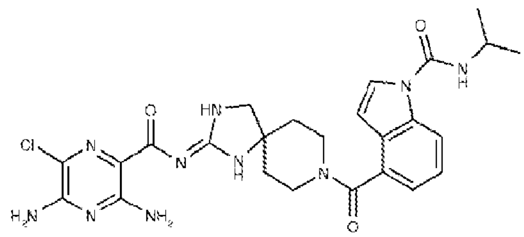

65 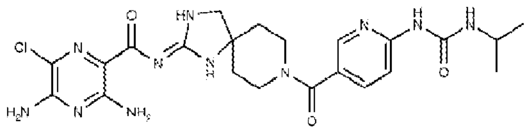

66 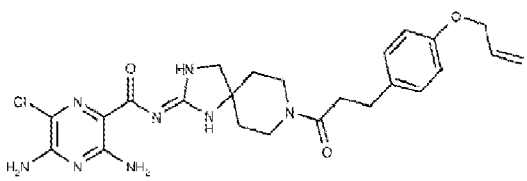

67 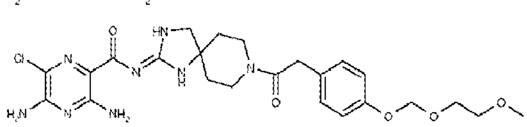

68 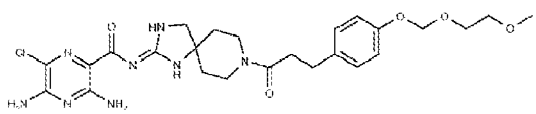

(continued)

69 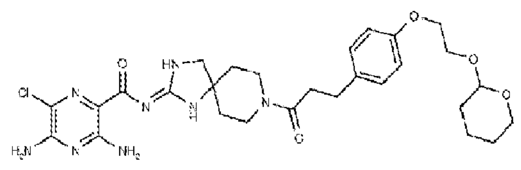

70 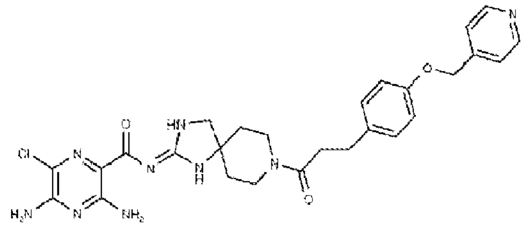

71 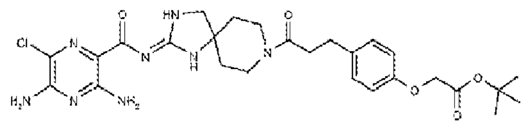

72 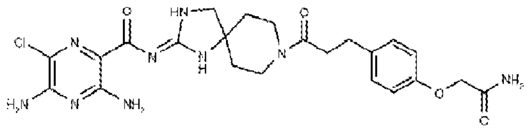

73 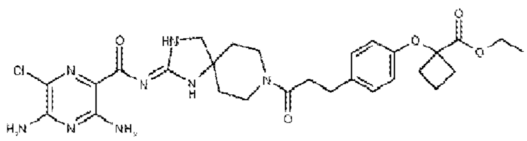

74 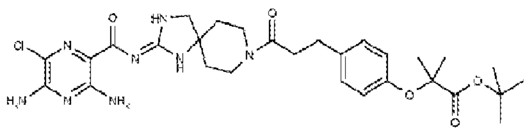

75 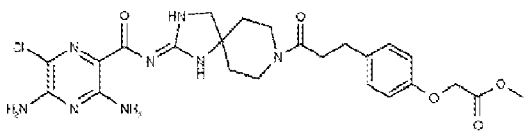

76 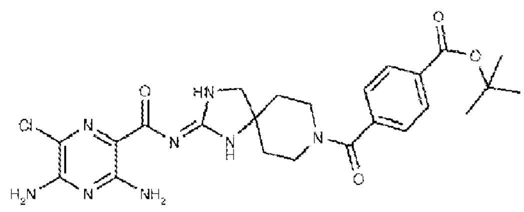

77 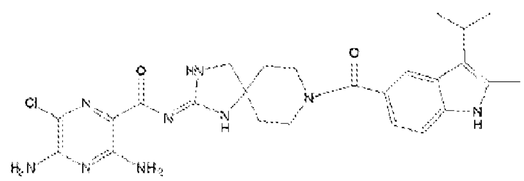

(continued)

78 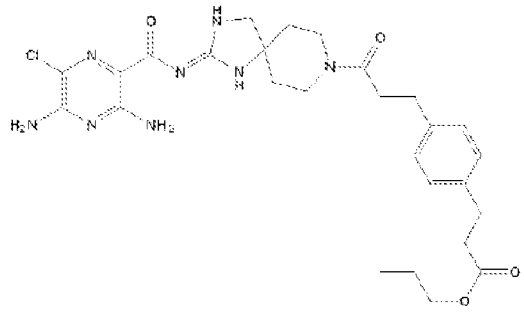

79 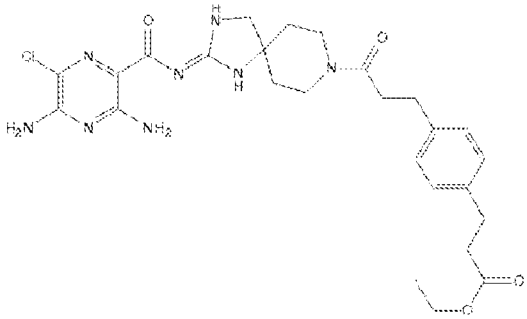

80 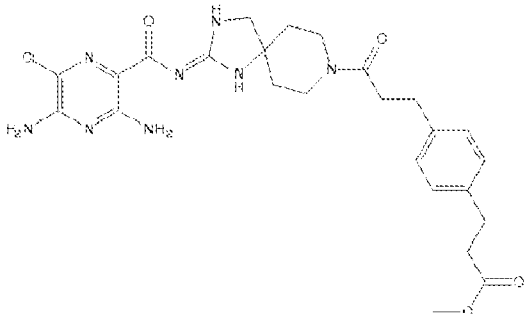

81 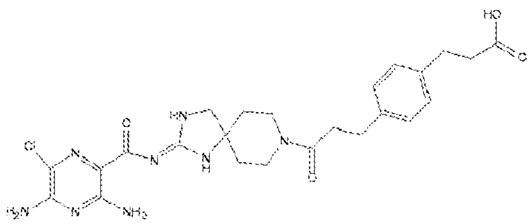

82 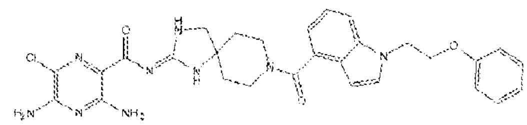

83 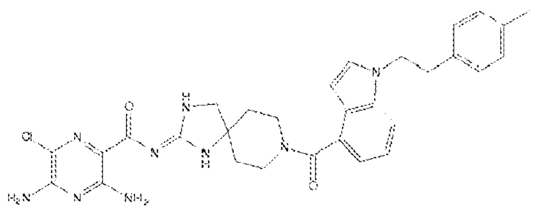

84 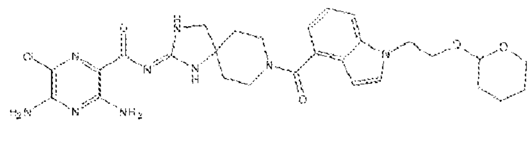

(continued)

85 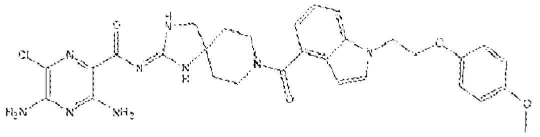

86 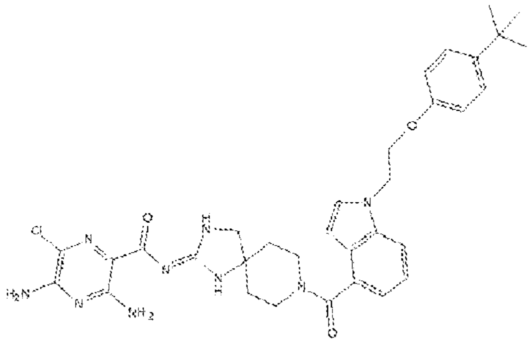

87 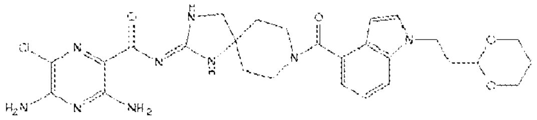

88 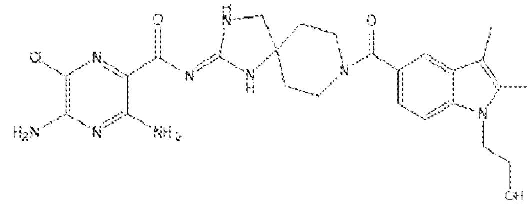

89 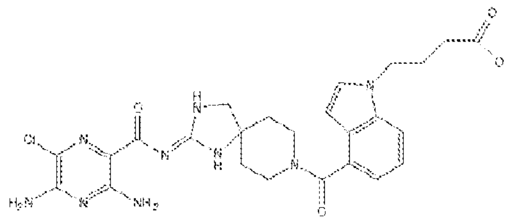

90 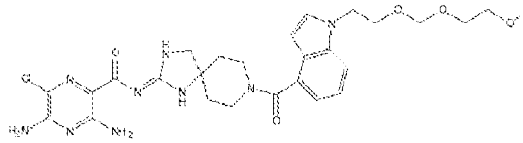

91 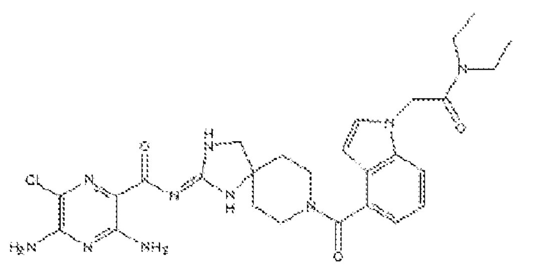

92 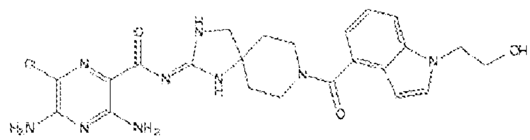

(continued)

93 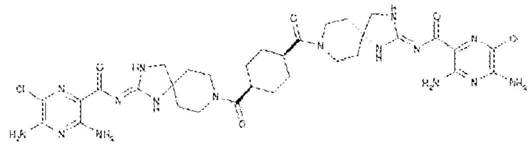

94 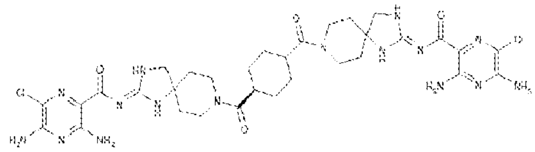

95 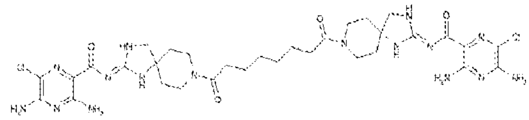

96 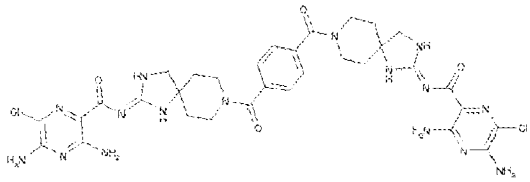

97 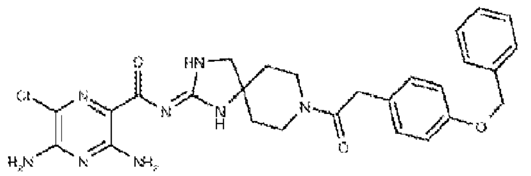

98 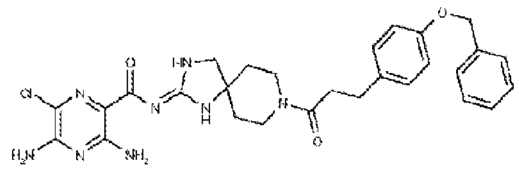

99 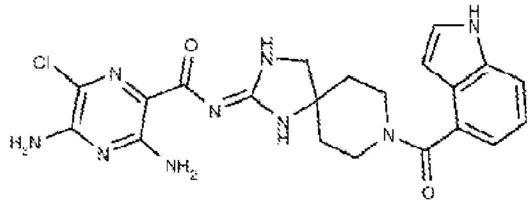

100 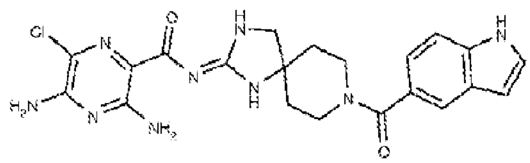

101 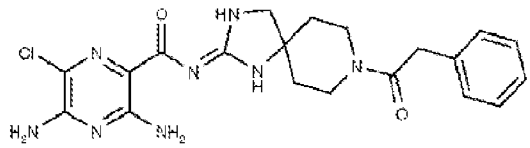

(continued)

102
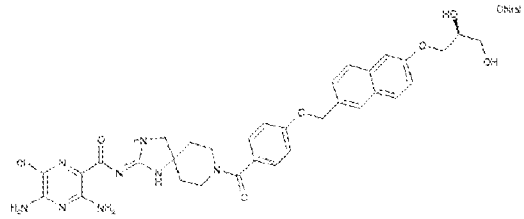

103
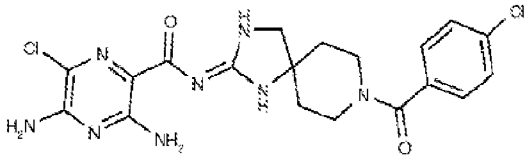

104
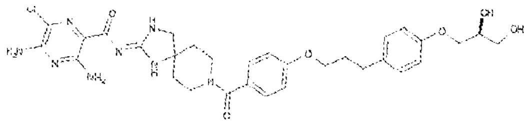

105
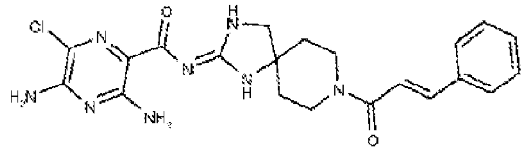

106
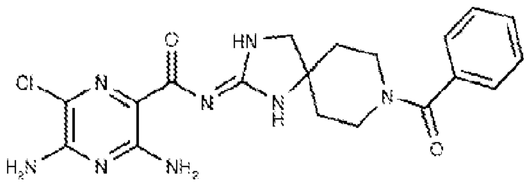

107
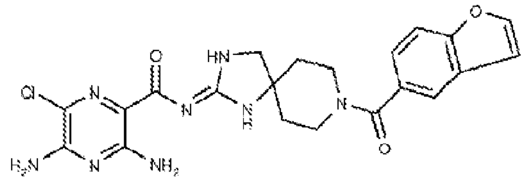

108
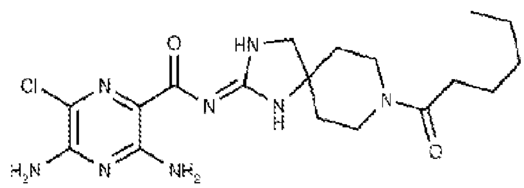

109
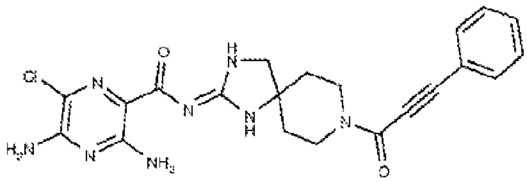

110
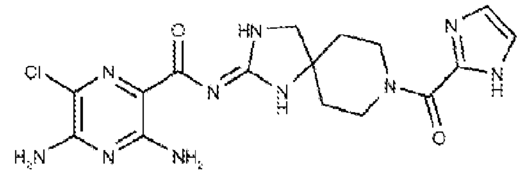

(continued)

111 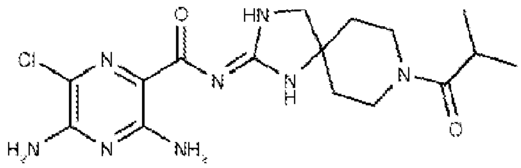

112 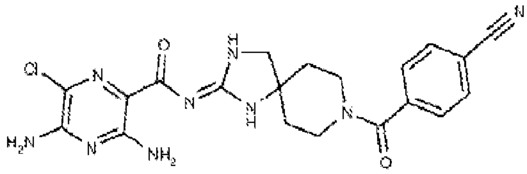

113 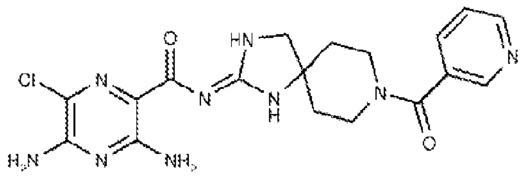

114 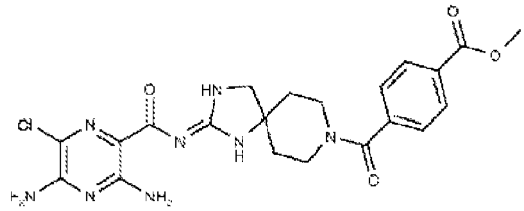

115 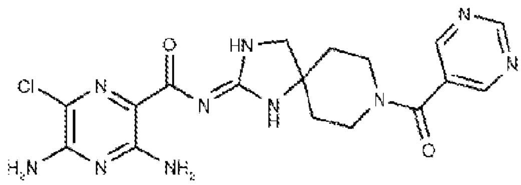

116 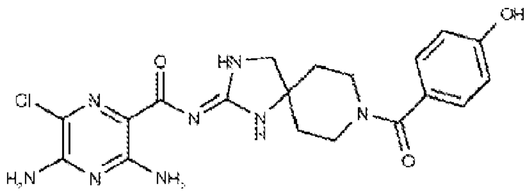

117 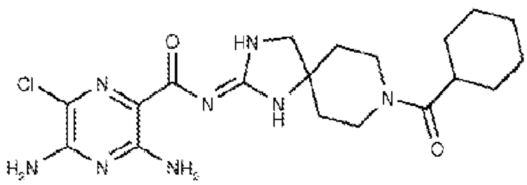

118 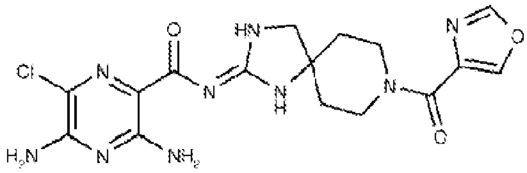

119 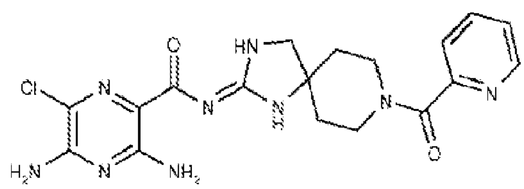

(continued)

120 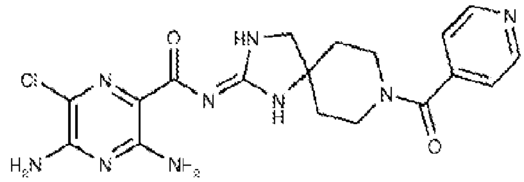

121 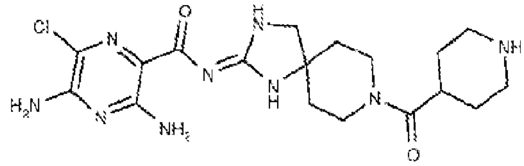

122 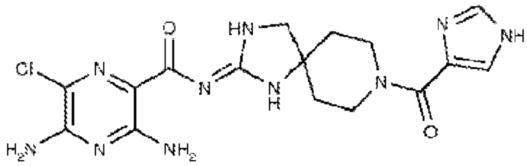

123 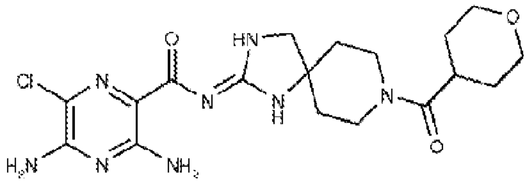

124 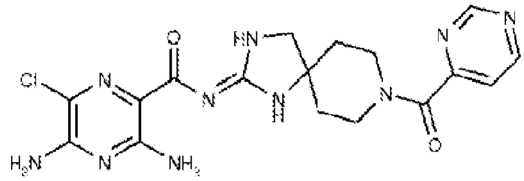

125 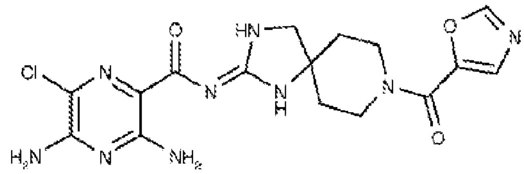

126 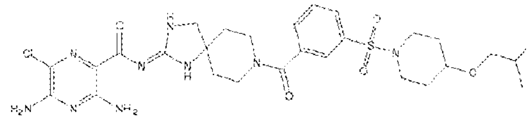

127 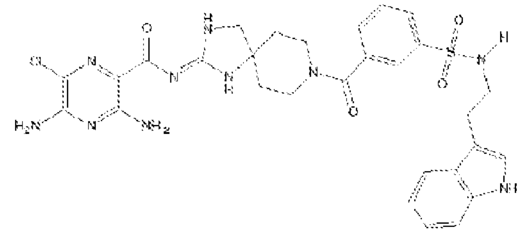

128 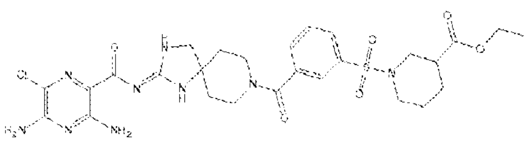

(continued)

129 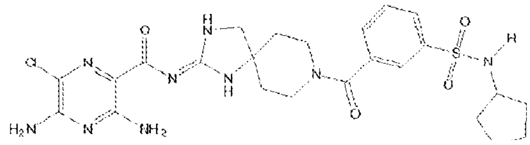

130 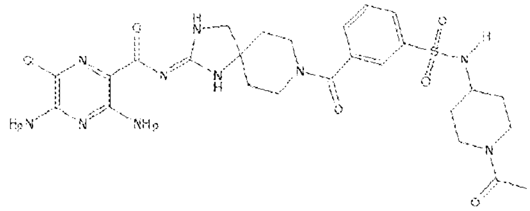

131 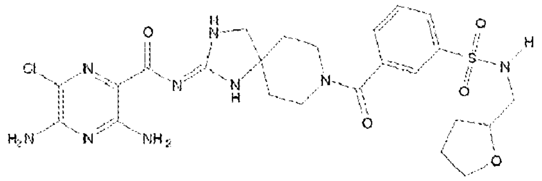

132 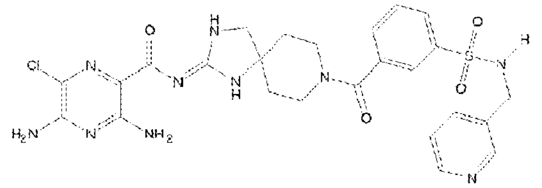

133 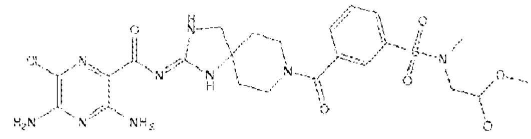

134 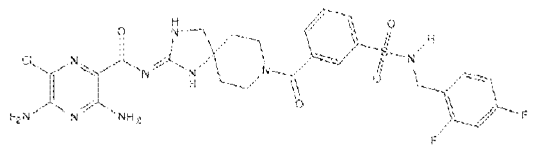

135 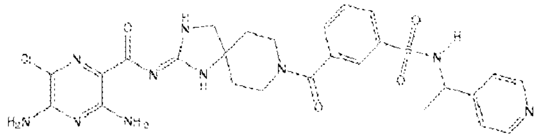

136 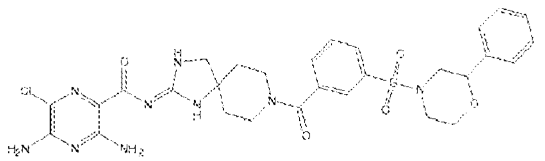

137 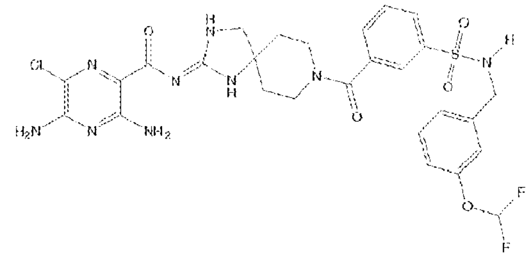

(continued)

138 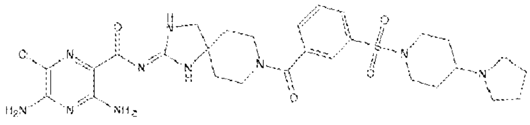

139 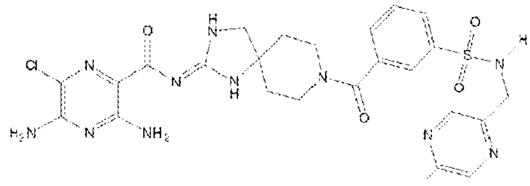

140 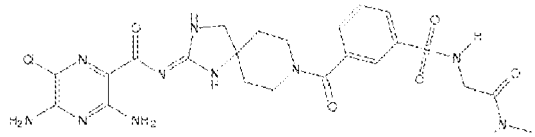

141 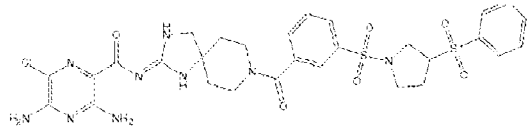

142 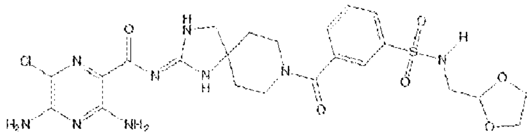

143 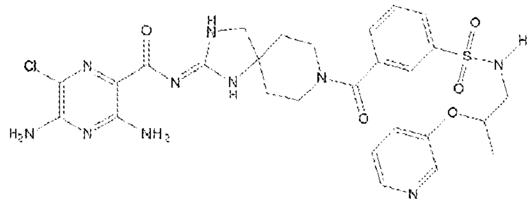

144 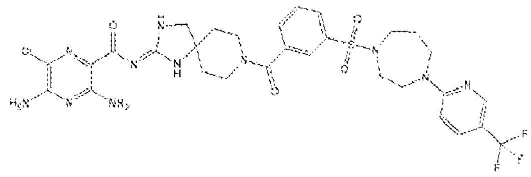

145 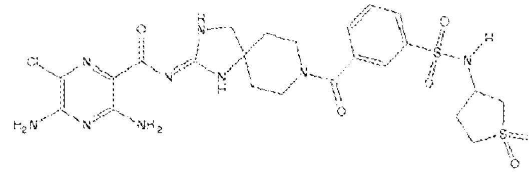

146 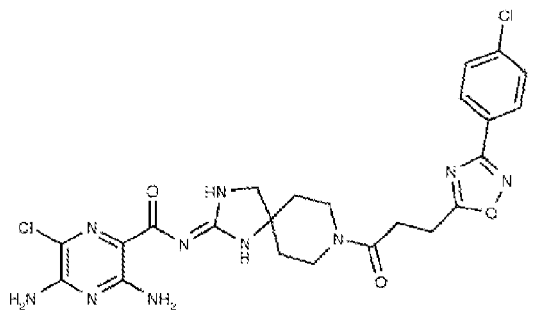

(continued)

147 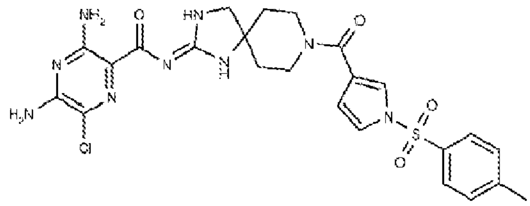

148 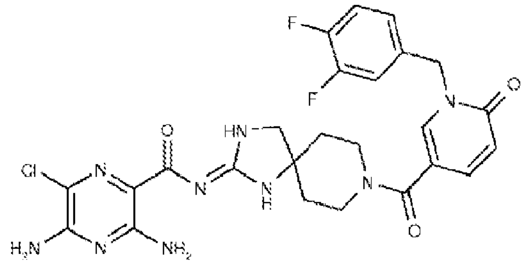

149 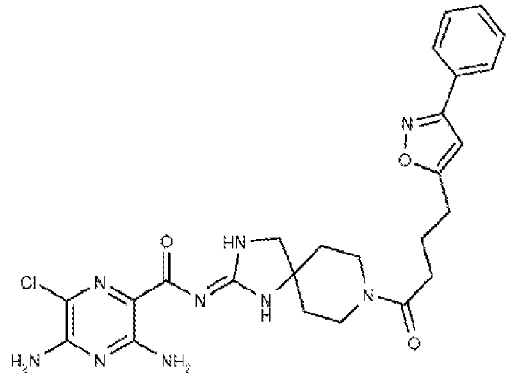

150 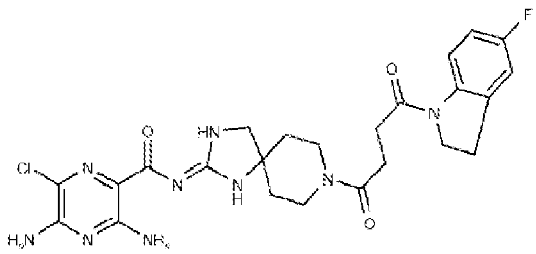

151 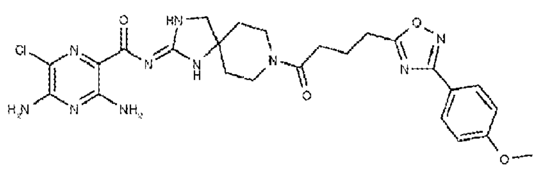

152 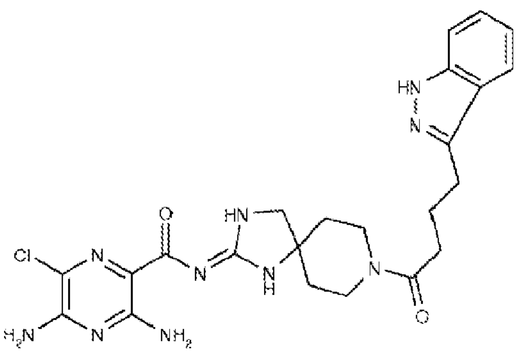

(continued)

153

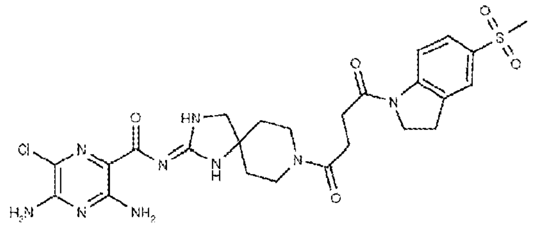

154

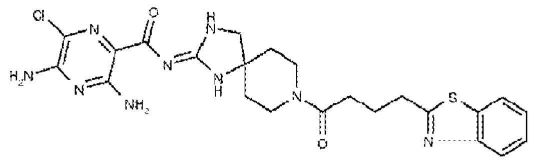

155

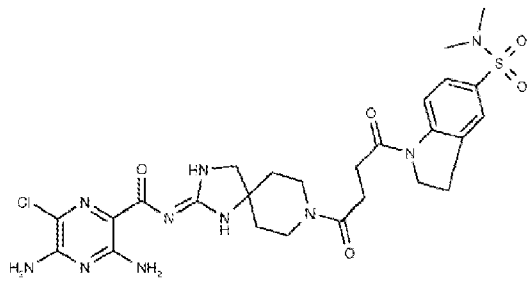

156

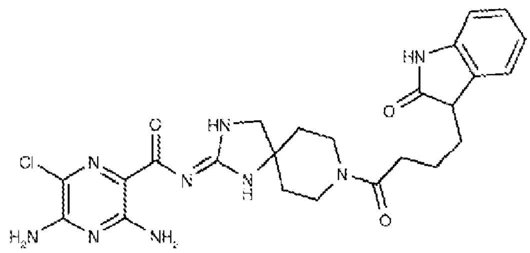

157

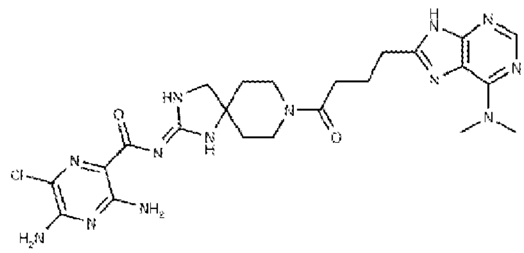

158

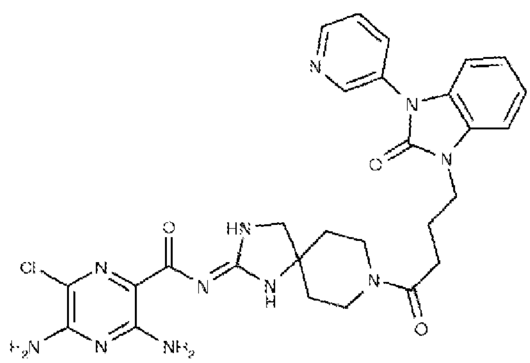

(continued)

159

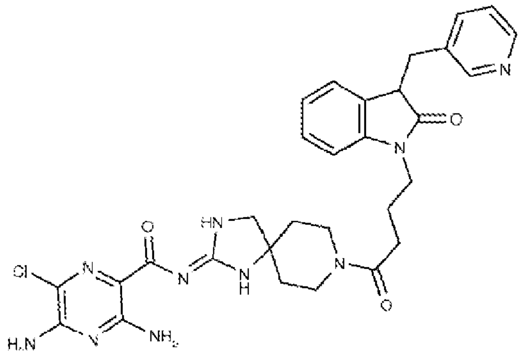

160

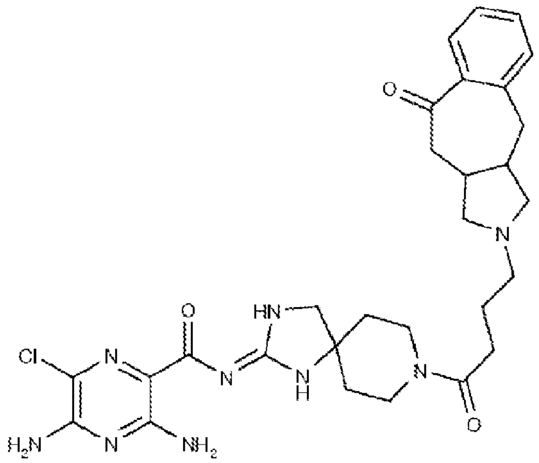

161

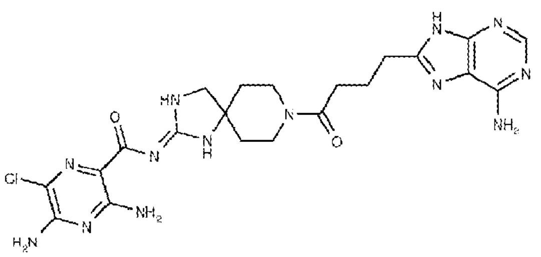

162

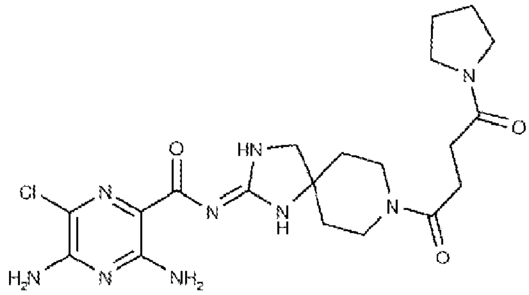

163

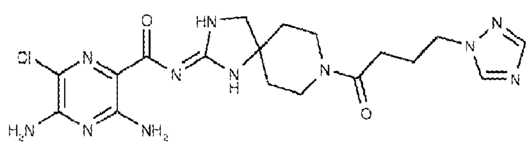

(continued)

164

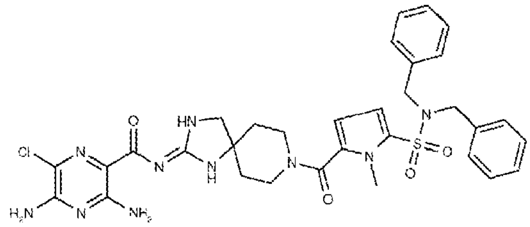

165

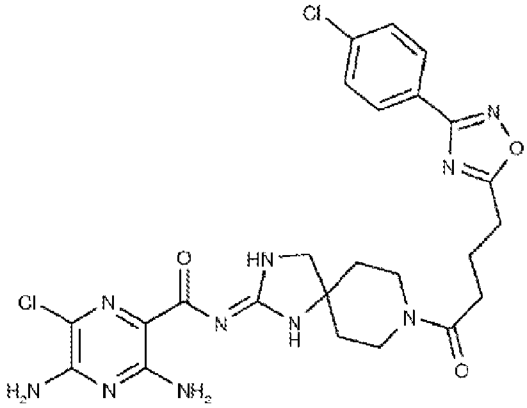

166

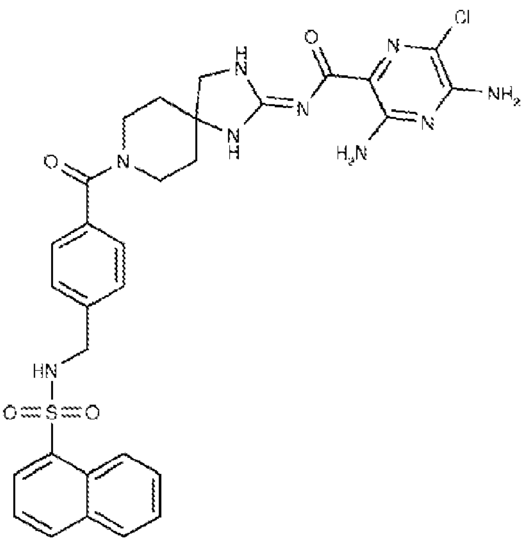

167

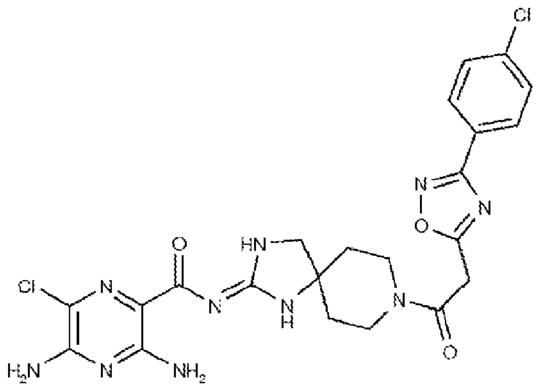

(continued)

168

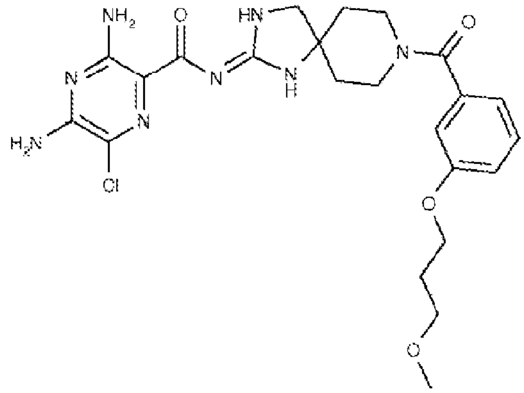

169

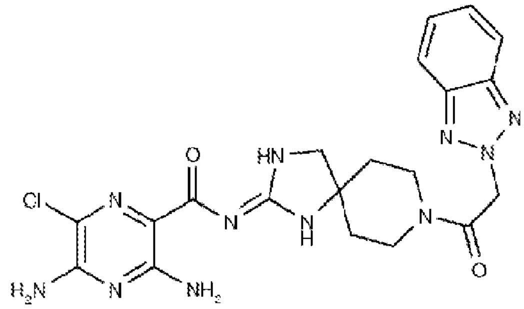

170

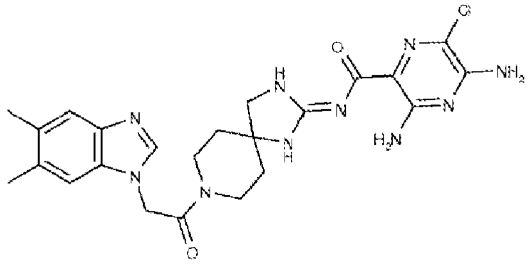

171

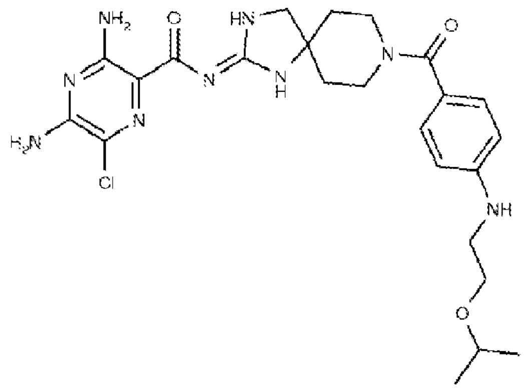

172

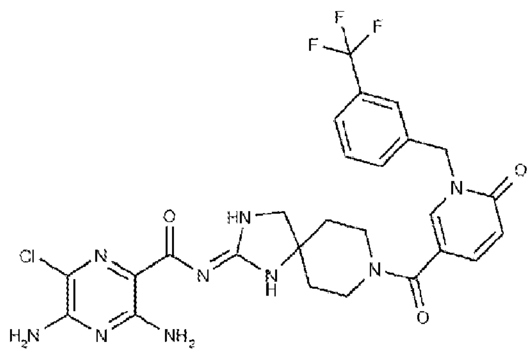

(continued)

173

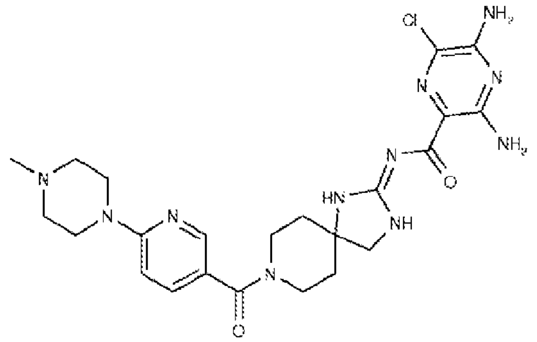

174

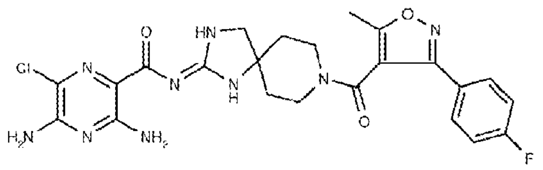

175

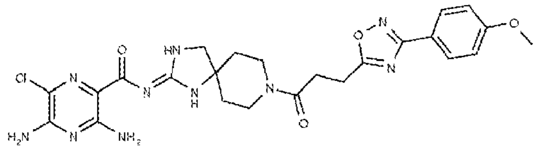

176

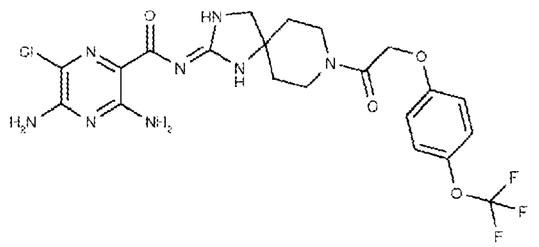

177

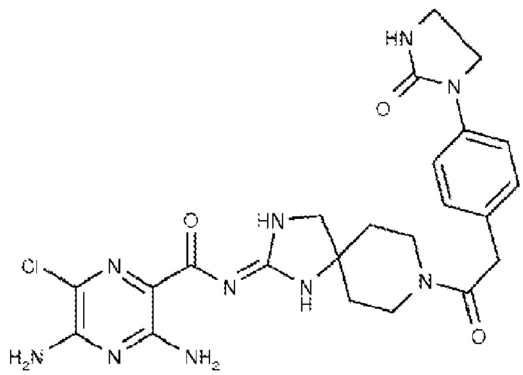

178

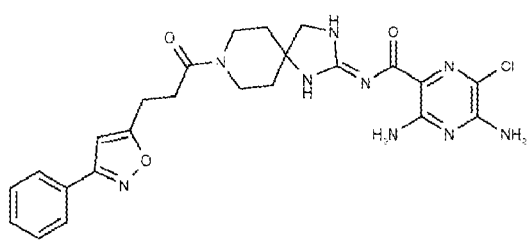

(continued)

179

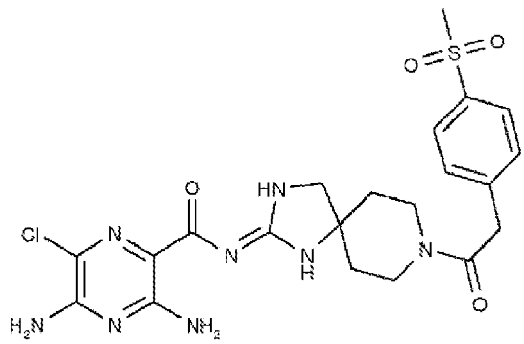

180

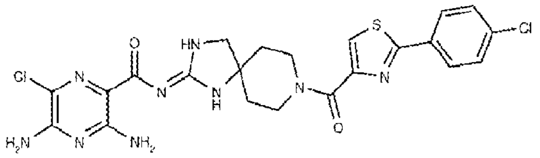

181

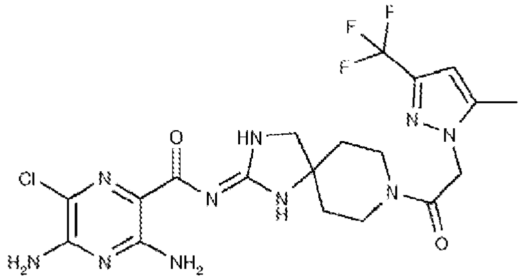

182

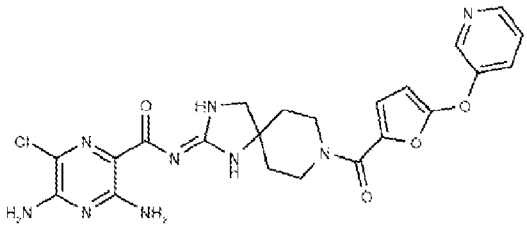

183

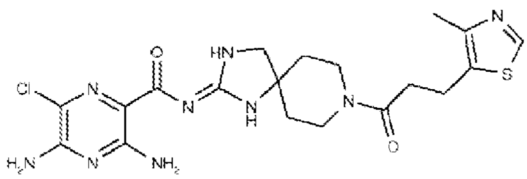

184

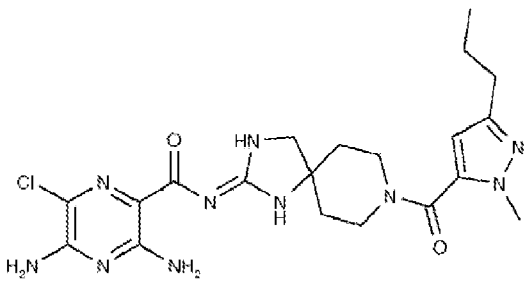

(continued)

185

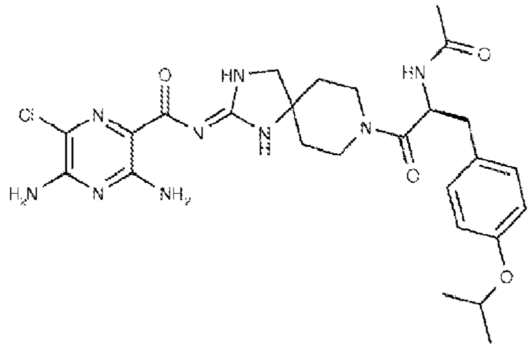

186

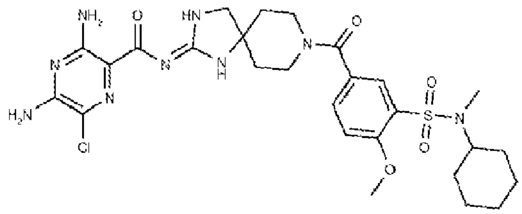

187

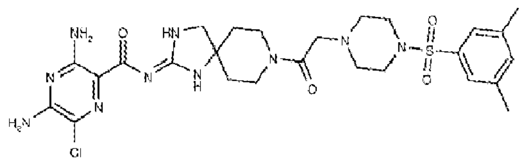

188

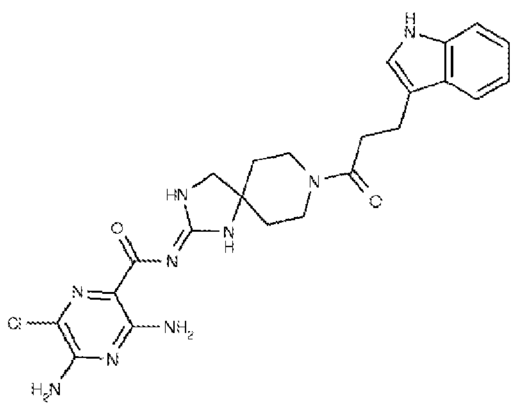

189

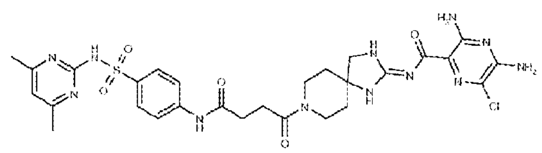

Chiral

190

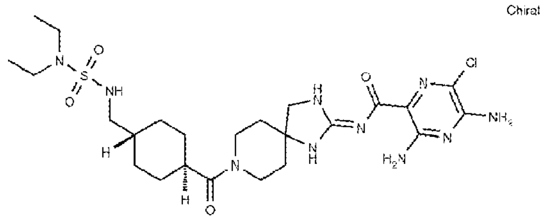

(continued)

191 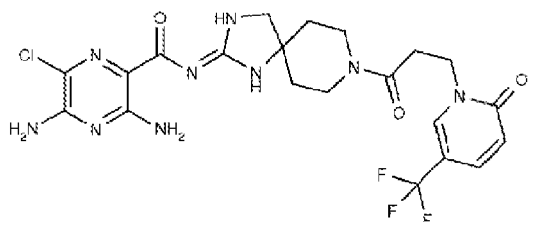

192 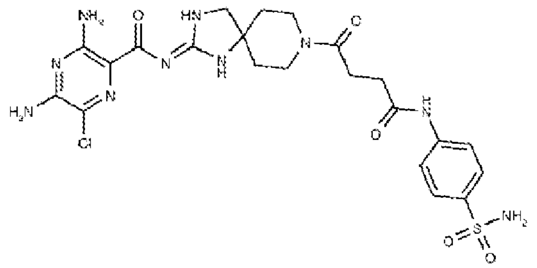

193 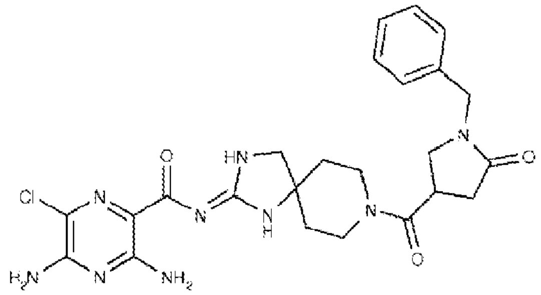

194 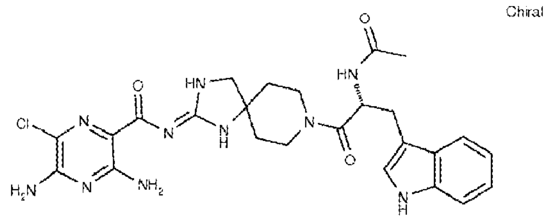


195 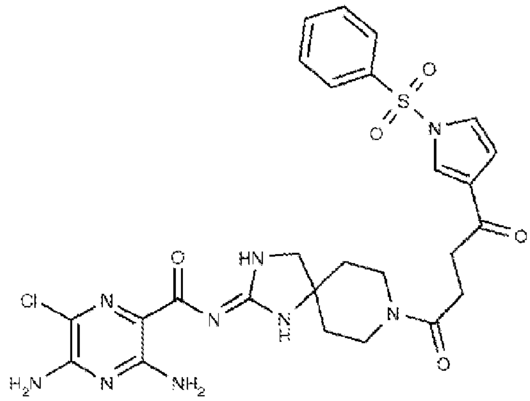

196 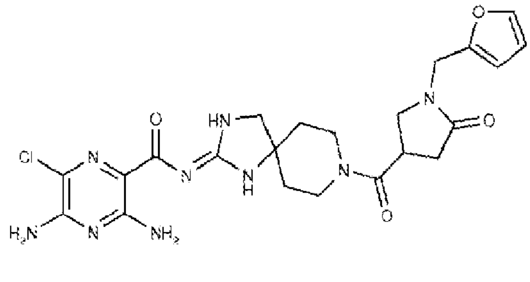

(continued)

197

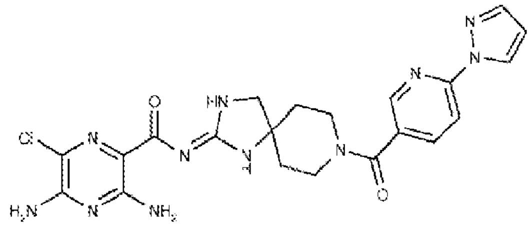

198

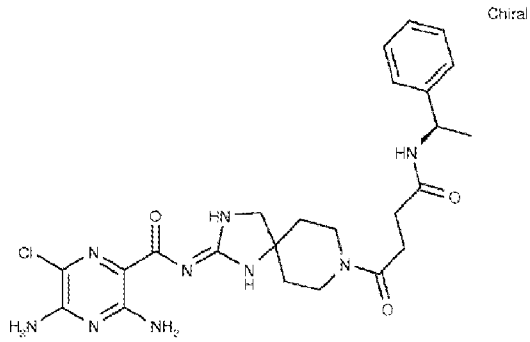

199

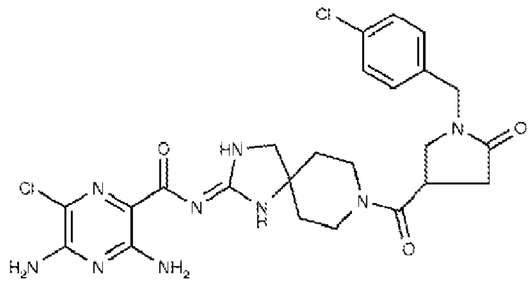

200

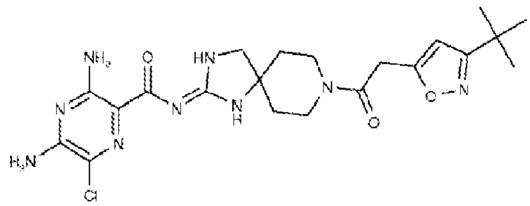

201

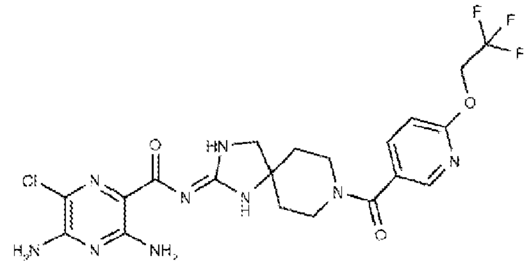

202

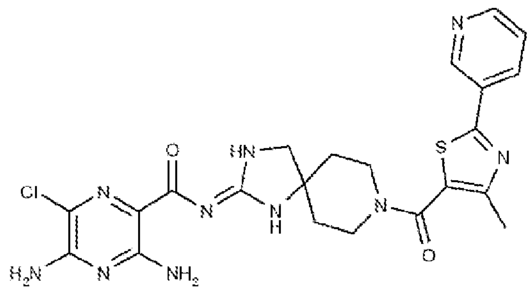

(continued)

203

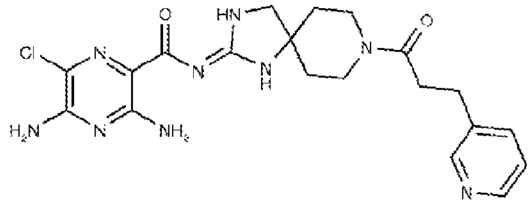

204

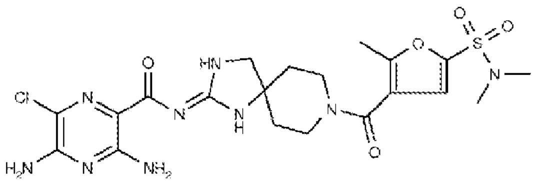

205

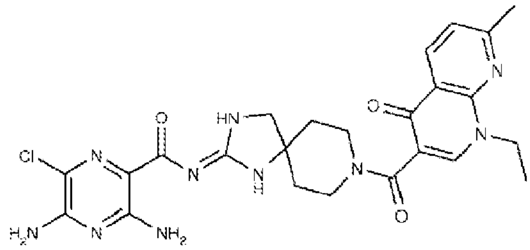

206

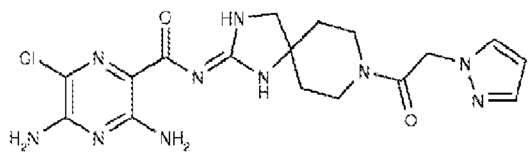

207

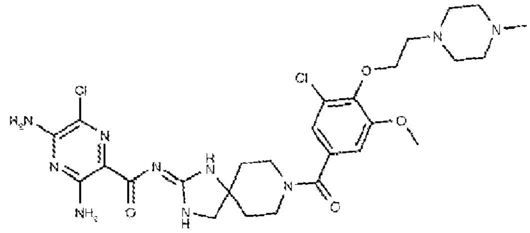

208

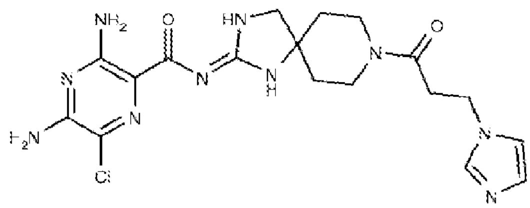

209

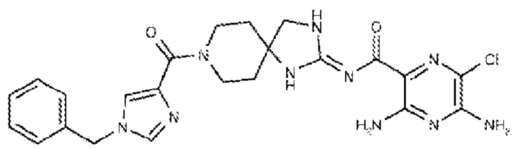

210

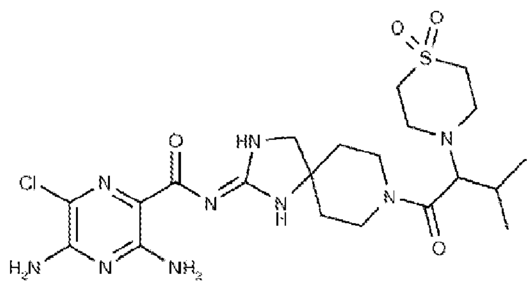

(continued)

211 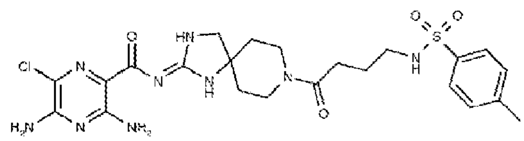

212 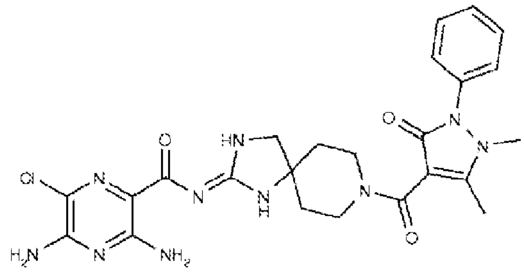

213 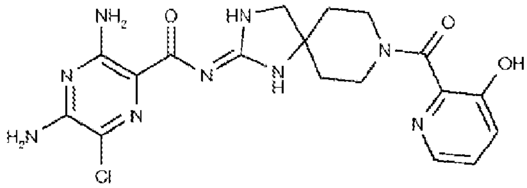

214 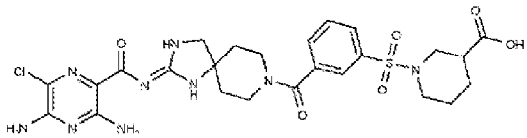

215 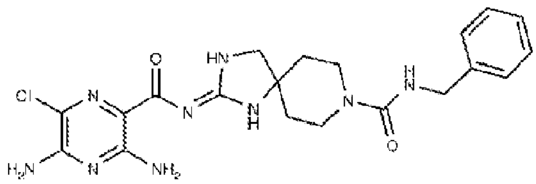

216 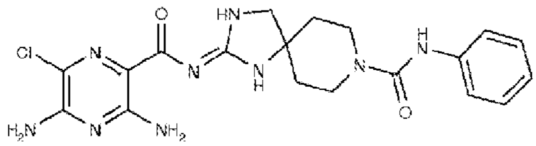

217 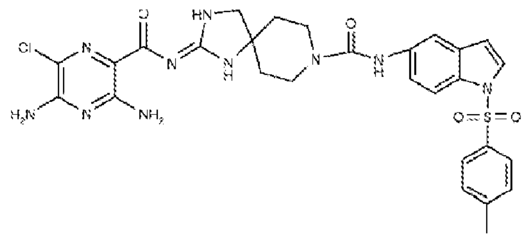

218 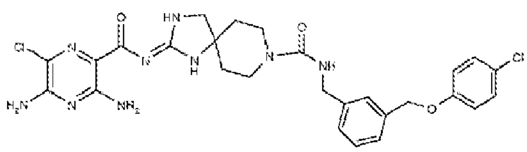

(continued)

219
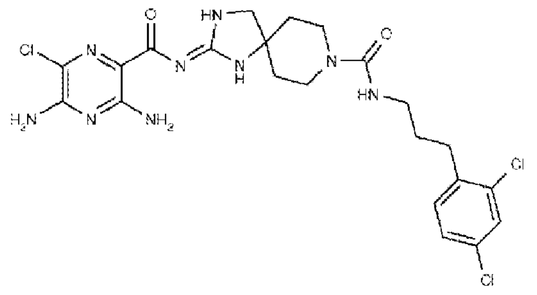

220
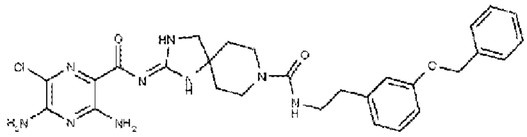

221
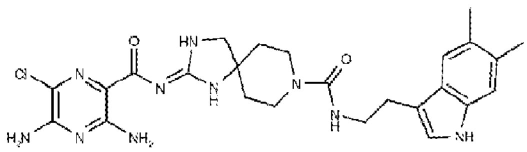

222
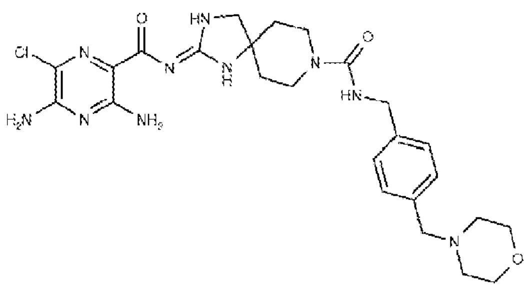

223
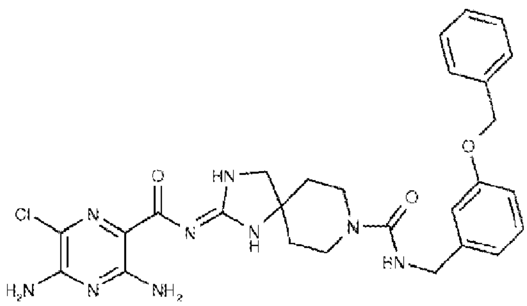

224
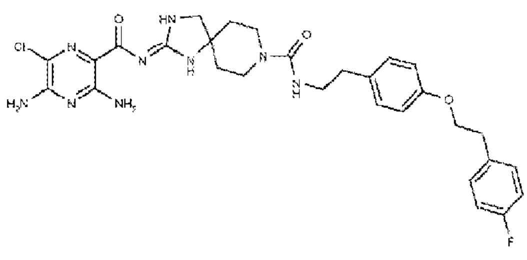

225
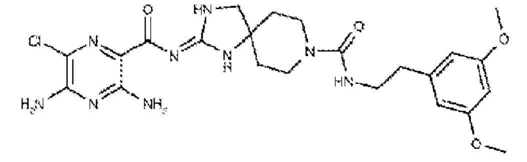

(continued)

226 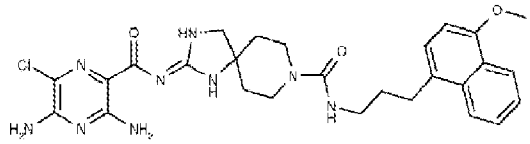

227 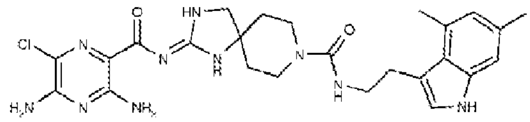

228 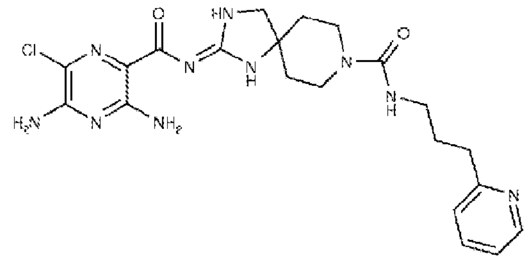

229 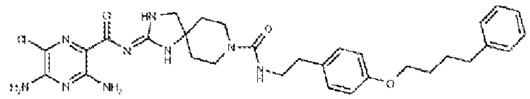

230 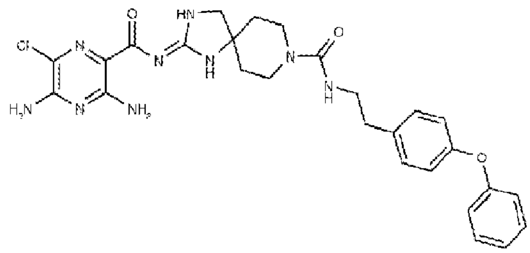

231 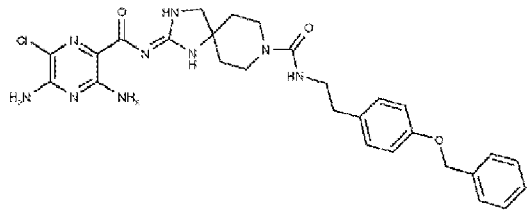

232 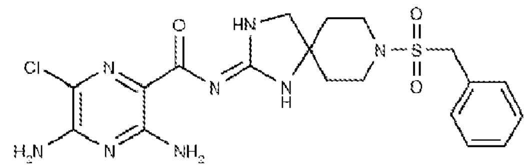

233 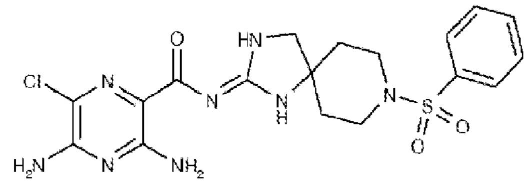

234 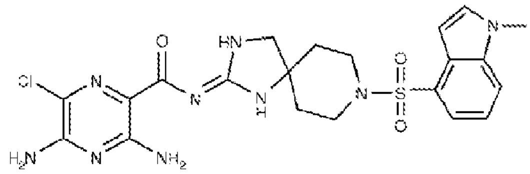

(continued)

235 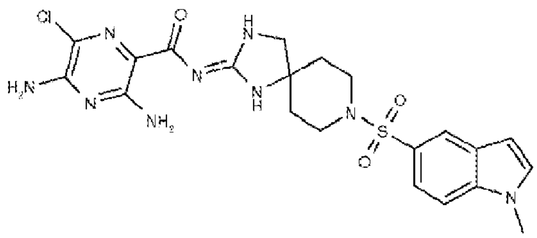

236 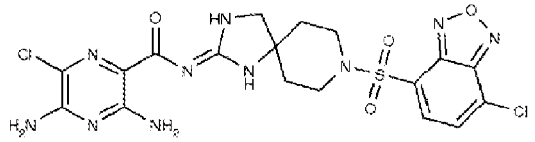

237 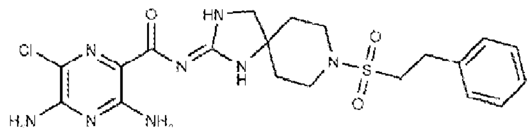

238 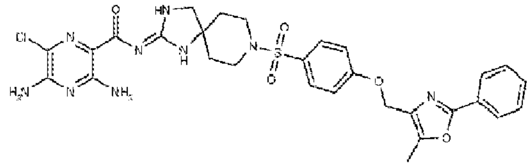

239 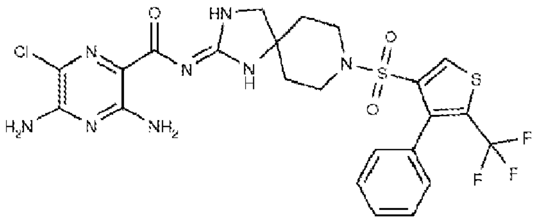

240 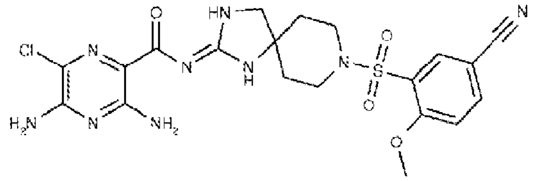

241 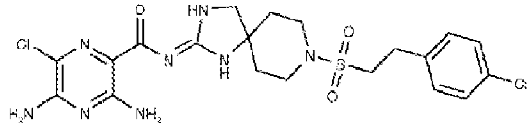

242 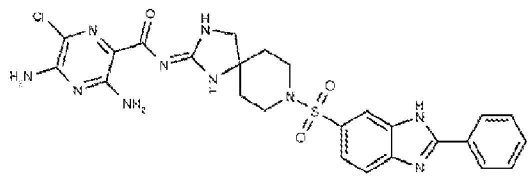

243 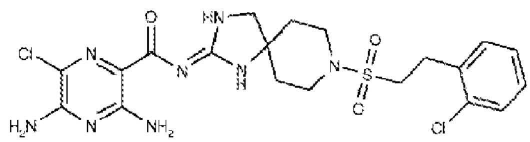

(continued)

244 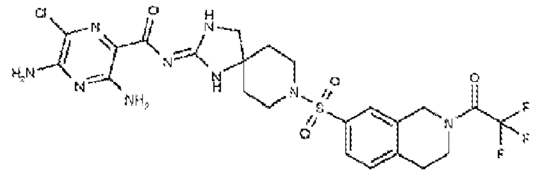

245 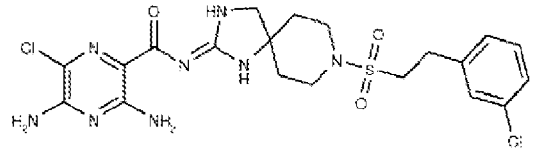

246 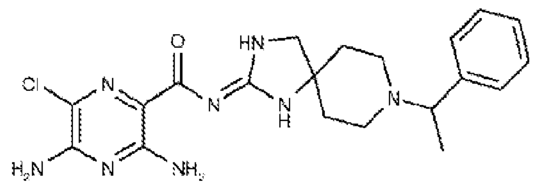

247 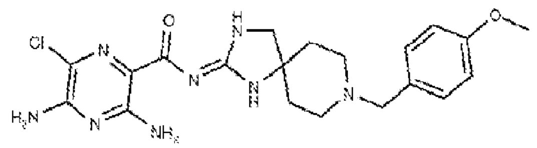

248 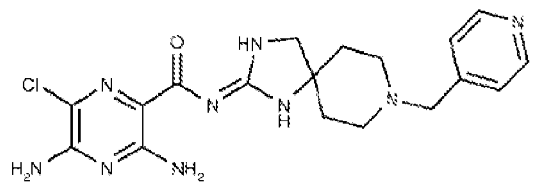

249 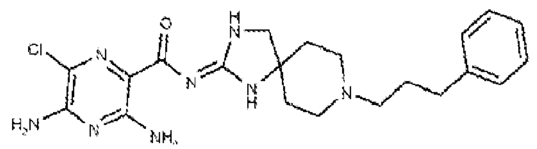

250 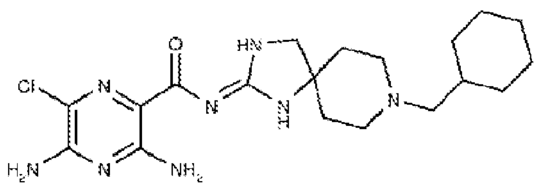

251 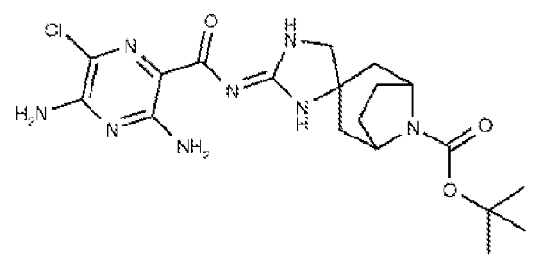

252 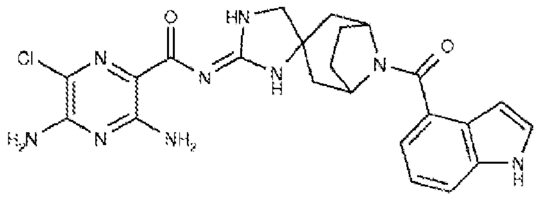

(continued)

253 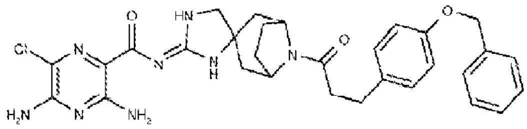

DEFINITIONS OF FORMULA IV, IV(1a), IV(1b), IV(1c) AND IV(2) TERMS

Terms used in the description of the compounds of Formula IV, IV(1a), IV(1b), IV(1c) and IV(2) have the following meanings:

"Optionally substituted" means the group referred to can be substituted at one or more positions by any one or any combination of the radicals listed thereafter.

"optionally substituted by one or more Z groups" denotes that the relevant group may include one or more substituents, each independently selected from the groups included within the definition of Z. Thus, where there are two or more Z group substituents, these may be the same or different.

"Halo" or "halogen", as used herein, may be fluorine, chlorine, bromine or iodine.

"$C_1$-$C_8$-Alkyl", as used herein, denotes straight chain or branched alkyl having 1-8 carbon atoms. If a different number of carbon atoms is specified, such as $C_6$ or $C_3$, then the definition is to be amended accordingly.

"$C_1$-$C_8$-Alkoxy", as used herein, denotes straight chain or branched alkoxy having 1-8 carbon atoms. If a different number of carbon atoms is specified, such as $C_6$ or $C_3$, then the definition is to be amended accordingly.

The term "alkylene" denotes a straight chain or branched saturated hydrocarbon chain containing between 1 and 8 carbon atoms. If a different number of carbon atoms is specified, such as $C_6$ or $C_3$, then the definition is to be amended accordingly.

"Amino-$C_1$-$C_8$-alkyl" and "amino-$C_1$-$C_8$-alkoxy" denote amino attached by a nitrogen atom to $C_1$-$C_8$-alkyl, e.g., $NH_2$-($C_1$-$C_8$)-, or to $C_1$-$C_8$-alkoxy, e.g., $NH_2$-($C_1$-$C_8$)-O-. If a different number of carbon atoms is specified, such as $C_6$ or $C_3$, then the definition is to be amended accordingly.

"$C_1$-$C_8$-Alkylamino" and "di($C_1$-$C_8$-alkyl)amino" denote $C_1$-$C_8$-alkyl, as hereinbefore defined, attached by a carbon atom to an amino group. The $C_1$-$C_8$-alkyl groups in di($C_1$-$C_8$-alkyl)amino may be the same or different. If a different number of carbon atoms is specified, such as $C_6$ or $C_3$, then the definition is to be amended accordingly.

"Amino-(hydroxy)-$C_1$-$C_8$-alkyl" denotes amino attached by a nitrogen atom to $C_1$-$C_8$-alkyl and hydroxy attached by an oxygen atom to the same $C_1$-$C_8$-alkyl. If a different number of carbon atoms is specified, such as $C_6$ or $C_3$, then the definition is to be amended accordingly.

"$C_1$-$C_8$-Alkylcarbonyl" and " $C_1$-$C_8$-alkoxycarbonyl", as used herein, denote $C_1$-$C_8$-alkyl or $C_1$-$C_8$-alkoxy, respectively, as hereinbefore defined, attached by a carbon atom to a carbonyl group. If a different number of carbon atoms is specified, such as $C_6$ or $C_3$, then the definition is to be amended accordingly.

"$C_3$-$C_8$-Cycloalkylcarbonyl", as used herein, denotes $C_3$-$C_8$-cycloalkyl, as hereinbefore defined, attached by a carbon atom to a carbonyl group. If a different number of carbon atoms is specified, such as $C_6$ or $C_3$, then the definition is to be amended accordingly.

"$C_7$-$C_{14}$-Aralkyl", as used herein, denotes alkyl, e.g., $C_1$-$C_4$-alkyl, as hereinbefore defined, substituted by a $C_6$-$C_{10}$-aromatic carbocyclic group, as herein defined. If a different number of carbon atoms is specified, such as $C_6$ or $C_3$, then the definition is to be amended accordingly.

"$C_3$-$C_{15}$-Carbocyclic group", as used herein, denotes a carbocyclic group having 3- to 15-ring carbon atoms that is saturated or partially saturated, such as a $C_3$-$C_8$-cycloalkyl. Examples of $C_3$-$C_{15}$-carbocyclic groups include but are not limited to cyclopropyl, cyclobutyl, cyclopentyl, cyclohexyl, cycloheptyl or cyclooctyl or a bicyclic group, such as bicyclooctyl, bicyclononyl including indanyl and indenyl and bicyclodecyl. If a different number of carbon atoms is specified, such as $C_6$, then the definition is to be amended accordingly.

"aryl" or "$C_6$-$C_{15}$-Aromatic carbocyclic group", as used herein, denotes an aromatic group having 6- to 15-ring carbon atoms. Examples of $C_6$-$C_{15}$-aromatic carbocyclic groups include, but are not limited to, phenyl, phenylene, benzenetriyl, naphthyl, naphthylene, naphthalenetriyl or anthrylene. If a different number of carbon atoms is specified, such as $C_{10}$, then the definition is to be amended accordingly.

"4- to 8-Membered heterocyclic group", "5- to 6- membered heterocyclic group", "3- to 10-membered heterocyclic group", "3- to 14-membered heterocyclic group", "4- to 14-membered heterocyclic group" and "5- to 14-membered heterocyclic group", refers, respectively, to 4- to 8-membered, 5- to 6-membered, 3- to 10-membered, 3- to 14-membered, 4- to 14-membered and 5- to 14-membered heterocyclic rings containing at least one ring heteroatom selected from the group consisting of nitrogen, oxygen and sulphur, which may be saturated, partially saturated or unsaturated (aromatic). The heterocyclic group includes single ring groups, fused ring groups and bridged groups. Examples of such heterocyclic

groups include, but are not limited to, furan, pyrrole, pyrrolidine, pyrazole, imidazole, triazole, isotriazole, tetrazole, thiadiazole, isothiazole, oxadiazole, pyridine, piperidine, pyrazine, oxazole, isoxazole, pyrazine, pyridazine, pyrimidine, piperazine, pyrrolidine, pyrrolidinone, morpholine, triazine, oxazine, tetrahyrofuran, tetrahydrothiophene, tetrahydrothiopyran, tetrahydropyran, 1,4-dioxane, 1,4-oxathiane, indazole, quinoline, indazole, indole, 8-aza-bicyclo[3.2.1]octane or thiazole.

A second aspect of the present invention provides for the use of a compound of formula (IV) in any of the aforementioned embodiments, in free or pharmaceutically acceptable salt form in combination with at least one component of Columns A, B, or C, for the manufacture of a medicament for the treatment of an inflammatory or allergic condition, particularly an inflammatory or obstructive airways disease or mucosal hydration.

An embodiment of the present invention provides for the use of a compound of formula (IV) in any of the aforementioned embodiments, in free or pharmaceutically acceptable salt form, for the manufacture of a medicament for the treatment of an inflammatory or allergic condition selected from cystic fibrosis, primary ciliary dyskinesia, chronic bronchitis, chronic obstructive pulmonary disease, asthma, respiratory tract infections, lung carcinoma, xerostomia and keratoconjunctivitis sire.

It is understood that any and all embodiments of the present invention may be taken in conjunction with any other embodiment to describe additional embodiments of the present invention. Furthermore, any elements of an embodiment are meant to be combined with any and all other elements from any of the embodiments to describe additional embodiments. It is understood by those skilled in the art that combinations of substituents where not possible are not an aspect of the present invention.

Throughout this specification and in the claims that follow, unless the context requires otherwise, the word "comprise", or variations, such as "comprises" or "comprising", will be understood to imply the inclusion of a stated integer or step or group of integers or steps but not the exclusion of any other integer or step or group of integers or steps.

Especially preferred specific compounds of formula (IV) are those described hereinafter in the Examples.

The compounds represented by formula (IV) may be capable of forming acid addition salts, particularly pharmaceutically acceptable acid addition salts. Pharmaceutically acceptable acid addition salts of the compound of formula (IV) include those of inorganic acids, e.g., hydrohalic acids, such as hydrofluoric acid, hydrochloric acid, hydrobromic acid or hydroiodic acid, nitric acid, sulfuric acid, phosphoric acid; and organic acids, e.g., aliphatic monocarboxylic acids, such as formic acid, acetic acid, trifluoroacetic acid, propionic acid and butyric acid; aliphatic hydroxy acids, such as lactic acid, citric acid, tartaric acid or malic acid; dicarboxylic acids, such as maleic acid or succinic acid; aromatic carboxylic acids, such as benzoic acid, p-chlorobenzoic acid, diphenylacetic acid, para-biphenyl benzoic acid or triphenylacetic acid; aromatic hydroxy acids, such as o-hydroxybenzoic acid, p-hydroxybenzoic acid, 1-hydroxynaphthalene-2-carboxylic acid or 3-hydroxynaphthalene-2-carboxylic acid; cinnamic acids, such as 3-(2-naphthalenyl) propenoic acid, para-methoxy cinnamic acid or para-methyl cinnamic acid; and sulfonic acids, such as methanesulfonic acid or benzenesulfonic acid. These salts may be prepared from compounds of formula (IV) by known salt-forming procedures.

Compounds of formula (IV) which may contain acidic, e.g., carboxyl, groups, are also capable of forming salts with bases, in particular, pharmaceutically acceptable bases, such as those well-known in the art; suitable such salts include metal salts, particularly alkali metal or alkaline earth metal salts, such as sodium, potassium, magnesium or calcium salts; or salts with ammonia or pharmaceutically acceptable organic amines or heterocyclic bases, such as ethanolamines, benzylamines or pyridine. These salts may be prepared from compounds of formula (IV) by known salt-forming procedures.

Stereoisomers are those compounds where there is an asymmetric carbon atom. The compounds exist in individual optically active isomeric forms or as mixtures thereof, e.g., as diastereomeric mixtures. The present invention embraces both individual optically active R and *S* isomers, as well as mixtures thereof. Individual isomers can be separated by methods well-known to those skilled in the art, e.g., chiral high performance liquid chromatography (HPLC).

Tautomers are one of two or more structural isomers that exist in equilibrium and are readily converted from one isomeric form to another.

More specifically, for example, compounds of Formula Ia where $R^6$ and/or $R^{11}$ are hydrogen may exist in one or both of the following tautomeric forms:

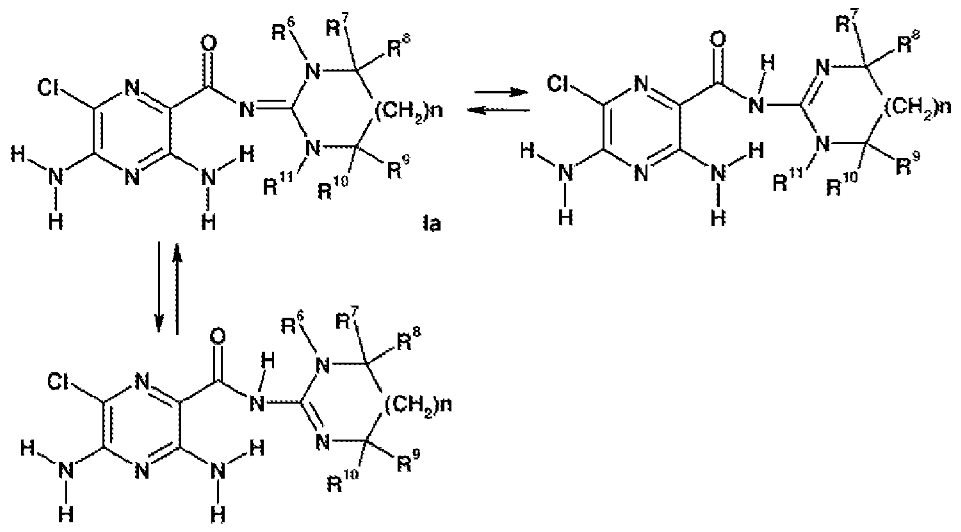

Ia

Compounds according to Formula IV may exist in corresponding tautomeric forms.

Examples of tautomers include but are not limited to those compounds defined in the claims.

The compounds of the invention may exist in both unsolvated and solvated forms. The term "solvate" is used herein to describe a molecular complex comprising the compound of the invention and one or more pharmaceutically acceptable solvent molecules, e.g., ethanol. The term "hydrate" is employed when said solvent is water.

Synthesis

Generally, compounds according to Formula IV can be synthesized by the routes described in Scheme 1 and the Examples.

For instance, intermediate 1 can be reacted with intermediate 2 in an organic solvent to provide compound 3 which can be isolated as the free base. The free base can then be converted to a salt form by treatment with an appropriate acid.

Intermediates can be prepared from methods known by those skilled in the art or are commercially available.

Scheme 1

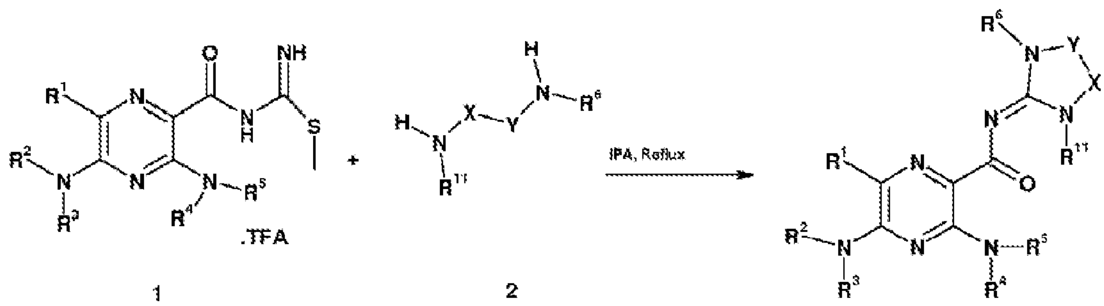


1 2 3

In Scheme 1, $R^1$, $R^2$, $R^3$, $R^4$, $R^5$, $R^6$ and $R^{11}$ are as defined above; Y is $CR^7R^8$ ; X is $CR^9R^{10}$; n is 0; and $R^7$, $R^8$, $R^9$ and $R^{10}$ are also as defined above. For compounds where n is 1 or 2, then the appropriate methylene or ethylene linking groups are inserted between X and Y in the diamine reactant 2.

The compounds of Formula 1 and Formula 2 above can be prepared according to conventional routes described in the literature.

Compounds of formula (IV), in free form, may be converted into salt form, and vice versa, in a conventional manners understood by those skilled in the art. The compounds in free or salt form can be obtained in the form of hydrates or solvates containing a solvent used for crystallization. Compounds of formula (IV) can be recovered from reaction mixtures and purified in a conventional manner. Isomers, such as stereoisomers, may be obtained in a conventional manner, e.g., by fractional crystallisation or asymmetric synthesis from correspondingly asymmetrically substituted, e.g., optically active, starting materials. The compounds of formula (IV) can be prepared, e.g., using the reactions

and techniques described below and in the Examples. The reactions may be performed in a solvent appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention.

The various substituents on the synthetic intermediates and final products shown in the following reaction schemes can be present in their fully elaborated forms, with suitable protecting groups where required as understood by one skilled in the art, or in precursor forms which can later be elaborated into their final forms by methods familiar to one skilled in the art. The substituents can also be added at various stages throughout the synthetic sequence or after completion of the synthetic sequence. In many cases, commonly used functional group manipulations can be used to transform one intermediate into another intermediate, or one compound of formula (IV) into another compound of formula (IV). Examples of such manipulations are conversion of an ester or a ketone to an alcohol; conversion of an ester to a ketone; interconversions of esters, acids and amides; alkylation, acylation and sulfonylation of alcohols and amines; and many others. Substituents can also be added using common reactions, such as alkylation, acylation, halogenation or oxidation. Such manipulations are well-known in the art, and many reference works summarize procedures and methods for such manipulations. Some reference works which gives examples and references to the primary literature of organic synthesis for many functional group manipulations, as well as other transformations commonly used in the art of organic synthesis are March's Organic Chemistry, 5th Edition, Wiley and Chichester, Eds. (2001); Comprehensive Organic Transformations, Larock, Ed., VCH (1989); Comprehensive Organic Functional Group Transformations, Katritzky et al. (series editors), Pergamon (1995); and Comprehensive Organic Synthesis, Trost and Fleming (series editors), Pergamon (1991). It will also be recognized that another major consideration in the planning of any synthetic route in this field is the judicious choice of the protecting group used for protection of the reactive functional groups present in the compounds described in this invention. Multiple protecting groups within the same molecule can be chosen such that each of these protecting groups can either be removed without removal of other protecting groups in the same molecule, or several protecting groups can be removed using the same reaction step, depending upon the outcome desired. An authoritative account describing many alternatives to the trained practitioner is Greene and Wuts, Protective Groups in Organic Synthesis, Wiley and Sons (1999).

Pharmacological activity

Having regard to their blockade of the epithelial sodium channel (ENaC), compounds of formula (IV), in free or pharmaceutically acceptable salt form, hereinafter alternately referred to as "agents of the invention", in combination with an CF Modulator modulator of Columns A, B, or C are useful in the treatment of conditions which respond to the blockade of the epithelial sodium channel, particularly conditions benefiting from mucosal hydration.

Diseases mediated by blockade of the epithelial sodium channel, include diseases associated with the regulation of fluid volumes across epithelial membranes. For example, the volume of airway surface liquid is a key regulator of mucociliary clearance and the maintenance of lung health. The blockade of the epithelial sodium channel will promote fluid accumulation on the mucosal side of the airway epithelium thereby promoting mucus clearance and preventing the accumulation of mucus and sputum in respiratory tissues (including lung airways). Such diseases include respiratory diseases, such as cystic fibrosis, primary ciliary dyskinesia, chronic bronchitis, chronic obstructive pulmonary disease (COPD), asthma, respiratory tract infections (acute and chronic; viral and bacterial) and lung carcinoma. Diseases mediated by blockade of the epithelial sodium channel also include diseases other than respiratory diseases that are associated with abnormal fluid regulation across an epithelium, perhaps involving abnormal physiology of the protective surface liquids on their surface, e.g., xerostomia (dry mouth) or keratoconjunctivitis sire (dry eye). Furthermore, blockade of the epithelial sodium channel in the kidney could be used to promote diuresis and thereby induce a hypotensive effect.

Treatment in accordance with the invention may be symptomatic or prophylactic.

Asthma includes both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g., of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, *e.g.,* of acute asthmatic or bronchoconstrictor attack, improvement in lung function or improved airways hyperreactivity. It may further be evidenced by reduced requirement for other, symptomatic therapy, i.e., therapy for or intended to restrict or abort symptomatic attack when it occurs, *e.g.,* anti-inflammatory *(e.g.,* cortico-steroid) or bronchodilatory. Prophylactic benefit in asthma may, in particular, be apparent in subjects prone to "morning dipping". "Morning dipping" is a recognized asthmatic syndrome, common to a substantial percentage of asthmatics and characterized by

asthma attack, e.g., between the hours of about 4-6 am, i.e., at a time normally substantially distant from any previously administered symptomatic asthma therapy.

Chronic obstructive pulmonary disease includes chronic bronchitis or dyspnea associated therewith, emphysema, as well as exacerbation of airways hyperreactivity consequent to other drug therapy, in particular, other inhaled drug therapy. The invention is also applicable to the treatment of bronchitis of whatever type or genesis including, e.g., acute, arachidic, catarrhal, croupus, chronic or phthinoid bronchitis.

The agents of the invention may also be useful as acid-sensing ion channel (ASIC) blockers. Thus they may be useful in the treatment of conditions which respond to the blockade of the acid-sensing ion channel.

The suitability of epithelial sodium channel blocker as a treatment of a disease benefiting from mucosal hydration, may be tested by determining the inhibitory effect of the channel blocker on ENaC in a suitable cell-based assay. For example single cells or confluent epithelia, endogenously expressing or engineered to over express ENaC can be used to assess channel function using electrophysiological techniques or ion flux studies. See methods described in: Hirsh et al., J Pharm Exp Ther (2004); Moody et al., Am J Physiol Cell Physiol (2005).

Epithelial sodium channel blockers, including the compounds of formula (IV), are also useful as co-therapeutic agents for use in combination with other drug substances, such as anti-inflammatory, bronchodilatory, antihistamine or anti-tussive drug substances, particularly in the treatment of cystic fibrosis or obstructive or inflammatory airways diseases such as those mentioned hereinbefore, e.g., as potentiators of therapeutic activity of such drugs or as a means of reducing required dosaging or potential side effects of such drugs.

The epithelial sodium channel blocker may be mixed with the CF Modulator active agent in a fixed pharmaceutical composition or it may be administered separately, before, simultaneously with or after the other drug substance.

Accordingly, the invention includes as a further aspect a combination of ENaC inhibitor and an CF Modulator modulator selected from at least one of Columns A, B, or C, optionally, with osmotic agents (hypcrtonic saline, dcxtran, mannitol, Xylitol) + modifiers of CFTR function, both wild-type and mutant (correctors + potentiators), e.g., those described in WO 2007/021982, WO 2006/099256, WO 2006/127588, WO 2004/080972, WO 2005/026137, WO 2005/035514, WO 2005/075435, WO 2004/111014, WO 2006/101740, WO 2004/110352, WO 2005/120497 and US 2005/0176761, an anti-inflammatory, bronchodilatory, antihistamine, anti-tussive, antibiotic or DNase drug substance, said epithelial sodium channel blocker and said drug substance being in the same or different pharmaceutical composition.

Suitable antibiotics include macrolide antibiotics, e.g., tobramycin (TOBI™).

Suitable DNase drug substances include dornase alfa (Pulmozyme™), a highly-purified solution of recombinant human deoxyribonuclease I (rhDNase), which selectively cleaves DNA. Dornase alfa is used to treat cystic fibrosis.

Other useful combinations of ENaC inhibitors and CF Modulator modulator selected from at least one of Columns A, B, or C include combinations with anti-inflammatory drugs, e.g. those with antagonists of chemokine receptors, e.g., CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists, such as Schering-Plough antagonists SC-351125, SCH-55700 and SCH-D; Takeda antagonists, such as *N*-[[4-[[[6,7-dihydro-2-(4-methyl-phenyl)-*5H*-benzo-cyclohepten-8-yl]carbonyl]amino]phenyl]-methyl]tetrahydro-N, *N*-dimethyl-*2H*-pyran-4-amin-ium chloride (TAK-770); and CCR-5 antagonists described in USP 6,166,037 (particularly claims 18 and 19), WO 00/66558 (particularly claim 8), WO 00/66559 (particularly claim 9), WO 04/018425 and WO 04/026873.

Suitable anti-inflammatory drugs include steroids, in particular, glucocorticosteroids, such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists, such as montelukast and zafirlukast; PDE4 inhibitors, such as cilomilast (Ariflo® GlaxoSmithKline), Roflumilast (Byk Gulden),V-11294A (Napp), BAY19-8004 (Bayer), SCII-351591 (Schering-Plough), Arofylline (Almirall Prodesfarma), PD189659/PD168787 (Parke-Davis), AWD-12-281 (Asta Medica), CDC-801 (Celgene), SeICID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; adenosine A2B receptor antagonists such as those described in WO 02/42298; and beta-2 adrenoceptor agonists, such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula (IV) of WO 0075114, which document is incorporated herein by reference, preferably compounds of the Examples thereof, especially a compound of formula:

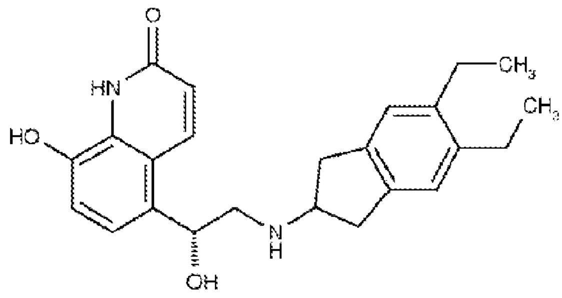

,

corresponding to indacaterol and pharmaceutically acceptable salts thereof, as well as compounds (in free or salt or solvate form) of formula (IV) of WO 04/16601, and also compounds of EP 1440966, JP 05025045, WO 93/18007, WO 99/64035, USP 2002/0055651, WO 01/42193, WO 01/83462, WO 02/66422, WO 02/70490, WO 02/76933, WO 03/2-[439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618, WO 04/46083, WO 04/80964, WO 04/108765 and WO 04/108676.

Suitable bronchodilatory drugs include anticholinergic or antimuscarinic agents, in particular, ipratropium bromide, oxitropium bromide, tiotropium salts and CHF 4226 (Chiesi), and glycopyrrolate, but also those described in EP 424021, USP 3,714,357, USP 5,171,744, WO 01/04118, WO 02/00652, WO 02/51841, WO 02/53564, WO 03/00840, WO 03/33495, WO 03/53966, WO 03/87094, WO 04/018422 and WO 04/05285.

Suitable dual anti-inflammatory and bronchodilatory drugs include dual beta-2 adrenoceptor agonist/muscarinic antagonists such as those disclosed in USP 2004/0167167, WO 04/74246 and WO 04174812.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine, as well as those disclosed in JP 2004107299, WO 03/099807 and WO 04/026841.

In accordance with the foregoing, the invention also provides as a further aspect a method for the treatment of a condition responsive to blockade of the epithelial sodium channel, e.g., diseases associated with the regulation of fluid volumes across epithelial membranes, particularly an obstructive airways disease, which comprises administering to a subject, particularly a human subject, in need thereof a compound of formula (IV), in free form or in the form of a pharmaceutically acceptable salt.

In another aspect the invention provides a compound of formula (IV), in free form or in the form of a pharmaceutically acceptable salt, for use in the manufacture of a medicament for the treatment of a condition responsive to blockade of the epithelial sodium channel, particularly an obstructive airways disease, e.g., cystic fibrosis and COPD.

The agents of the invention may be administered by any appropriate route, e.g. orally, e.g., in the form of a tablet or capsule; parenterally, e.g., intravenously; by inhalation, e.g., in the treatment of an obstructive airways disease; intranasally, e.g., in the treatment of allergic rhinitis; topically to the skin; or rectally. In a further aspect, the invention also provides a pharmaceutical composition comprising a compound of formula (IV), in free form or in the form of a pharmaceutically acceptable salt, optionally together with a pharmaceutically acceptable diluent or carrier. The composition may contain a co-therapeutic agent, such as an anti-inflammatory, broncho-dilatory, antihistamine or anti-tussive drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g., patches. Compositions for inhalation may comprise aerosol or other atomizable formulations or dry powder formulations.

When the composition comprises an aerosol formulation, it preferably contains, e.g., a hydro-fluoro-alkane (HFA) propellant, such as HFA134a or HFA227 or a mixture of these, and may contain one or more co-solvents known in the art, such as ethanol (up to 20% by weight), and/or one or more surfactants, such as oleic acid or sorbitan trioleate, and/or one or more bulking agents, such as lactose. When the composition comprises a dry powder formulation, it preferably contains, e.g., the compound of formula (IV) having a particle diameter up to 10 microns, optionally together with a diluent or carrier, such as lactose, of the desired particle size distribution and a compound that helps to protect against product performance deterioration due to moisture, e.g., magnesium stearate. When the composition comprises a nebulised formulation, it preferably contains, e.g., the compound of formula (IV) either dissolved, or suspended, in a vehicle containing water, a co-solvent, such as ethanol or propylene glycol and a stabilizer, which may be a surfactant.

Further aspects of the invention include:

a compound of formula (IV) in inhalable form, e.g., in an aerosol or other atomisable composition or in inhalable particulate, e.g., micronised form;

an inhalable medicament comprising a compound of formula (IV) in inhalable form;
a pharmaceutical product comprising a compound of formula (IV) in inhalable form in association with an inhalation device; and
an inhalation device containing a compound of formula IV in inhalable form.

Dosages of compounds of formula (IV) employed in practicing the present invention will of course vary depending, e.g., on the particular condition to be treated, the effect desired and the mode of administration. In general, suitable daily dosages for administration by inhalation are of the order of 0.005-10 mg, while for oral administration suitable daily doses are of the order of 0.05-100 mg.

Pharmaceutical Use and Assay

Compounds of formula (IV) and their pharmaceutically acceptable salts, hereinafter referred to alternatively as "agents of the invention", are useful as pharmaceuticals. In particular, the compounds have good ENaC blocker activity and may be tested in the following assays. *Cell culture*

Human Bronchial Epithelial cells (HBECs) (Cambrex) were cultured under air-liquid interface conditions to provide a well differentiated mucociliary phenotype.

HBECs were cultured using a modification of the method described by Gray and colleagues (Gray et al., 1996). Cells were seeded in plastic T-162 flasks and were grown in bronchial epithelial cell growth medium (BEGM; Cambrex) supplemented with bovine pituitary extract (52 [μg/mL), hydrocortisone (0.5 [μg/mL), human recombinant epidermal growth factor (0.5 ng/mL), epinephrine (0.5 [μg/mL), transferrin (10 μg/mL), insulin (5 μg/mL), retinoic acid (0.1 μg/mL), triiodothyronine (6.5 μg/mL), gentamycin (50 μg/mL) and amphotericin B (50 ng/mL). Medium was changed every 48 hours until cells were 90% confluent. Cells were then passaged and seeded (8.25 x $10^5$ cells/insert) on polycarbonate Snapwell inserts (Costar) in differentiation media containing 50% DMEM in BEGM with the same supplements as above but without triiodothyronine and a final retinoic acid concentration of 50 nM (all-trans retinoic acid). Cells were maintained submerged for the first 7 days in culture, after which time they were exposed to an apical air interface for the remainder of the culture period. At this time, media was changed to DMEM:F12 media containing 2% v/v Ultroser G for the remainder of culture. Amphotericin B was removed from all media 3 feeds prior to use in the Ussing Chambers. Cells were used between days 7 and 21 after establishment of the apical-air interface. At all stages of culture, cells were maintained at 37°C in 5% $CO_2$ in an air incubator.

*Short circuit current (ISC) measurements*

Snapwell inserts were mounted in Vertical Diffusion Chambers (Costar) and were bathed with continuously gassed Ringer solution (5% $CO_2$ in $O_2$; pH 7.4) maintained at 37°C containing (in mM): 120 NaCl, 25 $NaHCO_3$, 3.3 $KH_2PO_4$, 0.8 $K_2HPO_4$, 1.2 $CaCl_2$, 1.2 $MgCl_2$, and 10 glucose. The solution osmolarity was between 280 and 300 mOsmol/kg $H_2O$ for all physiological salt solutions used. Cells were voltage clamped to 0 mV (model EVC4000; WPI). RT was measured by applying a 1- or 2-mV pulse at 30-s intervals and calculating RT by Ohm's law. Data were recorded using a PowerLab workstation (ADInstruments).

Test compounds were prepared as a 10 mM stock solution in DMSO (95%). Serial 3-fold dilutions were freshly prepared in an appropriate vehicle (distilled $H_2O$ or Ringers solution). The initial concentration was added to the apical chamber as a 1000x concentrate in 5 μL, resulting in a final 1x concentration the 5 mL volume of the Ussing chamber. Subsequent additions of compound were added in a 3.3 μL volume of the 1000x serially diluted stock solution. At the completion of the concentration-response experiment, amiloride (10 μM) was added into the apical chamber to enable the total amiloride-sensitive current to be measured. An amiloride control $IC_{50}$ was established at the start of each experiment.

Results are expressed as the mean % inhibition of the amiloride-sensitive ISC. Concentration-response curves were plotted and $IC_{50}$ values generated using GraphPad Prism 3.02. Cell inserts were typically run in duplicate and the $IC_{50}$ calculated on the mean % inhibition data.

Compounds of the Examples, herein below, generally have $IC_{50}$ values in the data measurements described above below 10 μM. For example, the compounds of the Examples shown below have the indicated $IC_{50}$ values.

| EX | $IC_{50}$ (μM) |
|---|---|
| 5 | 0.065 |
| 11 | 1.686 |
| 19 | 0.018 |

(continued)

| EX | $IC_{50}$ (μM) |
|---|---|
| 23 | 0.0335 |
| 25 | 0.270 |
| 26 | 0.011 |
| 29 | 0.005 |
| 32 | 0.018 |
| 34 | 0.095 |
| 35 | 0.031 |
| 39 | 0.0055 |
| 40 | 0.0055 |
| 41 | 0.0095 |
| 42 | 0.011 |
| 43 | 0.013 |
| 44 | 0.0295 |
| 45 | 0.0426 |
| 48 | 0.0165 |
| 58 | 0.143 |
| 61 | 0.3465 |
| 62 | 0.013 |
| 64 | 0.0255 |
| 65 | 0.0395 |
| 70 | 0.074 |
| 71 | 0.042 |
| 76 | 0.012 |
| 86 | 0.008 |
| 91 | 0.0885 |
| 94 | 0.009 |
| 96 | 0.037 |
| 99 | 0.019 |
| 118 | 0.175 |
| 126 | 0.025 |
| 128 | 0.0115 |
| 141 | 0.002 |
| 146 | 0.006 |
| 147 | 0.016 |
| 185 | 0.062 |
| 215 | 0.036 |
| 220 | 0.0085 |
| 228 | 0.0935 |

(continued)

| EX | $IC_{50}$ (μM) |
|---|---|
| 232 | 0.054 |
| 235 | 0.364 |
| 238 | 0.0119 |
| 246 | 0.025 |
| 252 | 0.028 |

The invention is illustrated by the following Examples.

EXAMPLES

Compounds of Formula IV(b)

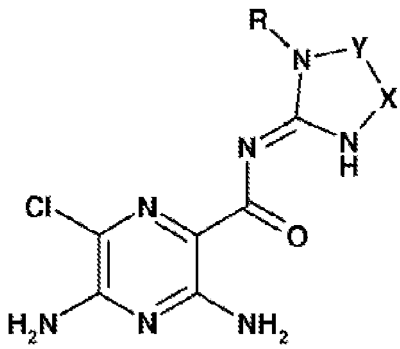

Ib

Formula IV(b)

are shown in Table II.D-2. Methods for preparing such compounds are described hereinafter. The table also shows mass spectrometry $[M+H]^+$ data.

Table II.D-2.

| Ex. | M/s $[M+H]^+$ |
|---|---|
| 1 | 284 |
| 2 | 270 |
| 3 | 270 |
| 4 | 408 |
| 5 | 461 |
| 6 | 418 |
| 7 | 404 |
| 8 | 376/378 |
| 9 | 400 |
| 10 | 328 |
| 11 | 310 |
| 12 | 415 |
| 13 | 404 |
| 14 | 270 |
| 15 | 296 |
| 16 | 310 |
| 17 | 390 |
| 18 | 390 |
| 19 | 464 |
| 20 | 464 |
| 21 | 464 |
| 22 | 464 |
| 23 | 517 |
| 24 | 418.2 |
| 25 | 418.2 |
| 26 | 446 |
| 27 | 356 |
| 28 | 506 |

| Ex. | M/s $[M+H]^+$ |
|---|---|
| 29 | 506.37 |
| 30 | 447.1 |
| 31 | 313.1 |
| 32 | 446.1 |
| 33 | 467.0 |
| 34 | 430.98 |
| 35 | 481.0 |
| 36 | 445.1 |
| 37 | 425 |
| 38 | 325 |
| 39 | 626.4 |
| 40 | 607.42 |
| 41 | 607.98 |
| 42 | 510.4 |
| 43 | 529.05 |
| 44 | 499.0 |
| 45 | 542.91 |
| 46 | 552.1 |
| 47 | 469.17 |
| 48 | 510.23 |
| 49 | 510.1 |
| 50 | 483.1 |
| 51 | 535.1 |
| 52 | 499.1 |
| 53 | 469.14 |
| 54 | 487.0 |
| 55 | 472.98 |
| 56 | 468.1 |

| Ex. | M/s $[M+H]^+$ |
|---|---|
| 57 | 480.1 |
| 58 | 521.1 |
| 59 | 528.2 |
| 60 | 469.08 |
| 61 | 597.07 |
| 62 | 530.21 |
| 63 | 553.54 |
| 64 | 529.54 |
| 65 | 530.46 |
| 66 | 513.40 |
| 67 | 547.42 |
| 68 | 561.04 |
| 69 | 601.10 |
| 70 | 564.10 |
| 71 | 587.50 |
| 72 | 530.10 |
| 73 | 599.10 |
| 74 | 615.20 |
| 75 | 545.10 |
| 76 | 529.41 |
| 77 | 524 |
| 78 | 571 |
| 79 | 557 |
| 80 | 543 |
| 81 | 529 |
| 82 | 588 |
| 83 | 586/588 |
| 84 | 597 |
| 85 | 618 |
| 86 | 644/646 |
| 87 | 582/584 |
| 88 | 540 |

| Ex. | M/s $[M+H]^+$ |
|---|---|
| 89 | 568/570 |
| 90 | 600/602 |
| 91 | 581/583 |
| 92 | 512/514 |
| 93 | 785 |
| 94 | $[M+2H]^{2+}$=393 |
| 95 | 787 |
| 96 | 779 |
| 97 | 549 |
| 98 | 563 |
| 99 | 468 |
| 100 | 468 |
| 101 | 443 |
| 102 | 675 |
| 103 | 463 |
| 104 | 653 |
| 105 | 455 |
| 106 | 429 |
| 107 | 469 |
| 108 | 423 |
| 109 | 453 |
| 110 | 419 |
| 111 | 395 |
| 112 | 454 |
| 113 | 430 |
| 114 | 487 |
| 115 | 431 |
| 116 | 445 |
| 117 | 435 |
| 118 | 420 |
| 119 | 430 |

| Ex. | M/s $[M+H]^+$ |
|---|---|
| 120 | 430 |
| 121 | 436 |
| 122 | 419 |
| 123 | 437 |
| 124 | 431 |
| 125 | 420 |
| 126 | 648.4 |
| 127 | 651.3 |
| 128 | 648.3 |
| 129 | 576.3 |
| 130 | 633.3 |
| 131 | 592.3 |
| 132 | 599.3 |
| 133 | 610.3 |
| 134 | 634.3 |
| 135 | 613.3 |
| 136 | 654.3 |
| 137 | 664.3 |
| 138 | 645.4 |
| 139 | 614.3 |
| 140 | 593.4 |
| 141 | 702.3 |
| 142 | 594.3 |
| 143 | 643.3 |
| 144 | 736.4 |
| 145 | 626.3 |
| 146 | 559.3 |
| 147 | 572.08 |
| 148 | 572.0 |
| 149 | 538.4 |
| 150 | 544.4 |
| 151 | 569.4 |

| Ex. | M/s $[M+H]^+$ |
|---|---|
| 152 | 511.4 |
| 153 | 604.3 |
| 154 | 528.3 |
| 155 | 633.4 |
| 156 | 526.3 |
| 157 | 556.4 |
| 158 | 604.4 |
| 159 | 617.4 |
| 160 | 594.4 |
| 161 | 528.4 |
| 162 | 478.3 |
| 163 | 462.3 |
| 164 | 691.04 |
| 165 | 573.05 |
| 166 | 648.06 |
| 167 | 545.3 |
| 168 | 517.07 |
| 169 | 484.04 |
| 170 | 511.04 |
| 171 | 530.08 |
| 172 | 603.99 |
| 173 | 530.19 |
| 174 | 527.99 |
| 175 | 555.07 |
| 176 | 608.05 |
| 177 | 527.07 |
| 178 | 524.1 |
| 179 | 520.99 |
| 180 | 545.95 |
| 181 | 514.98 |
| 182 | 512.01 |
| 183 | 478.01 |

| Ex. | M/s [M+H]+ |
|---|---|
| 184 | 475.08 |
| 185 | 572.09 |
| 186 | 634.09 |
| 187 | 619.12 |
| 188 | 496.02 |
| 189 | 685.08 |
| 190 | 599.2 |
| 191 | 542.01 |
| 192 | 579.03 |
| 193 | 526.05 |
| 194 | 553.09 |
| 195 | 614.3 |
| 196 | 516.06 |
| 197 | 496.01 |
| 198 | 528.04 |
| 199 | 560.14 |
| 200 | 490.05 |
| 201 | 528.06 |
| 202 | 527.02 |
| 203 | 458.1 |
| 204 | 540.02 |
| 205 | 539.11 |
| 206 | 433.05 |
| 207 | 635.19 |
| 208 | 447.09 |
| 209 | 509.09 |
| 210 | 542.00 |
| 211 | 564.06 |
| 212 | 539.11 |
| 213 | 445.96 |
| 214 | 620.1 |
| 215 | 458.1 |

| Ex. | M/s [M+H]+ |
|---|---|
| 216 | |
| 217 | 637.1 |
| 218 | 598.05 |
| 219 | 554.0 |
| 220 | 578.2 |
| 221 | 539.2 |
| 222 | 557.2 |
| 223 | 564.1 |
| 224 | 610.2 |
| 225 | 532.1 |
| 226 | 566.1 |
| 227 | 539.2 |
| 228 | 487.1 |
| 229 | 620.2 |
| 230 | 564.2 |
| 231 | 578.2 |
| 232 | 478.98 |
| 233 | |
| 234 | 517.9 |
| 235 | 518.1 |
| 236 | 540.9 |
| 237 | 493.1 |
| 238 | 652.2 |
| 239 | 615.1 |
| 240 | 520.1 |
| 241 | 527.0 |
| 242 | 581.1 |
| 243 | 527.1 |
| 244 | 616.1 |
| 245 | 527.0 |
| 246 | 429 |
| 247 | 445 |

| Ex. | M/s $[M+H]^+$ |
|---|---|
| 248 | 416 |
| 249 | 443 |
| 250 | 421 |

| Ex. | M/s $[M+H]^+$ |
|---|---|
| 251 | 451 |
| 252 | 494.15 |
| 253 | 589.20 |

General Synthesis Conditions

LCMS are recorded using a Phenomenex Gemini 50 mm x 3.0 mm, 3um column. Low pH methods use a gradient of 5-95% acetonitrile in water -0.1 % TFA, high pH methods use 5-95% acetonitrile in water -0.1% $NH_3$. $[M+H]^+$ refer to monoisotopic molecular weights.

| | |
|---|---|
| 9-BBN | 9-Borabicyclo [3.3.1]nonane |
| DBU | Diazabicyclo [5.4.0] undec-7-ene |
| DMF | dimethylformamide |
| DMSO | dimethyl sulfoxide |
| DCM | dichloromethane |
| DEAD | diethyl azodicarboxylate |
| DIAD | diisopropyl azodicarboxylate |
| DIPEA | diisopropylethylamine |
| EDCI | 1-ethyl-3-(3'-dimethylaminopropyl) carbodiimide |
| Er0Ac | ethyl acetate |
| HATU | 2- (7-Aza-1H-benzotriazole-1-y1)-1,1,3,3-tetramethyluronium hexafluorophosphate |
| HPLC | high performance liquid chromatography |
| IPA | Isopropyl alcohol (iso-propanol) |
| MeOII | methanol |
| MEMC1 | 2- methoxyethoxymethyl chloride |
| NMR | nuclear magnetic resonance |
| PS | polymer supported |
| PPTS | Pyridinium *para*-toluenesulfonate |
| PEAX | PE-anion exchange (e.g. Isolute® PE-AX columns from Biotage) |
| SCX-2 | strong cation exchange (e.g. Isolute® SCX-2 columns from Biotage) |
| TEA | triethylamine |
| THF | tetrahydrofuran |
| TFA | trifluoroacetic acid |

PREPARATION OF EXAMPLES

For clarity in describing the Examples described below. Examples 2, 9, and 10 are racemic mixtures. Examples 4 ,13 and 29 are mixtures of diastereomers. Examples 24 and 25 are single enantiomers wherein the stereochemistry of the unassigned stereocentre is not determined. All other examples are single enantiomers of defined stereochemistry.

Where not stated, the compounds are recovered from reaction mixtures and purified using conventional techniques such as flash chromatography, filtration, recrystallisation and trituration.

Example 1

3,5-Diamino-6-chloro-pyrazine-2-carboxylic acid [4,4-dimethyl-imidazolidin-(2Z)-ylidene]-amide

A suspension of 1-(3,5-diamino-6-chloro-pyrazine-2-carbonyl)-2-methyl-isothiourea (Intermediate A) (0.2 g, 0.517 mmol) in EtOH (2 ml) is treated with triethylamine (0.029 ml, 0.258 mmol) followed by 1,2-diamino-2-methylpropane (0.07 ml, 0.672 mmol) and stirred at reflux overnight. The resulting suspension is filtered under vacuum to afford the

title compound as a pale yellow solid; $[M+H]^+$ 284

Example 2

3,5-Diamino-6-chloro-pyrazine-2-carboxylic acid [4-methyl-imidazolidin-(2Z)-ylidene]-amide

This compound is prepared analogously to Example 1 by replacing 1,2-diamino-2-methylpropane with 1,2,diaminopropane; $[M+H]^+$ 270.

Example 3

3,5-Diamino-6-chloro-pyrazine-2-carboxylic acid [1-methyl-imidazolidin-(2Z)-ylidene]-amide

This compound is prepared analogously to Example 1 by replacing 1,2-diamino-2-methylpropane with N-methylenediamine; $[M+H]^+$270.

Example 4

3,5-Diamino-6-chloro-pyrazine-2-carboxylic acid (4,5-diphenyl-imidazolidin-2-ylidene)-amide

This compound is prepared analogously to Example 1 by replacing 1,2-diamino-2-methylpropane with 1,2 diphenylethylene diamine; $[M+H]^+$ 408.

Example 5

(4-{2-[(Z)-3,5-Diamino-6-chloro-pyrazine-2-carbonylimino]-imidazolidin-4-yl}-butyl)-carbamic acid benzyl ester

This compound is prepared analogously to Example 1 by replacing 1,2-diamino-2-methylpropane with ((S)-5,6-Diamino-hexyl)-carbamic acid benzyl ester (Intermediate B); $[M+H]^+$461.

Example 6

3,5-Diamiuo-6-chloro-pyrazine-2-carboxylic acid [1-[4-(4-methoxy-phenyl)-butyl]-imidazolidin-(2Z)-ylidene]-amide

This compound is prepared analogously to Example 1 by replacing 1,2-diamino-2-methylpropane with N*1*-[4-(4-methoxy-phenyl)-butyl]-ethane-1,2-diamine (Intermediate C); $[M+H]^+$ 418.

Example 7

3,5-Diamino-6-chloro-pyrazine-2-carboxylic acid [1-[4-(4-hydroxy-phenyl)-butyl]-imidazolidin-(2Z)-ylidene]-amide

This compound is prepared analogously to Example 1 by replacing 1,2-diamino-2-methylpropane with 4-[4-(2-amino-ethylamino)-butyl]-phenol (Intermediate C); $[M+H]^+$404.

Example 8

3,5-Diamino-6-chloro-pyrazine-2-carboxylic acid [(S)-4-(4-methoxy-benzyl)-imidazolidin-(2Z)-ylidene]-amide

This compound is prepared analogously to Example 1 by replacing 1,2-diamino-2-methylpropane with (S)-3-(4-methoxy-phenyl)-propane-1,2 diamine (Intermediate D); $[M+H]^+$376.

Example 9

3,5-Diamino-6-chloro-pyrazine-2-carboxylic acid [4-(3,4-dichloro-phenyl)-imidazolidin-(2Z)-ylidene]-amide

This compound is prepared analogously to Example 1 by replacing 1,2-diamino-2-methylpropane with 1-(3,4-Dichloro-phenyl)-ethane-1,2-diamine (Intermediate E); $[M+H]^+$400.

Example 10

3-{2-[(Z)-3,5-Diamino-6-chloro-pyrazine-2-carbonylimino]-imidazolidin-4-yl}-propionic acid

This compound is prepared analogously to Example 1 by replacing 1,2-diamino-2-methylpropane with 4,5-Diaminopentanoic acid dihydrochloride (Intermediate F); $[M+H]^+$328.

Examples 2-10

These compounds are recovered from reaction mixtures and purified using conventional techniques such as flash chromatography, filtration, capture release resin or preparative HPLC.

Example 11

3,5-Diamino-6-chloro-pyrazine-2-carboxylic acid (octahydro-benzoimidazol-2-ylidene)-amide

This compound is prepared analogously to Example 1 by replacing 1,2-diamino-2-methylpropane with cyclohexane-1,2-diamine. The reaction is carried out in propan-2-ol; $[M+H]^+$310.

Example 12

3,5-Diamino-6-chloro-pyrazinc-2-carboxylic acid [8-bcnzyl-1,3,8-triazaspiro[4.5]dec-(2Z)-ylidene]-amide

4-Amino-1-benzyl-piperidine-4-carbonitrile (Intermediate G) (200 mg, 0.91 mmol) in dry propan-2-ol (10 ml) is treated with triethylamine (0.25 ml) followed by 1-(3,5-diamino-6-chloro-pyrazine-2-carbonyl)-2-methyl-isothiourea (Intermediate A) (355 mg, 0.91 mmol). The mixture is heated at 70°C for 5 hours and then allowed to cool to room temperature. The precipitate is collected and washed with methanol to afford the title compound as a light yellow solid, 190 mg; $[M+H]^+$415.

Example 13

3,5-Diamino-6-chloro-pyrazine-2-carboxylic acid [4-[3-(4-methoxy-phenyl)-propyl]-imidazolidin-(2Z)-ylidene]-amide

This compound is prepared analogously to Example 12 by replacing 4-Amino-1-benzyl-piperidine-4-carbonitrile (Intermediate G) with 5-(4-methoxy-phenyl)-pentane-1,2-diamine (Intermediate I); $[M+H]^+$404.

Example 14

3,5-Diamino-6-chloro-pyrazine-2-carboxylic acid (tetrahydro-pyrimidin-2-ylidene)-amide

1-(3,5-Diamino-6-chloro-pyrazine-2-carbonyl)-2-methyl-isothiourea (Intermediate A) (1.0 g, 2.58 mmol) is suspended in propan-2-ol (10 ml) and 1,3-diaminopropane (0.32 ml, 3.9 mmol) is added. The mixture is heated at 60°C for 18 hours and then allowed to cool to room temperature and the solids present are collected by filtration. The solids are washed with THF and MeOH to yield the title compound as a yellow solid; $[M+H]^+$270.

Example 15

3,5-diamino-6-chloro-N-(1H-pyrrolo [1,2-c] imidazol-3 (2H,5H,6H,7H,7aH)-ylidene)pyrazine-2-carboxamide

1-(3,5-Diamino-6-chloro-pyrazine-2-carbonyl)-2-methyl-isothiourea (Intermediate A) (195 mg, 0.5 mmol) is suspended in propan-2-ol (10 ml) and (S)-2-(aminomethyl)pyrrolidine (100 mg, 1 mmol) is added. The mixture is heated at 60°C for 18 hours, allowed to cool to room temperature and the precipitate is removed by filtration. The filtrate is concentrated *in vacuo* and the residue purified by chromatography ($SiO_2$, DCM/MeOH) to afford the title compound as a light, yellow gum; $[M+H]^+$296.

Example 16

3,5-Diamino-6-chloro-pyrazine-2-carboxylic acid [1,3-diaza-spiro[4.4]non-(2Z)-ylidene]-amide

A solution of crude 1-aminomethyl-cyclopentylamine (Intermediate J) (80 mg, 0.70 mmol) in propan-2-ol (1.0 ml) is added to a suspension of 1-(3,5-diamino-6-chloro-pyrazine-2-carbonyl)-2-methyl-isothiourea (Intermediate A) (208 mg, 0.54 mmol) in propan-2-ol (1.08 ml) and heated at 70°C for 2 days. After cooling to room temperature, the reaction mixture is filtered under vacuum, and the solid is rinsed with MeOH. The filtrate is concentrated *in vacuo* to afford a bright yellow residue which is loaded onto a SCX-2 cartridge and eluted with 33% $NH_3$ (4 drops) in MeOH (5 ml x2). The methanolic ammonia fractions are combined and concentrated *in vacuo.* Purification using mass directed preparative LCMS eluting with 95% Water + 0.1% $NH_3$ : 5% Acetonitrile to affords the title compound; $[M+H]^+$ 310.

Example 17

3,5-Diamino-6-chloro-pyrazine-2-carboxylic acid [(R)-4-[3-(4-hydroxy-phenyl)-propyl]-imidazolidin-(2E)-ylidene] -amide

To a stirred solution of (4-((R)-4,5-Diamino-pentyl)-phenol (intermediate K) (1.5 g, 7.72 mmol) in propan-2-ol (100 ml) at 30°C is added in one portion 1-(3,5-Diamino-6-chloro-pyrazine-2-carbonyl)-2-methyl-isothiourea (Intermediate A) and the reaction is heated at 30°C for 18 hours followed by 50°C for a further 18 hours. The reaction mixture is filtered hot and the filtrate solvent is removed *in vacuo* to afford a yellow foam. The foam is purified by chromatography ($SiO_2$, DCM /MeOH/5% $NH_3$) to afford the title compound; $[M+H]^+$390.

Example 18

3,5-Diamino-6-chloro-pyrazine-2-carboxylic acid [(S)-4-[3-(4-hydroxy-phenyl)-propyl]-imidazolidin-(2E)-ylidene]-amide

This compound is prepared analogously to Example 17 replacing (4-((R)-4,5- Diamino-pentyl)-phenol (Intermediate K) with 4-((S)-4,5-Diamino-pentyl)-phenol (intermediate L; $[M+H]^+$390.

Example 19

3,5-Diamino-6-chloro-pyrazine-2-carboxylic acid [(R)-4-{3-[4-((S)-2,3-dihydroxy-propoxy)-phenyl]-propyl}imidazolidin-(2Z)-ylidene]-amide

To a stirred solution of 3,5-Diamino-6-chloro-pyrazine-2-carboxylic acid [(R)-4-[3-(4-hydroxy-phenyl)-propyl}-imidazolidin-(2E)-ylidene]-amide (Ex. 17) (1.0 g, 2.57 mmol) in 1,4 dioxane (38 ml) at 50°C is added in one portion 0.5 M KOH (5.3 ml, 2.7 mmol) followed by (S)-(-)-Glycidiol (0.170 ml, 2.57 mmol). The resulting mixture is heated at 50°C for 18 hours and then further (S)-(-)-Glycidiol (0.07 ml, 1.05 mmol) is added in one portion. The resulting mixture is heated at 50°C for 60 hours and then allowed to cool to room temperature. The solvent is removed *in vacuo* to afford an orange oil which is dissolved in EtOAc/MeOH 9:1 (100 ml) and washed with 1 M NaOH (50 ml). The organic layer is dried over $Na_2SO_4$ and the solvent is removed *in vacuo* to afford a brown/orange foam. Purification by chromatography ($SiO_2$, DCM/MeOH/$NH_3$) affords the title compound as a yellow foam; $[M+H]^+$464; $^1H$ NMR (400 MHz, DMSO-d6): 1.65-1.40 (m, 4H), 2.52 (m, 2H), 3.13 (dd, J=9.6, 7.1Hz, 1H), 3.42 (br d, J=4.7Hz, 2H), 3.62 (dd, J=9.6, 9.6Hz, 1H), 3.76 (m, 1H), 3.78 (m, 1H), 3.80 (m, 1H), 3.94 (dd, J= 9.5, 4.OHz, 1H), 4.62 (br s, 1H), 4.89 (br s, 1H), 6.68 (br s, 2H), 6.82 (d, J=8.5Hz, 2H), 7.09 (d, J=8.5Hz, 2H), 7.2-6.0 (br s, 1H), 8.18 (br s, 1H), 9.3-7.5 (br s, 1H).

Example 20

3,5-Diamino-6-chloro-pyrazine-2-carboxylic acid [(*S*)-4-{3-[4-((S)-2,3-dihydroxy-propoxy)-phenyl]-propyl}-imidazolidin-(2Z)-ylidene]-amide

To a solution of 3,5-Diamino-6-chloro-pyrazine-2-carboxylic acid [(S)-4-[3-(4-hydroxyphenyl)-propyl]-imidazolidin-(2E)-ylidene]-amide (Example 18) (37.5 mg, 0.09 mmol) in Ethanol (2 ml) is added triethylamine (63 μl, 0.45 mmol) and (S)-glycidol (6.07 μl, 0.09 mmol). The resulting mixture is heated at reflux for 18 hours and then allowed to cool to room temperature. The reaction mixture is diluted with MeOH (1 ml) and purified on a Waters 3000 prep HPLC system, (Microsorb C18, Water (0.1% TFA): MeCN) to afford the title compound; $[M+H]^+$464.

Example 21

(3,5-Diamino-6-chloro-pyrazine-2-carboxylic acid [(R)-4-{3-[4-((R)-2,3-dihydroxy-propoxy)-phenyl]propyl}-imidazolidin-(2Z)-ylidene]-amide

To a stirred solution of (R)-3-[4-((R)-4,5-Diamino-pentyl)-phenoxy]-propane-1,2-diol (Intermediate 0) (32.8 mg, 0.122 mmol) in propan-2-ol (3 ml) is added 1-(3,5- Diamino-6-chloropyrazine-2-carbonyl)-2-methyl-isothiourea (Intermediate A) (45.8 mg, 0.122 mmol) and the resultant reaction mixture is heated at 90°C for 18 hours. The reaction is allowed to cool to room temperature and diluted with DMSO (1.5 ml) and purified on a Waters 3000 preperative HPLC system (Microsorb™ C18, Water (0.1% TFA): MeCN). The fractions containing product are passed through a 1 g SCX-2 cartridge which is eluted with 1:1 Water:MeCN (20 ml), MeCN (20 ml) and 7M $NH_3$ in MeOH (20 ml). The ammonia elutions are concentrated *in vacuo* to afford the title compound; $[M+H]^+$464.

Example 22

3,5-Diamino-6-chloro-pyrazine-2-carboxylic acid [(S)-4-{3-[4-((R)-2,3-dihydroxy-propoxy)-phenyl]propyl}-imidazolidin-(2Z)-ylidene]-amide trifluroacetate

This compound is prepared analogously to Example 21 replacing (R)-3-[4-((R)-4,5-Diamino-pentyl)-phenoxy]-propane-1,2-diol (Intermediate O) with (R)-3-[4-((S)-4,5- Diamino-pentyl)-phenoxy]-propane-1,2-diol (Intermediate P); $[M+H]^+$464.

Example 23

3,5-Diamino-6-chloro-pyrazine-2-carboxylic acid [(R)-4-{3-[4-(2-morpholin-4-yl-2-oxo-ethoxy)-phenyl]-propyl}-imidazolidin-(2Z)-ylidene]-amide

This compound is prepared analogously to Example 21 replacing (R)-3-[4-((R)-4,5-Diamino-pentyl)-phenoxy]-propane-1,2-diol (Intermediate O) with 2-[4-((R)-4,5- Diamino-pentyl)-phenoxy]-1-morpholin-4-yl-ethanone (Intermediate Q); $[M+H]^+$517.

Examples 24 and 25

Both Enantiomers of 3,5-Diamino-6-chloro-pyrazine-2-carboxylic acid [4-[3-(4-methoxy-phenyl)-butyl]-imidazolidin-(2Z)-ylidene]-amide

The racemate of these compounds is prepared analogously to Example 12 replacing 4-Amino-1-benzyl-piperidine-4-carbonitrile (Intermediate G) with 5-(4-methoxy-phenyl)-hexane-1,2-diamine (Intermediate K). The enantiomers are separated by chiral HPLC:

Mobile phase: 100% EtOH (0.2% IPAm)
Column: Chirapak-AD 25cm x 4.6mm i.d
Flow rate: 1ml/min
UV 280nM
Concentration 1mg/mL
Inj Vol 10μL

Example 26

3,5-Diamino-6-chloro-pyrazine-2-carboyxlic acid [(S)-4-(4-benyloxy-2,2-dimethyl-butyl-imidazolidin-(2Z)-ylidene]-amide

Step 1

DEAD (4.49 ml, 28 mmol) is added to a stirred suspension of ((S)-5-benzyloxy-1-hydroxymethyl-3,-3-dimethyl-pentyl)-carbamic acid tert-butyl ester (prepared as described in EP 0702004 A2, Rueger et al., 10 g, 0.028 mmol), phthalimide (4.19 g, 0.028 mmol) and PS-triphenylphosphine (29.8 g, 56 mmol) in THF (500 ml), and the resulting reaction is stirred at room temperature for 3 days. The reaction is filtered to remove the PS-triphenylphosphine resin and the resin is washed with EtOAc (2 x 50 ml). The solvent is removed *in vacuo* and the residue is purified by flash

chromatography ($SiO_2$, EtOAc/iso-hexane) to afford [(*S*)-5-benzyloxy-1-(1,3-dioxo-1,3-dihydro-isoindol-2-ylmcthyl)-3,3-dimcthyl-pcntyl]-carbamic acid tcrt-butyl ester as a white solid; $[M+H]^+481$.

Step 2

Hydrazine (66.6 ml of a 1M solution in THF, 66.6 mmol) is added to a suspension of [(S)-5-benzyloxy-1-(1,3-dioxo-1,3-dihydro-isoindol-2-ylmethyl)-3,3-dimethyl-pentyl]- carbamic acid tert-butyl ester (4 g, 8.32 mmol) in ethanol (100 ml), and the resulting solution is heated at 40°C overnight. A fluffy white precipitate forms. The reaction is allowed to cool to room temperature and diethyl ether (100 ml) is added and the resulting white suspension cooled at 0°C for 30 minutes. The white precipitate is removed by filtration and the solvent removed *in vacuo.* The residue is then stirred with diethyl ether (100 ml) for 1 hour, filtered and the solvent is removed *in vacuo* to afford ((*S*)-1-Aminomethyl-5-benzyloxy-3,3-dimethyl-pentyl)-carbamic acid tert-butyl ester as a pale yellow oil; $[M+H]^+351$.

Step 3

Iodotrimethylsilane (1.63 ml, 11.94 mmol) is added dropwise to a solution of ((*S*)-1-Aminomethyl-5-benzyloxy-3,3-dimethyl-pentyl)-carbamic acid tert-butyl ester (2.79 g, 7.96 mmol) in DCM (30 ml) and the resulting yellow solution is stirred for 1 hour at room temperature. The reaction is filtered and the filtrate diluted with DCM (50 ml) and washed with 2 M NaOH (100 ml). The aqueous layer is allowed to stand overnight and any product which has oiled out of solution is extracted into EtOAc (100 ml). The organic layers are combined, dried over $MgSO_4$, and the solvent is removed *in vacuo* to yield (*S*)-Benzyloxy-4,4-dimethyl-hexane-1,2-diamine as a pale yellow oil; $[M+H]^+251$.

Step 4

A suspension of 1-(3,5-diamino-6-chloro-pyrazine-2-carbonyl)-2-methyl-isothiourea (Intermediate A) (2.56 g, 6.87 mmol) and (*S*)-Benzyloxy-4,4-dimethyl-hexane-1,2-diamine (1.72 g, 6.87 mmol) in propan-2-ol (50 ml) is heated at 90°C for 3 hours. The reaction is allowed to cool to room temperature, filtered to remove any insoluble material and the filter paper is washed with MeOH (50 ml). The filtrate is loaded on to a SCX-2 cartridge which has been pre-eluted with MeOH. The cartridge is eluted with MeOH and then 7M $NH_3$ in MeOH. Upon standing, a pale yellow solid crystallizes out of the $NH_3$ in MeOH solution. The solid is collected by filtration, washed with MeOH (20 ml) and dried *in vacuo* at 40°C to afford the title compound. $[M+H]^+446$.

Example 27

3,5-Diamino-6-chloro-pyrazine-2-carboxylic acid [(*S*)-4-(hydroxyl-2,2-dimethyl-butyl)-imidazolidin-(2Z)-ylidene]-amide

To a suspension of 3,5-Diamino-6-chloro-pyrazine-2-carboyxlic acid [(*S*)-4-(4-benyloxy-2,2-dimethyl-butyl-imidazolidin-(2Z)-ylidene]-amide (Ex. 26) (100 mg, 0.22 mmol) in DCM (5 ml) is added dropwise iodotrimethylsilane (0.061 ml, 0.448 mmol). The resulting yellow solution is heated at reflux for 2 days. The reaction is allowed to cool to room temperature and the yellow solid that has formed is collected by filtration, dissolved in MeOH (3 ml) and loaded onto a 10 g SCX-2 cartridge which has been pre-eluted with MeOH. The cartridge is eluted with MeOH (30 ml) and 7M $NH_3$ in MeOH (30 ml). The pale yellow 7M $NH_3$ in MeOH wash is concentrated *in vacuo* to afford the title compound as a yellow solid. $[M+H]^+356$.

Example 28

3,5-Diamino-6-chloro-pyrazine-2-carboxylic acid [(S)-4-{4-[4-(*S*)-2,3-dihydroxy-propoxy)-phenyl]-2,2-dimethyl-butyl}-imidazolidin-(2Z)-ylidene]-amide

Step 1

(S)-Glycidol (0.36 ml, 5.5 mmol) is added to a solution of 4-iodophenol (1 g, 4.5 mmol) and triethylamine (31 ml, 0.2 mmol) in ethanol (5 ml) and the resulting light brown solution is heated at reflux for 15 hours. The reaction is allowed to cool to room temperature and the solvent removed *in vacuo.* The residue is purified by chromatography ($SiO_2$, EtOAc/iso-hexane) to afford (*S*)-3-(4-Iodo-phenoxy)-propane-1,2-diol as a colorless oil.

Step 2

2,2-Dimethoxypropane (1.94 ml, 15.8 mmol) and PPTS (0.079 mg, 0.32 mmol) are added to a solution of (S)-3-(4-Iodo-phenoxy)-propane-1,2- diol (0.93 g, 3.16 mmol) in DMF (20 ml), and the resulting solution is left to stir at room temperature overnight. The solvent is removed *in vacuo* and the residue is purified by chromatography ($SiO_2$, EtOAc:Iso-hexane) to afford (R)-4-(4-Iodo-phenoxymethyl)-2,2-dimethyl-[1,3]dioxolane as a colorless oil.

Step 3

DEAD (0.63 ml, 4 mmol) is added to a suspension of ((*S*)-1-Hydroxymethyl-3,3-dimethyl-pent-4-enyl)-carbamic acid tert-butyl ester (1 g, 4 mmol), phthalimide (588 mg, 4 mmol) and PS-triphenylphosphine (3.72 g, 8 mmol) in THF (50 ml) and the resulting solution is stirred at room temperature overnight. The resin is removed by filtration, and the filtrate *concentrated in vacuo.* Purification by flash chromatography ($SiO_2$, EtOAc/iso-hexane) yields [(S)-1-(1,3-Dioxo-1,3-dihydro-isoindol-2-ylmethyl)- 3,3-dimethyl-pent-4-enyl]-carbamic acid tert-butyl ester as a white solid; $[M+H-BOC]^+$273.

Step 4

9-BBN (4.63 ml of a 0.5 M solution in THF, 0.23 mmol) is added to a solution of [(*S*)-1-(1,3-Dioxo-1,3-dihydro-isoindol-2-ylmethyl)-3,3-dimethyl-pent-4-enyl]-carbamic acid tert-butyl ester (0.43 g, 0.116 mmol) in THF (15 ml) and the resulting colorless solution is stirred at room temperature overnight. Anhydrous DMF (15 ml) is added to the solution, followed by 3 M aqueous $K_3PO_4$ solution (0.77 ml, 2.3 mmol), (R)-4-(4- Iodo-phenoxymethyl)-2, 2-dimethyl-[1,3]dioxolane (267 mg, 0.28 mmol) and Pd(dppf)$Cl_2$.DCM (47 mg, 0.058 mmol). The reaction is stirred at room temperature for 3 hours, 50°C for 2 hours and then is allowed to cool to room temperature and filtered through a pad of Celite™ (filter material) which is washed with EtOAc (3 x 50 ml). The combined filtrates are washed with sat. aq. $NaHCO_3$ solution (30 ml), dried ($MgSO_4$) and the solvent removed *in vacuo* to afford a black oil. Multiple chromatography ($SiO_2$, EtOAc/iso-hexane) yields [(S)-5-[4-((R)-2,2-Dimethyl-[1,3]dioxolan-4-ylmethoxy)-phenyl]-1-(1,3-dioxo-1,3-dihydro-isoindol-2-ylmethyl)-3,3-dimethyl-pentyl]-carbamic acid tert-butyl ester as a cream solid; $[M+H-BOC]^+$ 481.

Step 5

Hydrazine (2.2 ml of a 1M solution in THF, 2.2 mmol) is added to a solution of [(S)-5-[4-((R)-2,2-Dimethyl-[1,3]dioxolan-4-ylmethoxy)-phenyl]-1-(1,3-dioxo-1,3-dihydro-isoindol-2-ylmethyl)-3,3-dimethyl-pentyl]-carbamic acid tert-butyl ester (0.16 g, 0.28 mmol) in ethanol (5 ml), and the resulting colorless solution is heated at 45°C overnight. The reaction is allowed to cool to room temperature, and diethyl ether (30 ml) is added and the resulting white suspension cooled at 0°C for 30 minutes. The white solid is removed by filtration, and the solvent removed *in vacuo* to yield {(S)-1-Aminomethyl-5-[4-((R)-2,2-dimethyl-[1,3]dioxolan-4-ylmethoxy)-phenyl]-3,3-dimethyl-pentyl]-carbamic acid tert-butyl ester as a colorless oil; $[M+H]^+$ 451.

Step 6

A solution of {(S)-1-Aminomethyl-5-[4-((R)-2,2-dimethyl-[1,3]dioxolan-4-ylmethoxy)-phenyl]-3,3-dimethyl-pentyl} carbamic acid tert-butyl ester (0.13 g, 0.28 mmol) and TFA (1 ml) in DCM (5 ml) is stirred at room temperature for 1 hour, then loaded onto an SCX-2 cartridge which has been pre-eluted with MeOH. The cartridge is eluted with MeOH (2 x 5 ml), followed by 7M $NH_3$ in MeOH (2 x 5 ml) to yield (S)-3-[4- ((S)-5,6-Diamino-3,3-dimethyl-hexyl)-phenoxy]-propane-1,2-diol in 80% purity as a colorless oil; $[M+H]^+$ 311.

Step 7

A suspension of (S)-3-[4-((S)-5,6-Diamino-3,3-dimethyl-hexyl)-phenoxy]-propane-1,2-diol (60 mg, 0.19 mmol) and 1-(3,5-diamino-6-chloro-pyrazine-2-carbonyl)-2-methylisothiourea (Intermediate A) (72 mg, 0.19 mmol) in propan-2-ol (3 ml) is heated at 80°C for 35 minutes. The reaction mixture is allowed to cool to room temperature and diluted with MeOH until any solid dissolves. The solution is passed through a SCX-2 cartridge which is then eluted with further MeOH. The combined methanol elutions are concentrated *in vacuo.* Reverse phase chromatography (Isolute™ C18, Water/$CH_3CN$/0.1% TFA) yields the title compound as a yellow solid; $[M+H]^+$ 506.

Example 29

(E)-3,5-diamino-6-chloro-N-(4-(3-(4-((S)-2,3-dihydroxypropoxy)phenyl)propyl)-5-propylimidazolidin-2-ylidene)pyrazine-2-carboxamide hydrochloride

Step 1

4-(4-Methoxyphenyl)butyric acid (25 g, 129 mmol) is dissolved in 48% HBr (125 ml) and AcOH (125 ml). The resultant solution is heated at 150°C overnight. The resultant mixture is concentrated *in vacuo* and the residue taken up in EtOAc (500 ml). This solution is washed with water (500 ml), dried ($MgSO_4$) and concentrated to give 4-(4-Hydroxy-phenyl)-butyric acid as a tan solid; $^1H$ NMR (d6-DMSO): 1.72 (2H, tt, J =7.4 and 7.8 Hz), 2.18 (2H, t, J = 7.4 Hz), 2.45 (2H, t, J = 7.8 Hz), 6.66 (2H, dd, J = 1.98 and 9.3 Hz), 6.96 (2H, dd, J = 2.8 and 9.3 Hz), 9.12 (1H, s), 12.0 (1H, s).

Step 2

4-(4-Hydroxy-phenyl)-butyric acid (22.1 g, 123 mmol) is dissolved in THF (750 ml) and borane-dimethyl sulfide (23.3 ml, 245 mmol) is slowly added. The yellow suspension formed is heated at reflux for 3 hours until most of the solid slowly dissolves. The flask is removed from the heating mantle, and MeOH is slowly added until bubbling ceases and the residual solid has dissolved. The flask is cooled to room temperature and water (1 L) is added. The pH is corrected to 3 with AcOH, then the mixture is extracted with EtOAc (2 x 500 ml). The organics arc washed with brine, dried ($MgSO_4$) and concentrated. The crude product is slurried with silica (500 g) in 25% EtOAc/iso-hexanes (1 L). This is filtered, then flushed with 50% EtOAc/iso-hexanes (2 L) to elute the product. The organics are concentrated to give 4-(4-Hydroxy-butyl)-phenol as a brown oil which crystallizes on standing; $^1H$ NMR ($CDCl_3$): 1.55-1.72 (4H, m), 2.58 (2H, t, J = 7.0Hz), 3.1 (2H, br signal), 3.70 (2H, t, J = 6.4Hz), 6.77 (2H, d, J = 8.4Hz), 7.05 (2H, d, J = 8.4Hz).

Step 3

To 4-(4-Hydroxy-butyl)-phenol (32.7 g, 197 mmol) in acetone (600 ml) is added potassium carbonate (40.8 g, 295 mmol) followed by (S)-glycidol (13.7 ml, 207 mmol). The mixture is heated at reflux overnight. Further potassium carbonate (20 g) is added, followed by (S)-glycidol (5 g) and the mixture is heated at reflux for 72 hours. The suspension is cooled, filtered and the filtrate concentrated *in vacuo.* The residue is partitioned between EtOAc (500 ml) and 5% citric acid solution (500 ml). The organics are separated, dried ($MgSO_4$) and concentrated *in vacuo* to give (S)-3-[4-(4-Hydroxy-butyl)-phenoxy]-propane-1,2-diol as a brown oil; $^1H$ NMR ($CDCl_3$): 1.56-1.74 (4H, m), 2.20 (1H, t, J = 2.46Hz), 2.61 (2H, t, J = 7.6Hz), 3.68 (2H, t, J = 6.2Hz), 3.78 (1H, dd, J = 5.4 and 11.5Hz), 3.86 (1H, dd, J = 3.9 and 11.5Hz), 4.0-4.16 (3H, m), 6.85 (2H, d, J = 8.6Hz), 7.12 (2H, d, J = 8.6Hz).

Step 4

To (S)-3-[4-(4-Hydroxy-butyl)-phenoxy]-propane-1,2-diol (43 g, 179 mmol) in THF (700 ml) is added 2,2-dimethoxypropane (94 ml, 760 mmol) followed by PPTS (4.5 g, 17.9 mmol). The resultant mixture is stirred at room temperature overnight. The solution is concentrated *in vacuo* and the residue taken up in DCM (500 ml). This is washed with water, dried ($MgSO_4$) and concentrated *in vacuo.* The residue is purified through a silica plug (300 g) eluting with 10% followed by 25% EtOAc/*iso*-hexanes. The desired fractions are concentrated to give 4-[4-((R)-2,2-Dimethyl-[1,3]dioxolan-4-ylmethoxy)-phenyl]-butan-1-ol as a clear oil; $^1H$ NMR ($CDCl_3$): 1.42, (3H, s), 1.48 (3H, s), 1.53-1.73 (4H, m), 2.20 (1H, t, J = 2.5Hz), 2.60 (2H, t, J = 7.2Hz), 3.68 (2H, t, J = 6.4Hz), 3.92 (2H, dt, J = 5.8 and 8.5Hz), 4.07 (1H, dd, J = 5.4 and 9.5Hz), 4.19 (1H, dd, J = 6.4 and 8.5Hz), 4.49 (1H, p, J = 5.7Hz), 6.85 (2H, d, J = 8.7Hz), 7.11 (2H, d, J = 8.7Hz).

Step 5

To 4-[4-((R)-2,2-Dimethyl-[1,3]dioxolan-4-ylmethoxy)-phenyl]-butan-1-ol (5.0 g, 17.8 mmol) in DCM (180 ml) is added Dess-Martin periodinane (7.56 g, 17.8 mmol). The yellowish solution is stirred at room temperature for 1 hour. The resultant yellow suspension is treated with 1 N NaOH solution (200 ml) and stirred at room temperature for 30 minutes. The organic phase is separated, dried ($MgSO_4$) and concentrated to give 4-[4-((R)-2,2-Dimethyl-[1,3]dioxolan-4-ylmethoxy)-phenyl]-butyraldehyde as a clear oil; $^1H$ NMR ($CDCl_3$): 1.42, (3H, s), 1.48 (3H, s), 1.95 (2H, dt, J = 7.6 and 14.2Hz), 2.46 (2H, dt, J = 1.5 and 7.3Hz), 2.62 (2H, t, J = 7.6Hz), 3.90-3.96 (2H, m), 4.07 (1H, dd, J = 5.2 and 9.3Hz), 4.19 (1H, dd, J = 6.4 and 8.1Hz), 4.49 (1H, p, J = 5.8Hz), 6.86 (2H, d, J = 9.4Hz), 7.10 (2H, d, J = 9.4Hz), 9.77 (1H, t, J = 1.6Hz).

Step 6

To 4-[4-((R)-2,2-Dimethyl-[1,3]dioxolan-4-ylmethoxy)-phenyl]-butyraldehyde (4.28 g, 15.4 mmol) in THF (150 ml) is added *tert*-butyl sulfinamide (2.05 g, 16.9 mmol) followed by titanium ethoxide (6.5 ml, 30.8 mmol). The yellow solution formed is stirred at room temperature overnight. The solution is quenched with 1 N NaOH (200 ml) and EtOAc (100 ml) and stirred for 30 minutes at room temperature. The resultant mixture is filtered through Celite™ (filter material) and the organic phase is separated and dried ($MgSO_4$). *Concentration in vacuo* gives 2-Methyl-propane-2- sulfinic acid [4-[4-((R)-2,2-dimethyl-[1,3]dioxolan-4-ylmethoxy)-phenyl]-but-(E)-ylidene]-amide as a yellow oil; $[M+H]^+$ 382.23.

Step 7

To a solution of 2-Methyl-propane-2-sulfinic acid [4-[4-((R)-2,2-dimethyl-[1,3]dioxolan-4-ylmethoxy)-phenyl]-but-(E)-ylidene]-amide (4.51 g, 11.8 mmol) in THF (120 ml) at 0 °C is added vinylmagnesium bromide (11.8 ml of a 1 M solution in THF, 11.8 mmol) dropwise. After addition is complete, the mixture is stirred at 0 °C for 30 minutes then quenched with sat. aq. $NH_4Cl$ solution (20 ml). This mixture is allowed to warm to room temperature and diluted with water (50 ml) and EtOAc (50 ml). The organic phase is separated, dried ($MgSO_4$) and *concentrated in vacuo.* Purification by chromatography ($SiO_2$, EtOAc/iso-hexane) affords 2-Methyl-propane-2-sulfinic acid {4-[4-((R)-2,2-dimethyl-[1,3]dioxolan-4-ylmethoxy)-phenyl]-1-vinyl-butyl}-amide as a mixture of diastereomers as a gum; $[M+H]^+$410.39.

Step 8

A solution of 2-methyl-propane-2-sulfinic acid {4-[4-((R)-2,2-dimethyl-[1,3]dioxolan-4-ylmethoxy)-phenyl]-1-vinyl-butyl}-amide (1.0 g, 2.4 mmol) in DCM (25 ml) at -78 °C is saturated with oxygen, then ozone (generated using Fischer Technology Ozon Generator 500m) until a blue solution is obtained. Dimethyl sulfide (1.8 ml, 24 mmol) is then added and the mixture stirred to room temperature over 30 minutes. The resultant solution is washed with water (25 ml) and the organic phase is concentrated under high vacuum at low temperature to give 2-Methyl-propane-2-sulfinic acid {4-[4-((R)-2,2-dimethyl-[1,3] dioxolan-4-ylmethoxy)-phenyl]-1-formyl-butyl}-amide as an oil; $[M+H]^+$412.36.

Step 9

To a solution of 2-methyl-propane-2-sulfinic acid {4-[4-((R)-2,2-dimethyl-[1,3]dioxolan-4-ylmethoxy)-phenyl]-1-formyl-butyl}-amide in THF (20 ml) is added tert-butyl sulfinamide (323 mg, 2.7 mmol) followed by titanium ethoxide (1.0 ml, 4.8 mmol). The yellow solution formed is stirred at room temperature overnight. The solution is quenched with 1N NaOH (50 ml) and EtOAc (50 ml) and stirred for 30 minutes at room temperature. The resultant mixture is filtered through Celite™ (filter material) and the organic phase separated and dried ($MgSO_4$). Concentration gives 2- Methyl-propane-2-sulfinic acid (4-[4-((R)-2,2-dimethyl-[1,3]dioxolan--4-ylmethoxy)- phenyl]-1-{[(E)-2-methyl-propane-2-sulfinylimino]-methyl}-butyl)-amide as a mixture of diastereomers as a yellow oil; $[M+H]^+$515.38.

Step 10

To a solution of 2-methyl-propane-2-sulfinic acid (4-[4-((R)-2,2-dimethyl-[1,3]dioxolan-4-ylmethoxy)-phenyl]-1-{[(E)-2-methyl-propane-2-sulfinylimino]-methyl}-butyl)-amide (907 mg, 1.7 mmol) in THF (20 ml) at 0 °C n-propylmagncsium chloride (1.76 ml of a 2M solution in diethyl ether, 3.52 mmol). The solution is stirred at 0 °C for 30 minutes then at room temperature for 3 hours. A further portion of n-propylmagnesium (1.76 ml of a 2M solution in diethyl ether, 3.52 mmol) is added and the mixture is stirred at room temperature overnight. The resulting mixture is quenched with sat. aq. $NH_4Cl$ solution (50 ml) and extracted with EtOAc (2 x 50 ml). The organic phase is dried ($MgSO_4$) and concentrated *in vacuo.* The residue is dissolved in EtOAc (10 ml) and treated with 4M HCl/dioxan (10 ml). After 10 minutes, the solution is concentrated *in vacuo* and the residue diluted with DCM (100 ml). This is treated with sat. aq. $NaHCO_3$ solution (100 ml) and the organic phase is removed and dried ($MgSO_4$). The DCM solution is applied to a SCX-2 cartridge (10 g) and this is eluted with DCM and MeOH. The product is released with 2M $NH_3$ in MeOH, and the methanolic ammonia fraction concentrated to give (S)-3-[4-(4,5-Diamino-octyl)-phenoxyl-propane-1,2-diol as a mixture of diastereomers as a gum; $[M+H]^+$515.38.

Step 11

To a solution of (S)-3-[4-(4,5-Diamino-octyl)-phenoxy]-propane-1,2-diol (100 mg, 0.32 mmol) in propan-2-ol (5 ml) is added 1-(3,5-diamino-6-chloro-pyrazine-2- carbonyl)-2-methyl-isothiourea (Intermediate A) (121 mg, 0.32 mmol). The resulting suspension is heated at 90 °C for 2 hours then cooled and concentrated *in vacuo.* The residue is dissolved

in MeOH (20 ml) and applied to a 10 g SCX-2 cartridge. This is washed well with MeOH, water and MeCN, and then 2M $NH_3$ in MeOH. The methanolic ammonia fraction is concentrated then purified by chromatography ($SiO_2$, 5-10% 2M $NH_3$ in MeOH/DCM). Concentration of the relevant fractions gives the free base as a gum. This is dissolved in MeOH (10 ml) and treated with 1M HCl in diethyl ether (2 ml). Concentration yields the dihydrochloride salt of (E)-3,5-diamino-6-chloro-N-(4-(3-(4-((S)-2,3-dihydroxypropoxy)phenyl) propyl)-5-propylimidazolidin-2-ylidene)pyrazine-2-carboxamide as a yellow solid; $[M+H]^+$506.37, 508.36 for Cl isotopes.

Example 30

(3-{(S)-2-[(E)-3,5-Diamino-6-chloro-pyrazine-2-carbonylimino]-imidazolidin-4-yl}-propyl)-carbamic acid benzyl ester

1-(3,5-Diamino-6-chloro-pyrazine-2-carbonyl)-2-methyl-isothiourea (Intermediate A) (0.97 g, 3.72 mmol) is stirred in a three necked round bottom flask fitted with a bleach trap and condenser and ((S)-4,5-Diamino-pentyl)-carbamic acid benzyl ester (Intermediate S) (0.85 g, 3.38 mmol) in propan-2-ol (20 ml) is added. The reaction mixture is stirred at 85 °C for 66 hours. Purification by catch and release resin (SCX-2) followed by elution through a silica pad flushed with EtOAc, ethanol and MeOH yields the title compound as an orange foam; $[M+H]^+$447.1.

Example 31

3,5-Diamino-6-chloro-pyrazine-2-carboxylic acid [(S)-4-(3-amino-propyl)-imidazolidin-(2E)-ylidene]-amide

To a solution of (3-{(S)-2-[(E)-3,5-Diamino-6-chloro-pyrazine-2-carbonylimino]-imidazolidin-4-yl}-propyl)-carbamic acid benzyl ester (Ex. 30) (0.44 g, 0.98 mmol) in DCM (20 ml) is added iodotrimethylsilane (0.27 ml, 1.96 mmol) in a dropwise manner. The orange suspension is stirred at room temperature for 65 minutes. Purification by catch and release resin (SCX-2) eluting with MeOH followed by 7M $NH_3$ in MeOH yields the title compound as a yellow foam; $[M+H]^+$ 313.1.

Example 32

3,5-Diamino-6-chloro-pyrazine-2-carboxylic acid [(S)-4-[3-(3-benzyl-ur eido)-propyl] -imidazolidin-(2E)-ylidene]-amide

To a solution of 3,5-Diamino-6-chloro-pyrazine-2-carboxylic acid [(S)-4-(3-amino-propyl)- imidazolidin-(2E)-ylidene]-amide (Ex. 31) (0.040 g, 0.128 mmol) in DMF (2 ml) is added 1,1'-carbonyldiimidazole (0.023 g, 0.141 mmol) and the reaction mixture is stirred for 1 hour at room temperature. Benzylamine (0.014 ml, 0.128 mmol) is added and additional benzylamine (0.014 ml, 0.128 mmol) is added at hourly intervals for a total of 3 hours. Purification is by diluting the reaction with 2N NaOH (30 ml) and extracting the product into EtOAc (40 ml). The organic phase is washed with 2N NaOH (30 ml), dried over $MgSO_4$ and the solvent evaporated *in vacuo* to yield a yellow oil. The oil is dissolved in methanol (0.75 ml) and diethyl ether (5 ml) added to triturate a yellow solid. This solid is filtered off and the filtrate formed a further precipitate. This yellow solid is collected by filtration and rinsed with diethyl ether to give the title compound; $[M+H]^+$446.1.

Example 33

3,5-Diamino-6-chloro-pyrazine-2-carboxylic acid [(S)-4-(3-phenyl methanesulfonylamino-propyl)-imidazolidin-(2E)-ylidene]-amide

To a solution of 3,5-Diamino-6-chloro-pyrazine-2-carboxylic acid [(S)-4-(3-amino-propyl)- imidazolidin-(2E)-ylidene]-amide (Ex. 31) (0.030 g, 0.096 mmol) in DMF (5 ml) at 5 °C is added alpha-toluensulfonyl chloride (0.018 g, 0.096 mmol) and triethylamine (0.013 ml, 0.096 mmol). The solution is stirred for 10 minutes. Purification by reverse phase chromatography (Isolute™ C18, 0-100% MeCN in water -0.1%TFA) to affords the title compound as a yellow solid; $[M+H]^+$ 467.0.

Example 34

3,5-Diamino-6-chloro-pyrazine-2-carboxylic acid [(S)-4-(3-phenylacetylamino-propyl)-imidazolidin-(2E)-ylidene]-amide

To a solution of 3,5-Diamino-6-chloro-pyrazine-2-carboxylic acid [(S)-4-(3-amino-propyl)

-imidazolidin-(2E)-ylidene]-amide (Ex. 31) (0.030 g, 0.96 mmol) in DMF (2 ml), phenylacetyl chloride (0.013

ml, 0.096 mmol) is added. The yellow solution is stirred at room temperature for 10 minutes. Purification by catch and release resin (SCX-2) eluting with MeOH and 7M $NH_3$ in MeOH affords the title compound; $[M+H]^+$ 430.98.

Example 35

3,5-Diamino-6-chloro-pyrazine-2-carboxylic acid [(S)-4-(4-phenylmethanesulfonylamino-butyl)-imidazolidin-(2E)-ylidene]-amide

To a suspension of 3,5-Diamino-6-chloro-pyrazine-2-carboxylic acid [(S)-4-(4-amino-butyl)

-imidazolidin-(2E)-ylidene]-amide (Intermediate T) (0.023 g, 0.071 mmol) in DMF (2 ml) is added triethylamine (0.010 ml, 0.071 mmol) followed by alpha-toluenesulfonyl chloride (0.014 g, 0.071 mmol). The suspension is stirred at room temperature for 30 minutes. Purification by reverse phase chromatography (Isolute™ C18, 0-100% MeCN in water -0.1%TFA) followed by catch and release resin (SCX-2) eluting with MeOH and 7M $NH_3$ in MeOH gives the title compound as a yellow solid; $[M+H]^+$ 481.0.

Example 36

3,5-Diamino-6-chloro-pyrazine-2-carboxylic acid [(S)-4-(4-phenylacetyl amino-butyl)-imidazolidin-(2E)-ylidcnc]-amidc

To a suspension of 3,5-Diamino-6-chloro-pyrazine-2-carboxylic acid [(S)-4-(4-amino-butyl)

-imidazolidin-(2E)-ylidene]-amide (Intermediate T) (0.032 g, 0.098 mmol) in DMF (1 ml) is added triethylamine (0.014 ml, 0.098 mmol) followed by phenylacetyl chloride (0.013 ml, 0.098 mmol). The suspension is stirred at room temperature for 90 minutes before a further 0.5 equivalents of phenylacetyl chloride (0.006 ml, 0.049 mmol) is added. The reaction is left to stir at room temperature for a further 18 hours. Purification by reverse phase chromatography (Isolute™ C18, 0-100% MeCN in water -0.1 %TFA) followed by catch and release resin (SCX-2) eluting with MeOH and 7M $NH_3$ in MeOH affords the title compound as an off-white solid; $[M+H]^+$ 445.1.

Example 37

2- [(E)-3,5-Diamino-6-chloro-pyrazine-2-carbonylimino]-1,3,8-triaza-spiro [4.5] decane-8-carboxylic acid *tert*-butyl ester trifluoroacetate

A suspension of 4-amino-4-aminomethyl-piperidine-1-carboxylic acid tert-butyl ester (Intermediate U) (218 g, 0.95 mol) in *tert*-butanol (6 L) and 1-(3,5-diamino-6-chloro

-pyrazine-2-carbonyl)-2-methyl-isothiourea (Intermediate A) (338 g, 0.82 mol) is stirred at 40°C overnight. The temperature is then raised to 85 °C and the suspension stirred at this temperature for a further 4 days. The reaction mixture is concentrated *in vacuo* and the residue is taken up in water (1 L), sonicatcd and heated to 45-50 °C. The solid is collected by vacuum filtration and washed with ice cold water, then dried under vacuum at 50 °C overnight to afford the title compound as a yellow solid; $[M+H]^+$ 425.1.

Example 38

3,5-Diamino-6-chloro-pyrazine-2-carboxylic acid [1,3,8-triaza-spiro[4.5]dec-(2E)-ylidene]-amide dihydrochloride

To a stirred solution of 4M HCl in dioxane (1 L) is added 2-[(E)-3,5-diamino-6-chloro-pyrazine-2-carbonylimino]-1,3,8-triaza-spiro[4,5]decane-8-carboxylic acid *tert-butyl* ester trifluoroacetate (Ex. 37) (104 g, 193 mmol). The resulting thick suspension is stirred at room temperature for 2 hours. The product is isolated by vacuum filtration, rinsing with dioxane. The solid is dried under vacuum at 50 °C to afford the title compound as a dihydrochloride salt as a dark yellow solid; $[M+H]^+$= 325.

Example 39

3,5-Diamino-6-chloro-pyrazine-2-carboxylic acid [8-[(S)-3-phenyl-2-(toluene-4-sulfonylamino)-propionyl]-1,3,8-triaza-spiro[4.5]dec-(2E)-ylidene]-amide

To a solution of Tosyl-L-phenylalanine (1.0 g, 3.13 mmol) in DMF (25 ml) is added N-methyl morpholine (1.033 ml, 9.39 mmol) and 3,5-Diamino-6-chloro-pyrazine-2-carboxylic acid [1,3,8-triaza-spiro[4.5]dec-(2E)-ylidene]-amide dihydrochloride (Ex. 38) (1.37 g, 3.44 mmol), followed by HATU (1.31 g, 3.44 mmol) and the resulting solution is stirred at room temperature for 20 minutes. The crude product is diluted with water (300 ml) and the resultant solid is isolated. Purification by reverse phase chromatography (Isolute™ C18, 0-100% MeCN in water -0.1%TFA) followed by catch and release resin (SCX-2) eluted with MeOH and 7M $NH_3$ in MeOH yields a yellow solid which is triturated with MeOH and diethyl ether to give the title compound as a free base. The free base is stirred in 5M HCl at 100 °C for 30 minutes forming a gum. MeOH (5 ml) is added to the gum and then all solvent is removed *in vacuo.* The residue is triturated with MeOH and diethyl ether to give the title compound; $[M+H]^+$ 626.4; $^1H$ NMR (DMSO-d6): 1.12 - 1.71 (4H, m), 2.36 - 2.38 (3H, s), 2.59 - 2.83 (2H, m), 2.93 - 3.52 (4H, m), 3.41 - 3.60 (2H, m), 4.42 (1H, m), 7.12 (2H, d, J = 6.9 Hz), 7.17 - 7.28 (3H, m), 7.35 (2H, d, J = 7.7 Hz), 7.54 - 7.37 (2H, br), 7.57 (2H, d, J = 7.7 Hz), 8.12 (1H, d, J = 9.0 Hz), 7.70 - 8.26 (2H, br), 9.22 (1H, s), 9.95 (1H, s), 10.99 (1H, s).

Example 40

3,5-Diamino-6-chloro-pyrazine-2-carboxylic acid [8-(1-benzenesulfonyl-1H-indole-3-carbonyl)-1,3,8-triaza-spiro [4.5] dec-(2E)-ylidene]-amide

To a solution of 1-(phenylsulfonyl)-1H-indole-3-carboxylic acid (1.0 g, 3.32 mmol) in DMF (15 ml) is added HATU (1.388 g, 3.65 mmol) and N-methyl morpholine (1.095 ml, 9.96 mmol) and the solution is stirred at room temperature for 5 minutes. 3,5-Diamino-6-chloro-pyrazine-2-carboxylic acid [1,3,8-triaza-spiro[4.5]dec-(2E)-ylidene]-amide dihydrochloride (Ex. 38) (1.452 g, 3.65 mmol) is added and the reaction stirred at room temperature for 45 minutes. The reaction mixture is diluted with water (100 ml) and the precipitate that forms is isolated by filtration. The crude product is suspended in 2N NaOH and extracted into EtOAc. The organic portion is dried over $MgSO_4$ and concentrated *in vacuo* to yield a brown solid. The solid is suspended in a 1:1 mixture of water (+0.1 % TFA) and acetonitrile. A fine brown solid is removed by filtration and the yellow filtrate is concentrated *in vacuo* until 10 ml of solvent remains and a pale yellow solid has precipitated. This solid is washed with 2N NaOII (60 ml) and then suspended in 2N NaOII (100 ml) and extracted into EtOAc (2 x 100 ml). The organic phases are combined, dried over $MgSO_4$ and concentrated *in vacuo* to yield a pale cream solid. The cream solid is suspended in a 1:4 mixture of EtOAc : iso-hexane (100 ml) and the solid filtered off to give the free base of the title compound, which is suspended in 5 N HCl (20 ml) and stirred for 2 hours. MeOH (20 ml) is added to dissolve all solid and the solvent is concentrated *in vacuo* until a yellow solid precipitates. This solid is filtered off, rinsed with water and dried at 40 °C for 18 hours to give the title compound; $[M+H]^+$ 607.42; $^1H$ NMR (DMSO-d6): 1.86 - 1.92 (4H, m), 3.42 - 3.63 (4H, m), 3.68 (2H, s), 7.34 (1H, dd, J + 7.5 Hz, J = 7.5 Hz), 7.43 (1H, dd, J = 7.5 Hz, J = 7.5 Hz), 7.36 - 7.55 (2H, br), 7.62 (1H, d, J= 7.5 Hz), 7.63 (2H, m), 7.73 (1H, m), 7.99 (1H, d, J = 7.5 Hz), 8.06 (2H, obs), 8.07 (1H, s), 7.50 - 8.16 (2H, br), 9.18 (1H, s), 9.77 (1H, s), 11.09 (1H, s).

Example 41

3,5-Diamino-6-chloro-pyrazine-2-carboxylic acid [843-(3-isopropoxy-propylsulfamoyl)-benzoyl]-1,3,8-triaza-spiro[4.5]dec-(2E)-ylidene]-amide

To a solution of 3-(3-Isopropoxy-propylsulfamoyl)-benzoic acid (Intermediate V) (1.10 g, 3.65 mmol) in DMF (20 ml) is added HATU (1.53 g, 4.02 mmol) and N-methyl morpholine (1.204 ml, 10.95 mmol) and the solution is stirred at room temperature for 5 minutes. 3,5-Diamino-6-chloropyrazine-2-carboxylic acid [1,3,8-triaza-spiro[4.5]dec-(2E)-ylidene]

-amide dihydrochloride (Ex. 38) (1.60 g, 4.02 mmol) is added and the reaction stirred at room temperature for 45 minutes. The reaction mixture is diluted with 2N NaOH (150 ml) and the crude product extracted into EtOAc (2 x 250 ml). The organic phase is dried over $MgSO_4$ and the solvent evaporated *in vacuo* to yield a yellow oil. Purification on a Waters preparative Delta 3000 HPLC using a gradient of water (+0.1 % TFA) and acetonitrile yields a yellow oil. 2N NaOH is added to the oil and the product is extracted into EtOAc (2 x 400 ml). The organic phases are combined, dried over $MgSO_4$ and the solvent concentrated *in vacuo* to a volume of approximately 150 ml. To this solution is added iso-hexane (400 ml) and a pale yellow solid precipitates. This solid is collected by filtration and rinsed with iso-hexane to afford the title compound; $[M+H]^+$ 607.98; $^1H$ Nmr (DMSO): 1.00 (6H, d, J = 6.0 Hz), 1.55 (2H, m), 1.69 - 1.79 (4H, m),

2.81 (2H, t, 5.9 Hz), 3.29 (2H, tr, J = 6.0 Hz), 3.42 (1H, m), 3.44 (2H, br), 3.29 - 3.82 (4H, m), 6.15 - 7.30 (3H, br), 7.66 (1H, d, J = 7.4 Hz), 7.70 (1H, dd, J= 7.4 Hz, J= 7.4 Hz), 7.76 (1H, s), 7.86 (1H, d, J = 7.4 Hz), 7.44 - 8.00 (1H, br), 8.00 - 9.05 (3H, br).

Example 42

3,5-Diamino-6-chloro-pyrazine-2-carboxylic acid [8-[2-(5-phenyl-4H-[1,2,4]triazol-3-yl)-acetyl]-1,3,8-triaza-spiro [4.5] dec- (2E)-ylidene] -amide

(5-Phenyl-4H[1,2,4]traizol-3-yl)acetic acid (0.48 g, 2.364 mmol), HATU (0.988 g, 2.6 mmol), 5-Diamino-6-chloro-pyrazine-2-carboxylic acid [1,3,8-triaza-spiro[4.5]dec-(2E)-ylidene]

-amide dihydrochloride (Ex. 38) (1.033 g, 2.60 mmol), DMF (20 ml) and N-methyl morpholine (0.78 ml, 7.08 mmol) are added to a round bottomed flask and stirred at room temperature for 20 minutes. The crude product is precipitated from the reaction mixture by adding water (200 ml) and is isolated by filtration. Purification by reverse phase chromatography (Isolute™ C18, 0-100% MeCN in water -0.1%TFA) yields a yellow semi-solid. This is dissolved in MeOH (100 ml) and left to stand. An off white solid precipitates and this is collected by filtration to give the title compound; [M+H]+ 510.0; $^1$H NMR (DMSO-d6): 1.78 - 1.94 (4H, m), 3.67 (2H, s), 3.30 -3.82 (4H, m), 4.05 - 4.08 (2H, m), 7.45 - 7.55 (3H m), 7.01 - 7.75 (3H, br), 8.05 (2H, d, J = 7.1 Hz), 7.78 - 8.33 (2H, br), 9.24 (1H, s), 9.85 (1H, s), 11.04 (1H, s).

Example 43

3,5-Diamino-6-chloro-pyrazine-2-carboxylic acid [8-[3-(3-isopropyl-ureido)-benzoyl]-1,3,8-triaza-spiro [4.5] dec- (2E)-ylidene]-amide

3-(3-Isopropyl-ureido)-benzoic acid (Intermediate W) (1.08 g, 4.86 mmol) and HATU (2.03 g, 5.35 mmol) are stirred in DMF (25 ml) at room temperature and N-methyl morpholine (1.60 ml, 14.59 mmol) is added. The solution is stirred at room temperature for 5 minutes and 5-Diamino-6-chloropyrazine-2-carboxylic acid [1,3,8- triaza-spiro[4.5]dec-(2E)-ylidene]

-amide dihydrochloride (Ex. 38) (2.13 g, 5.35 mmol) is added. The brown solution is stirred at room temperature for 45 minutes. The crude product is precipitated by the addition of 2N NaOH and collected by filtration. The solid is purified by reverse phase chromatography (Isolute™ C18, 0-100% MeCN in water -0.1%TFA). The clean fractions are concentrated *in vacuo* to approximately 30 ml and 2N NaOH added. The off white solid is collected by filtration and rinsed with water to give the title compound; [M+H]+ 529.05; $^1$H NMR (DMSO-d6): 1.09 (6H, d, J = 6.5 Hz), 1.67 - 1.73 (4H, m), 3.42 (2H, br), 3.75 (1H, septet, J = 6.5 Hz), 3.31 -3.79 (4H, br), 6.15 (1H, d, J = 7.5 Hz), 6.70 (2H, br), 6.40 - 7.01 (1H, br), 6.86 (1H, d, J = 7.2 Hz), 7.26 (1H, dd, J = 8.3 Hz, J = 7.2 Hz), 7.31 (1H, d, J = 8.3 Hz), 7.53 (1H, s), 8.36 (1H, br), 8.48 (1H, br), 8.55 (1H, s), 8.00 - 9.00 (1H, br).

Example 44

3,5-Diamino-6-chloro-pyrazine-2-carboxylic acid [8-(2-Benzo [b]thiophen-3-yl-acetyl)-1,3,8-triaza-spiro [4.5] dec-(2E)-ylidene]-Amide

This compound is prepared analogously to Example 43 by replacing 3-(3-Isopropyl-ureido)-benzoic acid (Intermediate W) with benzo[b]thiophene-3-acetic acid. $[M-H]^+$ 499.0; $^1$H NMR (DMSO-d6): 1.59 - 1.74 (4H, m), 3.42 (2H, s), 3.48 - 3.95 (4H, m), 3.97 (2H, s), 6.20 - 7.11 (3H, br), 7.38 (1H, m), 7.39 (1H, m), 7.50 (1H, s), 7.83 (1H, d, J = 7.3 Hz), 7.97 (1H, d, J = 7.6 Hz), 7.75 - 9.30 (3H, br).

Example 45

3,5-Diamino-6-chloro-pyrazine-2-carboxylic acid [8-[5-oxo-1-(3-pyrrol-1-yl-propyl)-pyrrolidine-3-carbonyl]-1,3,8-triaza-spiro [4.5] dec- (2E)-ylidcnc] -amide

A solution of 5-Oxo-1-(3-pyrrol-1-yl-propyl)-pyrrolidine-3-carboxylic acid (Intermediate X) (1.15 g, 4.85 mmol), HATU (2.03 g, 5.33 mmol), DMF (20 ml) and N-methyl morpholine (1.60 ml, 14.54 mmol) is stirred at room temperature for 5 minutes before 5-Diamino-6-chloro-pyrazine-2-carboxylic acid [1,3,8-triaza-spiro[4.5]dec-(2E)-ylidene]-amide dihydrochloride (Ex. 38) (1.731 g, 5.33 mmol) is added. After stirring for 60 minutes at room temperature EtOAc (200 ml)

is added and the organic phase is washed with 2N NaOH (2 x 100 ml) and brine (100 ml). The organic phase is dried over $MgSO_4$ and the solvent evaporated *in vacuo.* Purification by reverse phase chromatography (IsoluteTM C18, 0-100% MeCN in water -0.1%TFA) followed by catch and release resin (SCX-2) eluting with MeOH and 7M $NH_3$ in MeOH yields a yellow oil. The oil is dissolved in DCM (10 ml) and product is precipitated out of solution by the addition of iso-hexane to yield a yellow solid which is filtered and rinsed with iso-hexane to yield the title product; $[M+H]^+$542.8; $^1$H NMR (DMSO-d6): 1.64 - 1.70 (4H, m), 1.84 - 1.89 (2H, m), 2.43 - 2.51 (2H, m), 3.39 - 3.43 (2H, m), 3.43 - 3.50 (2H, m), 3.55 (1H, m), 3.40 - 3.69 (4H, m), 3.84 (2H, m), 5.97 (2H, m), 6.65 - 6.74 (2H, br), 6.75 (2H, m), 6.2 - 7.6 (1H, br), 7.6 -9.5 (1H, br).

Example 46

3,5-Diamino-6-chloro-pyrazine-2-carboxylic acid [8-(6,7,8,9-tetrahydro-5H-carbazole-3-carbonyl)-1,3,8-triaza-spiro [4.5] dec- (2E)-ylidene]-amide

To a stirring solution of 6,7,8,9-Tetrahydro-5H-carbazole-3-carboxylic acid (0.05 g, 0.25 mmol) and HATU (0.11 g, 0.28 mmol) in dry DMF (5 ml) is added N-methyl morpholine (0.08 ml, 0.76 mmol). After 5 minutes stirring at room temperature, 5-Diamino-6-chloro-pyrazine-2-carboxylic acid [1,3,8-triaza-spiro[4.5]dec-(2E)-ylidene]- amide dihydrochloride (Ex. 38) (0.10 g, 0.28 mmol) is added and the reaction is left to stir at room temperature for 1 hour. Purification by reverse phase chromatography (Isolute™ C18, 0-100% MeCN in water) yields the title compound as a yellow powder; $[M+H]^+$ 524.2.

Example 47

3,5-Diamino-6-chloro-pyrazine-2-carboxylic acid [8-(1H-indazole-3-carbonyl)-1,3,8-triaza-spiro [4.5] dec- (2E)-ylidene]-amide

To a stirring solution of 1H-indazole-3-carboxylic acid (0.041 g, 0.25 mmol) and HATU (0.096 g, 0.25 mmol) in dry DMF (4 ml) is added N-methyl morpholine (0.08 ml, 0.76 mmol). After 5 minutes, 5-Diamino-6-chloro-pyrazine-2-carboxylic acid [1,3,8-triaza-spiro[4.5]dec-(2E)-ylidene]-amide dihydrochloride (Ex. 38) (0.10 g, 0.25 mmol) is added and the reaction left to stir at room temperature for 1 hour. Purification by reverse phase chromatography (Isolute™ C18, 0-100% MeCN in water-0.1%TFA) yields an oily residue that is ultrasonicated in acetonitrile to give a yellow suspension. The yellow solid is collected by filtration and rinsed with acetonitrile to afford the title compound; $[M+H]^+$ 469.17.

Example 48

3,5-Diamino-6-chloro-pyrazine-2-carboxylic acid [8-[2-(2,3-dimethyl-1H-indol-5-yl)-acetyl]-1,3,8-triaza-spiro [4.5] dec-(2E)-ylidene]-amide

This compound is prepared analogously to Example 47 by replacing 1H-indazole-3-carboxylic acid with 2-(2,3-dimethyl-1H-indol-5-yl)acetic acid with2-(2,3-dimethyl-1H-indol-5-yl)acetic acid. $[M+H]^+$ 510.23.

Example 49

3,5-Diamino-6-chloro-pyrazine-2-carboxylic acid [8-(1,2,3-trimethyl-1H-indole-5-carbonyl)-1,3,8-triaza-spiro [4.5] dec-(2E)-ylidene] -amide

To a stirring solution of 1,2,3-trimethyl-1H-indole-5-carboxylic acid (0.051 g, 0.25 mmol) and HATU (0.11 g, 0.28 mmol) in dry DMF (5 ml) is added N-methyl morpholine (0.083 ml, 0.76 mmol). After 5 minutes 5-Diamino-6-chloro-pyrazine-2- carboxylic acid [1,3,8-triaza-spiro[4.5]dec-(2E)-ylidene]-amide dihydrochloride (Ex. 38) (0.10 g, 0.28 mmol) is added and the reaction left to stir at room temperature for 1 hour. Purification by chromatography ($SiO_2$, MeOH/DCM) yields the title compound; $[M+H]^+$ 510.1.

Example 50

3,5-Diamino-6-chloro-pyrazine-2-carboxylic acid [8-(1-methyl-1H-indazole-3-carbonyl)-1,3,8-triaza-spiro [4.5] dec-(2E)-ylidene] -amide

This compound is prepared analogously to Example 46 by replacing 6,7,8,9- Tetrahydro-5H-carbazole-3-carboxylic acid with 1-methyl-1H-indazole-3-carboxylic acid. $[M+H]^+$ 483.1.

Example 51

3,5-Diamino-6-chloro-pyrazine-2-carboxylic acid [8-(4-benzyloxy-benzoyl)-1,3,8-triaza-spiro [4.5] dec- (2E)-ylidene]-amide

5-Diamino-6-chloro-pyrazine-2-carboxylic acid [1,3,8-triaza-spiro[4.5]dec-(2E)-ylidene]

-amide dihydrochloride (Ex. 38) (0.05 g, 0.13 mmol), 4-(benzyloxy)benzoic acid (0.029 g, 0.13 mmol), HATU (0.05 g, 0.13 mmol), N-methyl morpholine (0.041 ml, 0.38 mmol) and DMF (2 ml) are stirred together at room temperature for 72 hours. The reaction mixture is diluted with EtOAc (25 ml) and washed with water (25 ml) and sat. $NaHCO_3$ (25 ml). The organic phase is dried over $MgSO_4$ and evaporated *in vacuo* to yield a yellow oil. The oil is dissolved in ethyl acetate and a drop of methanol and iso-hexane are added. The resulting pale yellow solid is collected by filtration to give the title compound; $[M+H]^+$ 535.1.

Example 52

3,5-Diamino-6-chloro-pyrazine-2-carboxylic acid [8-(3-2,3-dihydro-benzofuran-5-yl-propionyl)-1,3,8-triaza-spiro [4.5] dec- (2E)-ylidene]-amide

This compound is prepared analogously to Example 51 by replacing 4-(benzyloxy)benzoic acid with 3-(2,3-dihydrobenzofuran-5-yl) propanoic acid. $[M+H]^+$499.1.

Example 53

3,5-Diamino-6-chloro-pyrazine-2-carboxylic acid [8-(1H-pyrrolo[2,3-b]pyridine-4-carbonyl)-1,3,8-triaza-spiro [4.5] dec-(2E)-ylidene] -amide

This compound is prepared analogously to Example 47 by replacing 1H-indazole-3-carboxylic acid with 1H-pyrrolo[2,3-b]pyridine-4-carboxylic acid; $[M+H]^+$ 469.14.

Example 54

3,5-Diamino-6-chloro-pyrazine-2-carboxylic acid [8-[3-(4-methoxy-phenyl)- propionyl]-1,3,8-triaza-spiro [4.5] dec-(2E)-ylidene]-amide

5-Diamino-6-chloro-pyrazine-2-carboxylic acid [1,3,8-triaza-spiro[4.5]dec-(2E)-ylidene]- amide dihydrochloride (Ex. 38) (0.05 g, 0.13 mmol), 3-(4-methoxyphenyl)-propionic acid (0.023 g, 0.13 mmol), HATU (0.048 g, 0.13 mmol), N-methyl morpholine (0.041 ml, 0.38 mmol) and DMF (2 ml) are stirred together at room temperature for 48 hours. The reaction mixture is diluted with EtOAc (50 ml) and product is extracted into 1 M HCl. The aqueous phase is basified to pH 12 with 2 N NaOH and product extracted into EtOAc (50 ml). The organic phase is dried over $MgSO_4$ and the solvent evaporated *in vacuo* to yield a brown glass. The product is triturated with MeOH and EtOAc to give a pale brown solid as the title compound; $[M+H]^+$ 487.0.

Example 55

3,5-Diamino-6-chloro-pyrazine-2-carboxylic acid [8-[3-(4-hydroxy-phenyl)-propionyl]-1,3,8-triaza-spiro [4.5] dec-(2E)-ylidene]-amide

This compound is prepared analogously to Example 49 by replacing 1,2,3-trimethyl-1H-indole-5-carboxylic acid with 13-(4-hydroxyphenyl)propionic acid; $[M+H]^+$ 472.98.

Example 56

3,5-Diamino-6-chloro-pyrazine-2-carboxylic acid [8-(1H-indole-2-carbonyl)-1,3,8-triaza-spiro [4.5] dec- (2E)-ylidene]-amide

This compound is prepared analogously to Example 46 by replacing 6,7,8,9-Tetrahydro-5H-carbazole-3-carboxylic acid with 1H-indole-2-carboxylic acid; $[M+H]^+$468.1.

Example 57

3,5-Diamino-6-chloro-pyrazine-2-carboxylic acid [8-(quinoline-5-carbonyl)-1,3,8-triaza-spiro [4.5] dec- (2E)-ylidene]-amide

This compound is prepared analogously to Example 46 by replacing 6,7,8,9-Tetrahydro-5H-carbazole-3-carboxylic acid with quinoline-5-carboxylic acid; $[M+H]^+$480.1.

Example 58

3,5-Diamino-6-chloro-pyrazine-2-carboxylic acid [8-(4-methyl-2-phenyl-pyrimidine-5-carbonyl)-1,3,8-triaza-spiro [4.5] dec-(2E)-ylidene] -amide

This compound is prepared analogously to Example 45 by replacing 5-Oxo-1-(3- pyrrol-1-yl

-propyl)-pyrrolidine-3-carboxylic acid (Intermediate X) with 4-methyl-2-phenylpyrimidine -5-carboxylic acid; $[M+H]^+$ 521.1.

Example 59

3,5-Diamino-6-chloro-pyrazine-2-carboxylic acid [8-(4-benzyl-morpholine-2- carbonyl)-1,3,8-triaza-spiro [4.5] dec-(2E)-ylidene] -amide

This compound is prepared analogously to Example 51 by replacing 4-(benzyloxy)benzoic acid with 4-benzyl-2-morpholinecarboxylic acid hydrochloride;$[M+H]^+$ 528.2.

Example 60

3,5-Diamino-6-chloro-pyrazine-2-carboxylic acid [8-(1H-pyrrolo[2,3-b]pyridine-5-carbonyl)-1,3,8-triaza-spiro[4.5]dec-(2E)-ylidene]-amide

This compound is prepared analogously to Example 47 by replacing 1H-indazole-3-carboxylic acid with 1H-pyrrolo[2,3-b]pyridine-5-carboxylic acid; $[M+H]^+$ 469.1.

Example 61

3,5-Diamino-6-chloro-pyrazine-2-carboxylic acid [8-[4-(4,6-dimethoxy-pyrimidin-2-ylmethoxy)-benzoyl]-1,3,8-triaza-spiro [4.5] dec-(2E)-ylidene]-amide

This compound is prepared analogously to Example 51 by replacing 4-(benzyloxy)benzoic acid with 4-((4,6-dimethoxypyrimidin-2-yl)methoxy)benzoic acid; $[M+H]^+$ 597.07.

Example 62

3,5-Diamino-6-chloro-pyrazine-2-carboxylic acid [8-[2-(3-isopropyl-ureido)-pyridine4-carbonyl]-1,3,8-triaza-spiro [4.5] dec-(2E)-ylidene] -amide

This compound is prepared analogously to Example 51 by replacing 4-(benzyloxy)benzoic acid with 2-(3-Isopropyl-ureido)-isonicotinic acid (intermediate Y) $[M+H]^+$ 530.2; $^1H$ NMR (DMSO-d6): 1.13 (6H, d, J = 6.5), 1.77-1.94 (4H, m), 3.66 (2H, d, J =11), 3.25-3.99 (5H, m), 6.97 (1H, br m), 7.50 (1H, br s), 7.31-7.60 (2H, br s), 7.61 (1H, br s),

7.74-8.25 (2H, br s), 8.28 (1H, d, J = 5.5), 9.08-9.21 (1H, br s), 9.60-9.80 (1H, br s), 9.70-10.25 (1H, br s), 11.07 (s, 1H).

Example 63

4-12-[(E)-3,5-Diamino-6-chloro-pyrazine-2-carbonylimino]-1,3,8-triaza-spiro[4.5]decane-8-carbonyl}-indole-1-carboxylic acid isopropylamide

This compound is prepared analogously to Example 51 by replacing 4-(benzyloxy) benzoic acid with 1-isopropylcarbamoyl-1H-indole-4-carboxylic acid (Intermediate Z): $[M+H]^+$ 553.5.

Example 64

3,5-Diamino-6-chloro-pyrazine-2-carboxylic acid [8-[4-(3-isopropyl-ureido)-benzoyl]-1,3,8-triaza-spiro [4.5]dec-(2E)-ylidene]-amide

This compound is prepared analogously to Example 51 by replacing 4-(benzyloxy)benzoic acid with 4-(3-isopropyl-ureido)-benzoic acid (Intermediate AA); $[M+H]^+$ 529.5.

Example 65

3,5-Diamino-6-chloro-pyrazine-2-carboxylic acid [8-[6-(3-isopropyl-ureido)-pyridine-3-carbonyl]-1,3,8-triaza-spiro [4.5] dec- (2E)-ylidene]-amide

This compound is prepared analogously to Example 51 by replacing 4-(benzyloxy)benzoic acid with 6-(3-isopropyl-ureido)-nicotinic acid (Intermediate AB); $[M+H]^+$ 530.5.

Example 66

3,5-Diamino-6-chloro-pyrazine-2-carboxylic acid [8-[3-(4-allyloxy-phenyl)-propionyl]-1,3,8-triaza-spiro [4.5] dec-(2E)-ylidene]-amide

This compound is prepared analogously to Example 51 by replacing 4-(benzyloxy)benzoic acid with 3-(4-allyloxy)phenyl)propanoic acid; $[M+H]^+$ 513.4.

Example 67

3,5-Diamino-6-chloro-pyrazine-2-carboxylic acid [8-{2-[4-(2-methoxy-ethoxymethoxy)-phenyl]-acetyl}-1,3,8-triaza-spiro [4.5] dec-(2E)-ylidene]-amide

This compound is prepared analogously to Example 51 by replacing 4-(benzyloxy)benzoic acid with [4-(2-methoxy-ethoxymethoxy)-phenyl]-acetic acid (Intermediate AC); 547.4.

Example 68

3,5-Diamino-6-chloro-pyrazine-2-carboxylic acid [8-{3-[4-(2-methoxy-ethoxymethoxy)-phenyl}-propionyl}-1,3,8-triaza-spiro [4.5] dec-(2E)-ylidene]-amide

This compound is prepared analogously to Example 2.13 by replacing 4-(benzyloxy)benzoic acid with 3-[4-(2-Methoxy-ethoxymethoxy)-phenyl]-propionic acid (Intermediate AD); $[M+H]^+$ 561.0.

Example 69

3,5-Diamino-6-chloro-pyrazine-2-carboxylic acid [8-(3-{4[2-(tetrahydro-pyran-2yloxy)-ethoxy]-phenyl}-propionyl)-1,3,8-triaza-spiro[4.5]dec-(2E)-ylidene]-amide

This compound is prepared analogously to Example 51 by replacing 4-(benzyloxy)benzoic acid with 3-{4-[2-(tetrahydro-pyran-2-yloxy)-ethoxy]-phenyl}-propionic acid (Intermediate AE); $[M+H]^+$ 601.1.

Example 70

3,5-Diamino-6-chloro-pyrazine-2-carboxylic acid [8-{3-[4-(pyridin-4-ylmethoxy)-phenyl]-propionyl}-1,3,8-triaza-spiro [4.5] dec- (2E)-ylidene]-amide

This compound is prepared analogously to Example 51 by replacing 4-(benzyloxy)benzoic acid with 3[4-(Pyridin-4-ylmethoxy)-phenyl]-propionic acid (Intermediate AF); $[M+H]^+$ 564.1.

Example 71

[4-(3-{2-[(E)-3,5-Diamino-6-chloro-pyrazine-2-carbonylimino]-1,3,8-triaza-spiro [4.5] dec-8-yl}-3-oxo-propyl)-phenoxy] -acetic acid tert-butyl

This compound is prepared analogously to Example 51 by replacing 4-(benzyloxy)benzoic acid with 3-(4-tert-butoxycarbonylmethoxy-phenyl)-propionic acid (Intermediate AG); $[M+H]^+$ 587.5.

Example 72

3,5-Diamino-6-chloro-pyrazine-2-carboxylic acid [8-[3-(4-carbamoylmethoxy-phenyl)-propionyl]-1,3,8-triaza-spiro [4.5] dec- (2E)-ylidene]-amide

This compound is prepared analogously to Example 51 by replacing 4-(benzyloxy)benzoic acid with 3-(4-Carbamoylmethoxy-phenyl)-propionic acid (Intermediate AH); $[M+H]^+$ 530.1.

Example 73

1-[4-(3-{2-[(E)-3,5-Diamino-6-chloro-pyrazine-2-carbonylimino]-1,3,8-triaza-spiro[4.5]dec-8-yl}-3-oxo-propyl)-phenoxy]-cyclobutanecarboxylic acid ethyl ester

This compound is prepared analogously to Example 51 by replacing 4-(benzyloxy)benzoic acid with 1-[4-(2-Carboxy-ethyl)-phenoxy]-cyclobutanecarboxylic acid ethyl ester (Intermediate AI); $[M+H]^+$ 599.1.

Example 74

2-[4-(3-{2-[(E)-3,5-Diamino-6-chloro-pyrazine-2-carbonylimino]-1,3,8-triaza-spiro[4.5]dec-8-yl}-3-oxo-propyl)-phenoxy]-2-methyl-propionic acid tert-butyl ester

This compound is prepared analogously to Example 51 by replacing 4-(benzyloxy)benzoic acid with 2-[4-(2-Carboxy-ethyl)-phenoxy]-2-methyl-propionic acid tert-butyl ester (Intermediate AJ); $[M+H]^+$ 615.2.

Example 75

[4 -(3 -{2- [(E)-3,5 -Diamino-6-chloro-pyrazine-2-carbonylimino]-1,3,8-triaza-spiro [4.5] dec-8-yl}-3-oxo-propyl)-phenoxy]-acetic acid methyl ester

This compound is prepared analogously to Example 51 by replacing 4-(benzyloxy)benzoic acid with 3-(4-Methoxycarbonylmethoxy-phenyl)-propionic acid (Intermediate AK); $[M+H]^+$ 545.1.

Example 76

4-{2-[(E)-3,5 -Diamino-6-chloro-pyrazine-2 -carbonylimino]-1,3,8-triaza-spiro [4 .5] decane-8-carbonyll-benzoic acid tert-butyl ester

This compound is prepared analogously to Example 51 by replacing 4-(benzyloxy)benzoic acid with 4-(tert-Butoxycarbonyl)benzoic acid; $[M+H]^+$ 529.4.

Example 77

3,5-Diamino-6-chloro-pyrazine-2-carboxylic acid [8-(3-isopropyl-2-methyl-1H-indole-5-carbonyl)-1,3,8-triaza-spiro [4.5] dec- (2E)-ylidene]-amide

This compound is prepared analogously to Example 51 by replacing 4-(benzyloxy)benzoic acid with 3-isopropyl-2-methyl-1H-indole-5-carboxylic acid; $[M+H]^+$ 524.

Example 78

3- [4- (3- {2-[(E)-3,5-Diamino-6-chloro-pyrazine-2-carbonylimino]-1,3,8-triaza-spiro [4.5] dec-8-yl}-3-oxo-propyl)-phenyl]-propionic acid propyl ester

This compound is prepared analogously to Example 51 by replacing 4-(benzyloxy)benzoic acid with 344-(2-Propoxycarbonyl-ethyl)-phenyl]-propionic acid (intermediate AL); $[M+H]^+$571.

Example 79

3-[4-(3-{2-[(E)-3,5-Diamino-6-chloro-pyrazine-2-carbonylimino]-1,3,8-triaza-spiro [4.5] dec-8-yl}-3-oxo-propyl)-phenyl]-propionic acid ethyl ester

This compound is prepared analogously to Example 51 by replacing 4-(benzyloxy)benzoic acid with 344-(2-Ethoxycarbonyl-ethyl)-phenyl]-propionic acid (intermediate AM); $[M+H]^+$557.

Example 80

3- [4-(3-{2-[(E)-3,5-Diamino-6-chloro-pyrazine-2-carbonylimino]-1,3,8-triaza-spiro[4.5] dec-8-yl}-3-oxo-propyl)-phenyl]-propionic acid methyl ester

This compound is prepared analogously to Example 51 by replacing 4-(benzyloxy)benzoic acid with 3-[4-(2-Methoxycarbonyl-ethyl)-phenyl]-propionic acid (intermediate AN); $[M+H]^+$ 543.

Example 81

3-[4-(3-{2-[(E)-3,5-Diamino-6-chloro-pyrazine-2-carbonylimino]-1,3,8-triaza-spiro [4.5] dec-8-yl}-3-oxo-propyl)-phenyl]-propionic acid

This compound is prepared analogously to Example 51 by replacing 4-(benzyloxy)benzoic acid with 3,3'-(1,4-phenylene)dipropanoic acid; $[M+H]^+$ 529.

Example 82

3,5-Diamino-6-chloro-pyrazine-2-carboxylic acid [8-[1-(2-phenoxy-ethyl)-1H-indole-4-carbonyl]-1,3,8-triaza-spiro [4.5] dec-(2E)-ylidene] -amide

This compound is prepared analogously to Example 51 by replacing 4-(benzyloxy)benzoic acid with 1-(2-Phenoxy-ethyl)-1H-indole-4-carboxylic acid (Intermediate AO); $[M+H]^+$ 588.

Example 83

3,5-Diamino-6-chloro-pyrazine-2-carboxylic acid [8-[1-(2-p-tolyl-ethyl) 1H-indole-4-carbonyl]-1,3,8-triaza-spiro [4.5] dec-(2E)-ylidene]-amide

This compound is prepared analogously to Example 51 by replacing 4-(benzyloxy)benzoic acid with 1-(2-p-Tolyl-ethyl)-1H-indole-4-carboxylic acid (Intermediate AP); $[M+H]^+$ 586.

Example 84

3,5-Diamino-6-chloro-pyrazine-2-carboxylic acid [8-{1-[2-(tetrahydro-pyran-2-yloxy)-ethyl]-1H-indole-4-carbonyl}-1,3,8-triaza-spiro [4.5] dec- (2E)-ylidene]-amide

This compound is prepared analogously to Example 51 by replacing 4-(benzyloxy)benzoic acid with 1-[2-(Tetrahydro-pyran-2-yloxy)-ethyl]-1H-indole-4- carboxylic acid (Intermediate AQ); $[M+H]^+$ 597.

Example 85

3,5-Diamino-6-chloro-pyrazine-2-carboxylic acid [8-{1-[2-(4-methoxy-phenoxy)-ethyl]-1H-indole-4-carbonyl}-1,3,8-triaza-spiro [4.5] dec- (2E)-ylidene]-amide

This compound is prepared analogously to Example 51 by replacing 4-(benzyloxy)benzoic acid with 1-[2-(4-Methoxy-phenoxy)-ethyl]-1H-indole-4- carboxylic acid (Intermediate AR); $[M+H]^+$ 618.

Example 86

3,5-Diamino-6-chloro-pyrazine-2-carboxylic acid [8-{1-[2-(4-tert-butyl-phenoxy)-ethyl]-1H-indole-4-carbonyl}-1,3,8-triaza-spiro [4.5] dec-(2E)-ylidene]-amide

This compound is prepared analogously to Example 51 by replacing 4-(benzyloxy)benzoic acid with 1-[2-(4-tert-Butyl-phenoxy)-ethyl]-1H-indole-4- carboxylic acid (Intermediate AS); $[M+H]^+$ 644.

Example 87

3,5-Diamino-6-chloro-pyrazine-2-carboxylic acid [8-[1-(2-[1,3]dioxan-2-yl-ethyl)-1H-indole-4-carbonyl]-1,3,8-triaza-spiro [4.5] dec-(2E)-ylidene]-amide

This compound is prepared analogously to Example 51 by replacing 4-(benzyloxy)benzoic acid with 1-(2-[1,3]Dioxan-2-yl-ethyl)-1H-indole-4-carboxylic acid (Intermediate AT; $[M+H]^+$ 582.

Example 88

3,5-Diamino-6-chloro-pyrazine-2-carboxylic acid [8-[1-(2-hydroxy-ethyl)-2,3- dimethyl-1H-indole-5-carbonyl]-1,3,8-triaza-spiro[4.5] dec-(2E)-ylidene]-amide

This compound is prepared analogously to Example 51 by replacing 4-(benzyloxy)benzoic acid with 2,3-Dimethyl-1-[2-(tetrahydro-pyran-2-yloxy)-ethyl]-1H-indole-5-carboxylic acid (Intermediate AU); $[M-4-1]^+$ 540.

Example 89

4-(4-{2-[(E)-3,5-Diamino-6-chloro-pyrazine-2-carbonylimino]-1,3,8-triaza-spiro[4.5]decane-8-carbonyl}-indol-1-yl)-butyric acid methyl ester

This compound is prepared analogously to Example 51 by replacing 4-(benzyloxy)benzoic acid with 1-(4,4,4-Trimethoxy-butyl)-1H-indole-4-carboxylic acid (Intermediate AW); $[M+H]^+$ 568

Example 90

3,5-Diamino-6-chloro-pyrazine-2-carboxylic acid [8-{1-[2-(2-methoxy-ethoxymethoxy)-ethyl] -1H-indole-4-carbonyl}-1,3,8-triaza-spiro [4.5]dec-(2E)-ylidene]-amide

This compound is prepared analogously to Example 51 by replacing 4-(benzyloxy)benzoic acid with 1-[2-(2-Methoxy-ethoxymethoxy)-ethyl]-1H-indole-4- carboxylic acid (Intermediate AW); $[M+H]^+$ 600

Example 91

3,5-Diamino-6-chloro-pyrazine-2-carboxylic acid [8-(1-diethylcarbamoylmethyl-1H-indole-4-carbonyl)-1,3,8-triaza-spiro [4.5] dec-(2E)-ylidene]-amide

This compound is prepared analogously to Example 51 by replacing 4-(benzyloxy)benzoic acid with 1-Diethylcarbamoylmethyl-1H-indole-4-carboxylic acid (Intermediate AX); $[M+H]^+$ 581

Example 92

3,5-Diamino-6-chloro-pyrazine-2-carboxylic acid [8-[1-(2-hydroxy-ethyl)-1H-indole-4-carbonyl]-1,3,8-triaza-spiro [4.5] dec-(2E)-ylidene] -amide

*p*-Toluenesulfonic acid monohydrate (1.6 mg, 0.0084 mmol) is added to a stirred solution of 3,5-diamino-6-chloro-pyrazine-2-carboxylic acid [8-{1-[2-(tetrahydro-pyran-2-yloxy)-ethyl]

-1H-indole-4-carbonyl}-1,3,8-triaza-spiro[4.5]dec-(2E)-ylidene]- amide (Ex. 84) (50 mg, 0.084 mmol) in MeOH (3 ml) and the resulting solution is stirred at room temperature for 3 hrs, then heated at 50 °C for 16 hours. The solvent is removed *in vacuo* and the residue is dissolved in MeOH (3 ml) and loaded onto a 1 g PEAX cartridge which is eluted with MeOH (20 ml). The filtrate is concentrated *in vacuo* to afford the title compound; $[M+H]^+$ 512/514

Example 93

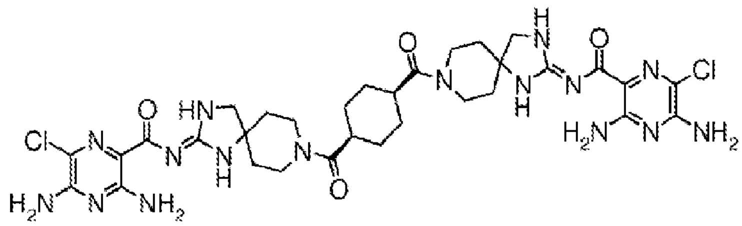

A mixture of 3,5-diamino-6-chloro-pyrazine-2-carboxylic acid [1,3,8-triazaspiro[4.5]dec

-(2*E*)-ylidene]-amide dihydrochloride (Ex. 38) (300 mg, 0.83 mmol), *cis*-1,4
-cyclohexanedicarboxylic acid (72 mg, 0.42 mmol), N-methyl morpholine (0.30 ml, 2.73 mmol) and HATU (315 mg, 0.83 mmol) in anhydrous DMF is stirred at room temperature for 16 hours. The reaction mixture is concentrated *in vacuo* and is subjected to column chromatography (basic alumina, 0-3% MeOH in DCM) to obtain off-white solid. The product is dissolved in DCM and reprecipitated by addition of diethyl ether. The supernatant solvent mixture is decanted and the product is washed again with diethyl ether and dried under vacuum to afford the compound shown as off-white solid; $[M+H]^+$ 785.

Example 94

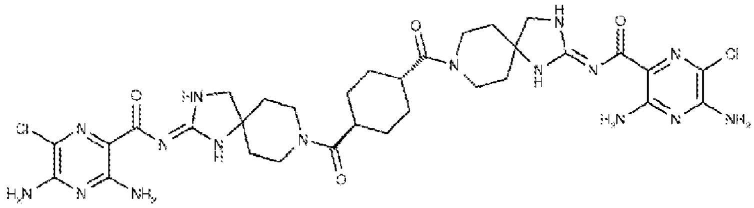

This compound is prepared analogously to Example 93 by replacing *cis*-1,4-cyclohexanedicarboxylic acid with *trans*-1,4-cyclohexanedicarboxylic acid; $[M+2H]^{2+}$ 393.

Example 95

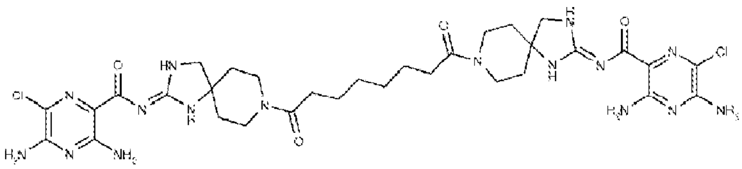

This compound is prepared analogously to Example 93 by replacing *cis*-1,4-cyclohexanedicarboxyfic acid with suberic acid; $[M+H]^+$ 787.

Example 96

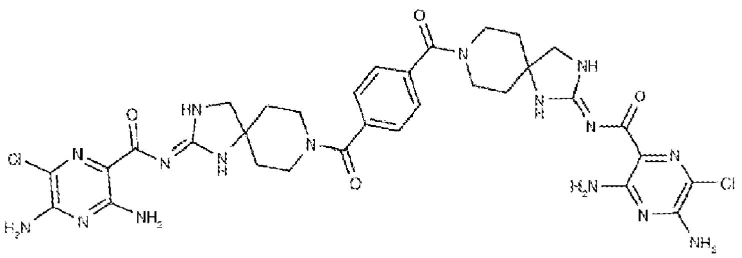

This compound is prepared analogously to Example 93 by replacing *cis-1,4*-cyclohexanedicarboxylic acid with terephthalic acid; $[M+H]^+$ 779.

Example 97

3,5-Diamino-6-chloro-pyrazine-2-carboxylic acid [8-[2-(4-benzyloxy-phenyl)-acetyl]-1,3,8-triaza-spiro [4.5] dec- (2*E*)-ylidene] -amide

A mixture of 3,5-diamino-6-chloro-pyrazine-2-carboxylic acid [1,3,8-triaza-spiro[4.5]dec

-(2*E*)-ylidene]-amide dihydrochloride (Ex. 38) (300 mg, 0.83 mmol), 4-benzyloxyphenylacetic acid (200 mg, 0.83 mmol), N-methyl morpholine (0.40 ml, 3.64 mmol) and HATU (315 mg, 0.83 mmol) in anhydrous DMF (20 ml) is stirred at room temperature for 16 hours. The reaction mixture is concentrated *in vacuo* and subjected to column chromatography (basic alumina, 0-3% MeOH in DCM) to obtain pale yellow solid. The product is dissolved in DCM and MeOH and reprecipitated by adding diethyl ether. The supernatant solvent mixture is decanted and the product is washed again with diethyl ether and dried under vacuum to afford the title compound as a pale yellow solid; $[M+H]^+$ 549.

Example 98

3,5-Diamino-6-chloro-pyrazine-2-carboxylic acid [8- [3-(4-benzyloxy-phenyl)-propionyl]-1,3,8-triaza-spiro [4.5] dec-(2E)-ylideneJ -amide

This compound is prepared analogously to Example 97 by replacing 4-benzyloxyphenylacetic acid with 3-(4-benzyloxyphenyl)propionic acid; $[M+H]^+$ 563.

Example 99

3,5-Diamino-6-chloro-pyrazine-2-carboxylic acid [8-(1H-indole-4-carbonyl)-1,3,8-triaza-spiro[4.5]dec-(2*E*)-ylidene]-amide

This compound is prepared analogously to Example 97 by replacing 4-benzyloxyphenylacetic acid with indole-4-carboxylic acid; $[M+H]^+$468.

Example 100

3,5-Diamino-6-chloro-pyrazine-2-carboxylic acid [8-(1H-indole-5-carbonyl)-1,3,8-triaza-spiro [4.5] dec-(2*E*)-ylidene]-amide

This compound is prepared analogously to Example 97 by replacing 4-benzyloxyphenylacetic acid with indole-5-carboxylic acid; $[M+H]^+$468.

Example 101

3,5-Diamino-6-chloro-pyrazine-2-carboxylic acid [8-phenylacetyl-1,3,8-triaza-spiro[4.5]dec-(2E)-ylidene]-amide

This compound is prepared analogously to Example 97 by replacing 4-benzyloxyphenylacetic acid with phenylacetic acid; $[M+H]^+$443.

Example 102

3,5-Diamino-6-chloro-pyrazine-2-carboxylic acid [8-{4-[6-((S)-2,3-dihydroxy-propoxy)-naphthalen-2-ylmethoxy]-benzoyl}-1,3,8-triaza-spiro[4,5]dec-(2E)-ylidene]-amide

Step 1

3,5-Diamino-6-chloro-pyrazine-2-carboxylic acid [8-{4-[6-((R)-2,2-dimethyl-[1,3]dioxolan-4-ylmethoxy)-naphthalen-2-ylmethoxy]-benzoyl}-1,3,8-triaza-spiro[4.5]dec -(2*E*)-ylidene]-amide is prepared analogously to Example 97 by replacing 4-benzyloxyphenylacetic acid with 4-[6-((R)-2,2-dimethyl-[1,3]dioxolan-4-ylmethoxy) -naphthalen-2-ylmethoxy]-benzoic acid (Intermediate AY); $[M+H]^+$ 715.

Step 2:

To a solution of 3,5-diamino-6-chloro-pyrazine-2-carboxylic acid [8-{4-[6-((R)-2, 2-dimethyl-[1,3]dioxolan-4-ylmethoxy)-naphthalen-2-ylmethoxy]-benzoyl}-1,3,8-triaza -spiro[4.5]dec-(2E)-ylidene]-amide (0.16 g, 0.22 mmol) in MeOII (10 ml) is added SCX-2 resin (-2 g), the resultant slurry is stirred for 0.5 hours and then the solvent is removed *in vacuo.* The slurry is loaded onto a column of SCX-2 resin (~3 g) and eluted with MeOH and then with 2 M $NH_3$ in MeOH. The methanolic ammonia fractions are concentrated *in vacuo* and the residue is triturated with diethyl ether to obtain 3,5-Diamino-6-chloro-pyrazine-2-carboxylic acid [8-{4-[6-((*S*)-2,3-dihydroxy-propoxy)-naphthalen-2-ylmethoxy]-benzoyl}-1,3,8-triaza-spiro [4.5] dec-(2E)-ylidene]-amide as yellow solid; $[M+H]^+$ 675.

Example 103

3,5-Diamino-6-chloro-pyrazine-2-carboxylic acid [8-(4-chloro-benzoyl)-1,3,8-triaza-spiro[4.5]dec-(2*E*)-ylidene]-amide

This compound is prepared analogously to Example 97 by replacing 4-benzyloxyphenylacetic acid with p-chlorobenzoic acid; $[M+H]^+$ 463.

Example 104

3,5-Diamino-6-chloro-pyrazine-2-carboxylic acid [8-(4-{3-[4-((*S*)-2,3-dihydroxy-propoxy)-phenyl]-propoxy}-benzoyl)-1,3,8-triaza-spiro [4,5]dec-(2*E*)-ylidene]-amide

This compound is prepared analogously to Example 102 by replacing 4-[6-((R)-2,2-dimethyl-[1,3]dioxolan-4-ylmethoxy)-naphthalen-2-ylmethoxy]-benzoic acid, (Intermediate AY) with 4-{3-[4-((R)-2,2-dimethyl-[1,3]dioxolan-4-ylmethoxy)-phenyl]-propoxy}-benzoic acid (Intermediate AZ); $[M+H]^+$ 653.

Example 105

3,5-Diamino-6-chloro-pyrazine-2-carboxylic acid [8-[(*E*)-(3-phenyl-acryloyl)]-1,3,8-triaza-spiro[4.5]dec-(2*E*)-ylidene]-amide

This compound is prepared analogously to Example 97 by replacing 4-benzyloxyphenylacetic acid with *trans*-cinnamic acid; $[M+H]^+$ 455.

Example 106

3,5-Diamino-6-chloro-pyrazine-2-carboxylic acid [8-benzoyl-1,3,8-triaza-spiro[4.5]dec-(2E)-ylidene]-amide

This compound is prepared analogously to Example 97 by replacing 4-benzyloxyphenylacetic acid with benzoic acid; $[M+H]^+$ 429.

Example 107

3,5-Diamino-6-chloro-pyrazine-2-carboxylic acid [8-(benzofuran-5-carbonyl)-1,3,8-triaza-spiro[4.5]dec-(2*E*)-ylidene]-amide

This compound is prepared analogously to Example 97 by replacing 4-benzyloxyphenylacetic acid with benzofuran-5-carboxylic acid; $[M+H]^+$ 469.

Example 108

3,5-Diamino-6-chloro-pyrazine-2-carboxylic acid [8-hexanoyl-1,3,8-triaza-spiro[4.5]dec-(2E)-ylidene]-amide

This compound is prepared analogously to Example 97 by replacing 4-benzyloxyphenylacetic acid with hexanoic acid; $[M+H]^+$ 423.

Example 109

3,5-Diamino-6-chloro-pyrazine-2-carboxylic acid [8-(3-phenyl-propynoyl)-1,3,8-triaza-spiro [4.5] dec-(2*E*)-ylidene]-amide

This compound is prepared analogously to Example 97 by replacing 4-benzyloxyphenylacetic acid with phenylpropiolic acid; $[M+H]^+$ 453.

Example 110

3,5-Diamino-6-chloro-pyrazine-2-carboxylic acid [8-(1H-imidazole-2-carbonyl)-1,3,8-triaza-spiro[4.5]dec-(2*E*)-ylidene]-amide

This compound is prepared analogously to Example 97 by replacing 4-benzyloxyphenylacetic acid with 2-imidazolecarboxylic acid; $[M+H]^+$ 419.

Example 111

3,5-Diamino-6-chloro-pyrazine-2-carboxylic acid [8-isobutyryl-1,3,8-triaza-spiro[4.5]dec-(2*E*)-ylidene]-amide

This compound is prepared analogously to Example 97 by replacing 4-benzyloxyphenylacetic acid with isobuteric acid; $[M+H]^+$ 395.

Example 112

3,5-Diamino-6-chloro-pyrazine-2-carboxylic acid [8-(4-cyano-benzoyl)-1,3,8-triaza-spiro [4.5]dec-(2*E*)-ylidene]-amide

This compound is prepared analogously to Example 97 by replacing 4-benzyloxyphenylacetic acid with p-cyanobenzoic acid; $[M+H]^+$ 454.

Example 113

3,5-Diamino-6-chloro-pyrazine-2-carboxylic acid [8-(pyridine-3-carbonyl)-1,3,8-triaza-spiro[4.5]dec-(2E)-ylidene]-amide

This compound is prepared analogously to Example 97 by replacing 4-benzyloxyphenylacetic acid with nicotinic acid; $[M+H]^+$ 430.

Example 114

4-{2-[(*E*)-3,5-Diamino-6-chloro-pyrazine-2-carbonylimino]-1,3,8-triaza-spiro[4.5]decane-8-carbonyl}-benzoic acid methyl ester

This compound is prepared analogously to Example 97 by replacing 4-benzyloxyphenylacetic acid with monomethyl terephthalate; $[M+H]^+$ 487.

Example 115

3,5-Diamino-6-chloro-pyrazine-2-carboxylic acid [8-(pyrimidine-5-carbonyl)-1,3,8-triaza-spiro[4.5]dec-(2*E*)-ylidene]-amide

This compound is prepared analogously to Example 97 by replacing 4-benzyloxyphenylacetic acid with pyrimidine-5-carboxylic acid; $[M+H]^+$ 431.

Example 116

3,5-Diamino-6-chloro-pyrazine-2-carboxylic acid [8-(4-hydroxy-benzoyl)-1,3,8-triaza-spiro [4.5]dec-(2*E*)-ylidene]-amide

This compound is prepared analogously to Example 97 by replacing 4-benzyloxyphenylacetic acid with 4-hydroxybenzoic acid; $[M+H]^+$ 445.

Example 117

3,5-Diamino-6-chloro-pyrazine-2-carboxylic acid [8-cyclohexanecarbonyl-1,3,8-triaza-spiro [4.5]dec-(2*E*)-ylidene]-amide

This compound is prepared analogously to Example 97 by replacing 4-benzyloxyphenylacetic acid with cyclohexanecarboxylic acid; $[M+H]^+$ 435.

Example 118

3,5-Diamino-6-chloro-pyrazine-2-carboxylic acid [8-(oxazole-4-carbonyl)-1,3,8-triaza-spiro [4.5]dec-(2*E*)-ylidene]-amide

This compound is prepared analogously to Example 97 by replacing 4-benzyloxyphenylacetic acid with oxazole-4-carboxylic acid; $[M+H]^+$ 420.

Example 119

3,5-Diamino-6-chloro-pyrazine-2-carboxylic acid [8-(pyridine-2-carbonyl)-1,3,8-triaza-spiro [4.5]dec-(2*E*)-ylidene]-amide

This compound is prepared analogously to Example 97 by replacing 4-benzyloxyphenylacetic acid with 2-picolinic acid; $[M+H]^+$ 430.

Example 120

3,5-Diamino-6-chloro-pyrazine-2-carboxylic acid [8-(pyridine-4-carbonyl)-1,3,8-triaza-spiro[4.5]dec-(2E)-ylidene]-amide

This compound is prepared analogously to Example 97 by replacing 4-benzyloxyphenylacetic acid with isonicotinic acid; $[M+H]^+$ 430.

Example 121

3,5 -Diamino-6-chloro-pyrazine-2-carboxylic acid [8-(piperidine-4-carbonyl)-1,3,8-triaza-spiro[4.5]dec-(2*E*)-ylidene]-amide hydrochloride

4 M HCl in dioxane (5 ml) is added to a solution of 4-{2-[(*E*)-3,5-diamino-6-chloro-pyrazine -2-carbonylimino]-1,3,8-triaza-spiro[4.5]decane-8-carbonyl}-piperidine-1-carboxylic acid *tert*-butyl ester (Intermediate BA) (0.14 g, 0.26 mmol) in dioxane (10 ml) and the reaction mixture is stirred at room temperature for 3 hours. The reaction mixture is concentrated *in vacuo* and the yellow solid obtained is triturated with DCM. The DCM layer is decanted and the compound is washed with MeOH and dried under vacuum to afford the title compound as yellow solid; $[M+H]^+$ 436.

Example 122

3,5-Diamino-6-chloro-pyrazine-2-carboxylic acid [8-(1H-imidazole-4-carbonyl)-1,3,8-triaza-spiro[4.5]dec-(2*E*)-ylidene]-amide

This compound is prepared analogously to Example 97 by replacing 4-benzyloxyphenylacetic acid with 4-imidazolecarboxylic acid; $[M+H]^+$ 419.

Example 123

3,5-Diamino-6-chloro-pyrazine-2-carboxylic acid [8-(tetrahydro-pyran-4-carbonyl)-1,3,8-triaza-spiro [4.5]dec-(2*E*)-ylidene]-amide

This compound is prepared analogously to Example 97 by replacing 4-benzyloxyphenylacetic acid with tetrahydropyran-4-carboxylic acid; $[M+H]^+$ 437.

Example 124

3,5-Diamino-6-chloro-pyrazine-2-carboxylic acid [8-(pyrimidine-4-carbonyl)-1,3,8-triaza-spiro[4.5]dec-(2*E*)-ylidene]-amide

This compound is prepared analogously to Example 97 by replacing 4-benzyloxyphenylacetic acid with pyrimidine-4-carboxylic acid; $[M+H]^+$ 431.

Example 125

3,5-Diamino-6-chloro-pyrazine-2-carboxylic acid [8-(oxazole-5-carbonyl)-1,3,8-triaza-spiro[4.5]dec-(2E)-ylidene]-amide

This compound is prepared analogously to Example 97 by replacing 4-benzyloxyphenylacetic acid with oxazole-5-carboxylic acid; $[M+H]^+$ 420.

Example 126

3,5-Diamino-6-chloro-pyrazine-2-carboxylic acid [8-[3-(4-isobutoxy-piperidine-1-sulfonyl)-benzoyl]-1,3,8-triaza-spiro[4.5]dec-(2E)-ylidene]-amide

Step 1

A solution of N,N-Diisopropylethylamine (0.0078ml, 0.045mmol) in THF (1 m1) is added to 4-Isobutoxy-piperidine (0.008g, 0.05mol) followed by a solution of 3-(Chlorosulfonyl)benzoic acid (9.93mg, 0.045mmol) and shaken at room temperature for 48 hours. The solution is evaporated under vacuum to afford 3-(4-Isobutoxy-piperidine-1-sulfonyl)-benzoic acid which is used without purification; $[M+H]^+$ 342.00.

Step 2

3-(4-Isobutoxy-piperidine-1-sulfonyl)-benzoic acid (0.03 mmol, 10.2 mg) is treated with a solution of HATU (11.4 mg, 0.03 mmol) in DMF (1 ml) followed by a solution of 3,5-Diamino-6-chloro-pyrazine-2-carboxylic acid [1,3,8-triaza-spiro[4.5]dec-(2E)-ylidene]-amide dihydrochloride (Ex. 38) (11.9 mg, 0.03 mmol) and N-methyl morpholine (0.010 ml, 0.03 mmol) in DMF (1 ml) and shaken at room temperature overnight. The solution is evaporated under vacuum, redissolved in DMSO (0.5 ml) and purified by mass-directed preparative HPLC. The purified fractions are evaporated under vacuum to afford the title compound; $[M+H]^+$ 648.4.

Examples 127-145

These compounds, namely

3,5-Diamino-6-chloro-pyrazine-2-carboxylic acid [8-{3-[2-(1H-indol-3-yl)-ethylsulfamoyl]-benzoybenzoyl}-1,3,8-triaza-spiro[4.5]dec-(2E)-ylidene]-amide (Ex. 127); 1-(3-{2-[(E)-3,5-Diamino-6-chloro-pyrazine-2-carbonylimino]-1,3,8-triaza-spiro[4.5]decane-8-carbonyl}-benzenesulfonyl)-piperidine-3-carboxylic acid ethyl ester (Ex. 128);
3,5-Diamino-6-chloro-pyrazine-2-carboxylic acid [8-(3-cyclopentylsulfamoyl-benzoyl)-1,3,8-triaza-spiro[4.5]dec-(2E)-ylidene]-amide (Ex. 127);
3,5-Diamino-6-chloro-pyrazine-2-carboxylic acid [8-(3-(1-acetyl-piperidin-4-ylsulfamoyl)-benzoyl]-1,3,8-triaza-spiro[4.5]dec-(2E)-ylidene]-amide (Ex. 130);
3,5-Diamino-6-chloro-pyrazine-2-carboxylic acid [8-{3-[(tetrahydro-furan-2-ylmethyl)-sulfamoyl]-benzoybenzoyl}-1,3,8-triaza-spiro[4.5]dec-(2E)-ylidene]-amide (Ex. 131);
3,5-Diamino-6-chloro-pyrazine-2-carboxylic acid [8-{3-[(pyridin-3-ylmethyl)-sulfamoyl]-benzoybenzoyl}-1,3,8-triaza-spiro[4.5]dec-(2E)-ylidene]-amide (Ex. 132);
3,5-Diamino-6-chloro-pyrazine-2-carboxylic acid [8-{3-[(2,2-dimethoxy-ethyl)-methyl-sulfamoyl]-benzoybenzoyl}-1,3,8-triaza-spiro[4.5]dec-(2E)-ylidene]-amide (Ex. 133);
3,5-Diamino-6-chloro-pyrazine-2-carboxylic acid [8-[3-(2,4-difluoro-benzylsulfamoyl)-benzoyl]-1,3,8-triaza-spiro[4.5]dec-(2E)-ylidene]-amide (Ex. 134);
3,5-Diamino-6-chloro-pyrazine-2-carboxylic acid [8-[3-(1-pyridin-4-yl-ethylsulfamoyl)-benzoyl]-1,3,8-triaza-spiro[4.5]dec-(2E)-ylidene]-amide (Ex. 135);
3,5-Diamino-6-chloro-pyrazine-2-carboxylic acid [8-[3-(2-phenyl-morpholine-4-sulfonyl)-benzoyl]-1,3,8-triaza-spiro[4.5]dec-(2E)-ylidene]-amide (Ex. 136);
3,5-Diamino-6-chloro-pyrazine-2-carboxylic acid [8-[3-(3-difluoromethoxy-benzylsulfamoyl)-benzoyl]-1,3,8-triaza-spiro[4.5]dec-(2E)-ylidene]-amide (Ex. 137);
3,5-Diamino-6-chloro-pyrazine-2-carboxylic acid [8-[3-(4-pyrrolidin-1-yl-piperidine-1-sulfonyl)-benzoyl]-1,3,8-triaza-spiro[4.5]dec-(2E)-ylidene]-amide (Ex. 138);
3,5-Diamino-6-chloro-pyrazine-2-carboxylic acid [8-{3-[(5-methyl-pyrazin-2-ylmethyl)-sulfamoyl]-benzoybenzoyl}-1,3,8-triaza-spiro[4.5]dec-(2E)-ylidene]-amide (Ex. 139);
3,5-Diamino-6-chloro-pyrazine-2-carboxylic acid [8-[3-(dimethylcarbamoylmethyl-sulfamoyl)-benzoyl]-1,3,8-triaza-spiro[4.5]dec-(2E)-ylidene]-amide (Ex. 140);
3,5-Diamino-6-chloro-pyrazine-2-carboxylic acid [8-[3-(3-benzenesulfonyl-pyrrolidine-1-sulfonyl)-benzoyl]-1,3,8-triaza-spiro[4.5]dec-(2E)-ylidene]-amide (Ex. 141);
3,5-Diamino-6-chloro-pyrazine-2-carboxylic acid [8-{3-[([1,3]dioxolan-2-ylmethyl)-sulfamoyl]-benzoybenzoyl}-1,3,8-triaza-spiro[4.5]dec-(2E)-ylidene]-amide (Ex. 142);
3,5-Diamino-6-chloro-pyrazine-2-carboxylic acid [8-{3-[2-(pyridin-3-yloxy)-propylsulfamoyl]-benzoybenzoyl}-1,3,8-triaza-spiro[4.5]dec-(2E)-ylidene]-amide (Ex. 143);

3,5-Diamino-6-chloro-pyrazine-2-carboxylic acid [8-{3-[4-(5-trifluoromethyl-pyridin-2-yl)-[1,4]diazepane-1-sulfonylFbenzoybenzoyl}-1,3,8-triaza-spiro[4.5]dec-(2E)-ylidene]-amide (Ex. 144);

3,5-Diamino-6-chloro-pyrazine-2-carboxylic acid [8-[3-(1,1-dioxo-tetrahydro-1lambda*6*-thiophen-3-ylsulfamoyl)-benzoyl]-1,3,8-triaza-spiro[4.5]dec-(2E)-ylidene]-amide (Ex. 145);

are made analogously to Examples 126 replacing 4-isobutoxy-piperidine in step 1 with the appropriate amines which are all commercially available. The compounds are recovered from the reaction mixture and purified using conventional techniques.

Example 146

3,5-Diamino-6-chloro-pyrazine-2-carboxylic acid [8-{3-[3-(4-chlorophenyl)-[1,2,4]oxadiazol-5-yl]-propionyl}-1,3,8-triaza-spiro[4.5]dec-(2E)-ylidene-amide trifluoroacetate

N-methyl morpholine (33 μl, 0.3 mmol) is added to 3-(3-p-Tolyl-[1,2,4]oxadiazol-5-yl) -propionic acid (0.1 mmol), followed by HATU (41.8 mg, 0.11 mmol) dissolved in peptide grade DMF (250 μl) and 3,5-Diamino-6-chloro-pyrazine-2-carboxyfic acid [1,3,8-triaza-spiro[4.5]dec-(2E)-ylidene]-amide dihydrochloride (Ex. 38) (40 mg, 0.1 mmol) dissolved in peptide grade DMF (250 μl). The reaction is scaled and shaken overnight at room temperature. Purification is by mass-directed preparative HPLC to give the title compound; $[M+H]^+$ 559.3.

Example 147

3,5-Diamino-6-chloro-pyrazine-2-carboxylic acid [8-[1-(toluene-4-sulfonyl)-1H-pyrrole-3-carbonyl]-1,3,8-triaza-spiro [4.5]dec-(2E)-ylidene]-amide

A solution of 1-(Toluene-4-sulfonyl)-1H-pyrrole-3-carboxylic acid (0.023 g, 0.085 mmol) in NMP (850 μl) is added to PS-carbodiimide (190 mg of 1.3 mmol/g loading, 0.24 mmol), followed by a solution of 3,5-Diamino-6-chloro-pyrazine-2-carboxylic acid [1,3,8-triaza-spiro[4.5]dec-(2E)-ylidene]- amide dihydrochloride (Ex. 38) (0.08 mmol) and N-methyl morpholine (8 μl, 0.08 mmol) in NMP (1 ml), and the resulting reaction mixture is shaken at room temperature. The reaction mixture is filtered and the resin is washed with NMP (1 ml). The collected filtrate is concentrated *in vacuo* and the residues are purified by mass-directed preparative HPLC. The purified fractions are evaporated under vacuum to afford the title compound; $[M+H]^+$ 572.08.

Example 148

3,5-Diamino-6-chloro-pyrazine-2-carboxylic acid [8-[1-(3,4-difluoro-benzyl)-6-oxo-1,6-dihydro-pyridine-3-carbonyl]-1,3,8-triaza-spiro [4.5]dec-(2E)-ylidene]-amide

A solution of 1-(3,4-Difluoro-benzyl)-6-oxo-1,6-dihydro-pyridine-3-carboxylic acid (0.15 mmol) in NMP (0.5 ml) is added to a solution of 3,5-Diamino-6-chloro-pyrazine-2-carboxylic acid [1,3,8-triaza-spiro[4.5]dec-(2E)-ylidene]-amide dihydrochloride (Ex. 38) (0.049 g, 0.15 mmol) and N-methyl morpholine (0.033 ml, 0.30 mmol) in NMP (1 ml), followed by a solution of HATU (0.11 g, 0.3 mmol) in NMP (0.5 ml). The reaction mixture is shaken at room temperature overnight. The reaction mixture is purified by mass-directed preparative HPLC. Fractions containing pure product are eluted through SCX-2 cartridges (Biotage 1g/6ml cartridge), and the cartridge is washed with MeOH (4 ml), followed by 3M $NH_3$ in MeOH solution (4 ml) to afford the title compound; $[M+H]^+$ 572.0.

Examples 149-213

These compounds,

namely 3,5-Diamino-6-chloro-pyrazine-2-carboxylic acid [8-[4-(3-phenyl-isoxazol-5-yl) -butyryl]-1,3,8-triaza-spiro[4.5] dec-(2E)-ylidene-amide (Ex. 149);

3,5-Diamino-6-chloro-pyrazine-2-carboxylic acid [8-[4-(5-fluoro-2,3-dihydro-indol-lyl)-4-oxo-butyryl]-1,3,8-triaza-spiro[4.5] dec-(2E)-ylidene-amide (Ex. 150);

3,5-Diamino-6-chloro-pyrazine-2-carboxylic acid [8-{4-[3-(4-methoxy-phenyl)-[1,2,4]oxadiazol-5yl]-butyryl}-1,3,8-triaza-spiro[4.5] dec-(2E)-ylidene-amide (Ex. 151);

3,5-Diamino-6-chloro-pyrazine-2-carboxylic acid [8-(4-1H-indazol-3-yl-butyryl)-1,3,8-triaza-spiro[4.5] dec-(2E)-ylidene-amide (Ex. 152);

3,5-Diamino-6-chloro-pyrazine-2-carboxylic acid [8-[4-(5-methanesulfonyl-2,3-dihydro-indol-1yl)-4-oxo-butyryl]-1,3,8-triaza-spiro[4.5] dec-(2E)-ylidene-amide (Ex. 153);
3,5-Diamino-6-chloro-pyrazine-2-carboxylic acid [8-(4-benzothiazol-2-yl-butyryl)-1,3,8-triaza-spiro[4.5] dec-(2E)-ylidene-amide (Ex. 154);
3,5-Diamino-6-chloro-pyrazine-2-carboxylic acid [8-[4-(5-dimethylsulfamoyl-2,3-dihydro-indol-1yl)-4-oxo-butyryl]-1,3,8-triaza-spiro[4.5] dec-(2E)-ylidene-amide (Ex. 155);
3,5-Diamino-6-chloro-pyrazine-2-carboxylic acid [8-[4-(2-oxo-2,3-dihydro-1H-indol-3yl)-butyryl]-1,3,8-triaza-spiro[4.5] dec-(2E)-ylidene-amide (Ex. 156);
3,5-Diamino-6-chloro-pyrazine-2-carboxylic acid [8-[4-(6-dimethylamino-9H-purin-8yl)-butryrl]-1,3,8-triaza-spiro[4.5] dec-(2E)-ylidene-amide (Ex. 157);
3,5-Diamino-6-chloro-pyrazine-2-carboxylic acid [8-[4-(2-oxo-3-pyridin-3yl-2,3-dihydro-benzoimidazol-1-yl)-butyryl]-1,3,8-triaza-spiro[4.5] dec-(2E)-ylidene-amide (Ex. 158);
3,5-Diamino-6-chloro-pyrazine-2-carboxylic acid [8-[4-(2-oxo-3-pyridine-3ylmethyl-2,3-dihydro-indol-1-yl)-butryrl]-1,3,8-triaza-spiro[4.5] dec-(2E)-ylidene-amide (Ex. 159);
3,5-Diamino-6-chloro-pyrazine-2-carboxylic acid [8-[4-(9-oxo-3,3a,4,9,10,10a-hexahydro-1H-2-aza-benzol[F]azulen-2yl)-butyryl]-1,3,8-triaza-spiro[4.5]dec-(2E)-ylidene-amide (Ex. 160);
3,5-Diamino-6-chloro-pyrazine-2-carboxylic acid [8-[4-(6-amino-9H-purine-8yl)-butyryl]-1,3,8-triaza-spiro[4.5] dec-(2E)-ylidene-amide (Ex. 161);
3,5-Diamino-6-chloro-pyrazine-2-carboxylic acid [8-(4-oxo-4-pyrrolidin-1-yl-butyryl)-1,3,8-triaza-spiro[4.5] dec-(2E)-ylidene-amide (Ex. 162);
3,5-Diamino-6-chloro-pyrazine-2-carboxylic acid [8-(4-[1,2,4]triazol-1-yl-butyryl)-1,3,8-triaza-spiro[4.5] dec-(2E)-ylidene-amide (Ex. 163);
3,5-Diamino-6-chloro-pyrazine-2-carboxylic acid [8-(5-dibenzylsulfamoyl-1-methyl-1H-pyrrole-2-carbonyl)-1,3,8-triaza-spiro[4.5]dec-(2E)-ylidene]-amide (Ex. 164);
3,5-Diamino-6-chloro-pyrazine-2-carboxylic acid [8-{4-[3-(4-chloro-phenyl)-[1,2,4]oxadiazol-5-yl]-butyrybenzoyl}-1,3,8-triaza-spiro[4.5]dec-(2E)-ylidene]-amide (Ex. 165);
3,5-Diamino-6-chloro-pyrazine-2-carboxylic acid [8-{4-[(naphthalene-1-sulfonylamino)-methyl]-benzoybenzoyl}-1,3,8-triaza-spiro[4.5]dec-(2E)-ylidene]-amide (Ex. 166);
3,5-Diamino-6-chloro-pyrazine-2-carboxylic acid [8-{2[3-(4-chlorophenyl)-[1,2,4] oxadiazol-5-ylFacetybenzoyl}-1,3,8-triaza-spiro[4.5]dec-(2E)-ylidene-amide (Ex. 167);
3,5-Diamino-6-chloro-pyrazine-2-carboxylic acid [8-[3-(3-methoxy-propoxy)-benzoyl]-1,3,8-triaza-spiro[4.5]dec-(2E)-ylidene]-amide (Ex. 168);
3,5-Diamino-6-chloro-pyrazine-2-carboxylic acid [8-(2-benzotriazol-2-yl-acetyl)-1,3,8-triaza-spiro[4.5]dec-(2E)-ylidene]-amide (Ex. 169)
3,5-Diamino-6-chloro-pyrazine-2-carboxylic acid [8-(2-benzotriazol-2-yl-acetyl)-1,3,8-triaza-spiro[4.5]dec-(2E)-ylidene]-amide (Ex. 170);
3,5-Diamino-6-chloro-pyrazine-2-carboxylic acid [8-[4-(2-isopropoxy-ethylamino)-benzoyl]-1,3,8-triaza-spiro[4.5]dec-(2E)-ylidene]-amide (Ex. 171);
3,5-Diamino-6-chloro-pyrazine-2-carboxylic acid [8-[6-oxo-1-(3-trifluoromethylbenzyl)-1,6-dihydro-pyridine-3-carbonyl]-1,3,8-triaza-spiro[4.5]dec-(2E)-ylidene]- amide (Ex. 172);
3,5-Diamino-6-chloro-pyrazine-2-carboxylic acid [8-[6-(4-methyl-piperazin-1-yl)-pyridine-3-carbonyl]-1,3,8-triaza-spiro[4.5]dec-(2E)-ylidene]-amide (Ex. 173);
3,5-Diamino-6-chloro-pyrazine-2-carboxylic acid [8-[3-(4-fluoro-phenyl)-5-methyl-isoxazole-4-carbonyl]-1,3,8-triaza-spiro[4.5]dec-(2E)-ylidene]-amide (Ex. 174);
3,5-Diamino-6-chloro-pyrazine-2-carboxylic acid [8-{3-[3-(4-methoxy-phenyl)-[1,2,4]oxadiazol-5-yl]-propionyl1-1,3,8-triaza-spiro[4.5]dec-(2E)-ylidene]-amide (Ex. 175);
3,5-Diamino-6-chloro-pyrazine-2-carboxylic acid [8-[2-(4-trifluoromethoxy-phenoxy)-acetyl]-1,3,8-triaza-spiro[4.5]dec-(2E)-ylidene]-amide (Ex. 176);
3,5-Diamino-6-chloro-pyrazine-2-carboxylic acid [8-{2-[4-(2-oxo-imidazolidin-1-yl)-phenyl]-acetybenzoyl}-1,3,8-triaza-spiro[4.5]dec-(2E)-ylidene]-amide (Ex. 177);
3,5-Diamino-6-chloro-pyrazine-2-carboxylic acid [8-[3-(3-phenyl-isoxazol-5-yl)-propionyl]-1,3,8-triaza-spiro[4.5]dec-(2E)-ylidene]-amide (Ex. 178);
3,5-Diamino-6-chloro-pyrazine-2-carboxylic acid [8-[2-(4-methanesulfonyl-phenyl)-acetyl]-1,3,8-triaza-spiro[4.5]dec-(2E)-ylidene]-amide (Ex. 179);
3,5-Diamino-6-chloro-pyrazine-2-carboxylic acid [8-[2-(4-chloro-phenyl)-thiazole-4-carbonyl]-1,3,8-triaza-spiro[4.5]dec-(2E)-ylidene]-amide (Ex. 180);
3,5-Diamino-6-chloro-pyrazine-2-carboxylic acid [8-[2-(5-methyl-3-trifluoromethyl-pyrazol-1-yl)-acetyl]-1,3,8-triaza-spiro[4.5]dec-(2E)-ylidene]-amide (Ex. 181);

3,5-Diamino-6-chloro-pyrazine-2-carboxylic acid [8-[5-(pyridin-3-yloxy)-furan-2-carbonyl]-1,3,8-triaza-spiro[4.5]dec-(2E)-ylidene]-amide (Ex. 182);
3,5-Diamino-6-chloro-pyrazine-2-carboxylic acid [8-[3-(4-methyl-thiazol-5-yl)-propionyl]-1,3,8-triaza-spiro[4.5]dec-(2E)-ylidene]-amide (Ex. 183);
3,5-Diamino-6-chloro-pyrazine-2-carboxylic acid [8-(2-methyl-5-propyl-2H-pyrazole3-carbonyl)-1,3,8-triaza-spiro[4.5]dec-(2E)-ylidene]-amide (Ex. 184);
3,5-Diamino-6-chloro-pyrazine-2-carboxylic acid [8-[(*S*)-2-acetylamino-3-(4-isopropoxy-phenyl)-propionyl]-1,3,8-triaza-spiro[4.5]dec-(2E)-ylidene]-amide (Ex. 185);
3,5-Diamino-6-chloro-pyrazine-2-carboxylic acid [8-[3-(cyclohexyl-methyl-sulfamoyl)-4-methoxy-benzoyl]-1,3,8-triaza-spiro[4.5]dec-(2E)-ylidene]-amide (Ex. 186);
3,5-Diamino-6-chloro-pyrazine-2-carboxylic acid [8-{2-[4-(3,5-dimethyl-benzenesulfonyl)-piperazin-1-yl]-acetybenzoyl}-1,3,8-triaza-spiro[4.5]dec-(2E)-ylidene]-amide (Ex. 187);
3,5-Diamino-6-chloro-pyrazine-2-carboxylic acid [8-(3-1H-indol-3-yl-propionyl)-1,3,8-triaza-spiro[4.5]dec-(2E)-ylidene]-amide (Ex. 188);
3,5-Diamino-6-chloro-pyrazine-2-carboxylic acid [8-{3-[4-(4,6-dimethyl-pyrimidin-2-ylsulfamoyl)-phenylcarbamoyl]-propionyll-1,3,8-triaza-spiro[4.5]dec-(2E)-ylidene]-amide (Ex. 189);
(Ex.190);
3,5-Diamino-6-chloro-pyrazine-2-carboxylic acid [8-[3-(2-oxo-5-trifluoromethyl-2H-pyridin-1-yl)-propionyl]-1,3,8-triaza-spiro[4.5]dec-(2E)-ylidene]-amide (Ex. 191);
3,5-Diamino-6-chloro-pyrazine-2-carboxylic acid [8-[3-(4-sulfamoyl-phenylcarbamoyl)-propionyl]-1,3,8-triaza-spiro[4.5]dec-(2E)-ylidene]-amide (Ex. 192);
3,5-Diamino-6-chloro-pyrazine-2-carboxylic acid [8-(1-benzyl-5-oxo-pyrrolidine-3-carbonyl)-1,3,8-triaza-spiro[4.5]dec-(2E)-ylidene]-amide (Ex. 193);
3,5-Diamino-6-chloro-pyrazine-2-carboxylic acid [8-[(R)-2-acetylamino-3-(1H-indol-3-yl)-propionyl]-1,3,8-triaza-spiro[4.5]dec-(2E)-ylidene]-amide (Ex. 194);
3,5-Diamino-6-chloro-pyrazine-2-carboxylic acid [8-[4-(1-benzenesulfonyl-1H-pyrrol3-yl)-4-oxo-butyryl]-1,3,8-triaza-spiro[4.5] dec-(2E)-ylidene-amide (Ex. 195);
3,5-Diamino-6-chloro-pyrazine-2-carboxylic acid [8-(1-furan-2-ylmethyl-5-oxo-pyrrolidine-3-carbonyl)-1,3,8-triaza-spiro[4.5]dec-(2E)-ylidene]-amide (Ex. 196);
3,5-Diamino-6-chloro-pyrazine-2-carboxylic acid [8-(6-pyrazol-1-yl-pyridine-3-carbonyl)-1,3,8-triaza-spiro[4.5]dec-(2E)-ylidene]-amide (Ex. 197);
3,5-Diamino-6-chloro-pyrazine-2-carboxylic acid [8-(3-((R)-1-phenyl-ethylcarbamoyl)-propionyl]-1,3,8-triaza-spiro[4.5]dec-(2E)-ylidene]-amide (Ex. 198);
3,5-Diamino-6-chloro-pyrazine-2-carboxylic acid [8-[1-(4-chloro-benzyl)-5-oxo-pyrrolidine-3-carbonyl]-1,3,8-triaza-spiro[4.5]dec-(2E)-ylidene]-amide (Ex. 199);
3,5-Diamino-6-chloro-pyrazine-2-carboxylic acid [8-[2-(3-tert-butyl-isoxazol-5-yl)-acetyl]-1,3,8-triaza-spiro[4.5]dec-(2E)-ylidene]-amide (Ex. 200);
3,5-Diamino-6-chloro-pyrazine-2-carboxylic acid [8-[6-(2,2,2-trifluoro-ethoxy)-pyridine-3-carbonyl]-1,3,8-triaza-spiro[4.5]dec-(2E)-ylidene]-amide (Ex. 201);
3,5-Diamino-6-chloro-pyrazine-2-carboxylic acid [8-(4-methyl-2-pyridin-3-yl-thiazole5-carbonyl)-1,3,8-triaza-spiro[4.5]dec-(2E)-ylidene]-amide (Ex. 202);
3,5-Diamino-6-chloro-pyrazine-2-carboxylic acid [8-(3-pyridin-3-yl-propionyl)-1,3,8-triaza-spiro[4.5]dec-(2E)-ylidene]-amide (Ex. 203);
3,5-Diamino-6-chloro-pyrazine-2-carboxylic acid [8-(5-dimethylsulfamoyl-2-methyl-furan-3-carbonyl)-1,3,8-triaza-spiro[4.5]dec-(2E)-ylidene]-amide (Ex. 204);
3,5-Diamino-6-chloro-pyrazine-2-carboxylic acid [8-(1-ethyl-7-methyl-4-oxo-1,4-dihydro-[1,8]naphthyridine-3-carbonyl)-1,3,8-triaza-spiro[4.5]dec-(2E)-ylidene]-amide (Ex. 205);
3,5-Diamino-6-chloro-pyrazine-2-carboxylic acid [8-(2-pyrazol-1-yl-acetyl)-1,3,8-triaza-spiro[4.5]dec-(2E)-ylidene]-amide (Ex. 206);
3,5-Diamino-6-chloro-pyrazine-2-carboxylic acid [8-{3-chloro-5-methoxy-4-[2-(4-methyl-piperazin-1-yl)-ethoxy]-benzoybenzoyl}-1,3,8-triaza-spiro[4.5]dec-(2E)-ylidene]-amide (Ex. 207);
3,5-Diamino-6-chloro-pyrazine-2-carboxylic acid [8-(3-imidazol-1-yl-propionyl)-1,3,8-triaza-spiro[4.5]dec-(2E)-ylidene]-amide (Ex. 208);
3,5-Diamino-6-chloro-pyrazine-2-carboxylic acid [8-(1-benzyl-1H-imidazole-4-carbonyl)-1,3,8-triaza-spiro[4.5]dec-(2E)-ylidene]-amide (Ex. 209);
3,5-Diamino-6-chloro-pyrazine-2-carboxylic acid [8-[2-(1,1-dioxo-1lambda*6*-thiomorpholin-4-yl)-3-methyl-butyryl]-1,3,8-triaza-spiro [4.5] dec- (2E)-ylidene]-amide (Ex. 210);
3,5-Diamino-6-chloro-pyrazine-2-carboxylic acid [8-[4-(toluene-4-sulfonylamino)-butyryl]-1,3,8-triaza-spiro [4.5]

dec- (2E)-ylidene]-amide (Ex. 211);
3,5-Diamino-6-chloro-pyrazine-2-carboxylic acid [8-(1,5-dimethyl-3-oxo-2-phenyl-2,3-dihydro-1H-pyrazole-4-carbonyl)-1,3,8-triaza-spiro [4.5] dec- (2E)-ylidene]-amide (Ex. 212);
3,5-Diamino-6-chloro-pyrazine-2-carboxylic acid [8-(3-hydroxy-pyridine-2-carbonyl)-1,3,8-triaza-spiro[4.5]dec-(2E)-ylidene]-amide (Ex. 213);

are made analogously to Examples 146, 147 or 148 replacing the carboxylic acid reagents with the appropriate carboxylic acids which are all commercially available or prepared as described in section 'Preparation of Intermediate Compounds'. The compounds are recovered from the reaction mixture and purified using conventional techniques.

Example 214

1-(3-{2-[(E)-3,5-Diamino-6-chloro-pyrazine-2-carbonylimino]-1,3,8-triayrazine-2-carbonylimino]-1,3,8-triazenesulfonyl)-piperidine-3 -carboxylic acid

1-(3-{2-[(E)-3,5-Diamino-6-chloro-pyrazine-2-carbonylimino]-1,3,8-triaza-spiro[4.5]decane-8-carbonyl}-benzenesulfonyl)-piperidine-3-carboxylic acid ethyl ester (Ex. 128) (0.29 g, 0.45 mmol) is dissolved in TIIF (4 ml) and 2M LiOII (0.22 ml, 0.45 mmol) added. The yellow solution is stirred at room temperature for 5 hours. On concentration *in vacuo* the resulting sticky yellow solid is ultrasonicated in water (15 ml) until complete dissolution. The pH is adjusted to pH 2 by addition of 1 N HCl. The resultant yellow solid is collected by filtration and rinsed with water to yield the title compound; $[M+H]^+$ 620.1.

Example 215

2-[(E)-3,5-Diamino-6-chloro-pyrazine-2-carbonylimino]-1,3,8-triaza-spiro [4.5] decane-8-carboxylic acid benzylamide

To a solution of benzylamine (0.017 ml, 0.154 mmol) in DMF (1 ml) is added 1,1'-carbonyldiimidazole (0.03 g, 0.17 mmol) and the resulting solution is stirred at room temperature for 45 minutes. To this is added 5-Diamino-6-chloro-pyrazine-2-carboxylic acid [1,3,8-triaza-spiro[4.5]dec-(2E)-ylidene]-amide dihydrochloride (Ex. 38) (0.05 g, 0.15 mmol) and the yellow suspension is stirred for 24 hours. Purification by reverse phase chromatography (Isolute™ C18, 0-100% MeCN in water - 0.1 %TFA) followed by catch and release resin (SCX-2) eluting with MeOH and 7M $NH_3$ in MeOH affords the title compound as an off white solid; $[M+H]^+$ 458.1.

Examples 216-231

These compounds, namely

2-[(((E)-3,5-Diamino-6-chloro-pyrazine-2-carbonylimino]-1,3,8-triaza-spiro [4.5] decane-8-carboxylic acid phenylamide (Ex. 216),
2-[((E)-3,5-Diamino-6-chloro-pyrazine-2-carbonylimino]-1,3,8-triaza-spiro [4.5] decane-8-carboxylic acid [1-(toluene-4-sulfonyl)-1H-indol-5-yl]-amide (Ex. 217);
2-[(((E)-3,5-Diamino-6-chloro-pyrazine-2-carbonylimino]-1,3,8-triaza-spiro [4.5] decane-8-carboxylic acid 3-(4-chloro-phenoxymethyl)-benzylamide (Ex. 218);
2-[(((E)-3,5-Diamino-6-chloro-pyrazine-2-carbonylimino]-1,3,8-triaza-spiro [4.5] decane-8-carboxylic acid [3-(2,4-dichloro-phenyl)-propyl]-amide (Ex. 219);
2-[(((E)-3,5-Diamino-6-chloro-pyrazine-2-carbonylimino]-1,3,8-triaza-spiro [4.5] decane-8-carboxylic acid [2-(3-benzyloxy-phenyl)-ethyl]amide (Ex. 220);
2-[(((E)-3,5-Diamino-6-chloro-pyrazine-2-carbonylimino]-1,3,8-triaza-spiro [4.5] decane-8-carboxylic acid [2-(5,6-dimethyl-1H-indol-3-yl)-ethyl]amide (Ex. 221);
2-[(((E)-3,5-Diamino-6-chloro-pyrazine-2-carbonylimino]-1,3,8-triaza-spiro [4.5] decane-8-carboxylic acid 4-morpholin-4-ylmethyl-benzylamide (Ex. 222);
2-[(((E)-3,5-Diamino-6-chloro-pyrazine-2-carbonylimino]-1,3,8-triaza-spiro [4.5] decane-8-carboxylic acid 3-benzyloxy-benzylamide (Ex. 223);
2-[(((E)-3,5-Diamino-6-chloro-pyrazine-2-carbonylimino]-1,3,8-triaza-spiro [4.5] decane-8-carboxylic acid (2-{442-(4-fluoro-phenyl)-ethoxy]-phenyll-ethyl)-amide (Ex. 224);
2-[(((E)-3,5-Diamino-6-chloro-pyrazine-2-carbonylimino]-1,3,8-triaza-spiro [4.5] decane-8-carboxylic acid [2-(3,5-dimethoxy-phenyl)-ethyl]-amide (Ex. 225);
2-[(((E)-3,5-Diamino-6-chloro-pyrazine-2-carbonylimino]-1,3,8-triaza-spiro [4.5] decane-8-carboxylic acid [3-(4-methoxy-naphthalen-1-yl)-propyl]-amide (Ex. 226);
2-[(((E)-3,5-Diamino-6-chloro-pyrazine-2-carbonylimino]-1,3,8-triaza-spiro [4.5] decane-8-carboxylic acid [2-(4,6-dimethyl-1H-indol-3-yl)-ethyl]amide (Ex. 227);
2-[(((E)-3,5-Diamino-6-chloro-pyrazine-2-carbonylimino]-1,3,8-triaza-spiro [4.5] decane-8-carboxylic acid (3-pyridin-2-yl-propyl)-amide (Ex. 228);
2-[(((E)-3,5-Diamino-6-chloro-pyrazine-2-carbonylimino]-1,3,8-triaza-spiro [4.5] decane-8-carboxylic acid {2-[4-(4-phenyl-butoxy)-phenyl]-ethyl}-amide (Ex. 229);
2-[(((E)-3,5-Diamino-6-chloro-pyrazine-2-carbonylimino]-1,3,8-triaza-spiro [4.5] decane-8-carboxylic acid [2-(4-phenoxy-phenyl)-ethyl]-amide (Ex. 230);
2-[(((E)-3,5-Diamino-6-chloro-pyrazine-2-carbonylimino]-1,3,8-triaza-spiro [4.5] decane-8-carboxylic acid [2-(4-benzyloxy-phenyl)-ethyl]-amide (Ex. 231);

are prepared by an analogous procedure to Example 215, replacing benzylamine with the appropriate amines which are either commercially available or synthesized as described in the section 'Preparation of Intermediate compounds'. The compounds are recovered from reaction mixtures and purified using conventional techniques such as flash chromatography, filtration, recrystallisation and trituration.

Example 232

3,5-Diamino-6-chloro-pyrazine-2-carboxylic acid [8-phenylmethanesulfonyl-1,3,8-triaza-spiro[4.5]dec-(2*E*)-ylidene]-amide

To a solution of 5-Diamino-6-chloro-pyrazine-2-carboxylic acid [1,3,8-triaza-spiro[4.5]dec-(2E)-ylidene]- amide dihydrochloride (Ex. 38) (0.05 g, 0.15 mmol) in DMF (2 ml) is added alpha-toluenesulfonyl chloride (0.04 g, 0.20 mmol) and triethylamine (0.02 ml, 0.15 mmol) and the yellow solution is stirred at room temperature for 2 hours. Purification by reverse phase chromatography (Isolute™ C18, 0-100% MeCN in water -0.1%TFA) followed by catch and release resin (SCX-2) eluting with MeOH and 7M $NH_3$ in MeOH affords the title compound as a yellow solid; $[M+H]^+$ 478.98.

Examples 233-245

The following compounds, namely

3,5-Diamino-6-chloro-pyrazine-2-carboxylic acid [8-benzenesulfonyl-1,3,8-triaza-spiro[4.5]dec-(2E)-ylidene]-amide (Ex. 233);
3,5-Diamino-6-chloro-pyrazine-2-carboxylic acid [8-(1-methyl-1H-indole-4-sulfonyl)-1,3,8-triaza-spiro[4.5]dec-(2E)-ylidene]-amide (Ex. 234);
3,5-Diamino-6-chloro-pyrazine-2-carboxylic acid [8-(1-methyl-1H-indole-5-sulfonyl)-1,3,8-triaza-spiro[4.5]dec-(2E)-ylidene]-amide (Ex. 235);
3,5-Diamino-6-chloro-pyrazine-2-carboxylic acid [8-(7-chloro-benzo[1,2,5]oxadiazole4-sulfonyl)-1,3,8-triaza-spiro[4.5]dec-(2E)-ylidene]-amide (Ex. 236);
3,5-Diamino-6-chloro-pyrazine-2-carboxylic acid [8-(2-phenyl-ethanesulfonyl)-1,3,8-triaza-spiro[4.5]dec-(2E)-ylidene]-amide (Ex. 237);
3,5-Diamino-6-chloro-pyrazine-2-carboxylic acid [8-[4-(5-methyl-2-phenyl-oxazol-4-ylmethoxy)-benzenesulfonyl]-1,3,8-triaza-spiro[4.5]dec-(2E)-ylidene]-amide (Ex. 238);
3,5-Diamino-6-chloro-pyrazine-2-carboxylic acid [8-(4-phenyl-5-trifluoromethyl-thiophene-3-sulfonyl)-1,3,8-triaza-spiro[4.5]dec-(2E)-ylidene]-amide (Ex. 239);
3,5-Diamino-6-chloro-pyrazine-2-carboxylic acid [8-(5-cyano-2-methoxy-benzenesulfonyl)-1,3,8-triaza-spiro[4.5]dec-(2E)-ylidene]-amide (Ex. 240); 3
,5-Diamino-6-chloro-pyrazine-2-carboxylic acid [8-[2-(4-chloro-phenyl)-ethanesulfonyl]-1,3,8-triaza-spiro[4.5]dec-(2E)-ylidene]-amide (Ex. 241);
3,5-Diamino-6-chloro-pyrazine-2-carboxylic acid [8-(2-phenyl-3H-benzoimidazole-5-sulfonyl)-1,3,8-triaza-spiro[4.5]dec-(2E)-ylidene]-amide (Ex. 242);
3,5-Diamino-6-chloro-pyrazine-2-carboxylic acid [8-[2-(2-chloro-phenyl)-ethanesulfonyl]-1,3,8-triaza-spiro[4.5]dec-(2E)-ylidene]-amide (Ex. 243);
3,5-Diamino-6-chloro-pyrazine-2-carboxylic acid [8-[2-(2,2,2-trifluoro-acetyl)-1,2,3,4-tetrahydro-isoquinoline-7-sulfonyl]-1,3,8-triaza-spiro[4.5]dec-(2E)-ylidene]-amide (Ex. 244);
3,5-Diamino-6-chloro-pyrazine-2-carboxylic acid [8-[2-(3-chloro-phenyl)-ethanesulfonyl]-1,3,8-triaza-spiro[4.5]dec-(2E)-ylidene]-amide (Ex. 245);

are prepared by an analogous procedure to Example 232, replacing alpha-toluenesulfonyl chloride with the appropriate sulfonyl chlorides which are either commercially available or synthesized as described in the section 'Preperation of Intermediate compounds'. The compounds are recovered from reaction mixtures and purified using conventional techniques such as flash chromatography, filtration, recrystallisation and trituration.

Example 246

3,5-Diamino-6-chloro-pyrazine-2-carboxylic acid [8-(1-phenyl-ethyl)-1,3,8-triaza-spiro[4.5]dec-(2E)-ylidene]-amide

A mixture of 1-(3,5-diamino-6-chloro-pyrazine-2-carbonyl)-2-methyl-isothiourea (Intermediate A)(1.7 g, 4.54 mmol) and 4-aminomethyl-1-(1-phenyl-ethyl)-piperidin4-ylamine (Intermediate BM) (1.6 g, 4.59 mmol) in propan-2-ol (50 ml) is stirred at 80 °C for 16 hours. The reaction mixture is concentrated *in vacuo* and purified by column chromatography (basic alumina, 0-2% MeOH in DCM) to obtain pale yellow solid. The compound obtained is further dissolved in MeOH and precipitated by adding diethyl ether. The supernatant solvent mixture is decanted and the product is washed again with diethyl ether and dried under vacuum to afford the title compound as off-white solid; $[M+H]^+$ 429.

Example 247

3,5-Diamino-6-chloro-pyrazine-2-carboxylic acid [8-(4-methoxy-benzyl)-1,3,8-triaza-spiro [4.5]dec-(2E)-ylidene]-amide

This compound is prepared analogously to Example 246 by replacing 4-aminomethyl-1-(1-phenyl-ethyl)-piperidin-4-ylamine (Intermediate BM) with 4-aminomethyl-1-(4-methoxy-benzyl)-piperidin-4-ylamine (Intermediate BN) $[M+H]^+$ 445.

Example 248

3,5 -Diamino-6-chloro-pyrazine-2-carboxylic acid [8-pyridin-4-ylmethyl-1,3,8-triaza-spiro [4.5]dec-(2E)-ylidene]-amide

This compound is prepared analogously to Example 246 by replacing 4-aminomethyl-1-(1-phenyl-ethyl)-piperidin-4-ylamine (Intermediate BM) with 4-aminomethyl-1-pyridin-4-ylmethyl-piperidin-4-ylamine (Intermediate BO); $[M+H]^+$ = 416.

Example 249

3,5-Diamino-6-chloro-pyrazine-2-carboxylic acid [8-(3-phenyl-propyl)-1,3,8-triaza-spiro [4.5]dec-(2E)-ylidene]-amide

This compound is prepared analogously to Example 246 by replacing 4-aminomethyl-1-(1-phenyl-ethyl)-piperidin-4-ylamine (Intermediate BM) with 4-aminomethyl-1-(3-phenyl-propyl)-piperidin-4-ylamine (Intermediate BP) $[M+H]^+$ 443.

Example 250

3,5-Diamino-6-chloro-pyrazine-2-carboxylic acid [8-cyclohexylmethyl-1,3,8-triaza-spiro [4.5]dec-(2E)-ylidene]-amide

This compound is prepared analogously to Example 246 by replacing 4-aminomethyl-1-(1-phenyl-ethyl)-piperidin-4-ylamine (Intermediate BM) with 4-aminomethyl-1-cyclohexylmethyl-piperidin-4-ylamine (Intermediate BQ) $[M+H]^+$ 421.

Example 251

(E)-*tert*-Butyl 2'-(3,5-diamino-6-chloropyrazine-2-carbonylimino)-8-azaspiro [bicyclo [3.2.1] octane-3,4'-imidazolidine]-8-carboxylate

This compound is prepared analogously to Example 246 by replacing 4-aminomethyl-1-(1-phenyl-ethyl )-piperidin-4-ylamine (Intermediate BM) with 3-amino-3-aminomethyl-8-aza-bicyclo [3.2.1] octane- 8-carboxylic acid *tert*-butyl ester (Intermediate BR) $[M+H]^+$ 451.

Example 252

(E)-N-(8-(1H-indole-4-carbonyl)-8-azaspiro[bicyclo[3.2.1]octane-3,4'-imidazolidine]-2'-ylidene)-3,5-diamino-6-chloropyrazine-2-carboxamide

Step 1

Iodotrimethylsilane (0.23 ml, 1.66 mmol) is added to a suspension of (E)-*tert*-Butyl 2'-(3,5-diamino-6-chloropyrazine-2-carbonylimino)-8-azaspiro [bicyclo [3.2.1] octane-3,4'-imidazolidine]-8-carboxylate (Ex. 251) (500 mg, 1.11 mmol) in DCM (10 ml). DMF (5 ml) is then added and the reaction is stirred at room temperature overnight. Iodotrimethylsilane (0.5 ml) is added and the reaction mixture is concentrated *in vacuo.* The yellow solid is suspended in DCM and collected by filtration. The solid is dissolved in 1:1 MeOH/DCM and loaded onto an SCX-2 cartridge eluted with DCM followed by MeOH and $NH_3$/MeOH. The methanolic ammonia fractions are concentrated *in vacuo* to afford (E)-3,5-diamino-6-chloro-N-(8-azaspiro [bicyclo[3.2.1]octane-3,4'-imidazolidine]-2'-ylidene)pyrazine-2-carboxamide as a yellow gum; $[M+H]^+$ 351.

Step 2

(E)-3,5-diamino-6-chloro-N-(8-azaspiro [bicyclo [3.2.1]octane-3,4'-imidazolidine]-2'-ylidene)pyrazine-2-carboxamide (170 mg, 0.49 mmol) is dissolved in DMF (10 ml) along with HATU (184 mg, 0.49 mmol) and 4-indole-carboxylic acid (78 mg, 0.49 mmol). N-Mcthyl morpholinc (160 ml, 1.45 mmol) is added and the solution stirred at room temperature overnight. The mixture is then concentrated *in vacuo.* EtOAc (100 ml) is added and the solution washed with water (100 ml). The organic phase is dried ($MgSO_4$) and concentrated *in vacuo.* Purification by flash chromatography ($SiO_2$, DCM/MeOH) gives the title compound as a yellow solid; $[M+H]^+$ 494.15, 496.27 for Cl isotopes.

Example 253

(E)-3,5-diamino-N-(8-(3-(4-(benzyloxy)phenyl)propanoyl)-8-azaspiro [bicyclo [3.2.1] octane-3,4'-imidazolidine]-2'-ylidene)-6-chloropyrazine-2-carboxamide

(E)-3,5-diamino-6-chloro-N-(8-azaspiro [bicyclo [3.2.1] octane-3,4'-imidazolidine]-2'-ylidene)pyrazine-2-carboxamide (prepared as described for Ex. 252) (280 mg, 0.798 mmol) is dissolved in DMF (8 ml) along with HATU (303 mg, 0.798 mmol) and 3-(4-benzyloxy-phenyl)-propionic acid (205 mg, 0.798 mmol). N-Methyl morpholine (0.263 ml, 2.394 mmol) is added and the solution stirred at room temperature for 6 hours. The mixture is then concentrated *in vacuo.* EtOAc (100 ml) is added and the solution washed with water (100 ml). The organic phase is dried ($MgSO_4$) and concentrated. The residue is dissolved in MeOH (20 ml) and dry loaded onto silica (5 g). Purification by flash chromatography ($SiO_2$, DCM/MeOH) gives the title compound as a tan solid; $[M+H]^+$ 589.20, 591.19 for Cl isotopes.

Preparation of Intermediate Compounds

Intermediate A

1-(3,5-Diamino-6-chloro-pyrazine-2-carbonyl)-2-methyl-isothiourea hydroiodide

Method 1

This compound is prepared according to Cragoe, Edward J., Jr.; Woltersdorf, Otto W., Jr.; De Solms, Susan Jane. Heterocyclic-substituted pyrazinoylguanidines, and a pharmaceutical composition containing them. EP 17152 Page 4

Method 2

Step 1

A stirred suspension of 3,5-diamino-6-chloro-pyrazine-2-carboxylic acid methyl ester (110 g, 542.9 mmol) in MeOH (500 ml) at 5-10°C (ice-bath) is treated dropwise with a suspension of lithium hydroxide (46.6 g, benzoyl}benzoyl} mmol) in water (500 ml). The reaction mixture is heated to 50 °C for 5 hours then cooled to room temperature and stirred overnight. The resulting precipitate is collected by filtration and dried in a vacuum oven to afford Lithium 3,5-diamino-6-chloro-pyrazine-2-carboxylic acid as the lithium salt (di-hydratc); $[M-Li]^-$ 187.

Step 2

A stirred suspension of S-methyl-iso-thiourea sulphate (10 g, 35.9 mmol) in toluene (75 ml) is treated with 4 M NaOH (15 ml) at room temperature. To the two-phase mixture is added di-tert butyl dicarbonate (3.27 g, 15 mmol) in one portion. The reaction mixture is stirred at room temperature for 1 hour, then heated to 60 °C overnight. The organic portion is separated, washed with brine solution, then dried over $Na2SO_4$, filtered and concentrated *in vacuo* to a viscous oil, which crystallized under high vacuum to afford tert-Butyl amino(methylthio) methylenecarbamate as a colorless solid.

Step 3

A stirring suspension of lithium 3,5-diamino-6-chloro-pyrazine-2-carboxylic acid (22.6 g, 98.03 mmol) in DMF (400 ml) is treated portionwise with HATU (41 g, 107.83 mmol), under an inert atmosphere of nitrogen. The reaction mixture is stirred at room temperature for 2 hours and then tert-butyl amino(methylthio)methylenecarbamate (20.5 g, 107.83 mmol) is added portion wise over a period of 10 minutes. The reaction mixture is stirred at room temperature for a further 1.5 hours then heated to 50 °C and stirred overnight. The resulting precipitate is hot filtered, washing with water and dried in a vacuum oven (40°C) overnight to afford tert-Butyl (3,5-diamino-6-chloropyrazine-2-carboxamido)(methylthio) methylene carbamate; $[M+H]^+$ 361.

Step 4

*tert*-Butyl (3,5-diamino-6-chloropyrazine-2-carboxamido)(methylthio) methylene carbamate (50 g, 139 mmol) is slurried in DCM (500 ml). TFA (53.4 ml, 693 mmol) is dissolved in DCM (100 ml) and added dropwise over 45 mins to form a brown solution. The solution is stirred at room temperature overnight, after which time a yellow precipitate has formed. The solid is collected by filtration, and dried *in vacuo* to yield the title compound; $[M+H]^+$ 261.1.

Intermediate B

((S)-5,6-Diamino-hexyl)-carbamic acid benzyl ester:

Step 1

A solution of BOC-lysinol-(Z)-OH (0.5 g, 1.36 mmol) in dry THF (1 ml) under an inert atmosphere of argon is treated with PS-triphenylphosphine (0.91 g, 3.00mmol/g loading). To this mixture is added phthalimide (0.2 g, 1.36 mmol) and DEAD (0.24 ml, 1.50 mmol) in dry THF (4 ml) and the reaction mixture is stirred at room temperature overnight. The resin is removed by filtration under vacuum and the filtrate is concentrated *in vacuo.* Purification of the crude white solid by chromatography on silica eluting with 20-50% EtOAc in *iso*-hexane (1% TEA) affords [(S)-5-Benzyloxycarbonylamino-1-(1,3-dioxo-1,3-dihydro-isoindol-2-ylmethyl)-pentybenzoyl}- carbamic acid tert-butyl ester as a white crystalline solid; $[M+H]^+$ 496.

Step 2

A solution of [(S)-5-benzyloxycarbonylamino-1-(1,3-dioxo-1,3-dihydro-isoindol-2-ylmethyl)-pentyl]-carbamic acid tert-butyl ester (0.63 g, 1.27 mmol) in DCM (5.1 ml) and EtOH (5.1 ml) is treated with hydrazine hydrate (0.318 g, 6.35 mmol) and the reaction mixture is stirred at room temperature overnight. A white precipitate forms which is removed by filtration and washed with DCM (3 x 10 ml). The filtrate is concentrated *in vacuo* and redissolved in DCM (15 ml) and MeOH (2 ml). Undissolved material is removed by filtration and the filtrate is concentrated *in vacuo.* The resulting oily yellow solid is purified by chromatography on silica eluting with 10-50% MeOH in DCM (1% TEA) to afford ((S)-1-Aminomethyl-5-benzyloxycarbonylamino-pentyl)-carbamic acid tert-butyl ester as a clear oil; $[M+H]^+$ 366.

Step 3

A solution of ((S)-1-aminomethyl-5-benzyloxycarbonylamino-pentyl)-carbamic acid tert-butyl ester (0.24 g, 0.657 mmol) in DCM (2.4 ml) is treated dropwise with TFA (0.6 ml) and stirred at room temperature for 3 days. The solvent is removed *in vacuo* to afford ((S)-5,6-Diamino-hexyl)-carbamic acid benzyl ester as a yellow oil; $[M+H]^+$ 266.

Intermediate C

A mixture of 4-[4-(2-amino-ethylamino)-butyl]-phenol and N*1*-[4-(4-methoxyphenyl)-butyl] -ethane-1,2-diamine

Step 1

A solution of 4-methoxyphenylbutryric acid (6.99 g, 36 mmol) in THF (70 ml) is treated with EDCI (7.6 g, 36.9 mmol) followed by N-ethylmorpholine (9.2 ml, 72 mmol). After stirring at room temperature for 1 hour, N-BOC-ethylene diamine (5.84 g, 36 mmol) is added and the resulting mixture is stirred at room temperature overnight. The reaction is quenched by addition of saturated sodium hydrogen carbonate solution and extracted with EtOAc. The organic portion is washed with citric acid solution, brine, dried ($MgSO_4$) and concentrated *in vacuo* until 25 ml of solvent remained. The suspension is filtered to afford {2-[4-(4-Methoxy-phenyl)-butyrylamino]-ethyl}-carbamic acid tert-butyl ester: as a white solid.

Step 2

A solution of {2-[4-(4-methoxy-phenyl)-butyrylamino]-ethyl}-carbamic acid tert-butyl ester (6 g, 17.88 mmol) in dry THF (60 ml) under an inert atmosphere of Argon is treated carefully with borane. THF complex (53.88 ml, 1M Borane in THF). The reaction mixture is heated at reflux for 2 hours and then allowed to cool to room temperature overnight. The mixture is quenched by addition of MeOH and then heated to 70°C for a further 2 hours. After cooling to room temperature, the solvent is removed *in vacuo* to afford {2-[4-(4-Methoxy-phenyl)-butylamino]-ethyl}-carbamic acid tert-butyl ester as a viscous oil; $[M+H]^+$ 323.

Step 3

A suspension of {2-[4-(4-methoxy-phenyl)-butylamino]-ethyl}-carbamic acid tert-butyl ester (5.85 g, 18.1 mmol) in HBr (30 ml of a 48% solution) is heated at reflux for 2 hours. After cooling to room temperature, the solvent is removed *in vacuo.* The crude residue is suspended in EtOAc and filtered to afford a solid which consisted of a mixture of 4-[4-(2-amino-ethylamino)-butyl]-phenol and N*1*-14-(4-methoxy-phenyl)-butyl]-ethane-1,2-diamine in approximately 1:1 ratio; $[M+H]^+$ 209 and 223.

Intermediate D

(S)-3-(4-methoxy-phenyl)-propane-1,2 diamine

(S)-2-Amino-3-(4-methoxy-phenyl)-propionamide is prepared according to the procedure described on page 3880, Method 2.1.3 of Journal of Physical Chemistry B, 108(12), 3879-3889; 2004 and is reduced analogously to Intermediate C.

Intermediate E

1-(3,4-Dichloro-phenyl)-ethane-1,2-diamine

This compound is prepared according to the procedure described on page 907, Method 5 in the Journal of Medicinal Chemistry (1973), 16(8), 901-8.

Intermediate F

4,5-Diaminopentanoic acid dihydrochloride

This compound is prepared according to the procedure described in 'Radiolabeling chelating compounds comprising sulfur atoms, with metal radionuclides.' EP 300431 page 12, Intermediate 3.

Intermediate G

4-Amino-1-benzyl-piperidine-4-carbonitrile

Step 1

To a solution of ammonium chloride (1.73 g, 32.3 mmol) in water (20 ml) is added a 30% ammonia solution (2 ml) followed by 1-benzyl-4-piperidone. After 20 minutes sodium cyanide (1.47 g, 30 mmol) is added portionwise over 15 minutes. After stirring for one hour, water (50 ml) is added and the products are extracted with DCM (3 x 50 ml), dried ($MgSO_4$) filtered and concentrated *in vacuo.* Purification by chromatography on silica eluting with 50-100% EtOAc in *iso*-hexane affords 4-Aminomethyl-1-benzyl-piperidine-4-ylamine; $[M+H]^+$ 216

Step 2

To a solution of lithium aluminum hydride (1 M in THF, 10.4 ml) in dry diethyl ether (15 ml), cooled to 0 °C, under an argon atmosphere is added dropwise 4-amino methyl-1-benzylpiperidine-4-ylamine (900 mg, 4.18 mmol) in dry diethyl ether (15 ml). The reaction mixture is heated at reflux for 24 h and then cooled to 0°C. Water (0.25 ml) is added followed by a 15% aqueous NaOH (0. 25 ml) and then water (0.7 ml). After warming to room temperature $MgSO_4$ (150 mg) is added and stirred for 15 minutes. The solids are removed by suction filtration and the filtrate evaporated to give an oil. The solids are extracted with refluxing diethyl ether (80 ml) using a Soxhlet extractor for 14 hours. The diethyl ether is removed *in vacuo* and the two oils combined and purified by chromatography on silica eluting with 10-25% 2M ammonia in methanol solution in dichloromethane to give 4-Amino-1-benzyl-piperidine-4-carbonitrile ; $[M+H]^+$ 220

Intermediate H

5-14-((R)-2,2-Dimethyl-[1,3]dioxolane-4-ylmethoxy)-phenybenzoyl}-pentane-1,2-diamine

Step 1

To 3-(4-hydroxyphenyl)-1-propanol (10 g, 66 mmol) and potassium carbonate (13.5 g, 100 mmol) in acetone (200 ml) is added (S)-glycidol (6.5 ml, 100 mmol). The mixture is heated at reflux for 18 hours. After cooling to room temperature the solvent is removed *in vacuo* and the residue partitioned between EtOAc and water. The aqueous layer is further extracted twice with EtOAc and the combined organic portions are washed with water, brine, dried ($MgSO_4$), filtered and concentrated *in vacuo.* The crude residue is purified by flash column chromatography on silica eluting with 1:1 EtOAc/*iso*-hexane to afford (S)-3-[4-(3-Hydroxy-propyl)-phenoxy]-propane-1,2-diol as a white solid; $^1H$ NMR ($CDCl_3$): 1.20 (1H, br), 1.85 (2H, pent, J = 6.8 Hz), 1.98 (1H, br), 2.58 (1H, br), 2.65 (2H, tr, J = 6.9 Hz), 3.56 (2H, tr, J = 6.8 Hz), 3.72 (1H, m), 3.83 (1H, m), 4.00 (2H, dd, J = 2.1 Hz, J = 6.5 Hz), 4.09 (1H, br), 6.82 (2H, d, J = 7.4 Hz), 7.10 (2H, d, J = 7.4 Hz).

Step 2

To (S)-3-[4-(3-hydroxy-propyl)-phenoxy]-propane-1,2-diol (benzoyl}.5 g, 50.9 mmol) in dry DMF (150 ml) is added pyridinium p-toluenesulfonate (1.28 g, 5 mmol) and 2,2-dimethoxypropane (31 ml, 250 mmol). The mixture is stirred at room temperature for 18 hours and then the solvent is removed *in vacuo.* The residue is dissolved in EtOAc (150 ml) and washed with water, saturated aqueous sodium hydrogen carbonate solution, brine, dried ($MgSO_4$) and *concentrated in vacuo.* The residue is purified by chromatography on silica eluting with 1:4 EtOAc/ *iso*-hexane to 1:1 EtOAc/ *iso*-hexane to afford (3-[4-((R)-2,2-Dimethyl-[1,3]dioxolan-4-ylmethoxy)-phenyl]-propan-1-ol as a colorless oil; $^1H$ NMR ($CDCl_3$): 1.25 (1H, br), 1.39 (3H, s), 1.43 (3H,s), 1.85 (2H, pent, J = 6.9 Hz), 2.63 (2H, tr, J = 6.9 Hz), 3.63 (2H, tr, J = 6.9 Hz), 3.90 (2H, m), 4.02 (1H, m), 4.12 (1H, m), 4.50 (1H, pent, J = 6.8 Hz), 6.82 (2H, d, J = 7.4 Hz), 7.10 (2H, d, J = 7.4 Hz).

Step 3

To (3-[4-((R)-2,2-dimethyl-[1,3]dioxolan-4-ylmethoxy)-phenyl]-propan-1-ol (12.2 g, 46 mmol) in dry ether (150 ml) is added TEA (12.8 ml, 92 mmol). The mixture is cooled to 0 °C and treated dropwise with methanesulfonyl chloride (5.3 ml, 69 mmol). The reaction mixture is allowed to warm to room temperature and then stirring continued for 3 hours. The resulting mixture is washed with water (2 x 100 ml), saturated aqueous sodium hydrogencarbonate, brine, dried ($MgSO_4$) and concentrated *in vacuo* to give Methanesulfonic acid 3-[4-((R)-2,2-dimethyl [1,3] dioxolan-4- ylmethoxy)-phenyl]-propylester as a white solid; $^1H$ NMR($CDCl_3$): 1.39 (3H, s), 1.43 (3H,s), 2.02 (2H, pent, J = 6.9 Hz), 2.63 (2H, tr, J

= 6.9 Hz), 3.00 (3H,s), 3.90 (2H, m), 4.05 (1H, m), 4.14 (3h, m), 4.46 (1H, pent, J = 6.8 Hz), 6.82 (2H, d, J = 7.4 Hz), 7.10 (2H, d, J = 7.4 Hz).

Step 4

Methanesulfonic acid 3-[4-((R)-2,2-dimethyl [1,3] dioxolan-4-ylmethoxy)-phenyl]-propylester (11.8 g, 34.2 mmol) in acetone (200 ml) is treated with lithium bromide (8.9 g, 100 mmol) and then heated at reflux for 5 h. After cooling to room temperature, the mixture is concentrated *in vacuo.* The resulting residue is dissolved in EtOAc (150 ml), washed with water (2 x 50 ml), brine, dried ($MgSO_4$), filtered and concentrated *in vacuo* to give an oil. Purification by chromatography on silica eluting with 4:1 *iso*-hexane / EtOAc gives (R)-4-[4-(3-Bromo-propyl)-phenoxymethyl]-2,2-dimethl-[1,3] dioxolanc as a colorless oil which solidifies; $^1$H NMR ($CDCl_3$): 1.39 (3H, s), 1.43 (3H,s), 2.02 (2H, pent, J = 6.9 Hz), 2.63 (2H, tr, J = 6.9 Hz), 3.38 (2H, tr, J = 6.9 Hz), 3.90 (2H, m), 4.02 (1H, m), 4.15 (1H, m), 4.46 (1H, pent, J = 6.9 Hz), 6.82 (2H, d, J = 7.4 Hz), 7.10 (2H, d, J = 7.4 Hz).

Step 5

A solution of N-(diphenylmethylene) aminoacetonitrile (5.14 g, 23.4 mmol) in DCM (12 ml) is treated with (R)-4-[4-(3-bromo-propyl)-phenoxymethyl]-2,2-dimethl-[1,3] dioxolane (8.1 g, 24 mmol) in DCM (12 ml) and cooled to 0 °C. 48% aqueous NaOH (20 ml) is added followed by benzyltriethylammonium chloride (530 mg, 2.4 mmol) and the resulting mixture is allowed to warm to room temperature. After stirring vigorously for 4 hours mixture is diluted with DCM (100 ml) and the aqueous portion is removed. The organic layer is washed with water (2 x 50 ml), brine, dried ($MgSO_4$), filtered and concentrated *in vacuo* . The crude product is purified by chromatography on silica eluting with 15:1 iso-hexane/diethyl ether to 4:1 *iso*-hexane/diethyl ether to yield 2-(Benzhydrylidene-amino)-544-((R)-2,2-dimethyl-[1,3]dioxolan-4-ylmethoxy)-phenyl]pentanenitrile as a yellow oil; $^1$H NMR ($CDCl_3$): mix of diastereoisomers 1.39 (3H, s), 1.43 (3H,s), 1.71 (2H, m), 1.80-1.98 (2H, m), 2.52 (2H, tr, J = 7.0 Hz) 3.90,(2H, m), 4.02 (1H, m), 4.10-4.22 (2H, m), 4.47 (1H, pent, J = 6.9 Hz), 6.82 (2H, d, J = 7.4 Hz), 7.05 (2H, d, J = 7.4 Hz), 7.19 (2H, m), 7.35 (2H, tr, J = 7.2 Hz), 7.40-7.50 (4H, m), 7.60 (2H, d, J = 7.1 Hz).

Step 6

To a solution of 2-(benzhydrylidene-amino)-5-[4-((R)-2,2-dimethyl-[1,3]dioxolan-4-ylmethoxy)-phenyl]pentanenitrile (7.2 g, 15.5 mmol) in THF (50 ml) is added a 2M HCl (aq) (5 ml). The solution is heated at 40 °C for 4 hours and then allowed to cool to room temperature. The pH is adjusted to pH 9-10 using saturated aqueous sodium hydrogen carbonate solution and the organic solvent is removed *in vacuo.* The crude residue is dissolved in EtOAc (100 ml) and washed with water, brine, dried ($MgSO_4$), filtered and concentrated *in vacuo.* The resulting residue is purified by chromatography on silica eluting with 5:1 to 1:1 *iso*-hexane/ethyl aEtOAc and 1% triethylamine to yield 2-Amino-5-[4-((R)-2,2-dimethyl-[1,3]dioxolan-4-ylmethoxy)-phenyl]-pentanenitrile as a colorless oil which solidifies; $^1$H NMR ($CDCl_3$): mixture of diastereoisomers 1.39 (3H, s), 1.43 (3H,s), 1.70-1.87 (4H, m), 2.60 (2H, tr, J = 7.1 Hz), 3.62 (1H, br), 3.90 (2H, m), 4.00-4.18 (2H, m), 4.48 (1H, pent, J = 6.9 Hz), 6.82 (2H, d, J = 7.4 Hz), 7.10 (2H, d, J = 7.4 Hz). $[M+H]^+$ 305.

Step 7

A solution of 2-amino-5-[4-((R)-2,2-dimethyl-[1,3]dioxolan-4-ylmethoxy)-phenyl]-pentanenitrile (1.7 g, 4.28 mmol) in a 2M ammonia in methanol solution (50 ml) is passed through a H-CUBE apparatus fitted with a Rancy nickel CatCart at 50 °C and a hydrogen pressure of 50 bar and a flow rate of 1.5 ml/min. After 5 hours of continuous cycling of the solution the reaction mixture is concentrated *in vacuo* to give 5-[4-((R)-2,2-Dimethyl-[1,3]dioxolane-4-ylmethoxy)-phenyl]-pentane-1,2-diamine as a light-yellow oil; $[M+H]^+$ 309.

Intermediate I

5-(4-Methoxy-phenyl)-pentane-1,2-diamine

This compound is prepared analogously to Intermediate H by replacing (3-[4-((R)-2,2-dimethyl-1,3]dioxolan-4-ylmethoxy)-phenyl]-propan-1-ol with 4-(4-methoxyphenyl) -1-butanol.

Intermediate J

1-Aminomethyl-cyclopentylamine

Step 1

To a cooled 0 °C solution of (1-cyano-cyclopentyl)-carbamic acid tert-butyl ester (430 mg, 2.04 mmol) in dry THF (4.3 ml) under an atmosphere of argon is added dropwise 1.0 M $LiAlH_4$ (6.13 ml, 6.13 mmol). The reaction mixture is allowed to warm to room temperature and stirred for 3.5 hours. The mixture is then re-cooled to 0 °C and cautiously quenched with water (0.4 ml) : 15% NaOH (0.8 ml): water (1.2 ml) (1:2:3 eq). The resultant mixture is filtered through Celite® (filter material) to remove the inorganic solids and rinsed with MeOH. The filtrate is concentrated *in vacuo,* to yield a white solid, which is purified by chromatography on silica eluting with 30% MeOH in DCM to afford (1-Aminomethyl-cyclopentyl)-carbamic acid tert-butyl ester; $[M+H]^+$ 215.

Step 2

Iodotrimethylsilane (0.091 ml, 0.67 mmol) is added dropwise to a solution of (1-aminomethyl-cyclopentyl)-carbamic acid tert-butyl ester (120 mg, 0.56 mmol) in DCM (2.4 ml) and left to stir overnight. The resulting suspension is quenched with MeOH (2.4 ml) and concentrated *in vacuo* to yield 1-Aminomethyl-cyclopentylamine as a dark oil, which is used without further purification.

Intermediate K

(4-((R)-4,5-Diamino-pentyl)-phenol

Steps 1 and 2

(R)-2-tert-Butoxycarbonylamino-5-(4-tert-butoxy-phenyl)-pentanoic acid ethyl ester is prepared according to the procedure of Ding, Chuanyong.; Ma, Rujian.; Rong, Guobin. Preparation of w-Phenyl-(2S)-N-Boc-amino Acid Ethyl esters; Chinese Journal of Organic Chemistry Vol 26(12) 2006, 1694 &1695, replacing Ethyl Boc-L-pyroglutamate with Ethyl Boc-D-pyroglutamate & Bromomethyl-benzene with 1-Bromo-4-tert-butoxy-benzene in Example 2a, using preparation steps 2.2, 2.3, and 2.5; $[M+H]^+$ 394.

Step 3

(R)-2-tert-Butoxycarbonylamino-5-(4-tert-butoxy-phenyl)-pentanoic acid ethyl ester (179 g, 460 mmol) is dissolved in 7M $NH_3$ in MeOH (400 ml, 2800 mmol) and stirred at room temperature for 4 days. The reaction is concentrated *in vacuo* keeping the temperature below 30 °C to afford [(R)-4-(4-tert-Butoxy-phenyl)-1-carbamoyl-butyl]- carbamic acid tert-butyl ester $[M+H]^+$ 364.

Step 4

A solution of [(R)-4-(4-tert-Butoxy-phenyl)-1-carbamoyl-butyl]-carbamic acid tertbutyl ester (167 g, 458 mmol) in 1 M HCl in $Et_2O$ (4000 ml) is stirred at room temperature for 3 days. After this time, a white solid forms which is collected by filtration and washed with $Et_2O$ to yield (R)-2-Amino-5-(4-hydroxy-phenyl)-pentanoic acid amide; $[M+H]^+$ 209.

Step 5

To a stirred solution of (R)-2-Amino-5-(4-hydroxy-phenyl)-pentanoic acid amide (5 g, 24.01 mmol) in THF (250 ml) is added imidazole (4.90 g, 72 mmol), followed by *tert*-butyldimethylchlorosilane (3.98 g, 26.4 mmol). The resulting solution is heated at 70 °C for 4 hours and then allowed to cool to room temperature. Dilution with $Et_2O$ (200 ml) washing with water (2 x100 ml) and brine (100 ml), drying $MgSO_4$, and concentration *in vacuo* yields (R)-2-Amino-5-[4-(tert-butyldimethyl-silanyloxy)- phenyl]-pentanoic acid amide; $[M+H]^+$ 323.

Step 6

A solution of (R)-2-Amino-5[4-(tert-butyl-dimethyl-silanyloxy)-phenyl]-pentanoic acid amide (7.74 g, 24 mmol) in THF is stirred at 5 °C and borane (96 ml of a 1 M solution in THF, 96 mmol) is added. The mixture is stirred at 5 °C

until a homogeneous mixture is obtained and then stirred at room temperature for 30 minutes and 35 °C for 3 hours. After this time, further borane (24 ml of a 1 M solution in THF, 24 mmol) is added and the reaction is heated at 35 °C for 18 hours. After this time, a further portion of borane (24 ml of a 1 M solution in THF, 24 mmol) is added and the reaction heated at 35 °C for a further 24 hours. After this time, the reaction is cooled to 10 °C, and quenched by adding dropwise to MeOH (50 ml) at -5 °C. After allowing to warm to room temperature the solvent is removed *in vacuo* to afford a yellow oil. The oil is dissolved in MeOH (250 ml) and SCX-2 silica (180 g, 0.63mmol/g, 120 mmol) is added. The silica suspension is shaken for 18 hours, the silica is removed by filtration, washed with MeOH (3 x 100 ml), then suspended in 7M $NH_3$ in MeOH and shaken for 18 hours. The silica is removed by filtration and the 7M $NH_3$ in MeOH is removed *in vacuo* to afford the title compound as a yellow oil; $[M+H]^+$ 195.

Intermediate L

4-((S)-4,5-Diamino-pentyl)-phenol

This compound is prepared analogously to Intermediate K (NVP-QBM333), replacing Ethyl Boc-D-pyroglutamate in step 1 with Ethyl Boc-L-pyroglutamate; $[M+H]^+$ 195.

Intermediate M

(R)-tert-butyl5-(4-hydroxyphenyl)pentane-1,2-diyldicarbamate

To a solution of (4-((R)-4,5-Diamino-pentyl)-phenol (Intermediate K) (775 mg, 1.99 mmol) in DCM (10 ml) is added triethylamine (1.14 ml, 8.08 mmol) and a solution of di-tert-butyl dicarbonate (1.33 g, 6.08 mmol) in DCM (10 ml) and the resulting solution is stirred at room temperature for 18 hours. The solvent is *removed in vacuo* and the residue purified by chromatography ($SiO_2$, EtOAc/iso-hexane) to afford the title compound; $[M+H]^+$ 395.

Intermediate N

(S)-tert-butyl 5-(4-hydroxyphenyl)pentane-1,2-diyldicarbamate

This compound is prepared analogously to Intermediate M, (R)-tert-butyl 5-(4-hydroxyphenyl)pentane-1,2-diyldicarbamate replacing Intermediate K, (4-((R)-4,5- Diamino-pentyl)-phenol with Intermediate L, 4-((S)-4,5-Diamino-pentyl)-phenol; $[M+H]^+$ 395.

Intermediate O

(R)-3-[4-((R)-4,5-Diamino-pentyl)-phenoxy]-propane-1,2-diol

Step 1

Triethylamine (8.37 µl, 0.06 mmol) and (R)-(+)-glycidol (96 µl, 1.442 mmol) are added to a solution of (R)-tert-butyl 5-(4-hydroxyphenyl)pentane-1,2-diyldicarbamate (Intermediate M) (474 mg, 1.20 mmol) in EtOH (5 ml) and the resulting solution is heated at 90 °C for 18 hours. The reaction is allowed to cool to room temperature and concentrated *in vacuo.* Purification by chromatography ($SiO_2$, EtOAc/iso-hexane) affords {(R)-2-tert-Butoxycarbonylamino-5-[4-((R)-2,3-dihydroxy-propoxy)-phenyl]-pentyl}-carbamic acid tert-butyl ester; $[M+H]^+$ 469.

Step 2

{(R)-2-tert-Butoxycarbonylamino-5-[4-((R)-2,3-dihydroxy-propoxy)-phenyl]-pentyl}-carbamic acid tert-butyl ester (94 mg, 0.201 mmol) is stirred with a solution of 1 M HCl in $Et_2O$ (3 ml) for 18 hours and then loaded onto a 1 g SCX-2 cartridge washed with MeOH (30 ml), followed by 7M $NH_3$ in MeOH (30 ml). The $NH_3$ fraction is concentrated *in vacuo* to give the title compound, (R)-3-[4-((R)-4,5-Diamino-pentyl)-phenoxy]-propane-1,2-diol Intermediate H (R)-3-[4-((R)-4,5-Diamino-pentyl)-phenoxy]-propane-1,2-diol; $[M+H]^+$ 269.

Intermediate P

(R)-3-[4-((S)-4,5-Diamino-pentyl)-phenoxy]-propane-1,2-diol

This compound is prepared analogously to Intermediate O replacing (R)-tert-butyl 5-(4-hydroxyphenyl)pentane-1,2-diyldicarbamate (Intermediate M with (S)-tert-butyl 5-(4-hydroxyphenyl)pentane-1,2-diyldicarbamate (Intermediate N); $[M+H]^+$ 269.

Intermediate Q

2-[4-((R)-4,5-Diamino-pentyl)-phenoxy]-1-morpholin-4-yl-ethanone

(R)-tert-butyl5-(4-hydroxyphenyl)pentane-1,2-diyldicarbamate (Intermediate M) (446 mg, 0.565 mmol) is dissolved in DMF (10 ml) and $Cs_2CO_3$ (368 mg, 1.131 mmol) and 2-bromo-1-morpholinethanone (118 mg, 0.565 mmol) are added. The reaction is stirred at room temperature for 40 minutes, then diluted with water (20 ml) and extracted with EtOAc (2 x 50 ml). The organic layers are dried over $MgSO_4$ and the solvent concentrated *in vacuo* to give a clear oil. Purification by chromatography on a Waters 3000 prep HPLC system (Microsorb™ C18 Water/ MeCN +0.1% TFA) yields a clear oil, which is dissolved in dioxane (4 ml) and treated with 4 M HCl in dioxane (4 ml) and stirred at room temperature for 4 days. Concentration *in vacuo* affords a white foam which is dissolved in MeOH (3 ml) and loaded onto a 10 g SCX-2 cartridge which is washed with MeOH (60 ml) and 7M $NH_3$ in MeOH (60 ml). The $NH_3$ fractions are combined and concentrated *in vacuo* to give the title compound as a colorless oil; $[M+H]^+$ 322.

Intermediate R

5-(4-Methoxy-phenyl)-hexane-1,2-diamine

This compound is prepared analogously to Intermediate I by replacing 4-(4-methoxyphenyl)-1-butanol with 4-(4-methoxyphenyl)-1-pentanol.

Intermediate S

((S)-4,5-Diamino-pentyl)-carbamic acid benzyl ester

Step 1

Concentrated HCl (15 ml) is added to a suspension of Na-BOC-N8-Z-L-omithine (5.00 g, 13.65 mmol) in 2,2-dimethoxypropane (150 ml). An endotherm occurs and the resulting solution is left to stir at room temperature for 6 hours. The solvent is then reduced *in vacuo* to approximately 50 ml and diethyl ether (100 ml) is added to turn the solution turbid. On stirring a thick white suspension forms. The white solid is collected by filtration and rinsed with diethyl ether (100 ml). The white solid is dissolved in MeOH (30 ml) and diethyl ether (200 ml) is added to precipitate a white solid that is collected by filtration and rinsed with diethyl ether. The solid is dissolved in DCM and washed with 2 N NaOH (75 ml). The organic phase is dried over $MgSO_4$ and the solvent evaporated *in vacuo* to yield (S)-2-Amino-5-benzyloxycarbonylaminopentanoic acid methyl ester as a colorless oil; $[M+H]^+$ 280.78.

Step 2

(S)-2-Amino-5-benzyloxycarbonylamino-pentanoic acid methyl ester (2.80 g, 9.99 mmol) and 7M $NH_3$ in MeOH (20 ml) is stirred at room temperature for 72 hours. The reaction mixture is evaporated to dryness *in vacuo* to yield a white solid. The white solid is suspended in diethyl ether before filtration and drying to yield ((S)-4-Amino-4-carbamoyl-butyl)-carbamic acid benzyl ester.

Step 3

((S)-4-Amino-4-carbamoyl-butyl)-carbamic acid benzyl ester (1.87 g, 7.071 mmol) is suspended in dry THF (40 ml) and cooled to 10 °C in an ice bath under nitrogen. Borane (28.3 ml of a 1 M solution in THF, 28.3 mmol) is added. The ice bath is removed and the suspension heated to 70 °C and then left to stir at this temperature for 3 hours. Further borane (28.3 ml of a 1 M solution in THF, 28.3 mmol) is added and then after an hour the same amount of 1M borane in THF is added again. After a final hour at 70 °C the reaction mixture is quenched with MeOH (40 ml). The solvent is

reduced *in vacuo* to approximately 50 ml. This is diluted with 5 M HCl (100 ml) and washed with diethyl ether (3 x 100 ml). The aqueous phase is basified to pH12 with 2N NaOH and product extracted into EtOAc (3 x 100 ml). The organic phases are combined, dried over $MgSO_4$ and the solvent evaporated in vacuo to yield the title compound as a colorless oil.

Intermediate T

3,5-Diamino-6-chloro-pyrazine-2-carboxylic acid [(S)-4-(4-amino-butyl)-imidazolidin-(2E)-ylidene]-amide

To a suspension of (4-{(S)-2-[(E)-3,5-Diamino-6-chloro-pyrazine-2-carbonylimino]

-imidazolidin-4-ybenzoyl}-butyl)-carbamic acid benzyl ester (Ex. 5) (0.110 g, 0.239 mmol) in dry DCM (20 ml) is added iodotrimethylsilane (0.130 ml, 0.956 mmol). The reaction mixture is stirred at room temperature for 3.5 hours. McOH is added to the suspension yielding a solution. Purification by catch and release resin (SCX-2) eluting with MeOH and 7 M $NH_3$ in MeOH yields the title compound as a brown oil; $[M+H]^+$ 327.1

Intermediate U

4-Amino-4-aminomethyl-piperidine-1-carboxylic acid tert-butyl ester

Step 1

To a solution of 4-amino-4-cyano-piperidine-1-carboxylic acid *tert*-butyl ester (11.5 g, 51.0 mmol) in pyridine (20 ml) at 0 °C is added trifluoroacetic anhydride (11.0 ml) slowly and the reaction mixture is stirred at 0 °C for 4 h. The reaction mixture is diluted with DCM, washed with brine, dried over $Na2SO_4$ and concentrated *in vacuo.* The residue obtained is dissolved in DCM and re-precipitated by adding petroleum ether. The supernatant solvent mixture is decanted and the product is washed again with petroleum ether and dried under vacuum to afford 4-Cyano-4-(2,2,2-trifluoro-acetylamino)-piperidine-1-carboxylic acid *tert*-butyl ester as an oil; $^1H$ NMR ($d_6$-DMSO): 1.40 (9H, s), 1.81-1.88 (2H, m), 2.26-2.32 (2H, m), 2.99-3.15 (2H, m), 3.793.82 (2H, m), 10.1 (1H, s).

Step 2

To a solution of cyano-4-(2,2,2-trifluoro-acetylamino)-piperidine-1-carboxylic acid tert-butyl ester (10.0 g, 31.0 mmol) in EtOH (150 ml) is added Raney nickel (~1.5 g) and the reaction mixture is stirred under an atmosphere of hydrogen for 3 days. A further quantity of Raney nickel (~1.5 g) is added and the reaction mixture is further stirred for 2 days. The reaction mixture is filtered through a plug of Celite™ (filter material) and the filtrate is concentrated *in vacuo* to obtain 4-Aminomethyl-4-(2,2,2-trifluoro-acetylamino)-piperidine-1-carboxylic acid tert-butyl ester as a viscous oil that is used crude without further purification.

Step 3

4-Amino-4-aminomethyl-piperidine-1-carboxylic acid *tert-butyl* ester

To a solution of 4-aminomethyl-4-(2,2,2-trifluoro-acetylamino)-piperidine-1-carboxylic acid tert-butyl ester in MeOH (70 ml) is added a 30% aqueous solution of ammonia (70 ml) and the reaction mixture is stirred at 80 °C overnight. The reaction mixture is concentrated *in vacuo* to 4-Amino-4-aminomethyl-piperidine-1-carboxylic acid *tert*-butyl ester as a brown oil that is used crude without further purification; $[M+H]^+$ 230.

Intermediate V

3-(3-Isopropoxy-propylsulfamoyl)-benzoic acid

3-Isopropoxypropylamine (1.1 eq.) is dissolved in THF with stirring at room temperature. N,N-diisopropylethylamine (1 eq.) is added followed by methyl 3-(chlorosulfonyl)benzoic acid (1 eq.). The reaction mixture is stirred at room temperature for 2 hours before the solvent is evaporated *in vacuo* to yield the crude titled product.

Intermediate W

3-(3-Isopropyl-ureido)-benzoic acid

A suspension of 3-Aminobenzoic acid (20 g, 145.8 mmol) in THF (300 ml) is heated to 60 °C to form a clear solution. I-propylisocyanate (14.9 g , 175 mmol) is added over 30 minutes. During the addition the product starts to precipitate. After complete addition toluene (300 ml) is added. The reaction mixture is stirred at 60 °C for 4.5 hours. The heating bath is removed and the mixture is stirred overnight at room temperature. Finally the suspension is filtered and washed with a mixture of 1:1 THF : toluene (200 ml). The product is dried at 60 °C for 18 hours to yield 3-(3-Isopropyl-ureido)-benzoic acid.

Intermediate X

5-Oxo-1-(3-pyrrol-1-yl-propyl)-pyrrolidine-3-carboxylic acid

Step 1

To a solution of 5-Oxo-pyrrolidine-3-carboxylic acid methyl ester (1 eq.) in dry DMF is added NaH (1.1 eq.) followed by 1-(3-bromo-propyl)-1H-pyrrole (1 eq.). The reaction mixture is stirred at room temperature overnight. Purification is by normal phase chromatography to yield 5-Oxo-1-(3-pyrrol-1-yl-propyl)-pyrrolidine-3-carboxylic acid methyl ester.

Step 2

To a cooled solution (0 °C) of 5-Oxo-1-(3-pyrrol-1-yl-propyl)-pyrrolidine-3-carboxylic acid methyl ester in THF, 0.2M LiOH is added and RM is stirred for 3 hours gradually warming to room temperature. Reaction mixture is acidified with 1N HCl and product extracted into ethyl acetate. The organic phase is washed with brine, dried over magnesium sulphate and the solvent evaporated *in vacuo* to yield 5-Oxo-1-(3-pyrrol-1-yl-propyl)-pyrrolidine-3-carboxylic acid.

Intermediate Y

2-(3-Isopropyl-ureido)-isonicotinic acid

Step 1

To a solution of ethyl 2-aminoisonicotinate (500 mg, 3.01 mmol) in DMF (10 ml) is added triethylamine (1.26 ml, 9.03 mmol) and then isopropyl isocyanate (512 mg, 6.02 mmol). The reaction mixture is heated in a microwave at 140 °C for 2 hours. The reaction mixture is diluted with EtOAc, washed with water (x5), brine, dried ($MgSO_4$) and concentrated *in vacuo*. Chromatography ($SiO_2$, MeOH/DCM) affords 2-(3-Isopropyl-ureido)-isonicotinic acid ethyl ester; $[M+H]^+$ 252.

Step 2

To a solution of 2-(3-Isopropyl-ureido)-isonicotinic acid ethyl ester (130 mg, 0.52 mmol) in MeOH (5 ml) is added 2 M NaOH (2.5 ml) and the resulting solution is stirred for 1.5 hours at room temperature. The solvent is removed *in vacuo* and sat. aq. $NH_4Cl$ solution is added. The pH of the aqueous phase is adjusted to 1 using 1 M HCl and the product extracted into EtOAc, dried ($MgSO_4$) the solvent removed *in vacuo* to afford 2-(3-Isopropyl-ureido)-isonicotinic acid as a white solid; $[M+H]^+$ 224.

Intermediate Z

1-Isopropylcarbamoyl-1H-indole-4-carboxylic acid

This compound is prepared analogously to Intermediate Y by replacing ethyl 2-aminoisonicotinate in step 1 with methyl indol-4-carboxylate; $[M+H]^+$ 247.

Intermediate AA

- 4-(3-Isopropyl-ureido)-benzoic acid

This compound is prepared analogously to Intermediate Y by replacing ethyl 2-aminoisonicotinate in step 1 with methyl 4-aminobenzoate; $[M+H]^+$ 237.

Intermediate AB

- 6-(3-Isopropyl-ureido)-nicotinic acid

This compound is prepared analogously to Intermediate Y by replacing ethyl 2-aminoisonicotinate in step 1 with methyl 6-aminonicotinate; $[M+H]^+$ 224.

Intermediate AC

- [4-(2-Methoxy-ethoxymethoxy)-phenyl]-acetic acid

Step 1

To a solution of methyl 4-hydroxyphenylacetate (200mg, 1.20 mmol) in DCM (5 ml) is added DIPEA (0.315 ml, 1.81 mmol), and then MEMC1 (0.204 ml, 1.81 mmol), and the resulting reaction mixture is stirred for 2 hours at room temperature. An additional portion of MEMC1 (0.102 ml, 1 mmol) and of DIPEA (0.158 ml, 1 mmol) are added, and the reaction mixture is stirred for a further 16 hours. An additional portion of MEMC1 (0.102 ml, 1 mmol) and of DIPEA (0.158 ml, 1 mmol) are added and the reaction mixture is stirred for 3 hours. The reaction mixture is diluted with DCM and washed with 0.5 M HCl, 1 M NaOH and then 0.5 M HCl, dried ($MgSO_4$) and concentrated *in vacuo* to afford [4-(2-Methoxy-ethoxymethoxy)-phenyl]-acetic acid methyl ester.

Step 2

To a solution of [4-(2-Methoxy-ethoxymethoxy)-phenyl]acetic acid methyl ester (192 mg, 0.76 mmol) in MeOH (3 ml) is added 2 M NaOH (3 ml). The reaction mixture is stirred for 16 hours at room temperature. The solvent is removed *in vacuo* and the residue dissolved in EtOAc and washed with. $NH_4Cl$ solution, dried ($MgSO_4$) and *concentrated in vacuo* to yield [4-(2-Methoxy-ethoxymethoxy)-phenyl]-acetic acid.

Intermediate AD

3-[4-(2-Methoxy-ethoxymethoxy)-phenyl]-propionic acid

This compound is prepared analogously to Intermediate AC by replacing methyl 4-hydroxyphenylacetate in step 1 with methyl-3-(4-hydroxyphenyl)propionate.

Intermediate AE

3-{4[2-(Tetrahydro-pyran-2-yloxy)-ethoxy]-phenyl}-propionic acid

Step 1

Methyl 3-(4-hydroxyphenyl)propianoate (0.1 g, 0.55 mmol) is dissolved in DMF (5 ml) and NaH (0.033 g of a 60% dispersion in mineral oil, 0.83 mmol) is added. The reaction mixture is stirred at room temperature for 15 minutes then 2-(2- bromethoxy)tehtrahydro-2-H-pyran (0.109 ml, 0.72 mmol) is added and the reaction mixture is left to stir for 18 hours. Dilution with EtOAc (50 ml), washing with water (25 ml), saturated $NaHCO_3$ (25 ml) and brine (25 ml), drying over $MgSO_4$, and concentration *in vacuo* yields 3-{442-(Tetrahydro-pyran-2-yloxy)-ethoxy]-phenyllpropionic acid methyl ester as a colorless oil; $[M+H]^+$ 309.

Step 2

3-{4-[2-(Tetrahydro-pyran-2-yloxy)-ethoxy]-phenyl}-propionic acid methyl ester (0.12 g, 0.39 mmol) is dissolved

in MeOH (3 ml) and 2M NaOH solution (3 ml) is added and the resulting solution is stirred at room temperature for 18 hours. The reaction mixture is diluted with saturated ammonium chloride solution (20 ml) and extracted with EtOAc (100 ml x 2). The organic phased are combined, dried over $MgSO_4$, the solvent removed *in vacuo* to yield the title compound as a colorless oil; $[M+H]^+$ = 295.

Intermediate AF

3-[4-(Pyridin-4-ylmethoxy)-phenyl]-propionic acid

Step 1

To a solution of Methyl 3-(4-hydroxyphenyl)propanoate (0.5 g, 2.77 mmol) in dry DMF (10 ml) is added potassium carbonate (0.76 g, 5.55 mmol) followed by 4-(bromomethyl)pyridine hydrobromide (0.7 g, 2.77 mmol). The reaction mixture is stirred at room temperature overnight then poured into water (80 ml) and extracted with EtOAc (40 ml). The organic phase is washed with brine, dried ($MgSO_4$) and the solvent removed *in vacuo* to yield a dark brown oil. Chromatography ($SiO_2$, EtOAc) yields 3-[4-(Pyridin-4-ylmethoxy)-phenyl]-propionic acid methyl ester as a colorless oil; $[M+H]^+$ 272.0.

Step 2

To a solution of 3-[4-(Pyridin-4-ylmethoxy)-phenyl]-propionic acid methyl ester (0.28 g, 1.03 mmol) in THF (5 ml) and MeOH (5 ml) at room temperature is added 2 N LiOH (0.52 ml, 1.032 mmol) and the resulting solution is stirred overnight. Further 2 N LiOH (0.103 ml) is added and the reaction mixture stirred for a further 1 hour. The reaction mixture is concentrated *in vacuo* and the residue is diluted with water (50 ml) followed by EtOAc. The aqueous phase is acidified to pH2 with 1 N IIC1, and extracted with DCM. The organic phase is concentrated to a third of its volume *in vacuo* until a white powder precipitates which is collected by filtration to yield the title compound; $[M+H]^+$ 258.0.

Intermediate AG

3-(4-tert-Butoxycarbonylmethoxy-phenyl)-propionic acid

Step 1

To a stirring solution of methyl 3-(4-hydroxyphenyl)propanoate (2 g, 11.10 mmol) in dry DMF (30 ml) at room temperature is added potassium carbonate (1.53 g, 10 mmol) followed by tert-butyl 2-bromoacetate (2.17 g, 11.10 mmol). The reaction mixture is purged with nitrogen, then stoppered and left stirring at room temperature for 7 days. The reaction mixture is poured into water (200 ml) and extracted with EtOAc (100 ml), washed with brine, dried ($MgSO_4$), filtered and evaporated *in vacuo* to yield a pale yellow oil. Flash chromatography ($SiO_2$, EtOAc/iso-hexane) yields 3-(4-tert-Butoxycarbonylmethoxy-phenyl)-propionic acid methyl ester as a clear oil.

Step 2

To a solution of 3-(4-tert-Butoxycarbonylmethoxy-phenyl)-propionic acid methyl ester (2.70 g, 9.17 mmol) in THF (80 ml) is added 0.2N lithium hydroxide (45.9 ml, 9.17 mmol) at 0 °C and the reaction mixture is stirred at 0 °C for 4.5 hours. 1M HCl (15 ml) is added and the product is extracted using EtOAc (x3). The organic phase is dried ($Na_2SO_4$) and concentrated *in vacuo* to yield a white solid. Flash chromatography ($SiO_2$, 10% EtOAc in $CH_2Cl_2$, then 20% EtOAc in $CH_2Cl_2$) yields 3-(4-tert-Butoxycarbonylmethoxy-phenyl)-propionic acid as a white solid.

Intermediate AH

3-(4-Carbamoylmethoxy-phenyl)-propionic acid

This compound is prepared analogously to Intermediate AG by replacing tert-butyl 2-bromoacetate in step 1 with 2-bromoacetamide; $[M+H]^+$ 530.1.

Intermediate AI

1-[4-(2-Carboxy-ethyl)-phenoxy]-cyclobutanecarboxylic acid ethyl ester

This compound is prepared analogously to Intermediate AG by replacing tert-butyl 2-bromoacetate in step 1 with ethyl 1-bromocyclobutane-carboxylate; $[M+H]^+$ 293.0.

Intermediate AJ

2-[4-(2-Carboxy-ethyl)-phenoxyl-2-methyl-propionic acid tert-butyl ester

This compound is prepared analogously to Intermediate AG by replacing tert-butyl 2-bromoacetate in step 1 with tert-butyl 2-bromoisobutyrate. $^1$H NMR (DMSO-d6): 1.40 (9H, s), 1.48 (6H, s), 2.49 (2H, t, J = 7.5), 2.75 (2H, t, J = 7.5), 6.71 (2H, d, J = 8.5), 7.11 (2H, d, J = 8.50), 12.10 (1H, s).

Intermediate AK

3-(4-Methoxycarbonylmethoxy-phenyl)-propionic acid

Step 1

To a solution of 3-(4-hydroxyphenyl)propanoic acid (3.32 g, 20 mmol) in dry DMF (20 ml) is carefully added 1,1'-carbonyldiimidazole (3.24 g, 20 mmol) portionwise. The reaction mixture is stirred at 40 °C for 2 hours after which time DBU (6.02 ml, 40 mmol) and tert-butanol (4.78 ml, 50 mmol) are added and the reaction mixture is now stirred at 65 °C for 2 days. The reaction mixture is allowed to cool to room temperature and poured into water (50 ml) and the product is extracted with diethyl ether (3 x 30 ml). The organics are combined, dried ($MgSO_4$) and the solvent removed *in vacuo* to give a yellow oil. Purification by flash chromatography ($SiO_2$, EtOAc/iso-hexane) yields 3-(4-Hydroxy-phenyl)-propionic acid tert-butyl ester as a colorless oil. $^1$H NMR (DMSO-d6) 9.1 (1H, s), 7.0 (2H, d, J = 8.45), 6.65 (2H, d, J = 8.45), 2.7 (2H, t, J = 7.28), 2.4 (2H, t, J = 7.28), 1.4 (9H, s).

Step 2

To a solution of -(4-Hydroxy-phenyl)-propionic acid tert-butyl ester (1 g, 4.50 mmol) in dry DMF (20 ml) at room temperature under argon is added potassium carbonate (0.62 g, 4.50 mmol) followed by methyl bromoacetate (0.43 ml, 4.50 mmol) and the reaction mixture is stirred at room temperature. The reaction mixture is diluted with EtOAc and washed with water, dried ($MgSO_4$) and evaporated *in vacuo* to yield a clear colorless liquid. Purification on a Waters 3000 prep HPLC system (C18, MeCN/water) yields 3-(4-Methoxycarbonylmethoxy-phenyl)-propionic acid tert-butyl ester as a pale yellow oil.

Step 3

To 3-(4-Methoxycarbonylmethoxy-phenyl)-propionic acid tert-butyl ester (0.097 g, 0.33 mmol) is added a 90% solution of TFA in DCM (2 ml) and the resulting solution is stirred at room temperature for 1 hour. The solvents are removed *in vacuo* to yield 3-(4-Methoxycarbonylmethoxy-phenyl)-propionic acid as an off-white powder; $[M+H-18]^+$ 256.0

Intermediate AL

3-[4-(2-Propoxycarbonyl-ethyl)-phenyl]-propionic acid

To a solution of 3,3'-(1,4-phenylene)dipropanoic acid (250 mg, 1.125 mmol) DCM (15 ml) is added 4-dimethylaminopyridine (137 mg, 1.125 mmol) and propanol (3 ml, 40.1 mmol). The solution is cooled to 0 °C and dicyclohexylcarbodiimide (232 mg, 1.125 mmol) is added and the resulting solution is stirred at 0 °C for 30 minutes and 2 hours at room temperature. Concentration *in vacuo* affords a white solid which is suspended in $Et_2O$ (50 ml) and filtered to remove any insoluble material. The filtrate is concentrated *in vacuo* and purification by chromatography ($SiO_2$, EtOAc/iso-hexane) affords the title compound.

Intermediate AM

3-[4-(2-Ethoxycarbonyl-ethyl)-phenyl]-propionic acid

This compound is prepared analogously to Intermediate AL replacing propanol with ethanol.

Intermediate AN

3-14-(2-Methoxycarbonyl-ethyl)-phenyl]-propionic acid

This compound is prepared analogously to Intermediate AL replacing propanol with methanol.

Intermediate AO

1-(2-Phenoxy-ethyl)-1H-indole-4-carboxylic acid

Step 1

NaH (60% dispersion in mineral oil, 68.5 mg, 1.71 mmol) is added to solution of methyl indole-4-carboxylate (200 mg, 1.142 mmol) in DMF (5 ml) and the resulting suspension is stirred at room temperature for 20 minutes. After this time (2-bromoethoxy)benzene (298 mg, 1.484 mmol) is added and the reaction is stirred at room temperature for 18 hours. Dilution with EtOAc (50 ml) and washing with water (25 ml x 2), saturated $NaHCO_3$ (25 ml) and brine (25 ml), drying over $MgSO_4$, concentration *in vacuo* and purification by chromatography ($SiO_2$, EtOAc/iso-hexane) affords 1-(2-Phenoxy-ethyl)-1H-indole-4-carboxylic acid methyl ester; $[M+H]^+$ 296.

Step 2

1-(2-Phenoxy-ethyl)-1H-indole-4-carboxylic acid methyl ester (185 mg, 0.626 mmol) is suspended in a mixture of MeOH (3 ml) and 2 M NaOH (2 ml). The suspension is stirred at room temperature for 2 hours, TIIF (1 ml) is added and the reaction is heated at 60 °C for 1 hour. The reaction is allowed to cool to room temperature and diluted with sat. $NH_4Cl$ solution (10 ml), extracted with EtOAc (10 ml x 3), dried over $MgSO_4$, and concentrated *in vacuo* to give the title compound; $[M+H]^+$ 282.

Intermediate AP

1-(2-p-Tolyl-ethyl)-1H-indole-4-carboxylic acid

This compound is prepared analogously to Intermediate AO replacing (2-bromoethoxy)benzene with 4-methylphenethyl bromide; $[M+H]^+$ 280.

Intermediate AQ

1-[2-(Tetrahydro-pyran-2-yloxy)-ethyl]-1H-indole-4-carboxylic acid

This compound is prepared analogously to Intermediate AO replacing (2-bromoethoxy)benzene with 2-(2-bromoethoxy)tetrahydro-2H-pyran; $[M+H]^+$ 290.

Intermediate AR

1-[2-(4-Methoxy-phenoxy)-ethyl]-1H-indole-4-carboxylic acid

This compound is prepared analogously to Intermediate AO replacing (2-bromoethoxy)benzene with 1-(2-bromoethoxy)-4-methoxybenzene; $[M+H]^+$ 312.

Intermediate AS

1[2-(4-tert-Butyl-phenoxy)-ethyl]-1H-indole-4-carboxylic acid

This compound is prepared analogously to Intermediate AO replacing (2-bromoethoxy)benzene with 1-(2-bromoethoxy)-4-tert-butylbenzene; $[M+H]^+$ 338.

Intermediate AT

1-(2-[1,3]Dioxan-2-yl-ethyl)-1H-indole-4-carboxylic acid

This compound is prepared analogously to Intermediate AO replacing (2-bromoethoxy)benzene with (2-bromethyl)1,3-dioxane; $[M+H]^+$ 276.

Intermediate AU

2,3-Dimethyl-[2-(tetrahydro-pyran-2-yloxy)-ethyl]-1H-indole-5-carboxylic acid

This compound is prepared analogously to Intermediate A replacing (2-bromoethoxy)benzene with (2-(2-bromoethoxy)tetrahydro-2H-pyran and replacing Methyl indole-4-carboxylate with 2,3-dimethyl-1H-indole-5-carboxylate; $[M+H]^+$ 318.

Intermediate AV

1-(4,4,4-Trimethoxy-butyl)-1H-indole-4-carboxylic acid

This compound is prepared analogously to Intermediate AO 1-(2-Phenoxy-ethyl)-1H-indole-4-carboxylic acid replacing (2-bromoethoxy)benzene with trimethyl 4- bromoorthobutyrate.

Intermediate AW

1-[2-(2-Methoxy-ethoxymethoxy)-ethyl]-1H-indole-4-carboxylic acid

Step 1

NaH (60% dispersion in mineral oil, 86 mg, 2.14 mmol) is added to a solution of methyl indole-4-carboxylate (250 mg, 1.427 mmol) in DMF (20 ml) and the resulting suspension is stirred at room temperature for 30 minutes. After this time (2-(2-bromoethoxy)tetrahydro-2H-pyran (388 mg, 1.86 mmol) is added and the reaction is stirred at room temperature for 22 hours. Dilution with EtOAc (50 ml), washing with water (25 ml x 3), saturated $NaHCO_3$ (25 ml x 2) and brine (25 ml), drying over $MgSO_4$, concentration *in vacuo* and purification by chromatography ($SiO_2$, DCM/MeOH) affords 1[2-(Tetrahydro-pyran-2-yloxy)-ethyl]-1H-indole-4-carboxylic acid methyl ester; $[M+H]^+$ 304.

Step 2

To a solution of 1[2-(Tetrahydro-pyran-2-yloxy)-ethyl]-1H-indole-4-carboxylic acid methyl ester (120 mg, 0.396 mmol) in MeOH (10 ml) is added p-toluenesulfonic acid monohydrate (7.25 mg, 0.04 mmol). The reaction is stirred at room temperature for 16 hours and the solvent is removed *in vacuo.* The residue is dissolved in MeOH (3 ml) and loaded onto a 1 g PEAX cartridge washed with MeOH (20 ml). The filtrate is concentrated *in vacuo* to give 1-(2-Hydroxy-ethyl)-1H-indole-4-carboxylic acid methyl ester; $[M+H]^+$ 220.

Step 3

To a solution of -(2-Hydroxy-ethyl)-1H-indole-4-carboxylic acid methyl ester in DCM (3 ml) is added DIPEA (0.129 ml, 0.739 mmol) and 1-Chloromethoxy-2-methoxy-ethane (0.084 ml, 0.739 mmol). The solution is stirred at room temperature for 72 hours. The reaction is diluted with DCM (50 ml) and washed with 0.5 M HCl (20 ml), 1 M NaOH (20 ml) and 0.5 M HCl (20 ml). The organic layer is dried over $MgSO_4$ and the solvent is removed *in vacuo.* Purification by chromatography ($SiO_2$, DCM/MeOH) affords 1-[2-(2-Methoxy-ethoxymethoxy)-ethyl]-1H-indole-4-carboxylic acid methyl ester; $[M+H]^+$ 308.

Step 4

To a solution of 1-[2-(2-Methoxy-ethoxymethoxy)-ethyl]-1H-indole-4-carboxylic acid methyl ester (69 mg, 0.225 mmol) in MeOH (2 ml) is added 2 M NaOH (1 ml) and the reaction is stirred at room temperature for 19.5 hours, then for 2 hours at 50 °C. The reaction is allowed to cool to room temperature and the solvent removed *in vacuo.* To the residue is added sat. $NH_4Cl$ (10 ml), and the product is extracted with EtOAc (5 x 25 ml), washed with brine (10 ml), dried over $Na_2SO_4$, and the solvent is removed *in vacuo,* to give the title compound 1-[2-(2-Methoxy-ethoxymethoxy)-ethyl]-1H-indole-4-carboxylic acid; $[M+H]^+$ 294.

Intermediate AX

1-Diethylcarbamoylmethyl-1H-indole-4-carboxylic acid

Step 1

Methyl indole-4-carboxylate (50 mg, 2.85 mmol) and 2-chloro-N,N-diethylacetamide (854 mg, 5.71 mmol) are dissolved in DMF (10 ml) and to the solution is added potassium carbonate (986 mg, 7.14 mmol). The reaction is heated using microwave radiation at 100 °C for 2 hours, then diluted with DCM (60 ml) and washed with water (5 x 10 ml). Drying over $MgSO_4$, concentration *in vacuo,* and trituration with Et20 affords 1-Diethylcarbamoylmethyl-1H-indole-4-carboxylic acid methyl ester; $[M+H]^+$ 289.

Step 2

To a solution of Diethylcarbamoylmethyl-1H-indole-4-carboxylic acid methyl ester (480 mg, 1.665 mmol) in MeOH (5 ml) is added 2 M NaOH (5 ml). The reaction is heated at 50 °C for 20 hours and then allowed to cool to room temperature. The solvent is removed *in vacuo* and the residue dissolved in water (10 ml). The pH of the solution is adjusted to 5 using 1 M HCl and the resulting solid is collected by filtration to give the title compound 1-Diethylcarbamoylmethyl-1H-indole-4-carboxylic acid; $[M+H]^+$ 275.

Intermediate AY

4- [6- ((R)-2,2-Dimethyl-[1,3]dioxolan-4-ylmethoxy)-naphthalen-2-ylmethoxy]-benzoic acid

Step 1

To a solution of methyl 6-hydroxy-2-naphthoate (4.55 g, 22.5 mmol) in anhydrous acetone (60 ml) are added S-(-)-glycidol (2.0 g, 27.0 mmol) and $K_2CO_3$ (9.3 g, 67.3 mmol). The reaction mixture is heated to reflux for 3 days. The reaction mixture is filtered through Celite™ (filter material) and the filtrate is concentrated *in vacuo* to afford 6-((*S*)-2,3-Dihydroxy-propoxy)-naphthalene-2-carboxylic acid methyl ester as a white solid; $^1H$ NMR (DMSO-$d_6$): 3.49 (2H, t, J = 6.0 Hz), 3.85-3.88 (1H, m), 3.89 (3H, s), 4.02 (1H, dd, J = 9.9, 6.0 Hz), 4.16 (1H, dd, J = 9.9, 4.0 Hz), 4.73 (1H, t, J = 6.0 Hz), 5.04 (1H, d, J = 5.2 Hz), 7.26 (1H, dd, J = 9.0, 2.0 Hz), 7.41 (1H, d, J = 2.0 Hz), 7.88-7.94 (2H, m), 8.04 (1H, d, J = 9.0 Hz), 8.55 (1H, s).

Step 2

To 6-((*S*)-2,3-dihydroxy-propoxy)-naphthalene-2-carboxylic acid methyl ester (0.9 g, 3.26 mmol) in anhydrous DMF (10 ml) is added 2,2-dimethoxypropane (2.0 ml, 16.3 mmol) and pyridinium *p*-toluenesulfonate (0.08 g, 0.32 mmol) and the reaction mixture is stirred at room temperature for 16 hours. The reaction mixture is concentrated *in vacuo* and the residue is dissolved in EtOAc. The EtOAc layer is washed with 10% NaHC03, water, and brine, dried over anhydrous $Na_2SO_4$ and the solvent is evaporated *in vacuo* to obtain 6-((*R*)-2,2-Dimethyl-[1,3]dioxolan-4-ylmethoxy)-naphthalcnc-2-carboxylic acid methyl ester as solid; $^1H$ NMR (DMSO-d6): 1.32 (3H, s), 1.37 (3H, s), 3.78-3.82 (1H, m), 3.88 (3H, s), 4.benzoyl}-4.20 (3H, m), 4.45-4.50 (1H, m), 7.26 (1H, dd, J = 9.0, 2.0 Hz), 7.45 (1H, d, J = 2.0 Hz), 7.88 (1H, d, J = 9.0 Hz), 7.93 (1H, d, J= 9.0 Hz), 8.04 (1H, d, J = 9.0 Hz), 8.55 (1H, s).

Step 3

To a solution of 6-((R)-2,2-dimethyl-[1,3]dioxolan-4-ylmethoxy)-naphthalene-2-carboxylic acid methyl ester (1.0 g, 3.16 mmol) in anhydrous THF (20 ml) at 0 °C is added LiAlH4 (1.9 ml of a 2M solution in THF, 3.8 mmol). The reaction

mixture is stirred at room temperature overnight. The reaction mixture is concentrated *in vacuo* and the residue is purified by column chromatography ($SiO_2$, DCM) to afford [6-((*R*)-2,2-Dimethyl-[1,3]dioxolan-4-ylmethoxy)-naphthalen-2-yl]-MeOH as a colorless viscous oil which solidified on standing; $^1$H NMR ($d_6$-DMSO): 1.32 (3H, s), 1.37 (3H, s), 3.78 (1H, dd, J = 8.3, 6.0 Hz), 4.01-4.15 (3H, m), 4.45-4.48 (1H, m), 4.60 (2H, d, J = 6.0 Hz), 5.24 (1H, t, J = 6.0 Hz), 7.14 (1H, dd, J = 8.5, 2.5 Hz), 7.32 (1H, d, J = 2.5 Hz), 7.41 (1H, dd, J = 8.5, 1.5 Hz), 7.33-7.80 (3H, m).

Step 4

A mixture of methyl 4-hydroxybenzoate (0.5 g, 3.28 mmol), [6-((*R*)-2,2-dimethyl-[1,3]dioxolan-4-ylmethoxy)-naphthalen-2-yl]-methanol (0.9 g, 3.12 mmol) and triphenylphosphine (0.83 g, 3.16 mmol) in DCM (20 ml) is cooled to 0 °C. Diethyl azodicarboxylate (0.5 ml, 3.17 mmol) is added dropwise. The reaction mixture is stirred at room temperature overnight. The reaction mixture is concentrated *in vacuo* and purified by column chromatography ($SiO_2$, EtOAc/iso-hexane) to obtain white solid. The product obtained is once again purified by column chromatography (neutral alumina, EtOAc/petroleum ether) to obtain 4-[6-((*R*)-2,2-Dimethyl-[1,3]dioxolan-4-ylmethoxy)

-naphthalen-2-ylmethoxy]-benzoic acid methyl ester as white solid; $^1$H NMR ($d_6$-DMSO): 1.32 (3H, s), 1.38 (3H, s), 3.77-3.82 (4H, m), 4.08-4.16 (3H, m), 4.46-4.49 (1H, m), 5.30 (2H, s), 7.15-7.21 (3H, m), 7.37 (1H, d, J = 2.0 Hz), 7.53 (1H, dd, J = 8.50, 1.5 Hz), 7.83 (2H, dd, J = 9.0, 6.0 Hz), 7.92 (3H, m).

Step 5

To a solution of 446-((R)-2,2-dimethyl-[1,3]dioxolan-4-ylmethoxy)-naphthalen-2-ylmethoxy]-benzoic acid methyl ester (0.46, 1.09 mmol) in THF/water (10 ml of a 1:1 mixture) is added lithium hydroxide (0.15 g, 3.57 mmol). The reaction mixture is stirred at room temperature overnight, then at 70 °C for 24 h. The reaction mixture is cooled to room temperature, neutralized with 1.5 M HCl and the white solid obtained is collected by vacuum filtration, washed with water and dried under vacuum to afford 4-[6-((*R*)-2,2-Dimethyl-[1,3]dioxolan-4-ylmethoxy)-naphthalen-2-ylmethoxyJ - benzoic acid. $[M]^-$ 407.

Intermediate AZ

4-{3-[4-((*R*)-2,2-Dimethyl-[1,3]dioxolan-4-ylmethoxy)-phenyl]-propoxy}-benzoic acid

This compound is prepared analogously to Intermediate AY by replacing [6-((R)-2,2-dimethyl-[1,3]dioxolan-4-ylmethoxy)-naphthalen-2-yl]-methanol in Step 4 with 3-[4-((R)-2,2-dimethyl-[1,3]dioxolan-4-ylmethoxy)-phenyl]-propan-1-ol; $^1$H NMR (DMSOd6): 1.30 (3H, s), 1.35 (3H, s), 1.97-2.01 (2H, m), 2.68 (2H, t, J = 7.5 Hz), 3.72-3.75 (1H, m), 3.93-4.00 (4H, m), 4.06-4.10 (1H, m), 4.38 (1H, dd, J = 6.0, 5.0), 6.87 (2H, d, J = 9.0 Hz), 6.92 (2H, d, J = 9.0 Hz), 7.14 (2H, d, J =, 9.0 Hz), 7.84 (2H, d, J = 9.0 Hz).

Intermediate BA

4-{2-[(*E*)-3,5-Diamino-6-chloro-pyrazine-2-carbonylimino]-1,3,8-triaza-spiro[4.5]decane-8-carbonyl}-piperidine-1-carboxylic acid tert-butyl ester

This compound is prepared analogously to Example 97 by replacing 4-benzyloxyphenylacetic acid with 1-Boc-piperidine-4-carboxylic acid; $[M+H]^+$ 536.

Intermediate BB

4-[(Naphthalene-1-sulfonylamino)-methyl]-benzoic acid

4 N NaOH solution (30 ml) is added to a suspension of 4-(aminomethyl)benzoic acid (5.01 g, 31.82 mmol) in acetone (100 ml). Toluene (100 ml) is added and the reaction is heated at 40 °C to obtain dissolution. The solution is cooled to 0 °C and treated with 1-naphthalene sulfonyl chloride (12 g, 51.35 mmol) in acetone (100 ml) and the resulting reaction mixture is stirred for 3 hours. The reaction is acidified using citric acid and concentrated *in vacuo.* The residue is taken up in EtOAc and washed with water. The aqueous layer is back extracted with EtOAc and the combined organic layers are washed with water, brine, dried ($Na_2SO_4$) and the solvent removed *in vacuo* to yield a light brown solid. Trituration with $Et_2O$ yields the title compound.

Intermediate BC

3-(Cyclohexyl-methyl-sulfamoyl)-4-methoxy-benzoic acid

Step 1

A solution of methyl 3-(chlorosulfonyl)-4-methoxybenzoate (2.0 g, 7.56 mmol) and diisopropylethylamine (1.94 ml, 11.34 mmol) in DCM (50 ml) is treated with N-methyl cyclohexylamine (0.70 ml, 9.07 mmol) at 0 °C. The solution is stirred at room temperature for 3 hours and N-methyl cyclohexylamine (0.70 ml, 9.07 mmol) is added. The solution is partitioned between DCM (250 ml) and 0.5 N HCl (100 ml). The organic layer is washed with 0.5 N HCl (2 x 100 ml), . $NaHCO_3$ (2 x 100 ml) and water (100 ml), dried over $MgSO_4$, and the solvent removed *in vacuo* to yield a yellow oil. Crystallisation ($iPr_2O$/EtOAc) yields 3-(Cyclohexyl-methyl-sulfamoyl)-4-m ethoxy-benzoic acid methyl ester as yellow crystals; $[M+H]^+$ 342.

Step 2

A solution of 3-(Cyclohexyl-methyl-sulfamoyl)-4-methoxy-benzoic acid methyl ester (1.50 g, 4.39 mmol) in 1,4 dioxane (40 ml) is treated with 2 N NaOH (10 ml) and the resulting solution is stirred at room temperature for 21 hours. The solvent is removed *in vacuo* and ice cold 2 N HCl (25 ml) is added and the white solid which forms is extracted into DCM (150 ml). The organic layer is washed with water, dried ($MgSO_4$) and the solvent removed *in vacuo* to yield the title compound as a white solid; $[M-1]^-$326.

Intermediate BD

3-Chloro-5-methoxy-442-(4-methyl-piperazin-1-yl)-ethoxy]-benzoic acid

Step 1

A mixture of 5-chlorovanillic acid (5.0 g, 24.6 mmol) and conc. HCl (5 ml) in MeOH (100 ml) is heated at reflux for 48 hours. The solvent is *removed in vacuo* and water is added to the residue to yield a white precipitate, which is collected by filtration, washed with water, and then dissolved in $Et_2O$. The solution is dried ($Na_2SO_4$) and the solvent removed *in vacuo* to yield 3-Chloro-4-hydroxy-5-methoxy-benzoic acid methyl ester as a white solid.

Step 2

Triphenylphosphine (6.4 g, 24.4 mmol) and DIAD (4.8 ml, 202.2 mmol) are added to a solution of 3-Chloro-4-hydroxy-5-methoxy-benzoic acid methyl ester (2.5 g, benzoyl}.5 mmol) in THF (40 ml) at 0 °C and the resulting solution is stirred for 2 hours at 0 °C and 16 hours at room temperature. The solvent is removed *in vacuo,* and water is added to the residue. The product is extracted in EtOAc, dried ($Na_2SO_4$) and the solvent removed *in vacuo* to afford a yellow oil. Flash chromatography ($SiO_2$, EtOAc/MeOH) yields 3-Chloro-5-methoxy-4-[2-(4-methyl-piperazin-1-yl)-ethoxy]- benzoic acid methyl ester as an orange solid.

Step 3

A solution of 3-Chloro-5-methoxy-4-[2-(4-methyl-piperazin-1-yl)-ethoxy]-benzoic acid methyl ester (3.7 g, 10.7 mmol) in 2 N NaOH (20 ml) and THF (40 ml) is heated at reflux for 1 hour. The reaction mixture is washed with $Et_2O$. The aqueous phase is concentrated *in vacuo,* and water (50 ml) is added. The pH is adjusted to 3-4 using 2 N HCl. To this solution is added DOWEX 50WX4 (previously washed with MeOH, 2 N HCl and water), and the resulting mixture is stirred at room temperature for 1 hour. The resin is filtered, washed with water, and the product is released from the resin by washing with $MeOH/NH_4OH$. The solution is concentrated *in vacuo,* diluted with DCM and MeOH, dried ($Na_2SO_4$) and the solvent removed *in vacuo* to yield the title compound as a light cream solid.

Intermediate BE

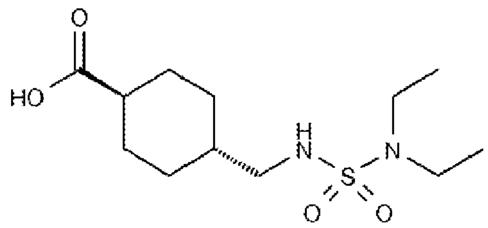

Step 1

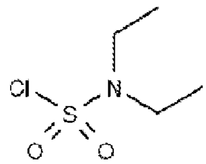

To a stirred solution of diethyl amine (500 ml, 4.8 mol) in $Et_2O$ (1200 ml) is added sulfuryl chloride (177.3 ml, 2.19 mol) over 80 minutes at -15 °C. The reaction is stirred at room temperature for 2.5 hours. $Et_2O$ (1000 ml) is added and the white solid present is removed by filtration, and washed with $Et_2O$ (2000 ml). The combined filtrates are concentrated under reduced pressure to yield as a colorless oil.

Step 2

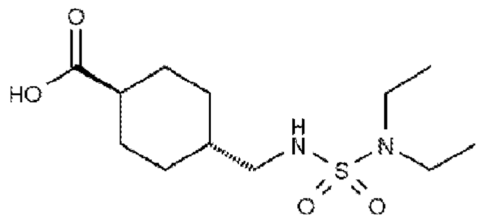

To a stirred solution of *trans*-4-(aminomethyl)-cyclohexane carboxylic acid (10 g, 63.6 mmol) in 1 N NaOH (153 ml) is added (10.91 g, 63.6 mmol) and the resulting mixture is stirred at room temperature for 15 hours. The reaction is cooled to 10 °C and conc. HCl solution (15 ml) is added and the mixture stirred for 10 minutes at this temperature. White crystals form which are isolated by filtration and washed with $Et_2O$ (40 ml) to yield the title compound.

Intermediate BF

3-(3-Phenyl-isoxazol-5-yl)-propionic acid

This compound is prepared as described by G. S. d'Alcontres; C Caristi; A Ferlazzo; M Gattuso, J. Chem. Soc. Perkin 1, (1976) 16, 1694.

Intermediate BG

3-(4-Chloro-phenoxymethyl)-benzylamine

This compound is prepared as described in US 2008200523.

Intermediate BH

2-{4-[2-(4-Fluoro-phenyl)-ethoxy]-phenyl}-ethylamine

Step 1

A suspension of 4-Hydroxybenzyl cyanide (7.9 g, 59.57 mmol), 1-(2-Bromo-ethyl)-4-fluoro-benzene (17.4 g, 71.48 mmol), potassium carbonate (19.8 g, 143 mmol) and sodium iodide (2.68 g, 17.87 mmol) in acetonitrile (120 ml) is heated at reflux for 44 hours. The reaction mixture is cooled and filtered and the solvent removed *in vacuo* to yield a dark brown oil. Flash chromatography ($SiO_2$, EtOAc/ iso-hexane) yields {442-(4-Fluoro-phenyl)-ethoxy}-phenyl}-acetonitrile as a yellow oil.

Step 2

2 N NaOH solution (45.2 ml, 90.3 mmol) is added to a solution of {4-[2-(4-Fluorophenyl)-ethoxy]-phenyl}-acetonitrile (3.29 g, 12.9 mmol) in EtOH (45.2 mol) followed by Al-Ni Alloy (2.5 g) and the resulting reaction mixture is stirred for 1 hour at room temperature. The reaction mixture is filtered and the EtOH removed *in vacuo.* The product is extracted into DCM (2 x 80 ml), dried ($MgSO_4$) and the solvent removed *in vacuo* to yield the title compound as a yellow oil.

Intermediate BI

2-(4,6-Dimethyl-1H-indol-3-yl)-ethylamine

This compound is prepared as described in EP 620222.

Intermediate BJ

2- [4- (4-Phenyl-butoxy)-phenyl]-ethylamine

This compound is prepared as described in WOP 2004016601.

Intermediate BK

4-(5-Methyl-2-phenyl-oxazol-4-ylmethoxy)-benzenesulfonyl chloride

This compound is prepared as described in WO 2005026134.

Intermediate BL

2-Phenyl-3H-benzoimidazole-5-sulfonyl chloride

This compound is prepared as described in EP 1205475.

Intermediate BM

4-Aminomethyl-1-(1-phenyl-ethyl)-piperidin-4-ylamine

Step 1

1-(1-Phenyl-ethyl)-piperidin-4-one is prepared according to the procedure described on page 525 of J. *Org. Chem.* 1991, 56(2), 513-528.

To a mixture of 1-(1-phenyl-ethyl)-piperidin-4-one (10.9 g, 53.6 mmol), ammonium chloride (4.3 g, 80.4 mmol) and 30% aqueous ammonia solution (30 ml) in water (30 ml) at room temperature is added sodium cyanide (4.0 g, 81.6 mmol) portion wise. The reaction mixture is stirred at room temperature for 18 hours, then diluted with water and extracted with DCM. The organic phase is washed with brine, dried over $Na_2SO_4$, filtered and concentrated *in vacuo* to obtain 4-Amino-1-(1-phenyl-ethyl)- piperidine-4-carbonitrile as a brown oil; $[M+H]^+$ 230.

Step 2

4-Aminomethyl-1-(1-phenyl-ethyl)-piperidin-4-ylaminen is prepared analogously to Intermediate U by replacing 4-amino-4-cyano-piperidine-1-carboxylic acid *tert*-butyl ester in Step 1 with 4-amino-1-(1-phenyl-ethyl)-piperidine-4-carbonitrile; $[M+H]^+$ 234.

Intermediate BN

4-Aminomethyl-1-(4-methoxy-benzyl)-piperidin-4-ylamine

This compound is prepared analogously to Intermediate BM by replacing 1-(1-phenylethyl)-piperidin-4-one with 1-(4-methoxybenzyl)piperidin-4-one in step 2; $^1H$ NMR (DMSO-d6): 1.46-1.64 (4H, m), 2.38-2.55 (4H, m), 2.67 (2H, s), 3.26 (2H, s), 4.08 (3H, s), 6.87 (2H, d, J = 8.2 Hz), 7.18 (2H, d, J = 8.2 Hz).

Intermediate BO

4-Aminomethyl-1-pyridin-4-ylmethyl-piperidin-4-ylamine

Step 1

To a solution of 4-aminomethyl-4-(2,2,2-trifluoro-acetylamino)-piperidine-1-carboxylic acid *tert*-butyl ester (Intermediate U, Step 2) (5.0 g, 15.4 mmol) in DCM (50 ml) at 0 °C is added pyridine (10 ml) followed by trifluoroacetic anhydride (3.5 ml, 25.3 mmol) and the reaction mixture is stirred at room temperature for 16 hours. The reaction mixture is diluted with DCM, washed with brine, dried over $Na_2SO_4$ and concentrated *in vacuo.* The residue obtained is dissolved in diethyl ether and re-precipitated by adding petroleum ether. The solvent mixture is decanted and the solid dried under vacuum to afford 4-(2,2,2-Trifluoro-acetylamino)-4-[(2,2,2-trifluoroacetylamino)-methyl]-piperidine-1-carboxylic acid *tert-butyl* ester; $[M+H]^+$ 420.

Step 2

To a solution of 4-(2,2,2-trifluoro-acetylamino)-4-[(2,2,2-trifluoro-acetylamino)-methyl]-piperidine-1-carboxylic acid *tert-butyl* ester (5.25 g, 12.5 mmol) in dioxane (50 ml) is added 4 M HCl in dioxane (15 ml) and the reaction mixture is stirred at room temperature for 3 hours. The reaction mixture is concentrated *in vacuo* and the off-white solid obtained dissolved in the minimum amount of MeOH and re-precipitated by adding diethyl ether. The supernatant solvent mixture is decanted and the product is washed again with diethyl ether and dried under vacuum to afford 2,2,2-Trifluoro-*N*-{4-[(2,2,2-trifluoro-acetylamino)-methyl]-piperidin-4-yl}-acetamide hydrochloride; $[M+H]^+$ 322.

Step 3

To a suspension of NaH (170 mg of a 60% dispersion in mineral oil, 4.25 mmol) in anhydrous DMF (20 ml) is added 2,2,2-trifluoro-*N*-{4-[(2,2,2-trifluoro-acetylamino)- methyl]-piperidin-4-yl}-acetamide hydrochloride) (500 mg, 1.4 mmol) followed by 4- bromomethylpyridine hydrobromide (350 mg, 1.4 mmol). The reaction mixture is stirred at room temperature for 3 hours. The reaction mixture is quenched with sat. $NH_4Cl$ solution and is concentrated *in vacuo.* The residue is purified by column chromatography (basic alumina, MeOH/DCM) to obtain 2,2,2-Trifluoro-*N*-[1-pyridin4-ylmethyl-4-(2,2,2-trifluoro-acetylamino)-piperidin-4-ylmethyl]-acetamide as off-white solid; $[M+H]^+$ 413.

Step 4

To a solution of 2,2,2-trifluoro-*N*-[1-pyridin-4-ylmethyl-4-(2,2,2-trifluoro-acetylamino)-piperidin-4-ylmethyl]-acetamide (200 mg , 0.49 mmol) in MeOH (10 ml) is added 30% aqueous ammonia solution (10 ml) and the reaction mixture is stirred at 60 °C for 3 h. The reaction mixture is concentrated *in vacuo* to obtain 4-Aminomethyl-1-pyridin-4-ylmethyl-piperidin-4-ylamine as a colorless gummy oil that is used without further purification; $^1H$ NMR (DMSO-d6): 1.63-1.77 (4H, m), 2.45-2.54 (4H, m), 2.49 (2H, s), 3.57 (3H, s), 7.30 (2H, d, J = 5.5 Hz), 8.68 (2H, d, J = 5.5 Hz).

Intermediate BP

4-Aminomethyl-1-(3-phenyl-propyl)-piperidin-4-ylamine

This compound is prepared analogously to Intermediate BO by replacing - bromomethylpyridine hydrobromide (Step 3) with 1-bromo-3-phenylpropane; $[M+H]^+$ 248.

Intermediate BQ

4-Aminomethyl-1-cyclohexylmethyl-piperidin-4-ylamine

This compound is prepared analogously to Intermediate BO by replacing - bromomethylpyridine hydrobromide (Step 3) with cyclohexylmethylbromide. This intermediate is used crude in the preparation of Example 250.

Intermediate BR

3-Amino-3-aminomethyl-8-aza-bicyclo[3.2.1]octane-8-carboxylic acid *tert*-butyl ester

This compound is prepared analogously to Intermediate BM by replacing 1-(1-phenyl-cthyl)-pipcridin-4-onc (Step 1) with *N*-Boc-nortropinone; $^1$H NMR (DMSO-d6): 1.40 (9H, s), 1.63-1.85 (8H, m), 2.79 (2H, s), 4.06 (2H, s).

**IV.** FORMULATIONS

In one aspect, the invention features a pharmaceutical formulation comprising an inhibitor of ENaC activity as provided in Column D and a modulator of CF Modulator activity as provided in Columns A, B, or C according to Table I. In some embodiments, the modulator of CF Modulator activity can include a compound of Formula I, or a compound of Formula II, or a compound of Formula III, or combinations thereof according to Table I. In some embodiments, the modulator of CF Modulator activity can include Compound 1, or Compound 2, or Compound 3 or combinations thereof according to Table I.

Table I is reproduced here for convenience.

Table I

| **Column A Embodiments** | | **Column B Embodiments** | | **Column C Embodiments** | | **Column D Embodiments** | |
|---|---|---|---|---|---|---|---|
| **Section** | | **Section** | **Heading** | **Section** | **Heading** | **Section** | **Heading** |
| **II.A**.1. | Compound of Formula I | **II.B.1.** | Compound of Formula II | **II.C.1.** | Compound of Formula III | **II.D.1.** | ENaC Compounds |
| | | | | | | | |
| **II.A.2.** | Compound 1 | **II.B.2.** | Compound 2 | **II.C.2**. | Compound 3 | **II.D.2** | ENaC Compounds of Formula IV |

According to Table I, the pharmaceutical composition comprises at least one component from Column D of Table I, and at least one component from Columns A, B, or C of Table 1. In one aspect Compound 1 is the component from Column A, Compound 2 is the component from Column B, and Compound 3 is the component of Column C.

In one embodiment, the composition comprises a homogeneous mixture comprising a composition according to Table I. In another embodiment, the composition comprises a nonhomogeneous mixture comprising a composition according to Table 1.

The pharmaceutical composition of Table I can be administered in one vehicle or separately. In another aspect, the pharmaceutical combination composition comprising an inhibitor of ENaC activity as exemplified in Column D of Table I, can be formulated into a unitary dosage unit, for example, a tablet, a capsule, a liquid suspension or solution for administration to the mammal in need thereof. The ENaC inhibitor can include an amorphous form, a substantially amorphous form or a crystalline form of the ENaC compound. Alternatively, each active agent can be formulated separately as a single dosage unit to be administered with the other active agent of the combination concurrently, or sequentially, i.e. prior to, or subsequent to each other, or within predetermined time periods apart, for example, within

5 minutes, within 30 minutes, within 1 hr., within 2 hrs, within 3 hrs. within 6 hrs., or within 12 hrs from administration of the other active agent. In some embodiments, the time period may be 24 hrs or more. For example, the first active agent (ENaC inhibitor or CF Modulator modulator) is administered on day 1, and the second active agent of the combination is administered the next day. The sequential administration regime is intended to only exemplify one of a number of possibilities of delayed administration of the second active agent from the first active agent and could be readily determined by one of ordinary skill in the art, for example, a prescribing physician.

The pharmaceutical compositions described herein may encompass one active agent or two different active agents selected from Table I, with the understanding that if the formulation includes two active agents, one of the active agents is an inhibitor of ENaC activity as exemplified by the components of Column D and the other active agent is a modulator of CF Modulator activity exemplified by the components of Columns A-C. In some embodiments, the pharmaceutical composition may contain more than one CF Modulator modulator as provided in Columns A-C.

In some embodiments, the pharmaceutical composition optionally comprises a pharmaceutically acceptable carrier, adjuvant or vehicle. In certain embodiments, these compositions optionally further comprise one or more additional therapeutic agents.

It will also be appreciated that certain of the Compounds of present invention can exist in free form for treatment, or where appropriate, as a pharmaceutically acceptable derivative, enantiomer, tautomer or a prodrug thereof. According to the present invention, a pharmaceutically acceptable derivative or a prodrug includes, but is not limited to, pharmaceutically acceptable salts, esters, salts of such esters, or any other adduct or derivative which upon administration to a patient in need thereof is capable of providing, directly or indirectly, a Compound as otherwise described herein, or a metabolite or residue thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. A "pharmaceutically acceptable salt" means any non-toxic salt or salt of an ester of a Compound of this invention that, upon administration to a recipient, is capable of providing, either directly or indirectly, a Compound of this invention or an inhibitory active metabolite or residue thereof.

Pharmaceutically acceptable salts are well known in the art. For example, S. M. Berge, et al. describe pharmaceutically acceptable salts in detail in J. Pharmaceutical Sciences, 1977, 66, 1-19, incorporated herein by reference. Pharmaceutically acceptable salts of the Compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and $N^+(C_{1-4}alkyl)_4$ salts. The present invention also envisions the quaternization of any basic nitrogen-containing groups of the Compounds disclosed herein. Water or oil-soluble or dispersible products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As described above, the pharmaceutically acceptable compositions of the present invention additionally comprise a pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the Compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated

vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compositions of the invention may be administered orally or parenterally, wherein the ENaC inhibitor compound and/or the CF Modulator modulator is/are present independently in the administered composition at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active Compounds of the composition, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a composition of the present invention, it is often desirable to slow the absorption of the composition from subcutaneous or intramuscular injection. This may be accomplished by the use of a liquid suspension of crystalline or amorphous material with poor water solubility. The rate of absorption of the composition then depends upon its rate of dissolution that, in turn, may depend upon crystal size and crystalline form. Alternatively, delayed absorption of a parenterally administered composition form is accomplished by dissolving or suspending the composition in an oil vehicle. Injectable depot forms are made by forming microencapsule matrices of the composition in biodegradable polymers such as polylactide-polyglycolide. Depending upon the ratio of composition to polymer and the nature of the particular polymer employed, the rate of composition release can be controlled. Examples of other biodegradable polymers include poly(orthoesters) and poly(anhydrides). Depot injectable formulations are also prepared by entrapping the composition in liposomes or microemulsions that are compatible with body tissues.

Compositions for rectal or vaginal administration are preferably suppositories which can be prepared by mixing the Compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active Compound.

Solid dosage forms for oral administration include capsules, tablets, mini-tablets, micro-tablets, particulates, micro and nano-particulates, pills, powders, and granules. In such solid dosage forms, the active Compound or combination of ENaC inhibitor and CF Modulator Compounds are mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium Compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of capsules, tablets, mini-tablets, micro-tablets, particulates, micro and nano-particulates, pills, and granules can be prepared with coatings and shells such as enteric coatings and other coatings well known in the pharmaceutical formulating art. They may optionally contain opacifying agents and can also be of a composition that they release the active ingrcdicnt(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active Compound or combination of Compounds can also be in microencapsulated form with one or more excipients as noted above. The solid dosage forms of capsules, tablets, mini-tablets, micro-tablets, particulates, micro and nano-particulates, pills, and granules can be prepared with coatings and shells such as enteric coatings, release controlling coatings and other coatings well known in the pharmaceutical formulating art. In such solid dosage forms the active Compound or combination of compounds may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a Compound or combination of Compounds of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a Compound to the body. Such dosage forms are prepared by dissolving or dispensing the Compound in the proper medium. Absorption enhancers can also be used to increase the flux of the Compound across the skin. The rate can be controlled by either providing a rate controlling membrane or by dispersing the Compound in a polymer matrix or gel.

It will also be appreciated that the compositions disclosed herein can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive Compound or combination of Compounds may be administered concurrently with another agent used to treat the same disorder), or they may achieve different effects (e.g., control of any adverse effects). As used herein, additional therapeutic agents that are normally administered to treat or prevent a particular disease, or condition, arc known as "appropriate for the disease, or condition, being treated".

In one embodiment, the additional agent is selected from a mucolytic agent, bronchodialator, an anti-biotic, an anti-infective agent, an anti-inflammatory agent, a CFTR modulator other than a Compound of the present invention, or a nutritional agent.

In one embodiment, the additional agent is an antibiotic. Exemplary antibiotics useful herein include tobramycin, including tobramycin inhaled powder (TIP), azithromycin, aztreonam, including the aerosolized form of aztreonam, amikacin, including liposomal formulations thereof, ciprofloxacin, including formulations thereof suitable for administration by inhalation, levoflaxacin, including aerosolized formulations thereof, and combinations of two antibiotics, e.g., fosfomycin and tobramycin.

In another embodiment, the additional agent is a mucolyte. Exemplary mucolytes useful herein includes Pulmozyme®.

In another embodiment, the additional agent is a bronchodialator. Exemplary bronchodialtors include albuterol, metaprotenerol sulfate, pirbuterol acetate, salmeterol, or tetrabuline sulfate.

In another embodiment, the additional agent is effective in restoring lung airway surface liquid. Such agents improve the movement of salt in and out of cells, allowing mucus in the lung airway to be more hydrated and, therefore, cleared more easily. Exemplary such agents include hypertonic saline, denufosol tetrasodium ([[(3S,5R)-5-(4-amino-2-oxopyrimidin-1-yl)-3-hydroxyoxolan-2-yl]methoxy-hydroxyphosphoryl] [[[(2R,3S,4R,5R)-5-(2,4-dioxopyrimidin-1-yl)-3, 4-dihydroxyoxolan-2-yl]methoxy-hydroxyphosphoryl]oxy-hydroxyphosphoryl] hydrogen phosphate), or bronchitol (inhaled formulation of mannitol).

In another embodiment, the additional agent is an anti-inflammatory agent, i.e., an agent that can reduce the inflammation in the lungs. Exemplary such agents useful herein include ibuprofen, docosahexanoic acid (DHA), sildenafil, inhaled glutathione, pioglitazone, hydroxychloroquine, or simavastatin.

In another embodiment, the additional agent is a CFTR modulator other than the components disclosed in Columns A-D, i.e., an agent that has the effect of modulating CFTR activity. Exemplary such agents include ataluren ("PTC124®"; 3-[5-(2-fluorophenyl)-1,2,4-oxadiazol-3-yl]benzoic acid), sinapultide, lancovutide, depelestat (a human recombinant neutrophil elastase inhibitor), cobiprostone (7-{(2R, 4aR, 5R, 7aR)-2-[(3S)-1,1-difluoro-3-methylpentyl]-2-hydroxy-6-oxooctahydrocyclopenta[b]pyran-5-yl}heptanoic acid), or (3-(6-(1-(2,2-difluorobenzo[d][1,3]dioxol-5-yl) cyclopropanccarboxamido)-3-mcthylpyridin-2-yl)bcnzoic acid. In another embodiment, the additional agent is (3-(6-(1-(2,2-difluorobenzo[d][1,3]dioxol-5-yl)cyclopropanecarboxamido)-3-methylpyridin-2-yl)benzoic acid.

In another embodiment, the additional agent is a nutritional agent. Exemplary such agents include pancrelipase (pancreating enzyme replacement), including Pancrease®, Pancreacarb®, Ultrase®, or Creon®, Liprotomase® (formerly Trizytek®), Aquadeks®, or glutathione inhalation. In one embodiment, the additional nutritional agent is pancrelipase.

The amount of additional therapeutic agent present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

A composition of the invention as disclosed herein may also be incorporated into compositions for coating an implantable medical device, such as prostheses, artificial valves, vascular grafts, stents and catheters. Accordingly, the present invention, in another aspect, includes a composition for coating an implantable device comprising a composition as disclosed herein or a pharmaceutically acceptable composition thereof, and in classes and subclasses herein, and a carrier suitable for coating said implantable device. In still another aspect, the present invention includes an implantable device coated with a composition comprising a composition as described herein or a pharmaceutically acceptable composition thereof, and a carrier suitable for coating said implantable device. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition.

In order that the invention described herein may be more fully understood, the following examples are set forth. It should be understood that these examples are for illustrative purposes only and are not to be construed as limiting this invention in any manner.

For illustrative purposes only, formulations IV.A, B and C which include the CF Modulator modulators are recited as either single component formulations, or formulations containing the combination of CF Modulator modulator component from Columns A, B, or C and an ENaC inhibitor component from Column D.

## V. METHODS OF USE

In yet another aspect, the present invention provides a method of treating a condition, disease, or disorder implicated by CFTR and/or ENaC dysfunction, the method comprising administering a pharmaceutical composition to a subject, preferably a mammal, in need thereof, the composition comprising a component from Column D (which includes an ENaC inhibitor, preferably an ENaC inhibitor that is a compound of Formula IV) and at least one component from Columns A, B, and C according to Table I. In one embodiment, the pharmaceutical composition comprises an ENaC inhibitor from Column D and a Compound of Formula I. In one embodiment, the pharmaceutical composition comprises an ENaC inhibitor from Column D and a Compound of Formula II. In one embodiment, the pharmaceutical composition comprises an ENaC inhibitor from Column D and a Compound of Formula III. In another embodiment, the pharmaceutical composition comprises an ENaC inhibitor from Column D and Compound 1. In another embodiment, the pharmaceutical composition comprises an ENaC inhibitor from Column D and Compound 2. In another embodiment, the pharmaceutical composition comprises an ENaC inhibitor from Column D and Compound 3. In a further embodiment, the pharmaceutical composition comprises an ENaC inhibitor from Column D and a Compound 1 formulation. In a further embodiment, the pharmaceutical composition comprises an ENaC inhibitor from Column D and a Compound 2 formulation. In a further embodiment, the pharmaceutical composition comprises an ENaC inhibitor from Column D and a Compound 3 formulation.

In various embodiments, the administration of the combined active agents can be performed by administering

each active agent of the combination as separate dosage units or as a single dosage unit. When administering the two active agents separately, each of the active agents can be administered concurrently, or one active agent can be administered prior to or after the other.

In certain embodiments, the present invention provides a method of treating a condition, disease, or disorder implicated by a deficiency of CFTR activity, the method comprising administering the pharmaceutical composition of the invention to a subject, preferably a mammal, in need thereof.

In yet another aspect, the present invention provides a method of treating, or lessening the severity of a condition, disease, or disorder implicated by CFTR mutation. In certain embodiments, the present invention provides a method of treating a condition, disease, or disorder implicated by a deficiency of the CFTR activity, the method comprising administering the pharmaceutical composition of the invention to a subject, preferably a mammal, in need thereof.

In another aspect, the invention also provides a method of treating or lessening the severity of a disease in a patient, the method comprising administering the pharmaceutical composition of the invention to a subject, preferably a mammal, in need thereof, and said disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's syndrome type III, Dent's disease, hyperekplexia, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

In some embodiments, the method includes treating or lessening the severity of cystic fibrosis in a patient comprising administering to said patient one of the compositions as defined herein. In certain embodiments, the patient possesses mutant forms of human CFTR. In other embodiments, the patient possesses one or more of the following mutations ΔF508, R117H, and G551D of human CFTR. In one embodiment, the method includes treating or lessening the severity of cystic fibrosis in a patient possessing the ΔF508 mutation of human CFTR comprising administering to said patient one of the compositions as defined herein. In one embodiment, the method includes treating or lessening the severity of cystic fibrosis in a patient possessing the G551D mutation of human CFTR comprising administering to said patient one of the compositions as defined herein. In one embodiment, the method includes treating or lessening the severity of cystic fibrosis in a patient possessing the ΔF508 mutation of human CFTR on at least one allele comprising administering to said patient one of the compositions as defined herein. In one embodiment, the method includes treating or lessening the severity of cystic fibrosis in a patient possessing the ΔF508 mutation of human CFTR on both alleles comprising administering to said patient one of the compositions as defined herein. In one embodiment, the method includes treating or lessening the severity of cystic fibrosis in a patient possessing the G551D mutation of human CFTR on at least one allele comprising administering to said patient one of the compositions as defined herein. In one embodiment, the method includes treating or lessening the severity of cystic fibrosis in a patient possessing the G551D mutation of human CFTR on both alleles comprising administering to said patient one of the compositions as defined herein.

In some embodiments, the method includes lessening the severity of cystic fibrosis in a patient comprising administering to said patient one of the compositions as defined herein. In certain embodiments, the patient possesses mutant forms of human CFTR. In other embodiments, the patient possesses one or more of the following mutations ΔF508, R117H, and G551D of human CFTR. In one embodiment, the method includes lessening the severity of cystic fibrosis in a patient possessing the ΔF508 mutation of human CFTR comprising administering to said patient one of the compositions as defined herein. In one embodiment, the method includes lessening the severity of cystic fibrosis in a patient possessing the G551D mutation of human CFTR comprising administering to said patient one of the compositions as defined herein. In one embodiment, the method includes lessening the severity of cystic fibrosis in a patient possessing the ΔF508 mutation of human CFTR on at least one allele comprising administering to said patient one of the compositions

as defined herein. In one embodiment, the method includes lessening the severity of cystic fibrosis in a patient possessing the ΔF508 mutation of human CFTR on both alleles comprising administering to said patient one of the compositions as defined herein. In one embodiment, the method includes lessening the severity of cystic fibrosis in a patient possessing the G551D mutation of human CFTR on at least one allele comprising administering to said patient one of the compositions as defined herein. In one embodiment, the method includes lessening the severity of cystic fibrosis in a patient possessing the G551D mutation of human CFTR on both alleles comprising administering to said patient one of the compositions as defined herein.

In some aspects, the invention provides a method of treating or lessening the severity of Osteoporosis in a patient comprising administering to said patient a composition as defined herein.

In certain embodiments, the method of treating or lessening the severity of Osteoporosis in a patient comprises administering to said patient a pharmaceutical composition as described herein.

In some aspects, the invention provides a method of treating or lessening the severity of Osteopenia in a patient comprising administering to said patient a composition as defined herein.

In certain embodiments, the method of treating or lessening the severity of Osteopenia in a patient comprises administering to said patient a pharmaceutical composition as described herein.

In some aspects, the invention provides a method of bone healing and/or bone repair in a patient comprising administering to said patient a composition as defined herein.

In certain embodiments, the method of bone healing and/or bone repair in a patient comprises administering to said patient a pharmaceutical composition as described herein.

In some aspects, the invention provides a method of reducing bone resorption in a patient comprising administering to said patient a composition as defined herein.

In some aspects, the invention provides a method of increasing bone deposition in a patient comprising administering to said patient a composition as defined herein.

In certain embodiments, the method of increasing bone deposition in a patient comprises administering to said patient a composition as defined herein.

In some aspects, the invention provides a method of treating or lessening the severity of COPD in a patient comprising administering to said patient a composition as defined herein.

In certain embodiments, the method of treating or lessening the severity of COPD in a patient comprises administering to said patient a composition as defined herein.

In some aspects, the invention provides a method of treating or lessening the severity of smoke induced COPD in a patient comprising administering to said patient a composition as defined herein.

In certain embodiments, the method of treating or lessening the severity of smoke induced COPD in a patient comprises administering to said patient a composition as defined herein.

In some aspects, the invention provides a method of treating or lessening the severity of chronic bronchitis in a patient comprising administering to said patient a composition as described herein.

In certain embodiments, the method of treating or lessening the severity of chronic bronchitis in a patient comprises administering to said patient a composition as defined herein.

According to an alternative embodiment, the present invention provides a method of treating cystic fibrosis comprising the step of administering to said mammal a composition as defined herein.

According to the invention an "effective amount" of the composition is that amount effective for treating or lessening the severity of one or more of the diseases, disorders or conditions as recited above.

Another aspect of the present invention provides a method of administering a pharmaceutical composition by orally administering to a patient at least once per day the composition as described herein. In one embodiment, the method comprises administering a composition to said patient a composition as defined herein once of Table I every 24 hours. In another embodiment, the method comprises administering to said patient a composition as defined herein every 12 hours. In a further embodiment, the method comprises administering a to said patient a composition as defined herein three times per day. In still a further embodiment, the method comprises administering to said patient a composition as defined herein.

The compositions, according to the method of the present invention, may be administered using any amount and any route of administration effective for treating or lessening the severity of one or more of the diseases, disorders or conditions as recited above.

In certain embodiments, the compositions of the present invention are useful for treating or lessening the severity of cystic fibrosis in patients who exhibit residual CFTR activity in the apical membrane of respiratory and non-respiratory epithelia. The presence of residual CFTR activity at the epithelial surface can be readily detected using methods known in the art, e.g., standard electrophysiological, biochemical, or histochemical techniques. Such methods identify CFTR activity using *in vivo* or *ex vivo* electrophysiological techniques, measurement of sweat or salivary $Cl^-$ concentrations, or *ex vivo* biochemical or histochemical techniques to monitor cell surface density. Using such methods, residual CFTR activity can be readily detected in patients heterozygous or homozygous for a variety of different mutations,

including patients homozygous or heterozygous for the most common mutation, ΔF508.

In another embodiment, the compositions of the present invention are useful for treating or lessening the severity of cystic fibrosis in patients who have residual CFTR activity induced or augmented using pharmacological methods or gene therapy. Such methods increase the amount of CFTR present at the cell surface, thereby inducing a hitherto absent CFTR activity in a patient or augmenting the existing level of residual CFTR activity in a patient.

In one embodiment, a composition as defined herein can be useful for treating or lessening the severity of cystic fibrosis in patients within certain genotypes exhibiting residual CFTR activity, e.g., class III mutations (impaired regulation or gating), class IV mutations (altered conductance), or class V mutations (reduced synthesis) (Lee R. Choo-Kang, Pamela L., Zeitlin, Type I, II, III, IV, and V cystic fibrosis. Transmembrane Conductance Regulator Defects and Opportunities of Therapy; Current Opinion in Pulmonary Medicine 6:521 - 529, 2000). Other patient genotypes that exhibit residual CFTR activity include patients homozygous for one of these classes or heterozygous with any other class of mutations, including class I mutations, class II mutations, or a mutation that lacks classification.

In one embodiment, a composition as defined herein can be useful for treating or lessening the severity of cystic fibrosis in patients within certain clinical phenotypes, e.g., a moderate to mild clinical phenotype that typically correlates with the amount of residual CFTR activity in the apical membrane of epithelia. Such phenotypes include patients exhibiting pancreatic insufficiency or patients diagnosed with idiopathic pancreatitis and congenital bilateral absence of the vas deferens, or mild lung disease.

The exact amount required will vary from subject to subject, depending on the species, age, and general condition of the subject, the severity of the infection, the particular agent, its mode of administration, and the like. The compositions of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compositions of the present invention will be decided by the attending physician within the scope of sound medical judgment. The specific effective dose level for any particular patient or organism will depend upon a variety of factors including the disorder being treated and the severity of the disorder; the activity of the composition employed; the specific composition employed; the age, body weight, general health, sex and diet of the patient; the time of administration, route of administration, and rate of excretion of the specific composition employed; the duration of the treatment; drugs used in combination or coincidental with the specific composition employed, and like factors well known in the medical arts. The term "patient", as used herein, means an animal, preferably a mammal, and most preferably a human.

## VI. ASSAYS

### A. PROTOCOL 1

Assays for Detecting and Measuring ΔF508-CFTR Potentiation Properties of Compounds

Membrane potential optical methods for assaying ΔF508-CFTR modulation properties of compounds

The assay utilizes fluorescent voltage sensing dyes to measure changes in membrane potential using a fluorescent plate reader (e.g., FLIPR III, Molecular Devices, Inc.) as a readout for increase in functional ΔF508-CFTR in NIH 3T3 cells. The driving force for the response is the creation of a chloride ion gradient in conjunction with channel activation by a single liquid addition step after the cells have previously been treated with compounds and subsequently loaded with a voltage sensing dye.

Identification of Potentiator Compounds

To identify potentiators of ΔF508-CFTR, a double-addition HTS assay format was developed. This HTS assay utilizes fluorescent voltage sensing dyes to measure changes in membrane potential on the FLIPR III as a measurement for increase in gating (conductance) of ΔF508 CFTR in temperature-corrected ΔF508 CFTR NIH 3T3 cells. The driving force for the response is a $Cl^-$ ion gradient in conjunction with channel activation with forskolin in a single liquid addition step using a fluorescent plate reader such as FLIPR III after the cells have previously been treated with potentiator compounds (or DMSO vehicle control) and subsequently loaded with a redistribution dye.

*Solution*

Bath Solution #1: (in mM) NaCl 160, KCl 4.5, $CaCl_2$ 2, $MgCl_2$ 1, HEPES 10, pH 7.4 with NaOH.

Chloride-free bath solution: Chloride salts in Bath Solution #1 (above) are substituted with gluconate salts.

*Cell Culture*

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at ~20,000/well in 384-well matrigel-coated plates and cultured for 2 hrs at 37 °C before culturing at 27 °C for 24 hrs. for the potentiator assay. For the correction assays, the cells are cultured at 27 °C or 37 °C with and without compounds for 16 - 24 hours. Electrophysiological Assays for assaying ΔF508-CFTR modulation properties of compounds.

*Ussing Chamber Assay*

Ussing chamber experiments were performed on polarized airway epithelial cells expressing ΔF508-CFTR to further characterize the ΔF508-CFTR modulators identified in the optical assays. Non-CF and CF airway epithelia were isolated from bronchial tissue, cultured as previously described (Galietta, L.J.V., Lantero, S., Gazzolo, A., Sacco, O., Romano, L., Rossi, G.A., & Zegarra-Moran, O. (1998) In Vitro Cell. Dev. Biol. 34, 478-481), and plated onto Costar® Snapwell™ filters that were precoated with NIH3T3-conditioned media. After four days the apical media was removed and the cells were grown at an air liquid interface for >14 days prior to use. This resulted in a monolayer of fully differentiated columnar cells that were ciliated, features that are characteristic of airway epithelia. Non-CF HBE were isolated from non-smokers that did not have any known lung disease. CF-HBE were isolated from patients homozygous for ΔF508-CFTR.

HBE grown on Costar® Snapwell™ cell culture inserts were mounted in an Using chamber (Physiologic Instruments, Inc., San Diego, CA), and the transepithelial resistance and short-circuit current in the presence of a basolateral to apical $Cl^-$ gradient ($I_{SC}$) were measured using a voltage-clamp system (Department of Bioengineering, University of Iowa, IA). Briefly, HBE were examined under voltage-clamp recording conditions ($V_{hold}$ = 0 mV) at 37 °C. The basolateral solution contained (in mM) 145 NaCl, 0.83 $K_2HPO_4$, 3.3 $KH_2PO_4$, 1.2 $MgCl_2$, 1.2 $CaCl_2$, 10 Glucose, 10 HEPES (pH adjusted to 7.35 with NaOH) and the apical solution contained (in mM) 145 NaGluconate, 1.2 $MgCl_2$, 1.2 $CaCl_2$, 10 glucose, 10 HEPES (pH adjusted to 7.35 with NaOH).

*Identification of Potentiator Compounds*

Typical protocol utilized a basolateral to apical membrane $Cl^-$ concentration gradient. To set up this gradient, normal ringers was used on the basolateral membrane, whereas apical NaCl was replaced by equimolar sodium gluconate (titrated to pH 7.4 with NaOH) to give a large $Cl^-$ concentration gradient across the epithelium. Forskolin (10 μM) and all test compounds were added to the apical side of the cell culture inserts. The efficacy of the putative ΔF508-CFTR potentiators was compared to that of the known potentiator, genistein.

*Patch-clamp Recordings*

Total $Cl^-$ current in ΔF508-NIH3T3 cells was monitored using the perforated-patch recording configuration as previously described (Rae, J., Cooper, K., Gates, P., & Watsky, M. (1991) J. Neurosci. Methods 37, 15-26). Voltage-clamp recordings were performed at 22 °C using an Axopatch 200B patch-clamp amplifier (Axon Instruments Inc., Foster City, CA). The pipette solution contained (in mM) 150 N-methyl-D-glucamine (NMDG)-Cl, 2 $MgCl_2$, 2 $CaCl_2$, 10 EGTA, 10 HEPES, and 240 μg/mL amphotericin-B (pH adjusted to 7.35 with HCl). The extracellular medium contained (in mM) 150 NMDG-Cl, 2 $MgCl_2$, 2 $CaCl_2$, 10 HEPES (pH adjusted to 7.35 with HCl). Pulse generation, data acquisition, and analysis were performed using a PC equipped with a Digidata 1320 A/D interface in conjunction with Clampex 8 (Axon Instruments Inc.). To activate ΔF508-CFTR, 10 μM forskolin and 20 μM genistein were added to the bath and the current-voltage relation was monitored every 30 sec.

*Identification of Potentiator Compounds*

The ability of ΔE508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I\Delta_{F508}$ with similar potency and efficacy observed in the optical assays. In all cells examined, the reversal potential before and during potentiator application was around -30 mV, which is the calculated $E_{Cl}$ (-28 mV).

*Cell Culture*

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the correction compound at 37 °C for measuring the activity of correctors.

*Single-channel recordings*

Gating activity of wt-CFTR and temperature-corrected ΔF508-CFTR expressed in NIH3T3 cells was observed using excised inside-out membrane patch recordings as previously described (Dalemans, W., Barbry, P., Champigny, G., Jallat, S., Dott, K., Dreyer, D., Crystal, R.G., Pavirani, A., Lecocq, J-P., Lazdunski, M. (1991) Nature 354, 526 - 528) using an Axopatch 200B patch-clamp amplifier (Axon Instruments Inc.). The pipette contained (in mM): 150 NMDG, 150 aspartic acid, 5 $CaCl_2$, 2 $MgCl_2$, and 10 HEPES (pH adjusted to 7.35 with Tris base). The bath contained (in mM): 150 NMDG-Cl, 2 $MgCl_2$, 5 EGTA, 10 TES, and 14 Tris base (pH adjusted to 7.35 with HCl). After excision, both wt- and ΔF508-CFTR were activated by adding 1 mM Mg-ATP, 75 nM of the catalytic subunit of cAMP-dependent protein kinase (PKA; Promega Corp. Madison, WI), and 10 mM NaF to inhibit protein phosphatases, which prevented current rundown. The pipette potential was maintained at 80 mV. Channel activity was analyzed from membrane patches containing ≤ 2 active channels. The maximum number of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o$ = I/i(N), where I = mean current, i = single-channel current amplitude, and N = number of active channels in patch.

*Cell Culture*

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

*Activity of the Compound 1*

Compounds of the invention are useful as modulators of ATP binding cassette transporters. Table 1-3 below illustrates the EC50 and relative efficacy of certain embodiments in Table 1. In Table 1-3 below, the following meanings apply. EC50: "+++" means <10 uM; "++" means between 10uM to 25 uM; "+" means between 25 uM to 60uM. % Efficacy: "+" means < 25%; "++" means between 25% to 100%; "+++" means > 100%.

| Cmpd # | EC50 (uM) | % Activity |
|---|---|---|
| 1 | +++ | ++ |

B. PROTOCOL 2

Assays for Detecting and Measuring ΔF508-CFTR Potentiation Properties of Compounds

Membrane potential optical methods for assaying ΔF508-CFTR modulation properties of compounds

The assay utilizes fluorescent voltage sensing dyes to measure changes in membrane potential using a fluorescent plate reader (e.g., FLIPR III, Molecular Devices, Inc.) as a readout for increase in functional ΔF508-CFTR in NIH 3T3 cells. The driving force for the response is the creation of a chloride ion gradient in conjunction with channel activation by a single liquid addition step after the cells have previously been treated with compounds and subsequently

loaded with a voltage sensing dye.

Identification of Potentiator Compounds

To identify potentiators of ΔF508-CFTR, a double-addition HTS assay format was developed. This HTS assay utilizes fluorescent voltage sensing dyes to measure changes in membrane potential on the FLIPR III as a measurement for increase in gating (conductance) of ΔF508 CFTR in temperature-corrected ΔF508 CFTR NIH 3T3 cells. The driving force for the response is a $Cl^-$ ion gradient in conjunction with channel activation with forskolin in a single liquid addition step using a fluorescent plate reader such as FLIPR III after the cells have previously been treated with potentiator compounds (or DMSO vehicle control) and subsequently loaded with a redistribution dye.

Solutions

Bath Solution #1: (in mM) NaCl 160, KCl 4.5, $CaCl_2$ 2, $MgCl_2$ 1, HEPES 10, pH 7.4 with NaOH.
Chloride-free bath solution: Chloride salts in Bath Solution #1 (above) are substituted with gluconate salts.

Cell Culture

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at ~20,000/well in 384-well matrigel-coated plates and cultured for 2 hrs at 37 °C before culturing at 27 °C for 24 hrs. for the potentiator assay. For the correction assays, the cells are cultured at 27 °C or 37 °C with and without compounds for 16 - 24 hours.Electrophysiological Assays for assaying ΔF508-CFTR modulation properties of compounds.

Ussing Chamber Assay

Ussing chamber experiments were performed on polarized airway epithelial cells expressing ΔF508-CFTR to further characterize the ΔF508-CFTR modulators identified in the optical assays. Non-CF and CF airway epithelia were isolated from bronchial tissue, cultured as previously described (Galietta, L.J.V., Lantero, S., Gazzolo, A., Sacco, O., Romano, L., Rossi, G.A., & Zegarra-Moran, O. (1998) In Vitro Cell. Dev. Biol. 34, 478-481), and plated onto Costar® Snapwell™ filters that were precoated with NIH3T3-conditioned media. After four days the apical media was removed and the cells were grown at an air liquid interface for >14 days prior to use. This resulted in a monolayer of fully differentiated columnar cells that were ciliated, features that are characteristic of airway epithelia. Non-CF HBE were isolated from non-smokers that did not have any known lung disease. CF-HBE were isolated from patients homozygous for ΔF508-CFTR.
HBE grown on Costar® Snapwell™ cell culture inserts were mounted in an Using chamber (Physiologic Instruments, Inc., San Diego, CA), and the transepithelial resistance and short-circuit current in the presence of a basolateral to apical $Cl^-$ gradient ($I_{SC}$) were measured using a voltage-clamp system (Department of Bioengineering, University of Iowa, IA). Briefly, HBE were examined under voltage-clamp recording conditions ($V_{hold}$ = 0 mV) at 37 °C. The basolateral solution contained (in mM) 145 NaCl, 0.83 $K_2HPO_4$, 3.3 $KH_2PO_4$, 1.2 $MgCl_2$, 1.2 $CaCl_2$, 10 Glucose, 10 HEPES (pH adjusted to 7.35 with NaOH) and the apical solution contained (in mM) 145 NaGluconate, 1.2 $MgCl_2$, 1.2 $CaCl_2$, 10 glucose, 10 HEPES (pH adjusted to 7.35 with NaOH).

Identification of Potentiator Compounds

Typical protocol utilized a basolateral to apical membrane $Cl^-$ concentration gradient. To set up this gradient, normal ringers was used on the basolateral membrane, whereas apical NaCl was replaced by equimolar sodium gluconate (titrated to pH 7.4 with NaOH) to give a large $Cl^-$ concentration gradient across the epithelium. Forskolin (10 μM) and all test compounds were added to the apical side of the cell culture inserts. The efficacy of the putative ΔF508-CFTR potentiators was compared to that of the known potentiator, genistein.

Patch-clamp Recordings

Total $Cl^-$ current in ΔF508-NIH3T3 cells was monitored using the perforated-patch recording configuration as previously described (Rae, J., Cooper, K., Gates, P., & Watsky, M. (1991) J. Neurosci. Methods 37, 15-26). Voltage-clamp recordings were performed at 22 °C using an Axopatch 200B patch-clamp amplifier (Axon Instruments Inc., Foster

City, CA). The pipette solution contained (in mM) 150 *N*-methyl-D-glucamine (NMDG)-Cl, 2 $MgCl_2$, 2 $CaCl_2$, 10 EGTA, 10 HEPES, and 240 μg/mL amphotericin-B (pH adjusted to 7.35 with HCl). The extracellular medium contained (in mM) 150 NMDG-Cl, 2 $MgCl_2$, 2 $CaCl_2$, 10 HEPES (pH adjusted to 7.35 with HCl). Pulse generation, data acquisition, and analysis were performed using a PC equipped with a Digidata 1320 A/D interface in conjunction with Clampex 8 (Axon Instruments Inc.). To activate ΔF508-CFTR, 10 μM forskolin and 20 μM genistein were added to the bath and the current-voltage relation was monitored every 30 sec.

Identification of Potentiator Compounds

The ability of ΔF508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I\Delta_{F508}$ with similar potency and efficacy observed in the optical assays. In all cells examined, the reversal potential before and during potentiator application was around -30 mV, which is the calculated $E_{Cl}$ (-28 mV).

Cell Culture

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the correction compound at 37 °C for measuring the activity of correctors.

Single-channel recordings

Gating activity of wt-CFTR and temperature-corrected ΔF508-CFTR expressed in NIH3T3 cells was observed using excised inside-out membrane patch recordings as previously described (Dalemans, W., Barbry, P., Champigny, G., Jallat, S., Dott, K., Dreyer, D., Crystal, R.G., Pavirani, A., Lecocq, J-P., Lazdunski, M. (1991) Nature 354, 526 - 528) using an Axopatch 200B patch-clamp amplifier (Axon Instruments Inc.). The pipette contained (in mM): 150 NMDG, 150 aspartic acid, 5 $CaCl_2$, 2 $MgCl_2$, and 10 HEPES (pH adjusted to 7.35 with Tris base). The bath contained (in mM): 150 NMDG-Cl, 2 $MgCl_2$, 5 EGTA, 10 TES, and 14 Tris base (pH adjusted to 7.35 with HCl). After excision, both wt- and ΔF508-CFTR were activated by adding 1 mM Mg-ATP, 75 nM of the catalytic subunit of cAMP-dependent protein kinase (PKA; Promega Corp. Madison, WI), and 10 mM NaF to inhibit protein phosphatases, which prevented current rundown. The pipette potential was maintained at 80 mV. Channel activity was analyzed from membrane patches containing ≤ 2 active channels. The maximum number of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o$ = I/i(N), where I = mean current, i = single-channel current amplitude, and N = number of active channels in patch.

Cell Culture

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

Examples: Activity of the Compounds of Formula II

Compounds of Formula II are useful as modulators of ATP binding cassette transporters. Examples of activities and efficacies of the compounds of Formula 11 are shown below in Table 2-15. The compound activity is illustrated with "+++" if activity was measured to be less than 2.0 μM, "++" if activity was measured to be from 2 μM to 5.0 μM, "+" if activity was measured to be greater than 5.0 μM, and "-" if no data was available. The efficacy is illustrated with "+++" if efficacy was calculated to be greater than 100 %, "++" if efficacy was calculated to be from 100 % to 25 %, "+" if efficacy

was calculated to be less than 25 %, and "-" if no data was available. It should be noted that 100 % efficacy is the maximum response obtained with 4-methyl-2-(5-phenyl-1H-pyrazol-3-yl)phenol.

Table 2-15

| Activities and Efficacies of the compounds of Formula II | | |
|---|---|---|
| Example Compound No. | Activity $EC_{50}$ ($\mu$m) | % Efficacy |
| 2 | +++ | ++ |
| 2-2 | +++ | ++ |
| 2-3 | +++ | ++ |
| 2-4 | +++ | ++ |
| 2-5 | +++ | +++ |
| 2-6 | +++ | +++ |
| 2-7 | +++ | ++ |
| 2-8 | +++ | ++ |
| 2-9 | +++ | ++ |
| 2-10 | +++ | +++ |
| 2-11 | +++ | ++ |
| 2-12 | +++ | ++ |
| 2-13 | +++ | ++ |
| 2-14 | +++ | ++ |

C. PROTOCOL 3

Assays for Detecting and Measuring ΔF508-CFTR Correction Properties of Compounds

Membrane potential optical methods for assaying ΔF508-CFTR modulation properties of compounds.

The optical membrane potential assay utilized voltage-sensitive FRET sensors described by Gonzalez and Tsien *(See* Gonzalez, J. E. and R. Y. Tsien (1995) "Voltage sensing by fluorescence resonance energy transfer in single cells" Biophys J 69(4): 1272-80, and Gonzalez, J. E. and R. Y. Tsien (1997) "Improved indicators of cell membrane potential that use fluorescence resonance energy transfer" Chem Biol 4(4): 269-77) in combination with instrumentation for measuring fluorescence changes such as the Voltage/Ion Probe Reader (VIPR) (See, Gonzalez, J. E., K. Oades, et al. (1999) "Cell-based assays and instrumentation for screening ion-channel targets" Drug Discov Today 4(9): 431-439).

These voltage sensitive assays are based on the change in fluorescence resonant energy transfer (FRET) between the membrane-soluble, voltage-sensitive dye, $DiSBAC_2(3)$, and a fluorescent phospholipid, CC2-DMPE, which is attached to the outer leaflet of the plasma membrane and acts as a FRET donor. Changes in membrane potential ($V_m$) cause the negatively charged $DiSBAC_2(3)$ to redistribute across the plasma membrane and the amount of energy transfer from CC2-DMPE changes accordingly. The changes in fluorescence emission were monitored using VIPR™ II, which is an integrated liquid handler and fluorescent detector designed to conduct cell-based screens in 96- or 384-well micro titer plates.

Identification of Correction Compounds

To identify small molecules that correct the trafficking defect associated with ΔF508-CFTR; a single-addition HTS assay format was developed. The cells were incubated in serum-free medium for 16 hrs at 37 °C in the presence or absence (negative control) of test compound. As a positive control, cells plated in 384-well plates were incubated for 16 hrs at 27 °C to "temperature-correct" ΔF508-CFTR. The cells were subsequently rinsed 3X with Krebs Ringers solution and loaded with the voltage-sensitive dyes. To activate ΔF508-CFTR, 10 $\mu$M forskolin and the CFTR potentiator, genistein (20 $\mu$M), were added along with $Cl^-$-free medium to each well. The addition of $Cl^-$-free medium promoted $Cl^-$ efflux in response to ΔF508-CFTR activation and the resulting membrane depolarization was optically monitored using the FRET-based voltage-sensor dyes.

Identification of Potentiator Compounds

To identify potentiators of ΔF508-CFTR, a double-addition HTS assay format was developed. During the first addition, a $Cl^-$-free medium with or without test compound was added to each well. After 22 sec, a second addition of $Cl^-$-free medium containing 2 - 10 μM forskolin was added to activate ΔF508-CFTR. The extracellular $Cl^-$ concentration following both additions was 28 mM, which promoted $Cl^-$ efflux in response to ΔF508-CFTR activation and the resulting membrane depolarization was optically monitored using the FRET-based voltage-sensor dyes.

Solutions

Bath Solution #1: (in mM) NaCl 160, KCl 4.5, $CaCl_2$ 2, MgCl, 1, HEPES 10, pH 7.4 with NaOH.
Chloride-free bath solution: Chloride salts in Bath Solution #1 (above) are substituted with gluconate salts.
CC2-DMPE: Prepared as a 10 mM stock solution in DMSO and stored at -20°C.
$DiSBAC_2(3)$: Prepared as a 10 mM stock in DMSO and stored at -20°C.

Cell Culture

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at 30,000/well in 384-well matrigel-coated plates and cultured for 2 hrs at 37 °C before culturing at 27 °C for 24 hrs for the potentiator assay. For the correction assays, the cells are cultured at 27 °C or 37 °C with and without compounds for 16 - 24 hours.

Electrophysiological Assays for assaying ΔF508-CFTR modulation properties of compounds

Ussing Chamber Assay

Using chamber experiments were performed on polarized epithelial cells expressing ΔF508-CFTR to further characterize the ΔF508-CFTR modulators identified in the optical assays. $FRT^{\Delta F508\text{-}CFTR}$ epithelial cells grown on Costar Snapwell cell culture inserts were mounted in an Ussing chamber (Physiologic Instruments, Inc., San Diego, CA), and the monolayers were continuously shortcircuited using a Voltage-clamp System (Department of Bioengineering, University of Iowa, IA, and, Physiologic Instruments, Inc., San Diego, CA). Transepithelial resistance was measured by applying a 2-mV pulse. Under these conditions, the FRT epithelia demonstrated resistances of 4 KΩ/ $cm^2$ or more. The solutions were maintained at 27 °C and bubbled with air. The electrode offset potential and fluid resistance were corrected using a cell-free insert. Under these conditions, the current reflects the flow of $Cl^-$ through ΔF508-CFTR expressed in the apical membrane. The $I_{SC}$ was digitally acquired using an MP100A-CE interface and AcqKnowledge software (ν3.2.6; BIOPAC Systems, Santa Barbara, CA).

Identification of Correction Compounds

Typical protocol utilized a basolateral to apical membrane $Cl^-$ concentration gradient. To set up this gradient, normal ringer was used on the basolateral membrane, whereas apical NaCl was replaced by equimolar sodium gluconate (titrated to pH 7.4 with NaOH) to give a large $Cl^-$ concentration gradient across the epithelium. All experiments were performed with intact monolayers. To fully activate ΔF508-CFTR, forskolin (10 μM) and the PDE inhibitor, IBMX (100 μM), were applied followed by the addition of the CFTR potentiator, genistein (50 μM).
As observed in other cell types, incubation at low temperatures of FRT cells stably expressing ΔF508-CFTR increases the functional density of CFTR in the plasma membrane. To determine the activity of correction compounds, the cells were incubated with 10 μM of the test compound for 24 hours at 37°C and were subsequently washed 3X prior to recording. The cAMP- and genistein-mediated $I_{SC}$ in compound-treated cells was normalized to the 27°C and 37°C controls and expressed as percentage activity. Preincubation of the cells with the correction compound significantly increased the cAMP- and genistein-mediated $I_{SC}$ compared to the 37°C controls.

Identification of Potentiator Compounds

Typical protocol utilized a basolateral to apical membrane $Cl^-$ concentration gradient. To set up this gradient,

normal ringers was used on the basolateral membrane and was permeabilized with nystatin (360 μg/ml), whereas apical NaCl was replaced by equimolar sodium gluconate (titrated to pH 7.4 with NaOH) to give a large $Cl^-$ concentration gradient across the epithelium. All experiments were performed 30 min after nystatin permeabilization. Forskolin (10 μM) and all test compounds were added to both sides of the cell culture inserts. The efficacy of the putative ΔF508-CFTR potentiators was compared to that of the known potentiator, genistein.

Solutions

Basolateral solution (in mM): NaCl (135), $CaCl_2$ (1.2), $MgCl_2$ (1.2), $K_2HPO_4$ (2.4), $KHPO_4$ (0.6), N-2-hydroxyethylpiperazine-N'-2-ethanesulfonic acid (HEPES) (10), and dextrose (10). The solution was titrated to pH 7.4 with NaOH.

Apical solution (in mM): Same as basolateral solution with NaCl replaced with Na Gluconate (135).

Cell Culture

Fisher rat epithelial (FRT) cells expressing ΔF508-CFTR ($FRT^{\Delta F508\text{-}CFTR}$) were used for Ussing chamber experiments for the putative ΔF508-CFTR modulators identified from our optical assays. The cells were cultured on Costar Snapwell cell culture inserts and cultured for five days at 37 °C and 5% $CO_2$ in Coon's modified Ham's F-12 medium supplemented with 5% fetal calf serum, 100 U/ml penicillin, and 100 μg/ml streptomycin. Prior to use for characterizing the potentiator activity of compounds, the cells were incubated at 27 °C for 16 - 48 hrs to correct for the ΔF508-CFTR. To determine the activity of corrections compounds, the cells were incubated at 27 °C or 37 °C with and without the compounds for 24 hours.

Whole-cell recordings

The macroscopic ΔF508-CFTR current ($I_{\Delta F508}$) in temperature- and test compound-corrected NIH3T3 cells stably expressing ΔF508-CFTR were monitored using the perforated-patch, whole-cell recording. Briefly, voltage-clamp recordings of $I_{\Delta F508}$ were performed at room temperature using an Axopatch 200B patch-clamp amplifier (Axon Instruments Inc., Foster City, CA). All recordings were acquired at a sampling frequency of 10 kHz and low-pass filtered at 1 kHz. Pipettes had a resistance of 5 - 6 MΩ when filled with the intracellular solution. Under these recording conditions, the calculated reversal potential for $Cl^-$ ($E_{Cl}$) at room temperature was -28 mV. All recordings had a seal resistance > 20 GΩ and a series resistance < 15 MΩ. Pulse generation, data acquisition, and analysis were performed using a PC equipped with a Digidata 1320 A/D interface in conjunction with Clampex 8 (Axon Instruments Inc.). The bath contained < 250 μl of saline and was continuously perfused at a rate of 2 ml/min using a gravity-driven perfusion system,

Identification of Correction Compounds

To determine the activity of correction compounds for increasing the density of functional ΔF508-CFTR in the plasma membrane, we used the above-described perforated-patch-recording techniques to measure the current density following 24-hr treatment with the correction compounds. To fully activate ΔF508-CFTR, 10 μM forskolin and 20 μM genistein were added to the cells. Under our recording conditions, the current density following 24-hr incubation at 27°C was higher than that observed following 24-hr incubation at 37 °C. These results are consistent with the known effects of low-temperature incubation on the density of ΔF508-CFTR in the plasma membrane. To determine the effects of correction compounds on CFTR current density, the cells were incubated with 10 μM of the test compound for 24 hours at 37°C and the current density was compared to the 27°C and 37°C controls (% activity). Prior to recording, the cells were washed 3X with extracellular recording medium to remove any remaining test compound. Preincubation with 10 μM of correction compounds significantly increased the cAMP- and genistein-dependent current compared to the 37°C controls.

Identification of Potentiator Compounds

The ability of ΔF508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I_{\Delta F508}$ with similar potency and efficacy observed in the optical assays. In all cells examined, the reversal potential before and during potentiator application was around -30 mV, which is the calculated $E_{Cl}$ (-28 mV).

Solutions

Intracellular solution (in mM): Cs-aspartate (90), CsCl (50), $MgCl_2$ (1), HEPES (10), and 240 μg/ml amphotericin-B (pH adjusted to 7.35 with CsOH).

Extracellular solution (in mM): N-methyl-D-glucamine (NMDG)-Cl (150), $MgCl_2$ (2), $CaCl_2$ (2), HEPES (10) (pH adjusted to 7.35 with HCl).

Cell Culture

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the correction compound at 37 °C for measuring the activity of correctors.

Single-channel recordings

The single-channel activities of temperature-corrected ΔF508-CFTR stably expressed in NIH3T3 cells and activities of potentiator compounds were observed using excised inside-out membrane patch. Briefly, voltage-clamp recordings of single-channel activity were performed at room temperature with an Axopatch 200B patch-clamp amplifier (Axon Instruments Inc.). All recordings were acquired at a sampling frequency of 10 kHz and low-pass filtered at 400 Hz. Patch pipettes were fabricated from Corning Kovar Sealing #7052 glass (World Precision Instruments, Inc., Sarasota, FL) and had a resistance of 5 - 8 MΩ, when filled with the extracellular solution. The ΔF508-CFTR was activated after excision, by adding 1 mM Mg-ATP, and 75 nM of the cAMP-dependent protein kinase, catalytic subunit (PKA; Promega Corp. Madison, WI). After channel activity stabilized, the patch was perfused using a gravity-driven microperfusion system. The inflow was placed adjacent to the patch, resulting in complete solution exchange within 1 - 2 sec. To maintain ΔF508-CFTR activity during the rapid perfusion, the nonspecific phosphatase inhibitor $F^-$ (10 mM NaF) was added to the bath solution. Under these recording conditions, channel activity remained constant throughout the duration of the patch recording (up to 60 min). Currents produced by positive charge moving from the intra- to extracellular solutions (anions moving in the opposite direction) are shown as positive currents. The pipette potential ($V_p$) was maintained at 80 mV.

Channel activity was analyzed from membrane patches containing ≤ 2 active channels. The maximum number of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o = I/i(N)$, where I = mean current, i = single-channel current amplitude, and N = number of active channels in patch.

Solutions

Extracellular solution (in mM): NMDG (150), aspartic acid (150), $CaCl_2$ (5), $MgCl_2$ (2), and HEPES (10) (pH adjusted to 7.35 with Tris base).

Intracellular solution (in mM): NMDG-Cl (150), $MgCl_2$ (2), EGTA (5), TES (10), and Tris base (14) (pH adjusted to 7.35 with HCl).

Cell Culture

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

Using the procedures described above, the activity, ($EC_{50}$), of Compound 3 has been measured and is shown in following Table.

Table

| IC50/EC50 Bins: +++ <= 2.0 < ++ <= 5.0 < + | | |
|---|---|---|
| Percent Activity Bins: + <= 25.0 < ++ <= 100.0 < +++ | | |
| Cmpd. | Binned EC50 | Binned MaxEfficacy |
| Compound 3 | +++ | +++ |

D. PROTOCOL 4

Methods for testing the combined effects of CFTR and ENaC modulators on fluid transport in cultures of CF HBE.

To test combinations of CFTR modulators and pharmacological agents that reduce epithelial sodium channel (ENaC) activity either directly or indirectly on epithelial cell fluid transport, the height of the airway surface liquid (ASL) on the apical surface of human bronchial epithelial (HBE) cells obtained from the bronchi of CF patients was measured using confocal immunofluorescent microscopy. The apical surface was washed 2 times with 300 μl absorption buffer (89 mM NaCl, 4 mM KCl, 1.2 mM $MgCl_2$, 1.2 mM $CaCl_2$, 1 mM HEPES, 16 mM Na-Gluconate, 10 mM Glucose) prewarmed to 37 °C. After the final wash, 20 μl of 10,000 Kd dextran conjugated to Alexa Fluor 488 in absorption buffer was added and allowed to equilibrate for 2 days prior to testing. To test the effect of pharmacological modulation on the ASL, CFTR modulators prepared in HBE differentiation media [Dulbeco's MEM (DMEM)/F12, Ultroser-G (2.0%; Pall Catalog #15950-017), Fetal Clone II (2%), Insulin (2.5 μg/ml), Bovine Brain Extract (0.25%; Lonza Kit#CC-4133, component#CC-4092C), Hydrocortisone (20 nM), Triodothyronine (500 nM), Transferrin (2.5 μg/ml: InVitrogen Catalog #0030124SA), Ethanolamine (250 nM), Epinephrine (1.5 μM), Phosphoethanolamine (250 nM), Retinoic acid (10 nM)] were applied to the basolateral side at desired concentration. ENaC modulators were prepared in 2000 μL of Fluorinert FC-770 (3M) at the final concentration and 100 μL of the solution was added to the apical surface. After 96 hours of treatment the ASL height was measured using a Quorum Wave FX Spinning Disc Confocal System on an Inverted Zeiss microscope and 20X objective. The images were acquired and processed using Volocity 4.0 using a suitably programmable computer (Cell Imaging Software package from Perkin Elmer, previously Improvision)

OTHER EMBODIMENTS

All publications and patents referred to in this disclosure are incorporated herein by reference to the same extent as if each individual publication or patent application were specifically and individually indicated to be incorporated by reference. Should the meaning of the terms in any of the patents or publications incorporated by reference conflict with the meaning of the terms used in this disclosure, the meaning of the terms in this disclosure are intended to be controlling. Furthermore, the foregoing discussion discloses and describes merely exemplary embodiments of the present invention. One skilled in the art will readily recognize from such discussion and from the accompanying drawings and claims, that various changes, modifications and variations can be made therein without departing from the spirit and scope of the invention as defined in the following claims.

The present invention further comprises the aspects defined in the following clauses (which form part of the present description but are not considered as claims in accordance with decision J 15/88 of Legal Board of Appeal of the European Patent Office):

1. A Pharmaceutical composition comprising:

A. an epithelial sodium channel (ENaC) inhibitor; and

B. at least one of:

I. a compound of Formula I:

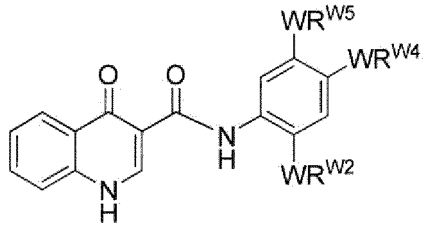

I

or a pharmaceutically acceptable salt thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is independently and optionally substituted with up to three substituents selected from -OR', $-CF_3$, $-OCF_3$, SR', S(O)R', $SO_2R'$, $-SCF_3$, halo, CN, -COOR', -COR', $-O(CH_2)_2N(R')_2$,$-O(CH_2)N(R')_2$, $-CON(R')_2$, $-(CH_2)_2OR'$, $-(CH_2)OR'$, $-CH_2CN$, optionally substituted phenyl or phenoxy, $-N(R')_2$, -NR'C(O)OR, -NR'C(O)R', $-(CH_2)_2N(R')_2$, or $-(CH_2)N(R')_2$;
$WR^{W5}$ is selected from hydrogen, $-OCF_3$, $-CF_3$, -OH, $-OCH_3$, $-NH_2$, -CN, $-CHF_2$, -NHR', $-N(R')_2$,-NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and
Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur; provided that:

i) $WR^{W2}$ and $WR^{W4}$ are not both -Cl; and
$WR^{W2}$, $WR^{W4}$ and $WR^{W5}$ are not $-OCH_2CH_2Ph$, $-OCH_2CH_2$(2-trifluoromethyl-phenyl), $-OCH_2CH_2$-(6,7-dimethoxy-1,2,3,4-tetrahydroisoquinolin-2-yl), or substituted 1*H*-pyrazol-3-yl; or

II. a compound of Formula II:

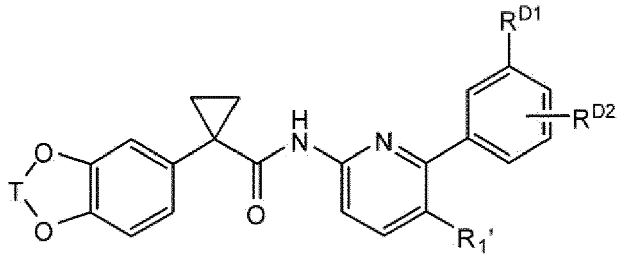

Formula II

or pharmaceutically acceptable salts thereof, wherein:

T is $-CH_2-$, $-CH_2CH_2-$, $-CF_2-$, $-C(CH_3)_2-$, or -C(O)-;
$R_1'$ is H, $C_{1-6}$ aliphatic, halo, $CF_3$, $CHF_2$, $O(C_{1-6}$ aliphatic); and
$R^{D1}$ or $R^{D2}$ is $Z^DR_9$
wherein:

$Z^D$ is a bond, CONH, $SC_2NH$, $SO_2N(C_{1-6}$ alkyl), $CH_2NHSO_2$, $CH_2N(CH_3)SO_2$, $CH_2NHCO$, COO, $SO_2$, or CO; and $R_9$ is H, $C_{1-6}$ aliphatic, or aryl; or

III. a Compound of Formula III:

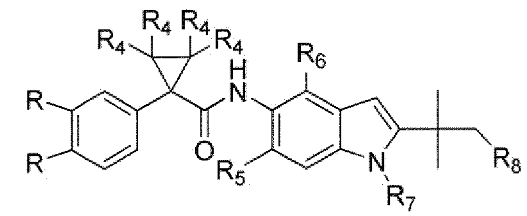

Formula III

or pharmaceutically acceptable salts thereof, wherein:

Each R is independently H, OH, $OCH_3$ or two R taken together form $-OCH_2O-$ or $-OCF_2O-$;
Each $R_4$ is independently H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

2. The pharmaceutical composition of clause 1, comprising an epithelial sodium channel inhibitor and a Compound of Formula I.

3. The pharmaceutical composition of any of clauses 1-2, wherein in the compound of Formula I each of $WR^{W2}$ and $WR^{W4}$ is independently selected from CN, $CF_3$, halo, $C_{2-6}$ straight or branched alkyl, $C_{3-12}$ membered cycloaliphatic, or phenyl, wherein said $WR^{W2}$ and $WR^{W4}$ is independently and optionally substituted with up to three substituents selected from -OR', $-CF_3$,$-OCF_3$, $-SCF_3$, halo, -COOR', -COR', $-0(CH_2)_2N(R')_2$, $-O(CH_2)N(R')_2$, $-CON(R')_2$, $-(CH_2)_2OR'$, $-(CH_2)OR'$, optionally substituted phenyl, $-N(R')_2$, -NC(O)OR', -NC(O)R', $-(CH_2)_2N(R')_2$, or$-(CH_2)N(R')_2$; and $WR^{W5}$ is selected from hydrogen, $-OCF_3$, $-CF_3$, -OH, $-OCH_3$, $-NH_2$, -CN, - NHR', $-N(R')_2$, -NHC(O)R', -NHC(O)OR', $-NHSO_2R'$, $-CH_2OH$, -C(O)OR', $-SO_2NHR'$, or$-CH_2NHC(O)O-(R')$.

4. The pharmaceutical composition of any of clauses 1-3, wherein in the compound of Formula I each of $WR^{W2}$ and $WR^{W4}$ is independently selected from -CN, $C_{2-6}$ straight or branched alkyl, $C_{3-12}$ membered cycloaliphatic, or phenyl, wherein each of said $WR^{W2}$ and $WR^{W4}$ is independently and optionally substituted with up to three substituents selected from -OR', - $CF_3$, $-OCF_3$, $-SCF_3$, halo, -COOR', -COR', $-O(CH_2)_2N(R')_2$, $-O(CH_2)N(R')_2$, $-CON(R')_2$,$-(CH_2)_2OR'$, $-(CH_2)OR'$, optionally substituted phenyl, $-N(R')_2$, -NC(O)OR', -NC(O)R',$-(CH_2)_2N(R')_2$, or $-(CH_2)N(R')_2$; and $WR^{W5}$ is selected from -OH, -CN, -NHR', $-N(R')_2$,-NHC(O)R', -NHC(O)OR', $-NHSO_2R'$, $-CH_2OH$, -C(O)OR', $-SO_2NHR'$, or $-CH_2NHC(O)O-(R')$.

5. The pharmaceutical composition of any of clauses 1-4, wherein in the compound of Formula I $WR^{W2}$ is a phenyl ring optionally substituted with up to three substituents selected from -OR', $-CF_3$, $-OCF_3$, -SR', -S(O)R', $-SO_2R'$, $-SCF_3$, halo, -CN, -COOR', -COR',$-O(CH_2)_2N(R')_2$, $-O(CH_2)N(R)_2$, $-CON(R')_2$, $-(CH_2)_2OR'$, $-(CH_2)OR'$, $-CH_2CN$, optionally substituted phenol or phenoxy, $-N(R')_2$, -NR'C(O)OR', -NR'C(O)R', $-(CH_2)_2N(R')_2$, or$-(CH_2)N(R')_2$; $WR^{W4}$ is $C_{2-6}$ straight or branched alkyl; and $WR^{W5}$ is -OH.

6. The pharmaceutical composition of any of clauses 1-5, wherein in the compound of Formula I each of $WR^{W2}$ and $WR^{W4}$ is independently $-CF_3$, -CN, or a $C_{2-6}$ straight or branched, alkyl.

7. The pharmaceutical composition of any of clauses 1-6, wherein in the compound of Formula I each of $WR^{W2}$ and $WR^{W4}$ is $C_{2-6}$ straight or branched alkyl optionally substituted with up to three substituents independently selected from -OR', $-CF_3$, $-OCF_3$, -SR', -S(O)R', $-SO_2R'$,$-SCF_3$, halo, -CN, -COOR', -COR', $-O(CH_2)_2N(R')_2$, $-O(CH_2)N(R')_2$, $-CON(R')_2$, $-(CH_2)_2OR'$,$-(CH_2)OR'$, $-CH_2CN$, optionally substituted phenyl or phenoxy, $-N(R')_2$, -NR'C(O)OR', - NR'C(O)R', $-(CH_2)_2N(R')_2$, or $-(CH_2)N(R)_2$.

8. The pharmaceutical composition of any of clauses 1-7, wherein in the compound of Formula I each of $WR^{W2}$ and $WR^{W4}$ is independently selected from optionally substituted n-propyl, isopropyl, n-butyl, sec-butyl, t-butyl, 1,1-dimethyl-2-hydroxyethyl, 1,1-dimethyl-2-(ethoxycarbonyl)-ethyl, 1,1-dimethyl-3-(t-butoxycarbonyl-amino) propyl, or n-pentyl.

9. The pharmaceutical composition of any of clauses 1-8, wherein in the compound of Formula I $WR^{W5}$ is selected from -CN, -NHR', $-N(R')_2$, $-CH_2N(R')_2$, $-NHC(O)R^7$, - NHC(O)OR', -OH, C(O)OR', or $-SO_2NHR'$.

10. The pharmaceutical composition of any of clauses 1-9, wherein in the compound of Formula I $WR^{W5}$ is selected from -CN, -NH($C_{1-6}$ alkyl), -N($C_{1-6}$ alkyl)$_2$, -NHC(O)( $C_{1-6}$ alkyl),$-CH_2NHC(O)O$($C_{1-6}$ alkyl), -NHC(O)O($C_{1-6}$ alkyl), -OH, -O($C_{1-6}$ alkyl), -C(O)O($C_{1-6}$ alkyl),$-CH_2O$($C_{1-6}$ alkyl), or $-SO_2NH_2$.

11. The pharmaceutical composition of any of clauses 1-10, wherein in the compound of Formula I $WR^{W5}$ is selected from -OH, $-CH_2OH$, -NHC(O)OMe, -NHC(O)OEt, -CN,$-CH_2NHC(O)O$(t-butyl), -C(O)OMe, or $-SO_2NH_2$.

12. The pharmaceutical composition of any of clauses 1-11, wherein in the compound of Formula I

a. $WR^{W2}$ is $C_{2-6}$ straight or branched alkyl;

b. $WR^{W4}$ is $C_{2-6}$ straight or branched alkyl or monocyclic or bicyclic aliphatic; and

c. $WR^{W5}$ is selected from -CN, -NH($C_{1-6}$ alkyl), -N($C_{1-6}$ alkyl)$_2$, -NHC(O)($C_{1-6}$ alkyl), -NHC(O)O($C_{1-6}$ alkyl), -$CH_2$C(O)O($C_{1-6}$ alkyl), -OH, -O($C_{1-6}$ alkyl), -C(O)O($C_{1-6}$ alkyl), or -$SO_2NH_2$.

13. The pharmaceutical composition of any of clauses 1-12, wherein in the compound of Formula I

a. $WR^{W2}$ is $C_{2-6}$ alkyl, -$CF_3$, -CN, or phenyl optionally substituted with up to 3 substituents selected from $C_{1-4}$ alkyl, -O($C_{1-4}$ alkyl), or halo;

b. $WR^{W4}$ is -$CF_3$, $C_{2-6}$ alkyl, or $C_{6-10}$ cycloaliphatic; and

c. $WR^{W5}$ is -OH, -NH($C_{1-6}$ alkyl), or -N($C_{1-6}$ alkyl)$_2$.

14. The pharmaceutical composition of any of clauses 1-13, wherein in the compound of Formula I $WR^{W2}$ is *tert*-butyl.

15. The pharmaceutical composition of any of clauses 1-14, wherein in the compound of Formula I $WR^{W4}$ is *tert*-butyl.

16. The pharmaceutical composition of any of clauses 1-15, wherein in the compound, of Formula I $WR^{W5}$ is -OH.

17. The pharmaceutical composition of any of clauses 1-16, wherein the compound of Formula I comprises Compound 1.

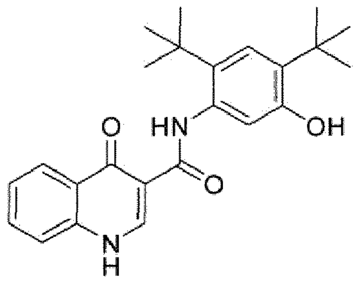

Compound 1

18. The pharmaceutical composition of clause 1, comprising an epithelial sodium channel inhibitor and a Compound of Formula II,

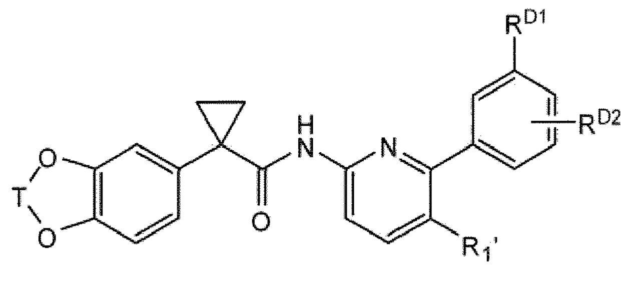

Formula II

or pharmaceutically acceptable salts thereof, wherein:

T is -$CH_2$-, -$CH_2CH_2$-, -$CF_2$-, -C($CH_3$)$_2$-, or -C(O)-; $R_1$' is H, $C_{1-6}$ aliphatic, halo, $CF_3$, $CHF_2$, O($C_{1-6}$ aliphatic); and $R^{D1}$ or $R^{D2}$ is $Z^DR_9$ wherein:

$Z^D$ is a bond, CONH, $SO_2NH$, $SO_2N$($C_{1-6}$ alkyl), $CH_2NHSO_2$, $CH_2N(CH_3)SO_2$, $CH_2NHCO$, COO, $SO_2$, or CO; and $R_9$, is H, $C_{1-6}$ aliphatic, or aryl.

19. The pharmaceutical composition of clause 18, wherein the Compound of Formula II comprises Compound 2.

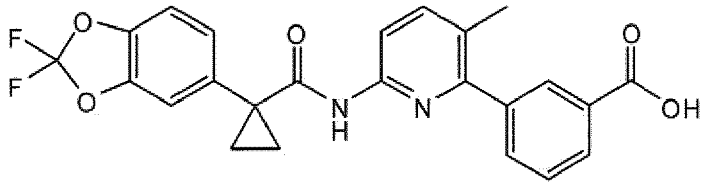

Compound 2

20. The pharmaceutical composition of clause 1, comprising an epithelial sodium channel inhibitor and a Compound of Formula III,

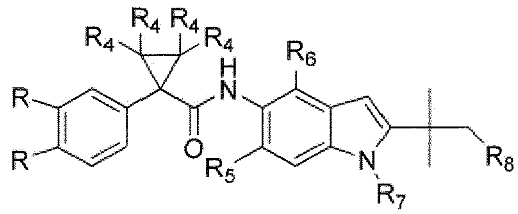

Formula III

or pharmaceutically acceptable salts thereof, wherein:

Each R is independently H, OH, $OCH_3$ or two R taken together form $-OCH_2O-$ or $-OCF_2O-$;
Each $R_4$ is independently H or alkyl;
$R_5$ is H or F;
$R_6$ is H or CN;
$R_7$ is H, $-CH_2CH(OH)CH_2OH$, $-CH_2CH_2N^+(CH_3)_3$, or-$CH_2CH_2OH$;
$R_8$ is H, OH, $-CH_2CH(OH)CH_2OH$, $-CH_2OH$, or $R_7$ and $R_8$ taken together form a five membered ring.

21. The pharmaceutical composition of clause 20, wherein the Compound of Formula III comprises Compound 3.

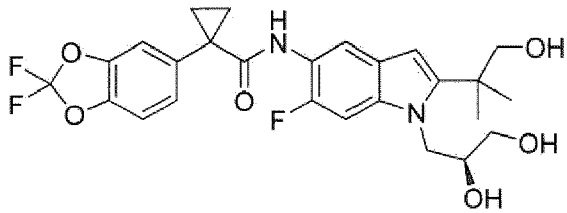

Compound 3

22. The pharmaceutical composition of any one of clauses 1 to 21, wherein the ENaC inhibitor is selected from amiloride, benzamil, dimethyl-amiloride, an ENaC inhibitor compound from PCT/EP2006/003387; PCT/EP2006/012314 and PCT/EP2006/012320, one compound of Formula IV, or a pharmaceutically acceptable salt, enantiomer and tautomer thereof.

23. The pharmaceutical composition of clause 22, wherein the ENaC inhibitor is amiloride.

24. A pharmaceutical composition comprising at least one of Compound 1, Compound 2, or Compound 3 and at least one compound of Formula IV,

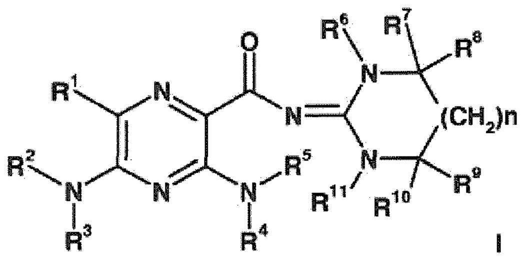

Formula IV

or pharmaceutically acceptable salts thereof, wherein:

$R^6$, $R^7$, $R^8$, $R^9$, $R^{10}$ and $R^{11}$ are each independently selected from H; $SO_2R^{16}$; aryl optionally substituted by one or more Z groups; a $C_3$-$C_{10}$ carbocyclic group optionally substituted by one or more Z groups; $C_3$-$C_{14}$ heterocyclic, group optionally substituted by one or more Z groups; $C_1$-$C_8$ alkyl optionally substituted by an aryl group, a $C_3$-$C_{10}$carbocyclic group optionally substituted by one or more Z groups or a $C_3$-$C_{14}$ heterocyclic group optionally substituted by one or more Z groups.

25. A pharmaceutical composition comprising at least one component from Columns A, B and C of Table I, and at least one component from Column D of Table I.

| **Column A Embodiments** | | **Column B Embodiments** | | **Column C Embodiments** | | **Column D Embodiments** | |
|---|---|---|---|---|---|---|---|
| **Section** | | **Section** | **Heading** | **Section** | **Heading** | **Section** | **Heading** |
| **II.A.1.** | Compound of Formula I | **II.B.1.** | Compound of Formula **II** | **II.C.1.** | Compound of Formula **III** | **II.D.1.** | ENaC Compounds |
| | | | | | | | |
| **II.A.2.** | Compound 1 | **II.B.2.** | Compound 2 | **II.C.2.** | Compound 3 | **II.D.2** | ENaC Compounds of Formula IV |

26. The pharmaceutical composition of clause 25, wherein the Column A component is Compound 1, the Column B component is Compound the Column C component is Compounds 3 and the Column D component is Compound of Formula IV.
27. The Pharmaceutical composition according to any of clauses 25-26, comprising Compound 1 and a Compound of Formula IV.
28. The pharmaceutical composition according to any of clauses 25-27, comprising Compound 2 and a Compound of Formula IV.
29. The pharmaceutical composition according to any of clauses 25-28, comprising Compound 3 and a Compound of Formula IV.
30. A method of treating a CFTR mediated disease in a human comprising administering to the human an effective amount of a pharmaceutical composition according to any of clauses 1-29.
31. The method of clause 30, wherein the CFTR mediated disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency. Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, potyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzabacher disease, neurodegenerative diseases such as Alzheimer's disease, Parkinson's disease, amyotrophic lateral sclerosis, progressive supranuclear palsy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's syndrome type III, Dent's disease, hyperekplexia, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

32. The method of any of clauses 30-31, wherein the CFTR mediated disease is cystic fibrosis, COPD, emphysema, or osteoporosis.

33. The method of any of clauses 30-32, wherein the CFTR mediated disease is cystic fibrosis.

34. The method according to any of clauses 30-33, wherein the patient possesses one or more of the following mutations of human CFTR: ΔF508, R117H, and G551D.

35. The method according to any of clauses 30-34, wherein the method includes treating or lessening the severity of cystic fibrosis in a patient possessing the ΔF508 mutation of human CFTR.

36. The method according to any of clauses 30-35, wherein the method includes treating or lessening the severity of cystic fibrosis in a patient possessing the G551D mutation of human CFTR.

37. The method according to any of clauses 30-36, wherein the method includes treating or lessening the severity of cystic fibrosis in a patient possessing the ΔF508 mutation of human CFTR on at least one allele.

38. The method according to any of clauses 30-37, wherein the method includes treating or lessening the severity of cystic fibrosis in a patient possessing the ΔF508 mutation of human CFTR on both alleles.

39. The method according to any of clauses 30-38, wherein the method includes treating or lessening the severity of cystic fibrosis in a patient possessing the G551D mutation of human CFTR on at least one allele.

40. The method according to any of clauses 30-39, wherein the method includes treating or lessening the severity of cystic fibrosis in a patient possessing the G551D mutation of human CFTR. on both alleles.

41. A kit comprising a pharmaceutical composition comprising a compound from at least one of Columns A B, and C, and at least one compound from Column D according to Table 1.

42. The kit of clause 41, wherein the kit comprises Compound 1 and a Compound of Formula IV.

43. The of clause 42, wherein the kit comprises Compound 2 and a Compound of Formula IV.

44. The kit of clause 43, wherein the kit comprises Compound 3 and a Compound of Formula IV.

## Claims

**1.** A pharmaceutical composition comprising:

A. an epithelial sodium channel (ENaC) inhibitor; and

B. at least one of:

I. a compound of Formula I:

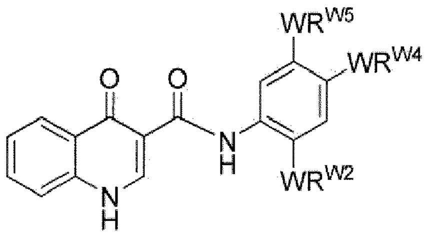

I

or a pharmaceutically acceptable salt thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is independently and optionally substituted with up to three substituents selected from -OR, $-CF_3$, $-OCF_3$, SR', S(O)R', $SO_2R'$, $-SCF_3$, halo, CN, -COOR',-COR', $-O(CH_2)_2N(R')_2$, $-O(CH_2)N(R')_2$, $-CON(R')_2$,
$-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$;
$WR^{W5}$ is selected from hydrogen, $-OCF_3$ $-CF_3$, -OH, $-OCH_3$, $-NH_2$, -CN, $-CHF_2$, -NHR', $-N(R')_2$,-NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and
Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R are taken together with the atom(s) to which they are bound to form an optionally substituted 3-12 membered saturated,
partially unsaturated, or fully unsaturated monocyclic or bicyclic ring having 0-4 heteroatoms independently selected from nitrogen, oxygen, or sulfur;
provided that:

i) $WR^{W2}$ and $WR^{W4}$ are not both -Cl; and
$WR^{W2}$, $WR^{W4}$ and $WR^{W5}$ are not $-OCH_2CH_2Ph$, $-OCH_2CH_2$(2-trifluoromethyl-phenyl), $-OCH_2CH_2$-(6,7-dimethoxy-1,2,3,4-tetrahydroisoquinolin-2-yl), or substituted 1*H*-pyrazol-3-yl; or

II. a compound of Formula II:

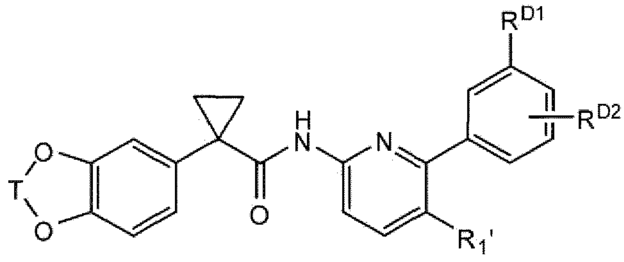

Formula II

or pharmaceutically acceptable salts thereof, wherein:

T is $-CH_2-$, $-CH_2CH_2-$, $-CF_2-$, $-C(CH_3)_2-$, or -C(O)-;
$R_1$' is H, $C_{1-6}$ aliphatic, halo, $CF_3$, $CHF_2$, O($C_{1-6}$ aliphatic); and
$R^{D1}$ or $R^{D2}$ is $Z^DR_9$
wherein:

$Z^D$ is a bond, CONH, $SO_2NH$, $SO_2N(C_{1-6}$ alkyl), $CH_2NHSO_2$, $CH_2N(CH_3)SO_2$, $CH_2NHCO$, COO, $SO_2$,
or CO; and $R_9$ is H, $C_{1-6}$ aliphatic, or aryl; or

III. a Compound of Formula III:

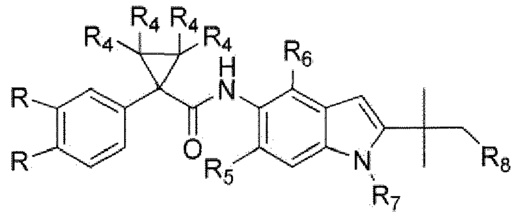

Formula III

or pharmaceutically acceptable salts thereof, wherein:

Each R is independently H, OH, $OCH_3$ or two R taken together form $-OCH_2O-$ or $-OCF_2O-$;
Each $R_4$ is independently H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

**2.** The pharmaceutical composition of claim 1, comprising an epithelial sodium channel inhibitor and a Compound of Formula I.

**3.** The pharmaceutical composition of any of claims 1-2, wherein in the compound of Formula I each of $WR^{W2}$ and $WR^{W4}$ is independently selected from CN, $CF_3$, halo, $C_{2-6}$ straight or branched alkyl, $C_{3-12}$ membered cycloaliphatic, or phenyl, wherein said $WR^{W2}$ and $WR^{W4}$ is independently and optionally, substituted with up to three substituents selected from-OR', $-CF_3$, $-OCF_3$, $-SCF_3$, halo, -COOR', -COR', $-O(CH_2)_2N(R')_2$, $-O(CH_2)N(R')_2$, $-CON(R')_2$, $-(CH_2)_2OR'$, $-(CH_2)OR'$, optionally substituted phenyl, $-N(R')_2$, -NC(O)OR',-NC(O)R', $-(CH_2)_2N(R')_2$, or $-(CH_2)N(R')_2$; and $WR^{W5}$ is selected from hydrogen, $-OCF_3$, $-CF_3$, -OH, $-OCH_3$, $-NH_2$, -CN, -NHR', $-N(R')_2$, -NHC(O)R', -NHC(O)OR', $-NHSO_2R'$, $-CH_2OH$, -C(O)OR', $-SO_2NHR'$, or $-CH_2NHC(O)O-(R')$.

**4.** The pharmaceutical composition of any of claims 1-3, wherein in the compound of Formula I each of $WR^{W2}$ and $WR^{W4}$ is $C_{2-6}$ straight or branched alkyl optionally substituted with up to three substituents independently selected from -OR', $-CF_3$, $-OCF_3$, -SR', -S(O)R', $-SO_2R'$, $-SCF_3$, halo, -CN, -COOR', -COR', $-O(CH_2)_2N(R')_2$, $-O(CH_2)N(R')_2$, $-CON(R')_2$, $-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$, in particular.

**5.** The pharmaceutical composition of any of claims 1-4, wherein in the compound of Formula I $WR^{W5}$ is selected from -NHR', $-N(R')_2$, $-CH_2N(R')_2$, -NHC(O)R',-NHC(O)OR', -OH, C(O)OR', or $-SO_2NHR'$-, or

**6.** The Pharmaceutical composition of any of claims 1-5, wherein in the compound of Formula I

a. $WR^{W2}$ is $C_{2-6}$ straight or branched alkyl, in particular *tert*-butyl,;
b. $WR^{W4}$ is $C_{2-6}$ straight or branched alkyl, in particular *tert*-butyl, or monocyclic or bicyclic aliphatic; and
c. $WR^{W5}$ is selected from -CN, -NH($C_{1-6}$ alkyl), -N($C_{1-6}$ alkyl)$_2$, -NHC(O)($C_{1-6}$ alkyl), -NHC(O)O($C_{1-6}$ alkyl), $-CH_2C(O)O$($C_{1-6}$ alkyl), -OH, -O($C_{1-6}$ alkyl), -C(O)O($C_{1-6}$ alkyl), or $-SO_2NH_2$.

**7.** The pharmaceutical composition of any of claims 1-6, wherein in the compound of Formula I

a. $WR^{W2}$ is $C_{2-6}$ alkyl, in particular *tert*-butyl, $-CF_3$, -CN, or phenyl optionally substituted with up to 3 substituents selected from $C_{1-4}$ alkyl, -O($C_{1-4}$ alkyl), or halo;
b. $WR^{W4}$ is $-CF_3$, $C_{2-6}$ alkyl, in particular *tert*-butyl, or cycloaliphatic; and
c. $WR^{W5}$ is -OH, -NH($C_{1-6}$ alkyl), or -N($C_{1-6}$ alkyl)$_2$.

**8.** The pharmaceutical composition of any of claims 1-7, wherein the compound of Formula I comprises (Compound 1.

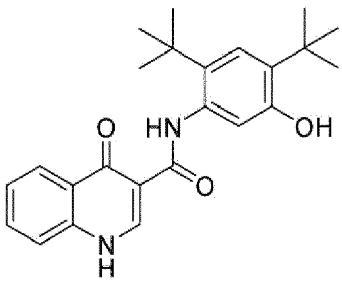

Compound 1

**9.** The pharmaceutical composition of claim 1, comprising an epithelial sodium channel inhibitor and a Compound of Formula II,

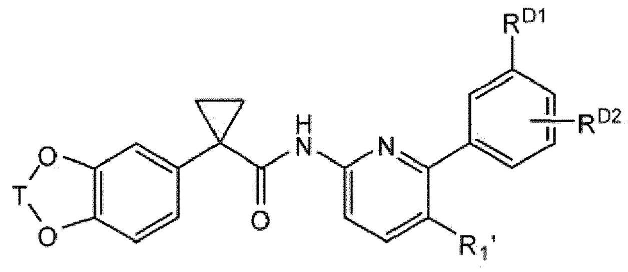

Formula II

or pharmaceutically acceptable salts thereof, wherein:

T is $-CH_2-$, $-CH_2CH_2-$, $-CF_2-$, $-C(CH_3)_2-$, or -C(O)-; $R_1$' is H, $C_{1-6}$ aliphatic, halo, $CF_3$. $CHF_2$, O($C_{1-6}$ aliphatic); and $R^{D1}$ or $R^{D2}$ is $Z^DR_9$ wherein:
$Z^D$ is a bound, CONH, $SO_2N$, $SO_2N$($C_{1-6}$ alkyl), $CH_2NHSO_2$, $CH_2N(CH_3)SO_2$, $CH_2NHCO$, COO, $SO_2$, or CO; and $R_9$ is H, $C_{1-6}$ aliphatic, or acryl.

**10.** The pharmaceutical composition of claim 9, wherein the Compound of Formula II comprises Compound 2.

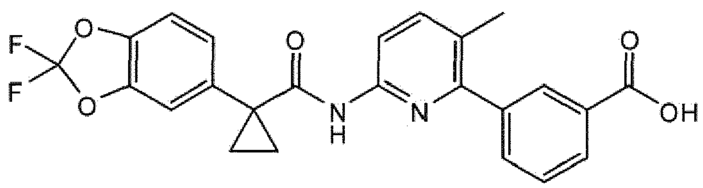

Compound 2

**11.** The pharmaceutical composition of claim 1, comprising an epithelial sodium channel inhibitor and a Compound of Formula III,

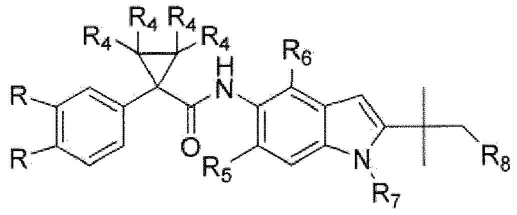

Formula III

or pharmaceutically acceptable salts thereof, wherein:

Each R is independently H, OH, $OCH_3$ or two R taken together form $-OCH_2O-$ or $-OCF_2O-$;
Each $R_4$ is independently H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

**12.** The pharmaceutical composition of claim 11, wherein the Compound of Formula III comprises Compound 3.

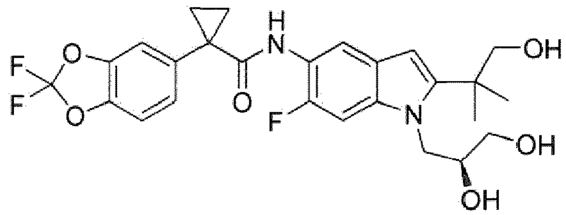

Compound 3

**13.** The pharmaceutical composition of any one of claims 1 to 12, wherein the ENaC inhibitor is selected from amiloride, benzamil, dimethyl-amiloride, an ENaC inhibitor compound from PCT/EP2006/003387; PCT/EP2006/012314 and PCT/EP2006/012320, one compound of Formula IV, or a pharmaceutically acceptable salt, enantiomer and tautomer thereof.

**14.** The Pharmaceutical composition of claim 13, wherein the ENaC inhibitor is amiloride.

**15.** A pharmaceutical composition comprising at least one of Compound 1, Compound 2, or Compound 3 and at least one compound of Formula IV,

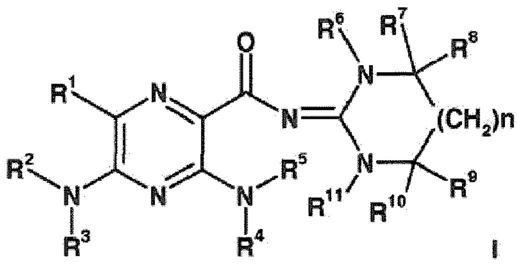

Formula IV

or pharmaceutically acceptable salts thereof, wherein:

$R^6$, $R^7$, $R^8$, $R^9$, $R^{10}$ and $R^{11}$ are each independently selected from H; $SO_2R^{16}$; aryl optionally substituted by one or more Z groups; a $C_3$-$C_{10}$ carbocyclic group optionally substituted by one or more Z groups; $C_3$-$C_{14}$

heterocyclic group optionally substituted by one or more Z groups; $C_1$-$C_8$ alkyl optionally substituted by an aryl group, a $C_3$-$C_{10}$ carbocyclic group optionally substituted by one or more Z groups or a $C_3$-$C_{14}$ heterocyclic, group optionally substituted by one or more Z groups.

**16.** A pharmaceutical composition comprising at least one component from Columns A, B and C of Table I, and at least one component from Column D of Table **I**.

| Column A Embodiments | | Column B Embodiments | | Column C Embodiments | | Column D Embodiments | |
|---|---|---|---|---|---|---|---|
| **Section** | | **Section** | **Heading** | **Section** | **Heading** | **Section** | **Heading** |
| **II.A.1.** | Compound of Formula I | **II.B.1.** | Compound of Formula **II** | **II.C.1.** | Compound of Formula III | **II.D.1.** | ENaC Compounds |
| | | | | | | | |
| **II.A.2.** | Compound 1 | **II.B.2.** | Compound 2 | **II.C.2.** | Compound 3 | **II.D.2** | ENaC Compounds of Formula IV |

**17.** The pharmaceutical composition according to any of claims 16, comprising Compound 1 and a Compound of Formula IV.

**18.** A pharmaceutical composition according to any of claims 1-17 for use in treating a CFTR mediated disease in a human

**19.** The composition for use of claim 18, wherein the CFTR mediated disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as **I**-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer’s disease, Parkinson’s disease, amyotrophic lateral sclerosis, progressive supranuclear palsy, Pick’s disease, several polyglutamine neurological disorders such as Huntington’s, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren’s disease, Osteoporosis, Osteopenia, bone healing and bone growth (including bone repair, bone regeneration, reducing bone resorption and increasing bone deposition), Gorham’s Syndrome, chloride channelopathies such as myotonia congenital (Thomson and Becker forms), Bartter’s syndrome type III, Dent’s disease, hyperekplexia, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aphasia.

**20.** The composition for use according to any of claims 18-19, wherein the patient possesses one or more of the following mutations of human CFTR: ΔF508, R117H, and G551D.

**21.** The composition for use according to any of claims 19-20, wherein the use includes treating or lessening the severity of cystic fibrosis in a patient possessing the ΔF508 mutation of human CFTR on both alleles.

**22.** A kit comprising a pharmaceutical composition comprising a compound from at least one of Columns A B, and C, and at least one compound from Column D according to Table I.

Europäisches Patentamt
European Patent Office
Office européen des brevets

# EUROPEAN SEARCH REPORT

Application Number
EP 14 18 4159

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 4 501 729 A (BOUCHER RICHARD C [US] ET AL) 26 February 1985 (1985-02-26)<br>* claim 5 * | 1-22 | INV.<br>A61K31/404<br>A61K31/443<br>A61K31/4965<br>A61K31/501<br>A61K45/06<br>A61P11/00<br>A61P19/10<br>A61P43/00 |
| Y | US 5 876 700 A (BOUCHER JR RICHARD C [US] ET AL) 2 March 1999 (1999-03-02)<br>* column 1, line 57 - line 67 *<br>* claim 7 * | 1-22 | |
| Y | WO 2006/002421 A2 (VERTEX PHARMA [US]; HADIDA RUAH SARAH S [US]; HAZLEWOOD ANNA R [US]; G) 5 January 2006 (2006-01-05)<br>* page 92, paragraph 253 - page 93; claims 21-31, 72 * | 1-8, 13-22 | |
| Y | WO 2007/134279 A2 (VERTEX PHARMA [US]; YOUNG CHRISTOPHER R [US]; ROWE CHARLES WILLIAM [US) 22 November 2007 (2007-11-22)<br>* claims 1, 21-23 * | 1-8, 13-22 | |
| Y | WO 2009/073757 A1 (VERTEX PHARMA [US]; KESHAVARZ-SHOKRI ALI [US]; ZHANG BEILI [US]; KRAWI) 11 June 2009 (2009-06-11)<br>* page 3, paragraph 12 *<br>* page 4, paragraph 13 *<br>* page 7, paragraph 56 * | 1,9,10, 13-22 | TECHNICAL FIELDS SEARCHED (IPC)<br>A61K |
| Y | US 2009/131492 A1 (RUAH SARA S HADIDA [US] ET AL HADIDA RUAH SARA S [US] ET AL) 21 May 2009 (2009-05-21)<br>* page 85; example 315; table 1 *<br>* page 111, paragraph 279 *<br>* claim 61 * | 1,11-22 | |

-/--

1

The present search report has been drawn up for all claims

| Place of search | Date of completion of the search | Examiner |
|---|---|---|
| Munich | 30 October 2014 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 4159

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2009/074575 A2 (NOVARTIS AG [CH]; BHALAY GURDIP [GB]; BUDD EMMA [GB]; BLOOMFIELD GRAHA) 18 June 2009 (2009-06-18) * page 59, last paragraph * * page 60, line 1 - line 2; claims 1, 13 * ----- | 1-22 | |

TECHNICAL FIELDS SEARCHED (IPC)

The present search report has been drawn up for all claims

1

| Place of search | Date of completion of the search | Examiner |
|---|---|---|
| Munich | 30 October 2014 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 18 4159

This annex lists the patent family members relating to the patent documents cited in the above-mentioned European search report. The members are as contained in the European Patent Office EDP file on
The European Patent Office is in no way liable for these particulars which are merely given for the purpose of information.

30-10-2014

| Patent document cited in search report | Publication date | Patent family member(s) | Publication date |
|---|---|---|---|
| US 4501729 A | 26-02-1985 | CA 1209918 A1 | 19-08-1986 |
| | | US 4501729 A | 26-02-1985 |
| US 5876700 A | 02-03-1999 | AU 4420496 A | 03-07-1996 |
| | | CA 2207585 A1 | 20-06-1996 |
| | | EP 0793483 A1 | 10-09-1997 |
| | | US 5656256 A | 12-08-1997 |
| | | US 5876700 A | 02-03-1999 |
| | | WO 9618385 A1 | 20-06-1996 |
| WO 2006002421 A2 | 05-01-2006 | AU 2005258320 A1 | 05-01-2006 |
| | | AU 2010249302 A1 | 06-01-2011 |
| | | AU 2010251787 A1 | 06-01-2011 |
| | | AU 2010251789 A1 | 06-01-2011 |
| | | BR PI0511321 A | 31-07-2007 |
| | | CA 2571949 A1 | 05-01-2006 |
| | | CA 2810655 A1 | 05-01-2006 |
| | | CN 101006076 A | 25-07-2007 |
| | | CN 101891680 A | 24-11-2010 |
| | | CN 101935301 A | 05-01-2011 |
| | | CN 103641765 A | 19-03-2014 |
| | | EP 1773816 A2 | 18-04-2007 |
| | | EP 2489659 A1 | 22-08-2012 |
| | | EP 2502902 A2 | 26-09-2012 |
| | | EP 2522659 A2 | 14-11-2012 |
| | | EP 2530075 A2 | 05-12-2012 |
| | | EP 2532650 A2 | 12-12-2012 |
| | | HK 1105970 A1 | 29-07-2011 |
| | | HK 1152707 A1 | 11-07-2014 |
| | | IL 180224 A | 31-10-2012 |
| | | JP 4947658 B2 | 06-06-2012 |
| | | JP 2008504291 A | 14-02-2008 |
| | | JP 2012056962 A | 22-03-2012 |
| | | JP 2012056963 A | 22-03-2012 |
| | | JP 2012062319 A | 29-03-2012 |
| | | JP 2012107069 A | 07-06-2012 |
| | | JP 2014156468 A | 28-08-2014 |
| | | NZ 552543 A | 30-09-2010 |
| | | NZ 587547 A | 28-09-2012 |
| | | NZ 587548 A | 25-05-2012 |
| | | NZ 587549 A | 26-10-2012 |
| | | NZ 587551 A | 12-01-2012 |
| | | NZ 598393 A | 30-05-2014 |
| | | RU 2382779 C2 | 27-02-2010 |
| | | US 2006074075 A1 | 06-04-2006 |
| | | US 2008071095 A1 | 20-03-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 18 4159

This annex lists the patent family members relating to the patent documents cited in the above-mentioned European search report. The members are as contained in the European Patent Office EDP file on
The European Patent Office is in no way liable for these particulars which are merely given for the purpose of information.

30-10-2014

| Patent document cited in search report | Publication date | Patent family member(s) | Publication date |
|---|---|---|---|
| | | US 2009227797 A1 | 10-09-2009 |
| | | US 2009298876 A1 | 03-12-2009 |
| | | US 2013035327 A1 | 07-02-2013 |
| | | US 2013331567 A1 | 12-12-2013 |
| | | US 2014051724 A1 | 20-02-2014 |
| | | US 2014155431 A1 | 05-06-2014 |
| | | US 2014163011 A1 | 12-06-2014 |
| | | WO 2006002421 A2 | 05-01-2006 |
| | | ZA 200700601 A | 26-11-2008 |
| WO 2007134279 A2 | 22-11-2007 | AT 534383 T | 15-12-2011 |
| | | AU 2007249269 A1 | 22-11-2007 |
| | | CA 2652072 A1 | 22-11-2007 |
| | | CN 101478964 A | 08-07-2009 |
| | | EP 2021797 A2 | 11-02-2009 |
| | | ES 2377840 T3 | 02-04-2012 |
| | | HK 1129581 A1 | 07-12-2012 |
| | | JP 2009536969 A | 22-10-2009 |
| | | US 2008090864 A1 | 17-04-2008 |
| | | US 2010069434 A1 | 18-03-2010 |
| | | US 2012122922 A1 | 17-05-2012 |
| | | WO 2007134279 A2 | 22-11-2007 |
| WO 2009073757 A1 | 11-06-2009 | AU 2008333845 A1 | 11-06-2009 |
| | | CA 2706920 A1 | 11-06-2009 |
| | | CN 101910156 A | 08-12-2010 |
| | | CN 103626744 A | 12-03-2014 |
| | | EA 201070698 A1 | 28-02-2011 |
| | | EP 2225230 A1 | 08-09-2010 |
| | | HK 1146819 A1 | 05-09-2014 |
| | | JP 2011506330 A | 03-03-2011 |
| | | JP 2013253111 A | 19-12-2013 |
| | | JP 2014088447 A | 15-05-2014 |
| | | KR 20100101130 A | 16-09-2010 |
| | | NZ 585880 A | 31-08-2012 |
| | | NZ 599889 A | 27-09-2013 |
| | | SG 186638 A1 | 30-01-2013 |
| | | US 2009170905 A1 | 02-07-2009 |
| | | US 2012277268 A1 | 01-11-2012 |
| | | US 2014011846 A1 | 09-01-2014 |
| | | WO 2009073757 A1 | 11-06-2009 |
| US 2009131492 A1 | 21-05-2009 | US 2009131492 A1 | 21-05-2009 |
| | | US 2010113555 A1 | 06-05-2010 |
| | | US 2011060024 A1 | 10-03-2011 |
| | | US 2011071206 A1 | 24-03-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 18 4159

This annex lists the patent family members relating to the patent documents cited in the above-mentioned European search report. The members are as contained in the European Patent Office EDP file on
The European Patent Office is in no way liable for these particulars which are merely given for the purpose of information.

30-10-2014

| Patent document cited in search report | Publication date | Patent family member(s) | Publication date |
|---|---|---|---|
| | | US 2013178471 A1 | 11-07-2013 |
| | | US 2014080826 A1 | 20-03-2014 |
| WO 2009074575 A2 | 18-06-2009 | AR 069637 A1 | 10-02-2010 |
| | | AU 2008334629 A1 | 18-06-2009 |
| | | CA 2707857 A1 | 18-06-2009 |
| | | CN 101939054 A | 05-01-2011 |
| | | CO 6310968 A2 | 22-08-2011 |
| | | CR 11470 A | 09-07-2010 |
| | | EA 201000886 A1 | 28-02-2011 |
| | | EC SP10010242 A | 31-08-2010 |
| | | EP 2231280 A2 | 29-09-2010 |
| | | EP 2444120 A1 | 25-04-2012 |
| | | EP 2520574 A1 | 07-11-2012 |
| | | HN 2010001165 A | 12-11-2012 |
| | | JP 5455922 B2 | 26-03-2014 |
| | | JP 2011506386 A | 03-03-2011 |
| | | KR 20100098682 A | 08-09-2010 |
| | | MA 31894 B1 | 01-12-2010 |
| | | NZ 585789 A | 30-03-2012 |
| | | PE 10962009 A1 | 25-08-2009 |
| | | TW 200930717 A | 16-07-2009 |
| | | US 2010130506 A1 | 27-05-2010 |
| | | US 2011237572 A1 | 29-09-2011 |
| | | US 2013012500 A1 | 10-01-2013 |
| | | US 2014171421 A1 | 19-06-2014 |
| | | WO 2009074575 A2 | 18-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61254180 A
- WO 2006002421 A
- WO 2005075435 A
- US 7741321 B
- US 7645789 B
- US 7495103 B
- US 7776905 B
- US 7659268 B
- US 20070244159 A1
- US 20080113985 A1
- US 20080019915 A1
- US 20080306062 A1
- US 20060074075 A1
- US 20090131492 A1
- US 20040006237 A
- US 20100184739 A
- US 114935 A
- US 20090131492 A
- EP 2008067110 W
- EP 2006003387 W
- EP 2006012314 W
- EP 2006012320 W
- WO 2007021982 A
- WO 2006099256 A
- WO 2006127588 A
- WO 2004080972 A
- WO 2005026137 A
- WO 2005035514 A
- WO 2004111014 A
- WO 2006101740 A
- WO 2004110352 A
- WO 2005120497 A
- US 20050176761 A
- US 6166037 A
- WO 0066558 A
- WO 0066559 A
- WO 04018425 A
- WO 04026873 A
- WO 0288167 A
- WO 0212266 A
- WO 02100879 A
- WO 0200679 A
- WO 0335668 A
- WO 0348181 A
- WO 0362259 A
- WO 0364445 A
- WO 0372592 A
- WO 0439827 A
- WO 0466920 A
- DE 10261874
- WO 0000531 A
- WO 0210143 A
- WO 0382280 A
- WO 0382787 A
- WO 0386294 A
- WO 03104195 A
- WO 03101932 A
- WO 0405229 A
- WO 0418429 A
- WO 0419935 A
- WO 0426248 A
- WO 9219594 A
- WO 9319749 A
- WO 9319750 A
- WO 9319751 A
- WO 9818796 A
- WO 9916766 A
- WO 0113953 A
- WO 03104204 A
- WO 03104205 A
- WO 0339544 A
- WO 04000814 A
- WO 04000839 A
- WO 04005258 A
- WO 04018450 A
- WO 04018451 A
- WO 04018457 A
- WO 04018465 A
- WO 04018431 A
- WO 04018449 A
- WO 04019944 A
- WO 04019945 A
- WO 04045607 A
- WO 04037805 A
- WO 0242298 A
- WO 0075114 A
- WO 0416601 A
- EP 1440966 A
- JP 05025045 B
- WO 9318007 A
- WO 9964035 A
- US P20020055651 A
- WO 0142193 A
- WO 0183462 A
- WO 0266422 A
- WO 0270490 A
- WO 0276933 A
- WO 032439 A

- WO 0342160 A
- WO 0342164 A
- WO 0372539 A
- WO 0391204 A
- WO 0399764 A
- WO 0416578 A
- WO 0422547 A
- WO 0432921 A
- WO 0433412 A
- WO 0437768 A
- WO 0437773 A
- WO 0437807 A
- WO 0439762 A
- WO 0439766 A
- WO 0445618 A
- WO 0446083 A
- WO 0480964 A
- WO 04108765 A
- WO 04108676 A
- EP 424021 A
- US P3714357 A
- US P5171744 A
- WO 0104118 A
- WO 0200652 A
- WO 0251841 A
- WO 0253564 A
- WO 0300840 A
- WO 0333495 A
- WO 0353966 A
- WO 0387094 A
- WO 04018422 A
- WO 0405285 A
- US P20040167167 A
- WO 0474246 A
- WO 04174812 A
- JP 2004107299 B
- WO 03099807 A
- WO 04026841 A
- EP 0702004 A2, Rueger
- EP 17152 A
- EP 300431 A
- US 2008200523 A
- EP 620222 A
- WO P2004016601 A
- WO 2005026134 A
- EP 1205475 A
- US 6099562 A
- US 5886026 A
- US 5304121 A

**Non-patent literature cited in the description**

- **GREGORY, R. J. et al.** *Nature,* 1990, vol. 347, 382-386
- **RICH, D. P. et al.** *Nature,* 1990, vol. 347, 358-362
- **RIORDAN, J. R. et al.** *Science,* 1989, vol. 245, 1066-1073
- **CUTTING, G. R. et al.** *Nature,* 1990, vol. 346, 366-369
- **DEAN, M. et al.** *Cell,* 1990, vol. 61, 863-870
- **KEREM, B-S. et al.** *Science,* 1989, vol. 245, 1073-1080
- **KEREM, B-S et al.** *Proc. Natl. Acad. Sci. USA,* 1990, vol. 87, 8447-8451
- **QUINTON, P. M.** *FASEB J.,* 1990, vol. 4, 2709-2727
- **DALEMANS et al.** *Nature Lond.,* 1991, vol. 354, 526-528
- **PASYK ; FOSKETT.** *J. Cell. Biochem.,* 1995, vol. 270, 12347-50
- **HWANG, T. C. et al.** *J. Gen. Physiol.,* 1998, vol. 111 (3), 477-90
- *CFTR mutations, http://www.genet.sickkids.on.ca/cftr*
- **GREENE ; WUTS.** Protective Groups in Organic Synthesis. John Wiley & Sons, 1999
- **P.J.KOCIENSKI.** Protecting Groups. Thieme, 1994
- March's Organic Chemistry. 2001
- Comprehensive Organic Transformations. VCH, 1989
- Comprehensive Organic Functional Group Transformations. Pergamon, 1995
- Comprehensive Organic Synthesis. Pergamon, 1991
- **GREENE ; WUTS.** Protective Groups in Organic Synthesis. Wiley and Sons, 1999
- **HIRSH et al.** *J Pharm Exp Ther,* 2004
- **MOODY et al.** *Am J Physiol Cell Physiol,* 2005
- *Journal of Physical Chemistry B,* 2004, vol. 108 (12), 3879-3889
- *Journal of Medicinal Chemistry,* 1973, vol. 16 (8), 901-8
- **DING, CHUANYONG. ; MA, RUJIAN. ; RONG, GUOBIN.** Preparation of w-Phenyl-(2S)-N-Boc-amino Acid Ethyl esters. *Chinese Journal of Organic Chemistry,* 2006, vol. 26 (12), 1694, , 1695
- **G. S. D'ALCONTRES ; C CARISTI ; A FERLAZZO ; M GATTUSO.** *J. Chem. Soc. Perkin 1,* 1976, vol. 16, 1694
- **S. M. BERGE et al.** *J. Pharmaceutical Sciences,* 1977, vol. 66, 1-19
- **E. W. MARTIN.** Remington's Pharmaceutical Sciences. Mack Publishing Co, 1980

- **LEE R. CHOO-KANG ; PAMELA L.** Zeitlin, Type I, II, III, IV, and V cystic fibrosis. Transmembrane Conductance Regulator Defects and Opportunities of Therapy. *Current Opinion in Pulmonary Medicine,* 2000, vol. 6, 521-529
- **GALIETTA, L.J.V. ; LANTERO, S. ; GAZZOLO, A. ; SACCO, O. ; ROMANO, L. ; ROSSI, G.A. ; ZEGARRA-MORAN, O.** *In Vitro Cell. Dev. Biol.,* 1998, vol. 34, 478-481
- **RAE, J. ; COOPER, K. ; GATES, P. ; WATSKY, M.** *J. Neurosci. Methods,* 1991, vol. 37, 15-26
- **DALEMANS, W. ; BARBRY, P. ; CHAMPIGNY, G. ; JALLAT, S. ; DOTT, K. ; DREYER, D. ; CRYSTAL, R.G. ; PAVIRANI, A. ; LECOCQ, J-P ; LAZDUNSKI, M.** *Nature,* 1991, vol. 354, 526-528
- **GALIETTA, L.J.V. ; LANTERO, S. ; GAZZOLO, A. ; SACCO, O. ; ROMANO, L ; ROSSI, G.A ; ZEGARRA-MORAN, O.** *In Vitro Cell. Dev. Biol.,* 1998, vol. 34, 478-481
- **DALEMANS, W. ; BARBRY, P. ; CHAMPIGNY, G. ; JALLAT, S. ; DOTT, K. ; DREYER, D. ; CRYSTAL, R.G. ; PAVIRANI, A. ; LECOCQ, J-P. ; LAZDUNSKI, M.** *Nature,* 1991, vol. 354, 526-528
- **GONZALEZ, J. E. ; R. Y. TSIEN.** Voltage sensing by fluorescence resonance energy transfer in single cells. *Biophys J,* 1995, vol. 69 (4), 1272-80
- **GONZALEZ, J. E. ; R. Y. TSIEN.** Improved indicators of cell membrane potential that use fluorescence resonance energy transfer. *Chem Biol,* 1997, vol. 4 (4), 269-77
- **GONZALEZ, J. E. ; K. OADES et al.** Cell-based assays and instrumentation for screening ion-channel targets. *Drug Discov Today,* 1999, vol. 4 (9), 431-439